# EUROPEAN PATENT SPECIFICATION

(11) **EP 2 275 542 B1**
(45) Date of publication and mention of the grant of the patent: **12.12.2012**
(21) Application number: 10179544.1
(22) Date of filing: 26.02.2007
(51) Int. Cl.: C12N 15/10, C12Q 1/68, G01N 33/68

(54) **Method for identifying useful proteins of brewery yeast**
Methode zur Identifizierung von nützlichen Proteinen in Brauereihefe
Procédé pour identifier des protéines utiles d'une levure de bière

(30) Priority: 28.02.2006 JP 2006117198
(43) Date of publication of application: 19.01.2011
(62) Divisional of application: 07705679.4
(73) Proprietor: Suntory Holdings Limited, Kita-ku, Osaka-shi Osaka 530-8203 (JP)
(72) Inventor: Nakao, Yoshihiro, Shimamoto-cho, Mishima-gun, Osaka 6188503 (JP); Kodama, Yukiko, Shimamoto-cho, Mishima-gun, Osaka 6188503 (JP); Shimonaga, Tomoko, Shimamoto-cho, Mishima-gun, Osaka 6188503 (JP); Kanamori, Takeshi, Shimamoto-cho, Mishima-gun, Osaka 6188503 (JP)
(74) Representative: Vossius & Partner

(56) References cited:
- WO-A-2004/079008
- WO-A-2006/024892
- WO-A-2006/024951
- JOUBERT R ET AL: "Identification by mass spectrometry of two-dimensional gel electrophoresis-separated proteins extracted from lager brewing yeast", ELECTROPHORESIS, WILEY-VCH VERLAG, WEINHEIM, DE, vol. 22, no. 14, August 2001 (2001-08), pages 2969-2982, XP002285923, ISSN: 0173-0835
- TAMAI Y ET AL: "Co-existence of two types of chromosome in the bottom fermenting yeast, Saccharomyces pastorianus.", YEAST (CHICHESTER, ENGLAND) JUL 1998, vol. 14, no. 10, July 1998 (1998-07), pages 923-933, XP002438055, ISSN: 0749-503X
- WILKINS M R ET AL: "Protein identification with N and C-terminal sequence tags in proteome projects", JOURNAL OF MOLECULAR BIOLOGY, LONDON, GB, vol. 278, no. 3, 8 May 1998 (1998-05-08), pages 599-608, XP004461360, ISSN: 0022-2836
- LINK A J ET AL: "Direct analysis of protein complexes using mass spectrometry", NATURE BIOTECHNOLOGY, NATURE PUBLISHING GROUP, NEW YORK, NY, US, vol. 17, July 1999 (1999-07), pages 676-682, XP002184653, ISSN: 1087-0156

## Description

### Technical Field

The invention relates to a method for identifying useful proteins of brewery yeast. More specifically, the invention relates to a method for identifying useful proteins of brewery yeast and genes encoding the proteins by proteome analysis using genome sequence information of brewery yeast.

The disclosure relates to a gene identified by the above-mentioned method and uses thereof, in particular to breeding of brewery yeast useful for improving productivity of alcoholic beverages (for example beer) and/or for improving flavor such as stabilization or enhancement of flavor, the alcoholic beverages produced by using the yeast, a production method thereof and the like.

### Background Art

In recent years, it is possible to introduce a target gene into desired cells and permits the gene to be expressed in the cell using genetic engineering techniques. Cells having desirable characters are prepared by gene engineering techniques with genes whose function have been analyzed using genome information.

In the production of fuel alcohol using industrial yeasts and alcoholic beverages using brewery yeasts, technologies for improving productivity and for stabilization and/or improvement of flavor have been successfully developed by using genetic engineering methods.

Proteome analysis is known as comprehensive protein analysis, and a method for identifying proteins and genes encoding the proteins as targets for genetic engineering. For example, it is used to identify the genes encoding said proteins separated by biochemical property and elucidate the function of the proteins by database searching (against known protein sequences).

An example of the most frequently used method in the analysis today is a peptide mass fingerprinting (PMF) method. A protein and a gene encoding the protein are identified by the PMF method, which comprises separating the protein by two-dimensional electrophoresis, digesting the protein with a protease such as trypsin, obtaining a mass spectrum of the resulting peptide mixture (peptide mass fingerprint) using a mass spectrometer, and comparing the mass spectrum obtained above with theoretical mass spectra calculated from amino acid sequences corresponding to nucleotide sequences from a genome data base.

A bottom fermenting yeast as one of the brewery yeast has been analyzed using a proteome analysis method (Joubert et al., Electrophoresis, 22, 2969 (2001)). However, it was not comprehensive analysis because almost half of the genome of bottom fermenting yeast has been unknown. Namely, the bottom fermenting yeast is an alloploid composed of at least two types of genome (Y. Tamai et al., Yeast, 10, 923 (1998)). One of the genomes is considered to be derived from *S*. *cerevisiae (Saccharomyces cerevisiae* type; may be abbreviated as "Sc" hereinafter) whose genome has been sequenced (for example, see A. Goffeau et al., Nature, 387, 5 (1997)). However, remaining genome (Non-*S*. *cerevisiae* type; may be abbreviated as "Non-Sc" hereinafter) has not been sequenced

### Disclosure of Invention

Under the above-mentioned situations, it is desired to analyze whole genome of the bottom fermenting yeast for the comprehensive proteome analyses to identify proteins and the genes encoding the proteins useful for improving productivity and/or flavor of the alcoholic beverages.

In view of the above-mentioned problems, the present inventors have sequenced genome of the bottom fermenting yeast. The bottom fermenting yeast was proved to have a genome structure having a Sc type nucleotide sequence group that shows over 94% identity and a Non-Sc type nucleotide sequence group that shows about 84% identity. The function of proteins encoded by the gene of the bottom fermenting yeast was inferred by comparing with the amino acid sequences that are registered in the genome database (DB) of *S. cerevisiae* whose genome sequences have been already elucidated. The results proved that the proteins of the bottom fermenting yeast are roughly divided into two types: those with nearly 100% amino acid identity to *S*. *cerevisiae* (Sc type) and those with 70 to 97% (Non-Sc type). The present inventors have made intensive studies based on these discoveries, and have completed the invention.

The invention provides a method for identifying desired proteins of yeast or genes encoding the proteins based on information of the analyzed genome sequence of the bottom fermenting yeast comprising two or more nucleotide sequences of SEQ ID NOs: 33 to 6236, SEQ ID NOs: 75337 to 82784, SEQ ID NOs: 166154 to 166181, SEQ ID NOs: 166490 to 167042 and SEQ ID NOs: 173125 to 174603.

In particular, the invention provides a method for identifying useful proteins of brewery yeast and genes encoding the proteins.

Specifically, the invention provides the following:
[1] A method for identifying a target protein of yeast or a gene encoding the target protein comprising the steps of:
   (A) a step of determining the amino acid sequence of the target protein selected from the following steps (A1) to (A4):
      Step (A1)
         (1) cultivating yeast under a predetermined cultivation condition;
         (2) extracting a protein sample from the cultivation product of the yeast;
         (3) separating the protein sample by a protein separation means, selecting a target peak or spot, and recovering the target protein or a fragment thereof contained in the peak or spot; and
         (4) determining the amino acid sequence of the target protein;
      Step (A2)
         (1) cultivating yeast under a predetermined cultivation condition;
         (2) extracting a protein sample from cultivated products of the yeast; and
         (3) determining the amino acid sequence of the one or more proteins contained in the protein sample;
      Step (A3)
         (1) cultivating the same strain of yeast under different cultivation conditions;
         (2) extracting a protein sample from each cultivation product obtained in step (1);
         (3) analyzing the protein sample and identifying a highly expressed or low expressed protein under each cultivation condition; and
         (4) determining the amino acid sequence of the protein identified in step (3);
      Step (A4)
         (1) cultivating different strains of yeast under the same cultivation condition;
         (2) extracting a protein sample from each cultivation product obtained in step (1);
         (3) analyzing the protein sample and identifying a protein whose expression level is different in each cultivation product; and
         (4) determining the amino acid sequence of the protein identified in step (3);
   (B) comparing the amino acid sequence determined in step (A) with the amino acid sequence determined in advance based on all or a part of genome sequence information of bottom fermenting yeast;
   (C) identifying the gene encoding the target protein based on the results of comparison; and
   (D) analyzing functions of the identified gene to identify characters given to the yeast by the gene;
   wherein all or a part of the genome sequence information of the bottom fermenting yeast comprises two or more nucleotide sequences of:
   SEQ ID Nos.: 33 to 6236,
   SEQ ID Nos.: 75337 to 82784,
   SEQ ID Nos.: 166154 to 166181,
   SEQ ID Nos.: 166490 to 167042; and
   SEQ ID Nos.: 173125 to 174603.
   In addition, as used in the present specification, the term "cultivation product" broadly means those obtained by cultivation of yeast (or a yeast strain), and includes broth (or culture), culture precipitates, yeast cells contained therein, culture supernatant and the like.
[2] A method for identifying a target protein of yeast or a gene encoding the target protein comprising the steps of:
   (1) referring to a database comprising all or a part of genome sequence information of bottom fermenting yeast based on the amino acid sequence of the target protein of the yeast;
   (2) identifying a gene encoding the target protein based on the result of reference; and
   (3) analyzing functions of the identified gene to identify characters given to the yeast by the gene;
   wherein all or a part of the genome sequence information of the bottom fermenting yeast comprises two or more nucleotide sequences of:
   SEQ I D Nos.: 33 to 6236,
   SEQ ID Nos.: 75337 to 82784,
   SEQ ID Nos.: 166154 to 166181,
   SEQ ID Nos.: 166490 to 167042; and
   SEQ ID Nos.: 173125 to 174603.
[3] The method according to [2], wherein the amino acid sequence of the target protein is obtained by separating one or more proteins from a protein extract derived from the yeast and determining the amino acid sequences of the one or more proteins.
[4] The method according to any one of [1] to [3], wherein all or a part of the genome sequence information of the bottom fermenting yeast comprises 40 or more nucleotide sequences selected from:
   SEQ ID Nos.: 33 to 6236,
   SEQ ID Nos.: 75337 to 82784,
   SEQ ID Nos.: 166154 to 166181,
   SEQ ID Nos.: 166490 to 167042; and
   SEQ ID Nos.: 173125 to 174603.
[5] The method according to any one of [1] to [3], wherein all or a part of the genome sequence information of the bottom fermenting yeast comprises 50 or more nucleotide sequences selected from:
   SEQ ID Nos.: 33 to 6236,
   SEQ ID Nos.: 75337 to 82784,
   SEQ ID Nos.: 166154 to 166181,
   SEQ ID Nos.: 166490 to 167042; and
   SEQ ID Nos.: 173125 to 174603.
[6] The method according to any one of [1] to [3], wherein all or a part of the genome sequence information of the bottom fermenting yeast comprises the nucleotide sequences selected from:
   SEQ ID Nos.: 33 to 6236,
   SEQ ID Nos.: 75337 to 82784,
   SEQ ID Nos.: 166154 to 166181,
   SEQ ID Nos.: 166490 to 167042; and
   SEQ ID Nos.: 173125 to 174603.
[7] The method according to any one of [1] to [3], wherein all or a part of the genome sequence information of the bottom fermenting yeast comprises the nucleotide sequences of SEQ ID Nos.: 33 to 6236.
[8] The method according to any one of [1] to [3], wherein all or a part of genome sequence information of the bottom fermenting yeast comprises two or more nucleotide sequences selected from SEQ ID Nos.: 33 to 6236.
[9] The method according to any one of [1] to [3], wherein all or a part of genome sequence information of the bottom fermenting yeast comprises nucleotide sequences of SEQ ID Nos.: 166154 to 166181.
[10] The method according to any one of [1] to [3], wherein all or a part of genome sequence information of the bottom fermenting yeast comprises two or more nucleotide sequences selected from SEQ ID Nos.: 166154 to 166181.

The method of the invention allows efficient identification of genes associated with desired fermentation characteristics and the proteins encoded by the genes, and thus allows comprehensive analysis of the function of the translation products of the genes contained in the genome of the bottom fermenting yeast.

In addition, the fermentation characteristics of the yeast can be controlled by identifying the nucleotide sequence of the gene considered to be involved in fermentation characteristics of brewery yeast with the method of the invention, and by high expression or disruption of the gene using gene engineering techniques.

The yeast showing good fermentation characteristics may be bred, and fuel alcohol or alcoholic beverages may be produced with improved productivity and quality using the yeast. For example, using Non-Sc MET17 gene identified by the present invention, yeast modified so as to express the gene in high level resulting in reduction of hydrogen sulfide, can be provided. Use of such yeast enables the concentration of hydrogen sulfide to be suppressed in a low level, and allows production of alcoholic beverages without off-flavor.

### Brief Description of the Drawings

Fig. 1 shows the cell growth with time upon beer fermentation test. The horizontal axis represents fermentation time while the vertical axis represents OD660 (optical density at 660 nm).
Fig. 2 shows the sugar consumption with time upon beer fermentation test. The horizontal axis represents fermentation time while the vertical axis represents apparent extract concentration (w/w%).
Fig. 3 shows the methionine concentration with time upon beer fermentation test. The horizontal axis represents the fermentation time, and the vertical axis represents the methionine concentration (mM).
Fig. 4 shows the cell growth of the parent strain and Non-ScMET17 highly expressed strain with time upon beer fermentation test. The horizontal axis represents fermentation time while the vertical axis represents OD660 (optical density at 660 nm).
Fig. 5 shows the sugar consumption with time upon beer fermentation test using
the parent strain and Non-ScMET17 highly expressed strain. The horizontal axis represents fermentation time while the vertical axis represents apparent extract concentration (w/w%).

### Best Mode for Carrying Out the Invention

### 1. Outline of the Invention

The present inventors created a database for identifying target yeast proteins and genes encoding the target proteins based on genome sequence analyzed by a method outlined in Japanese Patent Application Laid-Open (JP-A) No. 2004-283169. The data were not open to the public at the priority date of this application.

The database includes information of the genome sequence of the bottom fermenting yeast. The database specifically includes information of the nucleotide sequence listed in the attached sequence listing. The content of information of the nucleotide sequence (information of the nucleotide sequence of the genome of the bottom fermenting yeast) described in the attached sequence listing is as follows.

SEQ ID Nos.: 33 to 6236 represent nucleotide sequences of 6204 Non-Sc type open reading frames (may be abbreviated as ORF hereinafter). SEQ ID Nos.: 75337 to 82784 represent nucleotide sequences of 7448 Sc type ORFs. SEQ ID Nos.: 166154 to 166181 represent nucleotide sequences of 28 mitochondrial ORFs of the bottom fermenting yeast. While SEQ ID Nos.: 166490 to 167042 have not been identified as above ORFs, they represent 553 nucleotide sequences having significant similarity to the *S*. *cerevisiae* genes by homology search with NCBI-BlastX (http://www.ncbi.nlm.nih.gov/BLAST/). SEQ ID Nos.: 173125 to 174603 have been identified as other ORFs which represent 1479 nucleotide sequences encoding proteins which show significant similarity to the *S*. *cerevisiae* protein by homology search with NCBI-BlastP (http://www.ncbi.nlm.nih.gov/BLAST/).

Target proteins of yeast (or proteins having unknown functions) or genes encoding the target proteins can be identified by a proteome analysis method with use of the database based on yeast genome information. The proteome analysis methods include a method comprising the steps of: separating and purifying the target protein by a protein separation and purification method (for example two-dimensional electrophoresis (2-DE)) and high performance liquid chromatography (HPLC); and specifying the target protein by a protein identification method (for example, comparison of peptide maps obtained by mass spectral analysis or comparison of amino acid sequences obtained by amino acid sequencing). The target protein may be selected by utilizing known methods (for example two-dimensional electrophoresis (2-DE), two-dimensional fluorescence differential gel electrophoresis (2D-DIGE) or isotope-coded affinity tag method (ICAT method)). Functions of proteins having unknown functions in the protein sample can be comprehensively analyzed by using a shot gun method.

Accordingly, the invention provides a method for specifying the target protein of the yeast (or proteins having unknown functions) and the gene encoding the target protein based on the proteome analysis method using novel sequence information of the genome of bottom fermenting yeast. Optionally, the functions of the specified target protein and the gene encoding the target protein may be analyzed using known methods.

Specifically, the invention comprises the following aspects of the methods.

### (Embodiment 1)

A method for identifying a target protein of yeast or a gene encoding the target protein, the method comprising the steps of: (a) cultivating yeast under a give cultivation condition; (b) extracting a protein sample from cultivation products of the yeast; (c) separating the protein sample by a protein separation means to select a target peak or spot and recovering a target protein or a fragment thereof contained in the peak or spot;
(d) determining the amino acid sequence of the target protein; (e) comparing the amino acid sequence determined in Step (d) with the amino acid sequence determined in advance based on all or a part of genome sequence information of the bottom fermenting yeast; (f) identifying a gene encoding the target protein based on the results of comparison; and (g) analyzing the function of the identified gene to identify characteristics given by the gene to the yeast; wherein all or part of the genome sequence information of the bottom fermenting yeast comprises two or more nucleotide squences of SEQ ID NOs: 33 to 6236, SEQ ID NOs: 75337 to 82784, SEQ ID NOs: 166154 to 166181, SEQ ID NOs: 166490 to 167042 and SEQ ID NOs: 173125 to 174603.

### (Embodiment 2)

A method for identifying a target protein of yeast or a gene encoding the target protein comprising the steps of: (1) referencing a database comprising all or a part of genome sequence information of the bottom fermenting yeast based on the amino acid sequence (including information capable of specifying the amino acid sequence of the target protein, for example a mass spectrum pattern of a protein obtained by using a mass spectrometer) of the target protein of the yeast; (2) identifying the gene encoding the target protein based on the results of reference; and (3) analyzing the function of the identified gene to identify characteristics given by the gene to the yeast; wherein all or part of the genome sequence information of the bottom fermenting yeast comprises two or more nucleotide squences of SEQ ID NOs: 33 to 6236, SEQ ID NOs: 75337 to 82784, SEQ ID NOs: 166154 to 166181, SEQ ID NOs: 166490 to 167042 and SEQ ID NOs: 173125 to 174603.

### (Embodiment 3)

A method for identifying a target protein of yeast or a gene encoding the target protein comprising the steps of: (1) separating one or more proteins from a protein extract derived from the yeast (including a protein directly extracted from yeast, a secreted protein in broth, and the like) and determining an amino acid sequence of the one or more proteins (including a step for acquiring information for specifying the amino acid sequence); (2) referencing a database comprising all or a part of genome sequence information of the bottom fermenting yeast based on the amino acid sequence of the one or more proteins (including information capable of specifying the amino acid sequence); (3) identifying the gene encoding the one or more proteins; and (4) analyzing the function of the identified gene to identify characteristics given by the gene to the yeast; wherein all or part of the genome sequence information of the bottom fermenting yeast comprises two or more nucleotide squences of SEQ ID NOs: 33 to 6236, SEQ ID NOs: 75337 to 82784, SEQ ID NOs: 166154 to 166181, SEQ ID NOs: 166490 to 167042 and SEQ ID NOs: 173125 to 174603.

### (Embodiment 4)

A method for identifying a target protein of yeast or a gene encoding the target protein comprising the steps of: (1) cultivating yeast under a given cultivation condition; (2) extracting a protein sample from cultivated products of the yeast; (3) determining an amino acid sequence of the one or more proteins contained in the protein sample; (4) comparing the amino acid sequence determined in Step (3) with the amino acid sequence determined in advance based on all or a part of genome sequence information of the bottom fermenting yeast; (5) identifying a gene encoding the target protein based on the results of comparison; and (6) analyzing the function of the identified gene to identify characteristics given by the gene to the yeast; wherein all or part of the genome sequence information of the bottom fermenting yeast comprises two or more nucleotide squences of SEQ ID NOs: 33 to 6236, SEQ ID NOs: 75337 to 82784, SEQ ID NOs: 166154 to 166181, SEQ ID NOs: 166490 to 167042 and SEQ ID NOs: 173125 to 174603.

### (Embodiment 5)

A method for identifying a target protein of yeast or a gene encoding the target protein comprising the steps of: (1) cultivating the same strain of yeast under different cultivation conditions; (2) extracting a protein sample from each cultivation product obtained in Step (1); (3) analyzing the protein sample and specifying a highly expressed or low expressed protein under each cultivation condition; (4) determining the amino acid sequence of the protein specified in Step (3); (5) comparing the amino acid sequence determined in Step (4) with the amino acid sequence determined in advance based on all or a part of genome sequence information of the bottom fermenting yeast; (6) identifying the gene encoding the target protein based on the results of comparison; and (7) analyzing the function of the identified gene to identify characteristics given by the gene to the yeast; wherein all or part of the genome sequence information of the bottom fermenting yeast comprises two or more nucleotide squences of SEQ ID NOs: 33 to 6236, SEQ ID NOs: 75337 to 82784, SEQ ID NOs: 166154 to 166181, SEQ ID NOs: 166490 to 167042 and SEQ ID NOs: 173125 to 174603.

### (Embodiment 6)

A method for identifying a target protein of yeast for a gene encoding the target protein comprising the steps of: (1) cultivating different strains of yeast under the same cultivation condition; (2) extracting a protein sample from each fermentation product obtained in Step (1); (3) analyzing the protein sample and identifying a protein whose expression level is different (higher expression or lower expression) in each cultivation product; (4) determining the amino acid sequence of the protein specified in Step (3); comparing the amino acid sequence determined in Step (4) with the amino acid sequence determined in advance based on all or a part of genome sequence information of the bottom fermenting yeast; (6) identifying a gene encoding the target protein based on the results of comparison; and (7) analyzing the function of the identified gene to identify characteristics given by the gene to the yeast; wherein all or part of the genome sequence information of the bottom fermenting yeast comprises two or more nucleotide squences of SEQ ID NOs: 33 to 6236, SEQ ID NOs: 75337 to 82784, SEQ ID NOs: 166154 to 166181, SEQ ID NOs: 166490 to 167042 and SEQ ID NOs: 173125 to 174603.

As used herein, the term "target protein" refers to a useful protein to be specified by the methods of the invention, and all or part of the amino acid sequence is an object of search using a reference sequence database. The target protein includes a protein having unknown functions. Representative examples of the target protein include yeast proteins. While representative examples of the desired yeast proteins include proteins of the brewery yeast (for example the bottom fermenting yeast belonging to S. pastorianus and top fermenting yeast belonging to *S*. *cerevisiae*) and proteins of the baker's yeast, but are not limited thereto. An example of the preferable target protein includes a protein useful for brewing beer.

As used herein, the term "brewery yeast" refers to arbitrary yeast capable of being used for brewing of alcoholic beverages. Examples of yeast include those used for fermentation of beer, wine, sake, whisky and *shochu.*

The kind of beer is roughly divided into three categories depending on the kind of yeast and the method of fermentation. The three categories include natural fermentation beer fermented by utilizing wild yeast or microorganisms in a brewery; ale beer fermented at a temperature from 20 to 25 °C using top fermenting yeast belonging to *S. cerevisiae* with a short aging period thereafter; and lager beer fermented at a temperature from 6 to 15°C using bottom fermenting yeast belonging to *Saccharomyces pastorianus* with low temperature aging thereafter. Bottom fermenting yeast used for brewing of lager type beer is most widely used in brewing of beer.

As used herein, the term "a part of proteins" refers to a fragment(s) of a protein (for example a peptide fragment obtained by protease digestion of the protein). The length of the protein fragment preferably consists of at least four amino acid residues so that the original protein can be identified by the analysis of a reference database by using the amino acid sequence of the fragment (for example, see Wilkins et al., J. Mol. Biol., 278, 599 (1998)). Synthetic polypeptides obtained by a peptide synthesizer may be included in the protein fragment.

As used herein, the term "determining the amino acid sequence" is used to mean to include determining all or a part of the amino acid sequence of the target protein, as well as to include acquiring information capable of specifying the amino acid sequence of the target protein (for example a spectrum pattern obtained by using a mass spectrometer). While it is preferable to determine the complete amino acid sequence of the target protein, the invention may be practiced when an amino acid sequence having a length capable of extracting similar sequences can be determined by comparing with amino acid sequences in a reference database. For example, the target protein is considered to be screened when an identifying software, for example MASCOT, indicates the presence of at least one fragment having an expected value (P-value) of less than 0.05 in the amino acid sequence in a reference database showing a hit with the target database, and the fragment covers 5% or more, 10% or more, 20% or more, 30% or more, 40% or more, 50% or more, 60% or more, 70% or more, 80% or more, 90% or more, 95% or more, 97% or more of the total length of the target protein (naturally, the larger percentage is more preferable). The term "all or a part of the amino acid sequence" refers to all or a part of the amino acid sequence of the protein to be searched, and examples thereof include the amino acid sequence of a protein whose amino acid sequence has been known or fragments thereof, and the amino acid sequence whose amino acid sequence has not been known or fragments thereof, wherein the amino acid sequence is identified by mass spectrometric analysis or other methods according to the method for analyzing proteomes. While the amino acid sequence may be theoretically derived from a mass spectrometric pattern of the protein (may be abbreviated as "mass pattern" herein) obtained using a mass spectrometer, the mass pattern and the amino acid sequence theoretically estimated from the mass pattern may be considered to be equivalent to the amino acid sequence of all or a part of the above mentioned protein. Accordingly, the phrase "determining the amino acid sequence of the protein" is meant to include inference of all or a part of the amino acid sequence of the protein from the mass pattern. The mass pattern is usually represented by peaks of respective peptides in a polypeptide sample subjected to the analysis by taking m/z (mass/charge) of the ionized peptide or protein in the horizontal axis and a relative intensity in the vertical axis. Usually, the amino acid sequence is theoretical induced from the mass pattern using exclusive use software (for example MASCOT).

As used herein, the term "reference database" or "reference sequence database" refers to a nucleotide sequence database comprising all or a part of genome sequence information of the bottom fermenting yeast or an amino acid sequence database theoretically translated from the nucleotide sequence database. The term "theoretically translated amino acid sequence" refers to an amino acid sequence predicted to be coded by a predetermined nucleotide sequence according to universal genetic code when the amino acids are encoded by the gene on the nuclear (chromosomal) DNA and "The Yeast Mitochondrial Code (hitp://cvww.ncbi.nlm.hih.gov/Taxonomy/Utils/wprintc.cgi?mode=c#SG3)" when the amino acids are encoded by the gene on the mitochondria.

As used herein, the term "all of the genome sequence information of the bottom fermenting yeast" refers to an entire nucleotide sequence of the genome of the bottom fermenting yeast. As used herein, the term "a part of genome sequence information, of the bottom fermenting yeast" refers to at least a part of the nucleotide sequence of the genome of the bottom fermenting yeast (for example at least one contig (contiguous nucleotide sequence)). The length of "at least a part of the nucleotide sequence of the genome of the bottom fermenting yeast" is 8 bases or more, more preferably 12 bases or more, 15 bases or more, 18 bases or more, 21 bases or more, 24 bases or more, 27 bases or more or 30 bases or more, further preferably 40 bases or more, 50 bases or more, 60 bases or more, 70 bases or more, 80 bases or more, 90 bases or more or 100 bases or more, far more preferably 200 bases or more, 300 bases or more, 400 bases or more, 500 bases or more, 600 bases or more, 700 bases or more, 800 bases or more or 900 bases or more, and most preferably 1000 bases or more. Examples of the database comprising a part of genome sequence information of the bottom fermenting yeast include, for example, a nucleotide sequence database comprising at least one contig of the genome of the bottom fermenting yeast, a nucleotide sequence information comprising at least one open reading frame nucleotide sequence of the bottom fermenting yeast, and a nucleotide sequence information comprising at least one nucleotide sequence of open reading frame classified according to respective function. The open reading frame of the nucleotide sequence of the genome may be identified by using a program such as ORF Finder (http://www.ncbi.nih.gov/gorf/gorf.gtml). As used herein, the term "function of the gene" may be used to include function of the gene and /or function of translation product of the gene.

### 2. Selection of Target Protein

The object of the invention is to identify the target protein of yeast or the gene encoding the target protein and /or the function, and the method for selecting the target protein is not particularly restricted. The target protein is usually obtained from a protein extract derived from yeast by the methods of invention, for example from a cultivation product of yeast or yeast cells per se. However, according to the present invention, the target protein of yeast or the gene encoding the target protein and /or the function may be analyzed by a proteome analysis method using the amino acid sequence of the protein as a clue. Accordingly, the target protein of yeast or the gene encoding the target protein and /or the function may be analyzed by determining the amino acid sequence of the protein, even when a method for obtaining the target protein is not known.

Specifically, in methods of the present invention, a protein sample may be prepared from cultivation products obtained by cultivating the yeast under a predetermined condition.

In the methods of the present invention, protein samples obtained from two or more cultivation products may be prepared depending on the method for applied proteome analysis. For example, the same strain of yeast may be cultivated under different cultivation conditions, or different strains of yeast may be cultivated under the same cultivation condition, and the protein sample may be prepared from each cultivation product.

As used herein, the term "predetermined cultivation condition" may be any conditions capable of cultivating the yeast cells, and the compositions thereof are not particularly restricted. Accordingly, the "predetermined cultivation condition" may be appropriately selected as a condition suitable for cultivating the yeast cells in a liquid medium or solid medium, or as a specific condition which should be investigated with respect to specific characters of the cultivated products.

Examples of the cultivation condition include cultivation temperature, cultivation time, cultivation atmosphere, pH and the concentrations of substances (or kind of the medium) required for maintaining viability and growth of the yeast cells such as salts, carbon sources, nitrogen sources and vitamins. Naturally, various commercially available liquid media may be used. For example a YPD medium (2% (w/w) glucose, 1% (w/w) yeast extract, 2% (w/w) polypeptone) may be used. A preferable cultivation method is usually overnight shaking cultivation at a temperature from about 25 to 35°C. When a protein or a gene that exhibits its function under a specified culture condition is a target, the cultivation condition is changed depending on the function. For example, when a protein related to low temperature resistance or a gene encoding the target protein is a target, the yeast is cultivated at a low temperature, and a protein expressed in high level under the cultivation condition may be selected as a target protein.

### 3. Extraction of Protein from Protein Sample

In the methods of the present invention, the protein sample may be extracted from the cultivation product or yeast cells obtained as described above. Any protein extraction methods known in the art may be used as the extraction method. For example, the method described in O'Farrell, J. Biol. Chem., 250, 4007 (1975) may be used, wherein the protein may be extracted by dissolving the sample in a dissolution solution containing 8M urea, 2% NP-40, 2% carrier ampholyte and 5% 2-mercaptoethanol. Usually, in order to enhance extraction of the protein from the cells, the cells are homogenized before extraction using a homogenizer after suspending the cultivated yeast cells in a homogenizing buffer (for example 10 mmol/L Tris-HCl, pH 7.4, 5 mmol/L MgCl₂, 50 mg/L RNaseA, 1.6 mg/mL protease inhibitor (trade name COMPLETE; manufactured by Boehringer Mannheim Co.)), and a supernatant obtained by centrifugation is used for extracting the protein.

The protein is extracted at a low temperature (for example 4°C) for suppressing the protein degradation by protease contained in the yeast cells during the extraction process of the protein, and a protease inhibitor is preferably added. Examples of the protease inhibitor include serine protease inhibitors such as PMSF and chymostatin, cysteine protease inhibitors such as leupeptin, aspartic acid protease inhibitors such as pepstatin A, and metalloprotease inhibitors such as phosphoramidon and EDTA·Na₂. These protease inhibitors may be used alone, or in combination. Various commercially available protease inhibitors (for example trade name COMPLETE; manufactured by Boehringer Mannheim Co.) may be used.

### 4. Separation of Protein

According to the present invention, subsequently, the extracted protein is separated by protein separation methods. As used herein, the term "protein separation methods" refers to a method for separating various proteins contained in the protein sample according to the molecular weight and/or charges of each protein. Examples of the "protein separation methods" include electrophoresis (for example SDS-PAGE, isoelectric focusing and two-dimensional electrophoresis), liquid chromatography (for example ion-exchange chromatography, gel filtration chromatography, affinity chromatography, HPLC and FPLC), gas chromatography, centrifugation (for example ultra-centrifugation), precipitation (for example ammonium sulfate precipitation, organic solvent precipitation (for example acetone precipitation and ethanol precipitation)), pH treatment (for example acid treatment) and membrane separation (for example ultrafiltration).

The target protein may be preferably separated using two-dimensional electrophoresis, two-dimensional fluorescence differential gel electrophoresis or the like.

Simple and preferable protein separation methods may include two-dimensional electrophoresis. The two-dimensional electrophoresis is a method comprising two steps. The first step is one-dimensional gel electrophoresis using a disk or a planar gel. The second step is placing the first-dimensional gel at the top of a plate of an electrophoresis gel under a different principle or condition from the first-dimensional electrophoresis, and separating the protein by electrophoresis in a direction perpendicular to the direction of the first-dimensional electrophoresis. In the methods of the present invention, two-dimensional electrophoresis by O'Farell (1975, supra) may be favorably used (usually, isoelectric focusing is used as the first-dimensional electrophoresis followed by two-dimensional SDS-PAGE). Liquid chromatography (LC) may be also favorably used in the invention other than two-dimensional electrophoresis.

The proteins separated by gel electrophoresis may be detected by Coomassie brilliant blue staining or silver staining. The proteins may be stained with other pigments (for example, amido black, Ponceau S or fluorescent pigment). Various staining kits are commercially available.

In the methods of the present invention, subsequently, a separation pattern (or separation profile) of the proteins separated by the protein separation methods is analyzed to select a target peak(s) or spot(s), and the target protein or a fragment of the protein contained in the peak(s) or spot(s) are recovered. The "separation pattern (or separation profile)" as used herein is intended to mean an elution profile or a two-dimensional electrophoresis pattern indicated by the separated protein by the protein separation methods. For example, the profile means an elution profile when the protein sample is separated by liquid chromatography, and the pattern (or profile) means the pattern (or profile) of the protein distributed on the gel when the protein sample is separated by two-dimensional electrophoresis. The molecular weight of each separated protein is usually indicated by the position (or mobility) of the peak or spot in the separation pattern, and the concentration of each protein is shown by the intensity of each peak or spot. The desired peak or spot (target peak or target spot) is selected from these peaks or spots with reference to the position, mobility or intensity of the peak or spot.

The yeast cells may be independently cultivated in the above-mentioned cultivation process under the conditions in which at least one parameter (for example temperature, osmotic pressure and addition of ethanol) is higher or lower (stress condition) as compared with a usual cultivation condition (non-stress condition) suitable for cultivation of the yeast cells so that yeast cells having desired characteristics may be selected, if necessary. The separation pattern of the protein sample derived from a yeast strain cultivated under the stress condition may be compared with the separation pattern of the protein sample derived from a yeast strain cultivated under the non-stress condition, and peaks or spots expressed in higher levels or lower levels than those under the non-stress condition are selected.

In another embodiment of the present invention, a wild strain of the brewery yeast and a mutant strain or a strain that more evidently exhibits desired characteristics than the wild strain may be cultivated under a condition suitable for cultivating the yeast. The protein samples extracted from these cultivation products are separated by the protein separation methods, and the separation pattern of the protein sample derived from the wild strain is compared with that from the mutant strain or the strain that more evidently exhibits desired characteristics than the wild strain. Thus, the peak or spot of the protein expressed in a high level (or low level) is selected from the mutant strain or the strain that more evidently exhibits desired characteristics than the wild strain. An example of the strain that more evidently exhibits the desired characteristics than the wild strain includes a strain that provides more beer flavor components than the wild strain. Consequently, the method of the present invention may be used for identifying proteins that are the causes of emergence of phenotypes of the strain that more evidently exhibits the desired characteristics than the wild strain or genes encoding the target proteins.

In another embodiment, the separation pattern of the protein sample may be compared with the separation pattern of the protein sample extracted from a strain of *S*. *cerevisiae* whose genome has been published, when the protein sample extracted from a fermentation product of brewery yeast is separated, and then a target spot or peak is selected. Then, the target protein contained in the peak or spot or fragments of the protein are recovered, and a peak or spot characteristic in the separation pattern of the brewery yeast is selected. The protein specific to the bottom fermenting yeast or the gene encoding the target protein can be efficiently identified by determining the amino acid sequences of the proteins of these spots, and by searching and identifying the nucleotide sequence corresponding to each amino acid in the database of the open reading frame of the genome sequence of the bottom fermenting yeast. The separation pattern of the protein sample extracted from the yeast strain of *S*. *cerevisiae* available may be arranged into a database in advance as a two-dimensional electrophoresis database.

In a further embodiment of the present invention, the protein may be extracted from the yeast cells sampled from a fermentation broth of beer in order to identify a useful protein of the brewery yeast and to select a desired protein. Specifically, beer is fermented using the bottom fermenting yeast, the fermentation broth is sampled with time from the start of fermentation, and the cell growth and apparent extract concentration are observed. The yeast cells are recovered from the sampled fermentation broth by centrifugation in parallel with sampling, and the protein is extracted from the recovered yeast cells. The fermentation (cultivation) supernatant is also recovered, and the amino acid composition is analyzed using the recovered fermentation supernatant. The protein extracted from the cells is then separated by the protein separation means to prepare a separation pattern or separation profile. The separation patterns or separation profiles of the protein extract derived from the cells, which are sampled at two or more different times (for example after 8 hours and after 32 hours) from the start of fermentation, may be compared to one another. Then, a spot or peak that shows an increased intensity in accordance with the decrease of the contents of valine, leucine, isoleucine, methionine or the like, which are obtained from amino acid analysis of the fermentation supernatant, may be selected. A desired protein may be also selected from the brewed broth or fermented broth of wine or sake, not only from the broth of beer.

Subsequently, the target protein contained in the target peak or spot or the fragments thereof is recovered. When the protein separated by two-dimensional electrophoresis is subjected to peptide mass fingerprinting or to amino acid sequence analysis by mass analysis, the separated protein is specifically fragmented with protease or using a chemical degradation method usually in a gel or on a membrane filter by transferring the protein on the membrane filter after separating by two-dimensional electrophoresis. Both the digestion method in the gel and the digestion method on the membrane filter are used in the invention.

An Eckerskorn-Lottspeich method (Eckerskorn, C. & Lottspeich, F., Chromatographia, 28, 92 (1989)) or an improved method thereof may be used as the digestion method in the gel. In the Eckerskorn-Lottspeich method, the gel fraction including the desired spot (target protein portion) on the gel stained by Coomassie brilliant blue is cut out, and the gel fraction is soaked in alkaline solution. Then, the gel fraction is dried, and the gel fraction is rehydrated by adding a protease solution to the dry gel fraction in order to permit the protease to permeate into the gel. The protein is digested by allowing the protease to contact the protein. Trypsin and lysyl endopeptidase may be used as the protease. The protein may be fragmented on the membrane filter by interposing a PVDF membrane on which trypsin or lysyl endopeptidase is bound between the gel and blotting membrane filter when the protein separated by two-dimensional electrophoresis is blotted. The protein can be digested when the protein is transferred from the gel to the blotting membrane filter, and the protein is blotted on the blotting membrane filter as peptides.

In the methods of the present invention, the target protein can be separated with use of two-dimensional fluorescence differential gel electrophoresis (2D-DIGE) or isotope-coded affinity tag method (ICAT method; Nat. Biotechnol., 17, 994 (1999)). Since proteins obtained from two different cultivation products may be labeled with different fluorescent labels or isotope labels, respectively, according to 2D-DIGE or ICAT method, it is an advantage of these methods that differently expressed proteins in both cultivation products can be readily found.

### 5. Determination of Amino Acid Sequence of Target Protein

In the process of the present invention, subsequently, the amino acid sequence of the target protein or fragments thereof is determined. The amino acid sequence may be usually determined using a mass spectrometer (MS). In the mass spectrometric analysis, the mass of the sample is determined by ionizing a sample such as a protein or peptide using MS, separating the ions obtained according to mass/charge (m/z) ratios, and measuring the intensity of each separated ion peak. Various methods such as matrix-assisted laser desorption/ionization method (MALDI) method, electro-spray ionization (ESI) method, gas phase (EI, CI) method and field desorption ionization (FD) method may be used for ionization. An ion separation method compatible with the ionization method may be used for ion separation, and examples of the apparatus used for the method include a time-of-flight (TOF) mass spectrometer for MALDI and a quadrupole (QMS), ion trap or magnetic sector mass spectrometer for ESI. The mass spectrometer may be used in tandem. Examples of the apparatus include LC-ESI, MS/MS, Q-TOF MS and MALDI-TOF MS. Other methods for determining the amino acid sequence, for example a method for determining the amino acid sequence with a sequencer (for example gas phase sequencer), may be also used.

Mass spectrum (peptide mass fingerprint) of the peptide mixture obtained by protease digestion is produced by MS. While the mass spectroscopic method is not particularly restricted, representative examples include MALDI-TOF MS and ESI Q-TOF MS. The characteristics of the protein separated by two-dimensional electrophoresis can be elucidated (for example determination of the amino acid sequence) comparing the peptide mass finger print with the theoretical spectrum calculated from the amino acid sequence of the protein database. In addition, the gene encoding the protein can be identified by comparing the peptide mass finger print with the theoretical mass spectrum calculated from the amino acid sequence corresponding to the nucleotide sequence of the DNA database. An exclusive use software (for example MASCOT) may be usually used for such treatment.

A shot-gun analysis method (Nat. Biotechnol., 17, 676 (1999)) may be used for proteome analysis of the present invention. In the shot-gun analysis method, extracted proteins are digested with a protease, and digestion products are separated by multidimensional LC. Then, the amino acid sequences of the peptides in the digested products are analyzed by MS/MS, and a large amount of the sequence data are analyzed by a computer to sequentially identify the proteins. It is advantageous to use this shot-gun analysis method that proteins having unknown functions can be collectively analyzed from a cultivation product of a given yeast.

### 6. Identification of Gene

After determining the amino acid sequence of the target protein (or a part of the protein) as described above, the gene encoding the protein is determined from the amino acid sequence (or a part of the amino acid sequence) with reference to the reference database.

The reference database used in the methods of the invention comprises all or a part of genome sequence information of the bottom fermenting yeast.

Examples of the reference database used in the invention are described below.

### (Database: Reference Example 1)

Representative examples for identifying the target protein of yeast or the gene encoding the target protein, then comprehensively analyzing the functions of the translation products of the gene are genome sequence databases of the bottom fermenting yeast comprising the base sequences of the following SEQ ID Nos.:
SEQ ID Nos.: 33 to 6236,
SEQ ID Nos.: 75337 to 82784,
SEQ ID Nos.: 166154 to 166181,
SEQ ID Nos.: 166490 to 167042 and
SEQ ID Nos.: 173125 to 174603.

### (Database: Reference Example 2)

The database may be constructed by dividing into above-mentioned groups. For example, a database comprising one or plural sequence(s) of the following SEQ ID Nos. may be used for analyzing the functions of the translation products of the Non-Sc type genes of the bottom fermenting yeast:
SEQ ID Nos.: 33 to 6236

### (Database: Reference Example 3)

Furthermore, a database comprising one or plural sequence(s) of the following SEQ ID Nos. may be used for analyzing the functions of the translation products of the Sc type genes of the bottom fermenting yeast:
SEQ ID Nos.: 75337 to 82784

### (Database: Reference Example 4)

Furthermore, a database comprising one or plural sequence(s) of the following SEQ ID Nos. may be used for analyzing the functions of the translation products of mitochondrial ORF:
SEQ ID Nos.: 166154 to 166181

### (Database: Reference Example 5)

Examples of the databases used for the object of the invention are not restricted to those described above, and include databases comprising the following sequence infromation: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1600 or more, 1700 or more, 1800 or more, 1900 or more, 2000 or more, 2100 or more, 2200 or more, 2300 or more, 2400 or more, 2500 or more, 2600 or more, 2700 or more, 2800 or more, 2900 or more, 3000 or more, 3100 or more, 3200 or more, 3300 or more, 3400 or more, 3500 or more, 3600 or more, 3700 or more, 3800 or more, 3900 or more, 4000 or more, 4100 or more, 4200 or more, 4300 or more, 4400 or more, 4500 or more, 4600 or more, 4700 or more, 4800 or more, 4900 or more, 5000 or more, 5100 or more, 5200 or more, 5300 or more, 5400 or more, 5500 or more, 5600 or more, 5700 or more, 5800 or more, 5900 or more, 6000 or more, 6100 or more, 6200 or more, 6300 or more, 6400 or more, 6500 or more, 6600 or more, 6700 or more, 6800 or more, 6900 or more, 7000 or more, 7100 or more, 7200 or more, 7300 or more, 7400 or more, 7500 or more, 7600 or more, 7700 or more, 7800 or more, 7900 or more, 8000 or more, 8100 or more, 8200 or more, 8300 or more, 8400 or more, 8500 or more, 8600 or more, 8700 or more, 8800 or more, 8900 or more, 9000 or more, 9100 or more, 9200 or more, 9300 or more, 9400 or more, 9500 or more, 9600 or more, 9700 or more, 9800 or more, 9900 or more, 10000 or more, 10100 or more, 10200 or more, 10300 or more, 10400 or more, 10500 or more, 10600 or more, 10700 or more, 10800 or more, 10900 or more, 11000 or more, 11100 or more, 11200 or more, 11300 or more, 11400 or more, 11500 or more, 11600 or more, 11700 or more, 11800 or more, 11900 or more, 12000 or more, 12100 or more, 12200 or more, 12300 or more, 12400 or more, 12500 or more, 12600 or more, 12700 or more, 12800 or more, 12900 or more, 13000 or more, 13100 or more, 13200 or more, 13300 or more, 13400 or more, 13500 or more, 13600 or more, 13700 or more, 13800 or more, 13900 or more, 14000 or more, 14100 or more, 14200 or more, 14300 or more, 14400 or more, 14500 or more, 14600 or more, 14700 or more, 14800 or more, 14900 or more, 15000 or more, 15100 or more, 15200 or more, 15300 or more, 15400 or more, 15500 or more, 15600 or more, or 15700 or more nucleotide sequences selected from nucleotide sequences of SEQ ID Nos.: 33 to 6236, SEQ ID Nos.: 75337 to 82784, SEQ ID Nos.: 166154 to 166181, SEQ ID Nos.: 166490 to 167042 and SEQ ID Nos.: 173125 to 174603.

### (Database: Reference Example 6)

In addition, databases which can be used, may comprise the following sequences information: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1600 or more, 1700 or more, 1800 or more, 1900 or more, 2000 or more, 2100 or more, 2200 or more, 2300 or more, 2400 or more, 2500 or more, 2600 or more, 2700 or more, 2800 or more, 2900 or more, 3000 or more, 3100 or more, 3200 or more, 3300 or more, 3400 or more, 3500 or more, 3600 or more, 3700 or more, 3800 or more, 3900 or more, 4000 or more, 4100 or more, 4200 or more, 4300 or more, 4400 or more, 4500 or more, 4600 or more, 4700 or more, 4800 or more, 4900 or more, 5000 or more, 5100 or more, 5200 or more, 5300 or more, 5400 or more, 5500 or more, 5600 or more, 5700 or more, 5800 or more, 5900 or more, 6000 or more, 6100 or more, 6200 or more, 6300 or more, 6400 or more, 6500 or more, 6600 or more, 6700 or more, 6800 or more, 6900 or more, 7000 or more, 7100 or more, 7200 or more, 7300 or more, 7400 or more, 7500 or more, 7600 or more, 7700 or more, 7800 or more, 7900 or more, 8000 or more, 8100 or more, 8200 or more, 8300 or more, 8400 or more, 8500 or more, 8600 or more, 8700 or more, 8800 or more, 8900 or more, 9000 or more, 9100 or more, 9200 or more, 9300 or more, 9400 or more, 9500 or more, 9600 or more, 9700 or more, 9800 or more, 9900 or more, 10000 or more, 10100 or more, 10200 or more, 10300 or more, 10400 or more, 10500 or more, 10600 or more, 10700 or more, 10800 or more, 10900 or more, 11000 or more, 11100 or more, 11200 or more, 11300 or more, 11400 or more, 11500 or more, 11600 or more, 11700 or more, 11800 or more, 11900 or more, 12000 or more, 12100 or more, 12200 or more, 12300 or more, 12400 or more, 12500 or more, 12600 or more, 12700 or more, 12800 or more, 12900 or more, 13000 or more, 13100 or more, 13200 or more, 13300 or more, 13400 or more, 13500 or more, or 13600 or more nucleotide sequences selected from nucleotide sequences of SEQ ID Nos.: 33 to 6236, SEQ ID Nos.: 75337 to 82784 and SEQ ID Nos.: 166154 to 166181.

### (Database: Reference Example 7)

In addition, databases which can be used, may comprise the following sequence information: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1600 or more, 1700 or more, 1800 or more, 1900 or more, 2000 or more, 2100 or more, 2200 or more, 2300 or more, 2400 or more, 2500 or more, 2600 or more, 2700 or more, 2800 or more, 2900 or more, 3000 or more, 3100 or more, 3200 or more, 3300 or more, 3400 or more, 3500 or more, 3600 or more, 3700 or more, 3800 or more, 3900 or more, 4000 or more, 4100 or more, 4200 or more, 4300 or more, 4400 or more, 4500 or more, 4600 or more, 4700 or more, 4800 or more, 4900 or more, 5000 or more, 5100 or more, 5200 or more, 5300 or more, 5400 or more, 5500 or more, 5600 or more, 5700 or more, 5800 or more, 5900 or more, 6000 or more, 6100 or more, or 6200 or more nucleotide sequences selected from nucleotide sequences of SEQ ID Nos.: 33 to 6236 and SEQ ID Nos.: 166154 to 166181.

### (Database: Reference Example 8)

In addition, databases which can be used, may comprise the following sequence information: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1600 or more, 1700 or more, 1800 or more, 1900 or more, 2000 or more, 2100 or more, 2200 or more, 2300 or more, 2400 or more, 2500 or more, 2600 or more, 2700 or more, 2800 or more, 2900 or more, 3000 or more, 3100 or more, 3200 or more, 3300 or more, 3400 or more, 3500 or more, 3600 or more, 3700 or more, 3800 or more, 3900 or more, 4000 or more, 4100 or more, 4200 or more, 4300 or more, 4400 or more, 4500 or more, 4600 or more, 4700 or more, 4800 or more, 4900 or more, 5000 or more, 5100 or more, 5200 or more, 5300 or more, 5400 or more, 5500 or more, 5600 or more, 5700 or more, 5800 or more, 5900 or more, 6000 or more, 6100 or more, or 6200 or more nucleotide sequences selected from nucleotide sequences of SEQ ID Nos.: 33 to 6236.

### (Database: Reference Example 9)

In addition, databases which can be used, may comprise the following sequence information: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 200 or more, 300 or more, 400 or more, 500 or more, 600 or more, 700 or more, 800 or more, 900 or more, 1000 or more, 1100 or more, 1200 or more, 1300 or more, 1400 or more, 1600 or more, 1700 or more, 1800 or more, 1900 or more, 2000 or more, 2100 or more, 2200 or more, 2300 or more, 2400 or more, 2500 or more, 2600 or more, 2700 or more, 2800 or more, 2900 or more, 3000 or more, 3100 or more, 3200 or more, 3300 or more, 3400 or more, 3500 or more, 3600 or more, 3700 or more, 3800 or more, 3900 or more, 4000 or more, 4100 or more, 4200 or more, 4300 or more, 4400 or more, 4500 or more, 4600 or more, 4700 or more, 4800 or more, 4900 or more, 5000 or more, 5100 or more, 5200 or more, 5300 or more, 5400 or more, 5500 or more, 5600 or more, 5700 or more, 5800 or more, 5900 or more, 6000 or more, 6100 or more, 6200 or more, 6300 or more, 6400 or more, 6500 or more, 6600 or more, 6700 or more, 6800 or more, 6900 or more, 7000 or more, 7100 or more, 7200 or more, 7300 or more, or 7400 or more nucleotide sequences selected from nucleotide sequences of SEQ ID Nos.: 75337 to 82784.

### (Database: Reference Example 10)

In addition, databases which can be used, may comprise the following sequence information: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, or 27 or more nucleotide sequences selected from nucleotide sequences of SEQ ID Nos.: 166154 to 166181.

### (Database: Reference Example 11)

In addition, databases which can be used, may comprise the following sequence information: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 200 or more, 300 or more, 400 or more, or 500 or more nucleotide sequences selected from nucleotide sequences of SEQ ID Nos.: 166490 to 167042.

### (Database: Reference Example 12)

In addition, databases which can be used, may comprise the following sequence information: 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 200 or more, 300 or more, 400 or more, 500 or more, 600 or more, 700 or more, 800 or more, 900 or more, 1000 or more, 1100 or more, 1200 or more, 1300, or 1400 or more nucleotide sequences selected from nucleotide sequences of SEQ ID Nos.: 173125 to 174603.

In one preferable embodiment of the invention, amino acid sequence databases theoretically translated from the nucleotide sequence database for the genome of the bottom fermenting yeast may be used as the reference databases. For example, the amino acid sequence database corresponding to the database of the above-mentioned nucleotide sequence may be used. However, these databases are presented merely as examples, and the reference database used in the invention is by no means restricted to these examples.

As used herein, the term "refer to the reference database" is intended to mean to determine whether a nucleotide sequence or amino acid sequence corresponding to all or a part of the amino acid sequence of the target protein or the mass pattern thereof is present in the database by searching the reference database. Usually, such operations are conducted by running common software on a computer. While software such as BLAST, FASTA, Smith & Waterman, pep-pat (http://peppat.cbi.pku.edu.cn/) may be used for search of all or a part of the amino acid sequence of the target protein, but are not limited thereto. While commercially available software such as MASCOT may be used for search of all or a part of the mass pattern of the target protein, but is not limited thereto.

The reference database may be used by being stored in an external recording medium such as a hard disk of a computer or CD-ROM, or in a recording medium such as a server.

Not only amino acid sequence 100% identical to the sequence to be searched, but also amino acid sequences 60% or more, preferably 70% or more, more preferably 80% or more, further preferably 90% or more, and most preferably 95% or more, 96% or more, 97% or more, 98% or more, or 99% or more identical to the sequence to be searched are searched in the database by taking the possibility of mutation into consideration in searching the above-mentioned amino acid sequence. Software such as BLAST, FASTA, and Smith & Waterman may be used for such search.

As used herein, the term "identical to", for example, "an amino acid sequence at least 95% identical to the sequence to be searched" is intended to mean that the amino acid sequence to be searched is identical to the reference sequence, except that mismatch of up to 5 amino acid residues in a sequence consisting of 100 amino acids may be included in the reference amino acid sequence. "Mismatch" is caused by substitution, addition, deletion and/or insertion of one or more amino acid residue(s) in an arbitrary position of the sequence.

In a further aspect of the disclosure, results of search are analyzed using a comparative database associated with genome sequence information of *S*. *cerevisiae* in which genome sequence information has been published. The function of ORF identified in the reference nucleotide sequence may be deduced by homology search with the amino acid sequence of ORF of *S*. *cerevisiae.* Genome sequence information is registered as a database Saccharomyces Genome Database (SGD: http://www.yeastgenome.org/) and open to the public.

### 7. Analysis of Function of Identified Gene

When the gene encoding the target protein could be identified as described above, the function of said gene could be deduced by searching a known database. Such search is possible by using a sequence alignment algorithm, for example BLAST algorithm. Thus, the function of the gene may be considered to be similar with the gene identified by this search. As used herein, the term "analyze the function of the gene" is used to mean to deduce the function from known information.

To be more practice, Non-Sc gene of the bottom fermenting yeast may be identified by using the reference database used in the present invention. The function of such Non-Sc gene may be considered to be similar with the function of the gene hit by homology search for the comparative reference database, for example, the amino acid sequence and nucleotide sequence of ORF of *S*. *cerevisiae* registered in the Saccharomyces Genome Database (SGD: http://wwwyeastgenome.org/), and a non-redundant (nr) database described in National Center of Biotechnology Information (NCBI: http://www.ncbi.nlm.nih.gov/).

Annotations of the functions of 6204 Non-ScORFs (SEQ ID Nos.: 33 to 6236) and 28 mitochondrial ORFs (SEQ ID Nos.: 166154 to 166181) encoded in the genome of the bottom fermenting yeast are listed at the end of the present specification.

For confirming the function of the target protein and the gene encoding the target protein, the gene identified by the above-mentioned method is inserted into a vector. Then, the yeast cells are transformed with that vector to evaluate to the function of the target protein.

Such vector is usually constructed so that the vector comprises (a) a promoter capable of transcription in the yeast cells, (b) a polynucleotide (DNA) linked to the promoter in a sense direction of antisense direction and identified by the above-mentioned method, and (c) an expression cassette comprising a signal that functions in yeast as a constituting element with respect to termination of transcription of RNA molecules and polyadenylation.

For example, when the protein identified as described above is allowed to be expressed in a high level in fermentation of alcoholic beverages such as beer, a polynucleotide is introduced in a sense direction relative to the promoter so as to enhance expression of the polynucleotide (DNA) of the gene encoding the above-mentioned identified protein. When expression of the above-mentioned protein is to be suppressed in fermentation of alcoholic beverages such as beer, a polynucleotide is introduced in an antisense direction relative to the promoter so as to suppress expression of the polynucleotide (DNA) of the gene encoding the above-mentioned identified protein. Expression of the above-mentioned DNA or expression of the above-mentioned protein may be suppressed by disrupting the target gene (DNA). The gene can be disrupted by adding a single base or plurality of bases to, or deleting a single base or plurality of bases in regions related to expression of gene products in the target gene, for example within coding regions or promoter regions, or by deleting entire regions. Published reports may be referenced with respect to the method for disrupting these genes (for example, see Proc. Natl. Acad. Sci. USA, 76, 4951(1979), Methods in Enzymology, 101, 202(1983) and JP-A No. 6-253826).

The vectors available for introducing them into the yeast are any of vectors of a multiple copy (YEp) type, single copy (YCp) type and chromosome-integrated (YIp) type. For example, examples of YEp vector include YEp24 (J. R. Broach et al., Experimental Manipulation of Gene Expression, Academic Press, New York, 83 (1983)). Examples of YCp vector include YCp50 (M. D. Rose et al., Gene, 60, 237 (1987)). Examples of YIp vector include YIp50 (K. Struhl et al., Proc. Natl. Acad. Sci. USA, 76, 1035 (1979)). These vectors are readily available.

Examples of the promoter/terminator available for regulating expression of the gene in yeast include a promoter for glyceraldehyde triphosphate dehydrogenase gene (TDH3) and a promoter for 3-phosphoglycerate kinase gene (PGK1). These genes have been already cloned as described in detail in, for example, Tuite et al., EMBO J., 1, 603 (1982). They are readily available by known methods.

While auxotrophic makers cannot be used as the selection maker used for transformation in the brewery yeast, a geneticin resistance gene (G418r), copper resistant gene (CUP1) (Marin et al., Proc. Natl. Acad. Sci. USA, 81, 337 (1984)) and cerulenin resistant marker (fas2m, PDR4) may be used (J. Inokoshi et al., Biochemistry (seikagaku), 64, 660 (1992); Hussain et al., Gene, 101, 149 (1991)). The vectors constructed as described above are introduced into host yeast. Examples of the host yeast include those available for fermentation, for example brewery yeast for beer, wine and sake. While specific examples include yeast of genus *Saccharomyces,* brewery yeast such as *Saccharomyces pastorianus Weihenstephan* 34/70, *Saccharomyces carlsbergensis* NCYC453 and NCYC456, and *Saccharomyces cerevisiae* (*Saccharomyces cerevisiae*) NBRC1951, NBRC1952, NBRC1953, NBRC1954) may be used in the invention. While whisky yeast such as *Saccharomyces cerevisiae* NCYC90, wine yeast such as yeast for wine of brewing society of Japan #1, #3 and #4, and sake yeast such as yeast for sake of brewing society of Japan #7 and #9 may be also used, but are not limited thereto. Beer yeast, for example *Saccharomyces pastorianus*, is preferably used in the present disclosure.

Yeast may be transformed by a well known method used commonly. For example, an electroporation method "Meth. Enzym., 194, 182 (1990)", a spheroplast method "Proc. Natl. Acad. Sci. USA, 75, 1929 (1978)" and lithium acetate method "J. Bacteriology, 153, 163 (1983), Proc. Natl. Acad. Sci. USA, 75, 1929 (1978), Methods in Yeast Genetics, 2000 Edition : A Cold Spring Harbor Laboratory Course Manual" are available, but are not limited thereto.

More specifically, the host yeast is cultivated in a standard yeast nutrient medium (for example YEPD "Genetic Engineering. Vol.1, Plenum Press, New York, 117 (1979)") to OD 600 nm of from 1 to 6. The fermented yeast is collected by centrifugation, and is pretreated with an alkali metal ion, preferably lithium ion, at a concentration form 1 to 2M. The cells are allowed to stand still for about 60 minutes at about 30°C, and additionally allowed to stand still at about 30°C for about 60 minutes by mixing with DNA (from about 1 to 20 µg) to be introduced. Polyethylene glycol, preferably polyethylene glycol with a molecular weight of about 4000 dalton, is added to a final concentration of from about 20% to 50%. After allowing the cells to stand still at 30°C for about 30 minutes, the cells are heat-treated at about 42°C for about 5 minutes. Preferably, the cell suspension solution is washed with the standard yeast nutrient medium, and allowed to stand still for about 60 minutes at about 30°C by pouring the suspension solution into a fresh standard yeast nutrient medium. The cells are seeded thereafter on a standard agar medium containing an antibiotics used as a selection marker to obtain a transformant. References may be made to "Molecular Cloning (Methods in Yeast Genetics, A laboratory manual (Cold Spring Harbor Laboratory Press, Cold Spring Harbor, NY))" with respect to other standard cloning techniques.

Measurement of the expression level of the target gene is possible by quantificarion of mRNAs or proteins as the target gene products extracted from yeast cell culture. Methods known in the art may be used for quantification of the mRNA or protein. For example, mRNAs are quantified, for example, by Northern hybridization or qualitative RT-PCR, while proteins are quantified by Western blotting (Current Protocols in Molecular Biology, John Wiley & Sons 1994-2003).

The assimilation property of amino acids, sulfate ions and ammonia, low temperature resistance, foam stability, haze formation, ester formation and flocculation property of the yeast can be evaluated by a beer fermentation test and so on, and thus the function of the gene encoding the target protein can be evaluated.

### 8. Evaluation of yeast

Yeast favorable for brewing of desired alcoholic beverages may be selected by comparing characteristics of the test yeast with that of the standard yeast. The yeast transformed with above-mentioned vector, the yeast in which said gene expression is genetically controlled, the yeast subjected to mutation treatment and spontaneously mutated yeast may be used as the test yeast or standard yeast The mutation treatment can be applied by a method known in the art such as UV irradiation and EMS treatment (see, for example, Yasuji Ohsima, Seikagaku Jikkenho (Method of Biochemical Experiments), Vol. 39, p67-75, published by Japan Scientific Societies Press) or by them with appropriate modification.

Thus, yeast whose genome has been sequenced, mutant strains of said yeast and yeast strains selected from a culture collection may be subjected to proteome analysis to identify proteins showing variations of the expression level. Further, the proteome analysis of wild type of industrial strains and production strains having favorable phenotype can be used for identification of target proteins for breeding to improve productivity of desired products. To be more practice, when wild type strain and mutant strain which produces much amount of beer flavor compound are subjected to proteome analysis, spots corresponding to mutant strain may be identified, and consequently, protein contained in each spot may be analyzed and identified to show contribution to increasing the amount of beer flavor compound. Alternatively, spots showing different amounts of proteins among the conditions are found by the proteome analysis of the strains cultivated under different cultivation conditions, and the spots are subjected to search of the database. Consequently, the protein necessary for adaptation to the cultivation condition and the gene encoding the target protein may be identified.

Further, the methods of the present invention may enable not only a nucleotide sequence encoding a protein but also a nucleotide sequence locating upstream of the coding region to be searched. Therefore, for example, a nucleotide sequence that functions as a high-expression promoter may efficiently be selected by indentifying a protein showing a high level of expression in the bottom fermenting yeast cells by proteome analysis. Moreover, since modifications of proteins can cause changes in separation of proteins in proteome analysis, the modified protein may efficiently be identified by search using nucleotide sequence information and amino acid sequence information of the bottom fermenting yeast used in the method of the present invention, or search using a recording medium on which the nucleotide and amino acid sequence information are recorded.

Actually, through the proteome analysis of the proteins extracted from the bottom fermenting yeast cells in the beer brewing, the present inventors identified one of the proteins that showed the increases of expression levels along with the decrease of the methionine concentration in the beer fermentation broth as Non-ScMET17. Futhermore, according to this result, the present inventors identified and isolated the gene encoding Non-ScMET17. The gene was introduced into the yeast cells by gene engineering techniques and highly expressed in the transformant cells. This resulted in large decrease of H₂S production in the yeast cells during beer fermentation.

### Examples

Hereinafter, the present invention will be described in more detail with reference to working examples. The present invention, however, is not limited to the examples described below.

### Example 1: Proteome analysis of the yeast during beer fermention.

### 1. Beer fermentation test and sampling of the yeast cells

A fermentation test using bottom fermenting yeast *Saccharomyces pastorianus Weihenstephan* 34/70 strain (pYCGPYNot plasmid introduced strain) was performed under the following conditions.

| | |
|---|---|
| Wort extract concentration: | 12% |
| Wort content: | 2 L |
| Wort dissolved oxygen concentration: | about 8 ppm |
| Fermentation temperature: | 15°C constant |
| Yeast pitching rate: | 5g of wet yeast cell/1 L wort |

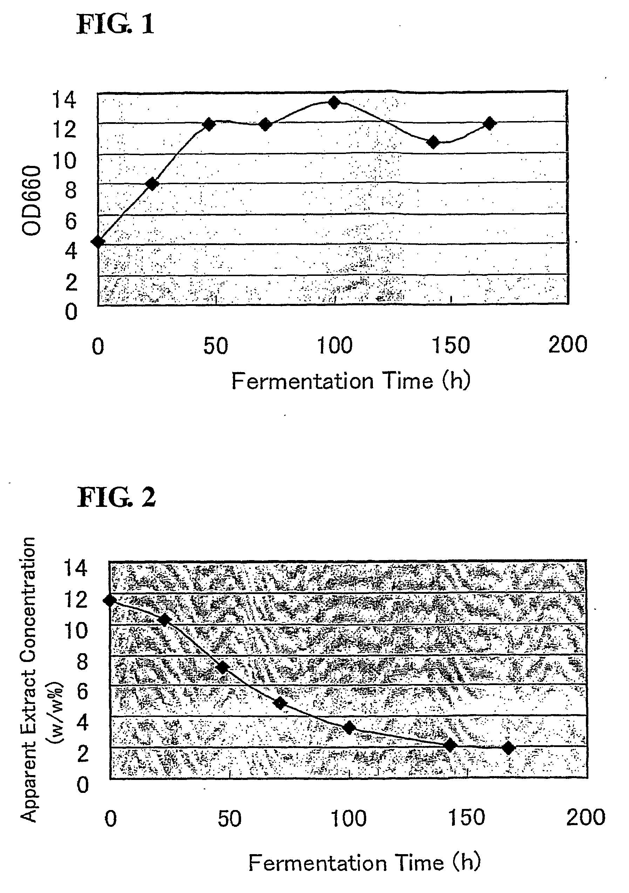

The fermentation broth was regularly sampled, and the time-course changes in the yeast cell growth (Fig. 1) and the apparent extract concentration (Fig. 2) were observed. At the same time, yeast cells corresponding to 90 mg (wet weight) were harversted by centrifugation (3,000 × g, 4°C, 10 minutes). The harversted cells were allowed to be preserved for about several days at -80°C. The frozen cells were defrosted immediately before use at room temperature. The supernatant of the fermentation broth was also recovered, and amino acid composition thereof was analyzed as described below.

### 2. Analysis of the amino acid composition of the supernatant of the fermentation broth

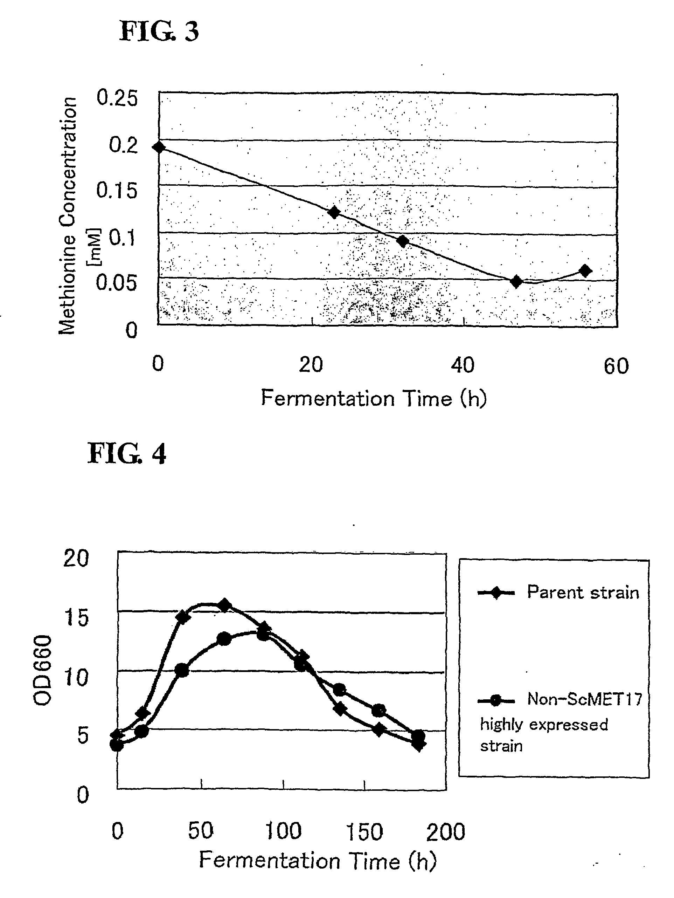

The amino acid composition of the supernatant of the fermentation broth was analyzed using a high speed amino acid analyzer (trade name: L-8800, manufactured by Hitachi Co.) and standard amino acid analysis column (trade name: P/N 855-3506, manufactured by Hitachi Co.). The time-course change in the methionine concentration is shown in Fig. 3.

### 3. Preparations of electrophoresis samples

Yeast cell extracts for two-dimensional electrophoresis were prepared by the following procedure. After washing once with lysis buffer (10 mmol/L, Tris-HCl (pH 8.0), 2 mmol/L MgCl₂), the cells were resuspended in 500 µL of lysis buffer containing Protease Inhibitor Cocktail for use with Fungal and Yeast Extracts (manufactured by SIGMA Co.) (hereinafter, represented by protease inhibitor cocktail) at a final concentration of 1% together with 0.5 g of dry-heat sterilized glass beads (425 to 600 µm, manufactured by SIGMA Co.). Cells were then disrupted by stirring at 4°C for 45 minutes using a TWIN 3-28 Tuple mixer (manufactured by Asahi TechnoGlass Co.). In this process, the sample was placed on ice for 2 minutes for every 15 minutes. After disruption, non-disrupted cells and insoluble substances were removed by centrifugation (15,000 × g, 4°C, 10 minutes), and the supernatant fraction was recovered. Benzonase nuclease (manufactured by Novagen Co.) was added to the supernatant fraction at a final concentration of 0.25 U/µL sample, and the sample was placed on ice for 30 minutes. Then, 5 mol/L of NaCl was added at a final concentration of 1 mol/L and the sample was placed on ice for another 5 minutes. Insoluble substances were removed by centrifugation (15,000 × g, 4°C, 5 minutes) to retrieve the supernatant fraction. The supernatant was divided into two portions, each portion was applied on a ultrafiltration spin column (trade name: Microcon YM-10, manufactured by Millipore Co.), and each supernatant was concentrated to a volume of 100 µL or less at 14,000 × g and 4°C. 400 µL of cold ultrapure water containing protease inhibitor cocktail at a final concentration of 1% was added thereto and mixed by pipetting, and each was concentrated to a volume of 100 µL or less at 14,000 x g and 4°C again. After desalting and concentration of the sample by repeating the same concentrating operation 3 times, the concentrated sample was recovered. While a part of the solute was precipitated in this concentration process, the precipitated solute was also recovered. The sample including the precipitate was solubilized by adding 4-fold volume of sample buffer (9.3 M urea, 2.3% (w/w) of CHAPS, 46.5 mM DTT, 0.002% (w/v) BPB), and the protein concentration of the sample was determined using 2D-Quant Kit (manufactured by Amersham Biosciences Co.). The sample corresponding to 111 µg of protein (usually, the volume of the sample was less than 50 µL) was mixed with 2.5 µL IPG Buffer (pH 4-7, manufactured by Amersham Biosciences Co.) and 430 µL of sample buffer, and was used as a electrophoresis sample after diluting to a total volume of 500 µL with ultra-pure water.

### 4. Separation of proteins by two-dimensional electrophoresis

The first-dimension separation of the proteins by isoelectric focusing was carried out as follows. After applying 450 µL of the electrophoresis sample (corresponding to 100 µg of protein) to 24 cm Strip Holder (manufactured by Amersham Biosciences Co.), a ready-made, dried immobilized pH gradient (IPG) strip gel (trade name: Immobiline DryStrip pH 4-7, 24cm, manufactured by Amersham Biosciences Co.) was placed onto the solution. Then, IPG Cover Fluid (manufactured by Amersham Biosciences Co.) was applied into the holder until the entire IPG strip was covered. The holder was positioned onto the platform of IPGphor isoelectric focusing apparatus (manufactured by Amersham Biosciences Co.), and rehydration of the gel and following isoelectric focusing were performed at a controlled temperature of 20°C. The electric current limit during isoelectric focusing was 50 µA/strip.
Rehydration: 10 hours
Isoelectric focusing, first step: 2 hours at a constant voltage of 500 V
Isoelectric focusing, second step: 1 hour under a gradient mode from 500 to 1000 V
Isoelectric focusing, third step: 3 hours under a gradient mode from 1000 to 8000 V
Isoelectric focusing, fourth step: 5.6 hours at a constant voltage of 8000 V

After isoelectric focusing, the IPG strip gel was taken out of the holder and the excess cover fluid was removed for the second-dimension separation, SDS-PAGE. When it was difficult to perform SDS-PAGE immediately after completing isoelectric focusing, the IPG strip gel was allowed to be stored in a hermetically sealable tube at - 80°C for several days. The gel after freeze storage was subjected to SDS-PAGE by the same procedure as the sample not preserved by freezing after defrosting at room temperature. SDS-PAGE was performed as follows. The IPG strip gel was immersed in 20 mL/strip of SDS equilibration buffer (50 mM Tris-HCl (pH 8.8), 6 M urea, 30% (v/v) glycerol, 2% (w/v) SDS, 1% (w/v) DTT, 0.002% (w/v) BPB), and was equilibrated by gently shaking at room temperature for 15 minutes. DALT Gel 12.5 (manufactured by Amersham Biosciences Co.) was attached to Ettan DALT Precast Gel Cassette (manufactured by Amersham Biosciences Co.). The equilibrated IPG strip gel was rinsed by dipping in cathode buffer (packaged in DALT Buffer Kit, manufactured by Amersham Biosciences Co.) and then attached so as to closely adhere on the top surface of DALT Gel 12.5. A piece of filter paper impregnated with molecular weight marker (trade name: LMW SDS Electrophoresis Calibration Kit, manufactured by Amersham Biosciences Co.) was also attached to the top surface of DALT Gel 12.5, and they were sealed with sealing solution (packaged in DALT Buffer Kit, manufactured by Amersham Biosciences Co.) melted by heating at 95°C. A prescribed volume of anode buffer (packaged in DALT Buffer Kit, manufactured by Amersham Biosciences Co.) was injected into the electrophoresis tank of Ettan DALT electrophoresis apparatus (manufactured by Amersham Biosciences Co.). The gel was inserted into the unit and the cathode chamber was filled with a prescribed volume of cathode buffer (packaged in DALT Buffer Kit, manufactured by Amersham Biosciences Co.). Electrophoresis was performed at a constant temperature of 25°C, and after running at 5 W/gel for 30 minutes, continued at 17 W/gel until the dye front of BPB included in the sealing solution reached the lower end of the gel.

### 5. Detection of protein spots

After completing SDS-PAGE, silver staining of the gel was performed with reference to the method by Shevchenko, A. et al (Anal. Chem., 68, 850 (1996)) and the protocol attached to Silver Staining Kit, Protein (manufactured by Amersham Biosciences Co.). The gel after electrophoresis was immersed in a fixing solution (40% (v/v) ethanol, 10% (v/v) acetic acid) for 30 minutes to overnight. After placing the gel for additional 30 minutes after exchange of the fixing solution, the gel was immersed in a sensitizing solution (30% (v/v) ethanol, 0.2% (w/v) Na₂S₂O₃, 6.8% (w/v) sodium acetate, 30 minutes), ultra-pure water (5 minutes) × 3, silver reaction solution (0.25% (w/v) AgNO₃, 20 minutes) and ultra-pure water (1 minute) × 2 in this order, and was developed by immersing in developing solution (2.5% Na₂CO₃, 0.04% (v/v) of 37% (w/v) formaldehyde) until the protein spots were stained until an appropriate chromatic intensity was reached. The gel was immersed in stopping solution (1.46% (w/v) EDTA·Na₂·2H₂O, 10 minutes), ultra-pure water (5 minutes) × 3 and preserving solution (8.7% (w/v) glycerol, 20 minutes) in this order thereafter to complete staining. The gel was gently shaken at room temperature in each step of staining, and the volume of all the solutions used was 250 mL/gel.

The proteins extracted from the cells were separated through the above-mentioned operations, and were able to be detected as spots.

### 6. Preparation of mass spectral analysis sample

The gels of two-dimensional electrophoresis carried out by using the yeast cells sampled at 8 and 32 hours after the start of fermentation test were compared to one another, and protein spots that showed increased intensities along with the decrease of methionine in the fermentation broth were selected and excised. When the amount of protein contained in a spot was not sufficient for mass spectral analysis, the identical spots were excised from two or more sheets of the gel on which the same electrophoresis sample was applied, and the excised gel pieces were collected in one fraction. The excised gel piece was transferred to a low adsorption tube, and was subjected to destaining and in-gel digestion steps described below. When it was difficult to perform the following treatments immediately, the gel pieces were stored at 4°C for a maximum period of several days with soaking in 500 µL/tube of fixing solution (33% (v/v) ethanol, 7.5% (v/v) acetic acid).

The gel pieces were destained by the following procedure. 100 µL of 50/50 30 mM K₃Fe(CN)₆ in 100 mM Na₂S₂O₃ was added to the gel pieces, and the solution was removed after shaking for 10 minutes at room temperature in the dark. Subsequently, the gel pieces were washed with 500 µL of ultra-pure water by shaking for 10 minutes at room temperature and then water was removed. This operation was performed three times. After that, the gel pieces were washed with 300 µL of 0.1M NH₄HCO₃/50% acetonitrile by shaking for 15 minutes at room temperature. This operation was repeated again.

After destaining, the proteins contained in the gel pieces were subjected to reductive alkylation as follows. 200 µL of reduction buffer (10 mM DTT, 98% 0.2M Tris, 2% EDTA·Na₂ (pH 8.0)) was added to the gel pieces and they were incubated at 37°C for 1 hour. After adding 4 µL of 4-vinylpyridine, the gel pieces were allowed to stand in dark for 1 hour, and 4 µL of 2-mercaptoethanol and 1 mL of ultra-pure water was further added. The supernatant was removed after allowing the gel pieces to stand for 30 minutes. Subsequently, 1 mL of ultra-pure water was added to the gel pieces and removed after incubation for 30 minutes. This operation was performed three times, and then, the gel pieces were incubated with 1 mL of 0.1M NH₄HCO₃/50% acetonitrile for 30 minutes. After removing the supernatant, the gel pieces were frozen at -80°C and were dried for 2 hours using SpeedVac vacuum drier (manufactured by Thermo Electron Co.).

The proteins in the dried gel were digested as follows. 5 µL of trypsin solution (0.1 µg/µL) was added to the gel pieces, and the solution was allowed to stand at 4°C for 10 minutes. 200 µL of digest buffer (50% 0.2M NH₄HCO₃, 10% acetonitrile, 0.5% 0.1 M CaCl₂) was added to the gel pieces and they were incubated at 37°C overnight. After retrieving the liquid phase, the gel was shaken for additional 30 minutes with 200 µL of 0.1% TFA/60% acetonitrile. This liquid phase and the liquid phase recovered in advance were combined, and the combined liquid phase was frozen at -80°C followed by vacuum drying. After adding 10 µL of 2% acetonitrile/0.1% TFA to the freeze-dried samples and shaking them for 10 minutes, insoluble fractions were removed by centrifugation for 5 minutes, and the supernatant was used as a sample for mass spectral analysis.

### 7. Mass spectral analysis and amino acid sequence analysis of target protein using liquid chromatography/tandem mass spectrometer (LC-MS/MS)

The sample for mass spectral analysis was separated using Paradigm MS-4 HPLC apparatus (manufactured by Michrom BioResources Co.) and Magic C18 column (manufactured by Michrom BioResources Co.). Depending on the separation states, either solvent A (2% acetonitrile, 0.1% formic acid) and solvent B (90% acetonitrile, 0.1% formic acid), or solvent A (2% acetonitrile, 0.1% TFA) and solvent B (90% acetonitrile, 0.1% TFA) was chosen as the combination of mobile phases. Separation was carried out at a constant flow rate of 1.0 µL/min under the following gradient mode:
First step: 5 to 65% gradient mode of solvent B for 50 minutes, and
Second step: 65 to 5% gradient mode of solvent B for 10 minutes.

Each fraction obtained was sequentially subjected to LCQ Advantage mass spectrometer (trade name: manufactured by Thermo Electron Co.).

### 8. Identification of target protein

Through the operations mentioned above, the list of peptide masses derived from the target protein was obtained. Based on this information, ORF corresponding to the protein was identified by searching the genome sequence database. Commercially available program, MASCOT (manufactured by MATRIX SCIENCE Co.), was used for identification of the protein. S-pyridylethyl (C) and ESI-TRAP were selected for Fixed Modifications and Instrument, respectively, as search parameters, and default setting values were used for the other parameters.

### 9. Search and identification of gene encoding protein regulated by methionine concentration

Among the proteins that showed the increases of expression levels along with the decrease of methionine concentration in the fermentation broth, the protein contained in the spot detected at a molecular weight of 50 kDa and pI of 6.0 was identified according to the method mentioned above. The result showed that the spot corresponded to the protein encoded by Non-Sc type O-acetyl homoserine-O-acetyl serine sulfhydrylase (Non-ScMET17) gene represented by SEQ ID No. 1817. The amino acid sequence of Non-ScMET17 gene was represented by SEQ ID No. 197022.

The results mentioned above indicated that identifying the protein extracted from the bottom fermenting yeast cells using the genome sequence database constructed in the present invention enabled the nucleotide sequence of the ORF corresponding to the protein to be searched. Moreover, since it was possible to search the nucleotide sequence locating upstream of the ORF together with the nucleotide sequence of the ORF, these results also indicated that identification of the protein whose level of expression was regulated by the methionine concentration in the fermentation broth enabled the nucleotide sequence that functions the methionine-concentration responsible promoter to efficiently be searched.

### Example 2: Introduction of Non-ScMET17 gene into yeast cells and expression of the gene in the cells

### 1. Cloning of novel O-acetyl homoserine-O-acetyl serine sulfhydrylase gene

Based on the nucleotide sequence information of the gene that encodes the protein identified in Example 1, two primers for amplification of whole length of the gene, Non-ScMET17_for (SEQ ID No: 197023) and Non-ScMET17_rv (SEQ ID No: 197024), were designed. Using these primers, PCR was performed using chromosome DNA of the genome-sequenced strain, *Saccharomyces pastorianus Weihenstephan* 34/70, as a template and DNA fragment (about 1.3 kb) comprising the whole length of Non-ScMET17 gene was obtained.

The amplified DNA fragment was inserted into pCR2.1-TOPO vector (manufactured by Invitrogen Co.) by TA cloning. The nucleotide sequence of Non-ScMET17 was analyzed by Sanger method (F. Sanger, Science, 14, 1215 (1981)) and verified.

### 2. Construction of Non-ScMET17 highly expressed strain

Non-ScMET17/pCR2.1-TOPO was digested with restriction enzymes SacI and NotI to prepare a DNA fragment comprising the entire length of the protein-encoding region of Non-ScMET17. This fragment was ligated to pYCGPYNot pre-treated with the restriction enzymes SacI and NotI, thereby constructing the Non-ScMET17 high expression vector, Non-ScMET17/pYCGPYNot. pYCGPYNot is a YCp-type yeast expression vector, and the gene inserted into the vector is highly expressed by the pyruvate kinase gene PYK1 promoter. This vector includes the geneticin-resistant gene, G418^{r}, as the selectable marker in the yeast, and also includes the ampicillin-resistant gene, Amp^{r}, as the selectable marker in *Escherichia coli.*

Using the high expression vector prepared by the above method, *Saccharomyces pastorianus Weihenstephan* 34/70 strain was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

### 3. Quantification of the amount of production of H₂S in test brewing of beer

Fermentation tests using the parent strain and Non-ScMET17 highly expressed strain obtained in the sections above were performed under the following conditions.

| | |
|---|---|
| Wort extract concentration : | 12% |
| Wort content: | 2L |
| Wort dissolved oxygen concentration: | about 8 ppm |
| Fermentation temperature: | 15°C constant |
| Yeast pitching rate: | 5 g wet yeast cell/L Wort |

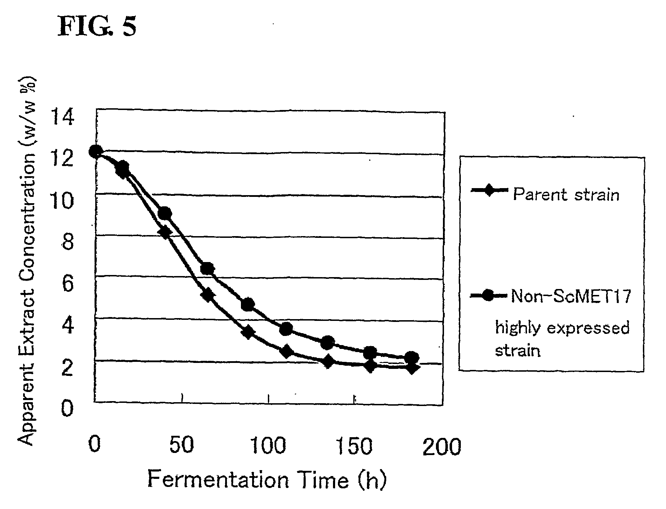

The fermentation broth was regularly sampled, and the time-course changes in amount of yeast cell growth (OD 660: Fig. 4) and apparent extract concentration (Fig.5) were observed. Quantification of H₂S production during fermentation was performed according to the method described by Takahashi et al. (Brauwissenschaft, 31, 1 (1978)). Measurements of H₂S to creat the standard curve were performed used samples containing known concentration of H₂S, and then the standard curve for H₂S quantification was created according to the peak areas of detected H₂S. The fermentation samples were analyzed under the same condition as samples for the standard curve and the amount of H₂S contained in the fermentation samples were determined by comparing the peak areas of H₂S thereof with the standard curve.

**Table 1: The amount of H₂S in fermentation broth at the end of fermentation**

| | Parent strain | Non-ScMET17 highly expressed strain |
|---|---|---|
| H₂S (ppb) | 22.1 | - |

| | | |
|---|---|---|
| (Note) - ; below detection limit (No H₂S peak was detected.) | | |

As shown in Table 1, the amount of H₂S produced by Non-ScMET17 expressed strain was below detection limit at the end of fermentation, though that by the parent strain was 22.1 ppb. This indicated that the amount of production of H₂S was largely reduced by expression of Non-ScMET17 in a high level.

### (References)

Putative functions of 6204 Non-Sc type ORFs (SEQ ID Nos.: 33 to 6236) and 28 mitochondrial ORFs (SEQ ID Nos.: 166154 to 166181) encoded in the genome of the bottom fermenting yeast by the present inventors are listed below.

The functions of genome sequence information as shown below were inferred by homology search using sequence alignment algorithm such as BLAST algorithm. When using BLAST algorithm, for example, the amino acid sequences or nucleotide sequences of ORFs of *S*. *cerevisiae* registered and released in Saccharomyces Genome Data.base (SGD: http://www.yeastgenome.org/) and/or non-redundant (nr) database of amino acid sequence or nucleotide sequence information provided by National Center of Biotechnology Information (NCBI: http://www.ncbi.nlm.nih.gov/) can be used for the comparative reference database.

### (SEQ ID Nos.: 33 to 6236)

33;putative polyol dehydrogenase

34;NAD-dependent (2R,3R)-2,3-butanediol dehydrogenase, a zinc-containing medium-chain alcohol dehydrogenase, produces 2,3-butanediol from acetoin during fermentation and allows using 2,3-butanediol as a carbon source during aerobic growth

35;Putative peroxisomal membrane protein required for import of peroxisomal proteins, functionally complements a Pichia pastoris pex22 mutation

36;Acetyl-coA synthetase isoform, expressed during growth on nonfermentable carbon sources and under aerobic conditions

37;Protein of unknown function; green fluorescent protein (GFP)-fusion protein localizes to the cytoplasm in a punctate pattern

38;Evolutionarily-conserved tail-anchored outer mitochondrial membrane GTPase which regulates mitochondrial morphology; cells lacking Gemlp contain collapsed, globular, or grape-like mitochondria; not required for pheromone-induced cell death

39;Protein localized to COPII-coated vesicles, forms a complex with Erv41p; involved in the membrane fusion stage of transport

40;G1 cyclin involved in cell cycle progression; activates Cdc28p kinase to promote the G1 to S phase transition; plays a role in regulating transcription of the other G1 cyclins, CLN1 and CLN2; regulated by phosphorylation and proteolysis

41;Pyruvate kinase, functions as a homotetramer in glycolysis to convert phosphoenolpyruvate to pyruvate, the input for aerobic (TCA cycle) or anaerobic (glucose fermentation) respiration

42;Hypothetical protein

43;Member of the DRG family of GTP-binding proteins; interacts with translating ribosomes

44;GTPase, required for general translation initiation by promoting Met-tRNAiMet binding to ribosomes and ribosomal subunit joining; homolog of bacterial IF2

45;Essential component of the MIND kinetochore complex (Mtw1p Including Nnflp-Nsl1p-Dsn1p) which joins kinetochore subunits contacting DNA to those contacting microtubules; critical to kinetochore assembly

46;Cytoplasmic Glc7p-interacting protein, potential Cdc28p substrate 47;One of two type V myosins; required for mother-specific HO expression, for the bud tip localization of ASH1 and IST2 mRNA; facilitates growth and orientation of ER tubules along with She3p

48;Integral membrane Ca(2+)-ATPase, potential aminophospholipid translocase required to form a specific class of secretory vesicles that accumulate upon actin cytoskeleton disruption; mutation affects maturation of the 18S rRNA

49;Essential nuclear protein, constituent of 66S pre-ribosomal particles; required for normal concentration of free 60S ribosomal subunits; required for maintenance of M1 satellite double-stranded RNA of the L-A virus

50;Putative GDP/GTP exchange factor required for mitotic exit at low temperatures; acts as a guanine nucleotide exchange factor (GEF) for Temlp, which is a key regulator of mitotic exit; physically associates with Ras2p-GTP

51;Protein O-mannosyltransferase, transfers mannose residues from dolichyl phosphate-D-mannose to protein serine/threonine residues; acts in a complex with Pmt1p, can instead interact with Pmt5p in some conditions; target for new antifungals

52;Component of the CCR4-NOT transcriptional complex, which is involved in regulation of gene expression; component of the major cytoplasmic deadenylase, which is involved in mRNA poly(A) tail shortening

53;One of two (see also PSK2) PAS domain containing S/T protein kinases; coordinately regulates protein synthesis and carbohydrate metabolism and storage in response to a unknown metabolite that reflects nutritional status

54;Regulatory subunit A of the heterotrimeric protein phosphatase 2A, which also contains regulatory subunit Cdc55p and either catalytic subunit Pph21p or Pph22p; required for cell morphogenesis and for transcription by RNA polymerase III

55;DNA N-glycosylase and apurinic/apyrimidinic (AP) lyase involved in base excision repair, localizes to the nucleus and mitochondrion

56;Endosomal SNARE related to mammalian syntaxin 8

57;Cystathionine gamma-lyase, catalyzes one of the two reactions involved in the transsulfuration pathway that yields cysteine from homocysteine with the intermediary formation of cystathionine;

58;Subunit of the mitochondrial sorting and assembly machinery (SAM complex); has a role in assembly of the TOM complex, which mediates protein import through the outer membrane; required for normal mitochondrial morphology and inheritance

59;Integral nuclear/ER membrane protein of unknown function, required for normal nuclear envelope morphology and sporulation

60;Mitochondrial protein of unknown function

61;Protein that forms a heterotrimeric complex with Erp1p, Emp24p, and Erv25p; member, along with Emp24p and Erv25p, of the p24 family involved in ER to Golgi transport and localized to COPII-coated vesicles

62;ATPase involved in protein folding and nuclear localization signal (NLS)-directed nuclear transport; member of heat shock protein 70 (HSP70) family; forms a chaperone complex with Ydj1p; localized to the nucleus, cytoplasm, and cell wall

63;Subunit of the nuclear pore complex (NPC), functions to anchor Nup2p to the NPC in a dynamic process that is controlled by the nucleoplasmic concentration of Gsplp-GTP; potential Cdc28p substrate

64;Protein that forms a heterotrimeric complex with Erp2p, Emp24p, and Erv25p; member, along with Emp24p and Erv25p, of the p24 family involved in ER to Golgi transport and localized to COPII-coated vesicles

65;Subunit of heterotrimeric Replication Factor A (RF-A), which is a highly conserved single-stranded DNA binding protein involved in DNA replication, repair, and recombination

66;N-succinyl-5-aminoimidazole-4-carboxamide ribotide (SAICAR) synthetase, required for 'de novo' purine nucleotide biosynthesis; red pigment accumulates in mutant cells deprived of adenine

67;Nonessential protein kinase with unknown cellular role

68;Protein kinase of the Mitotic Exit Network that is localized to the spindle pole bodies at late anaphase; promotes mitotic exit by directly switching on the kinase activity of Dbf2p

69;Hypothetical protein

70;Protein with roles in exocytosis and cation homeostasis; functions in docking and fusion of post-Golgi vesicles with plasma membrane; homolog of Sro7p and Drosophila lethal giant larvae tumor suppressor; interacts with SNARE protein Sec9p

71;Protein serine/threonine kinase essential for cell wall remodeling during growth; localized to sites of polarized growth and the mother-daughter bud neck; homolog of the alpha, beta, and gamma isoforms of mammalian protein kinase C (PKC)

72;Non-essential tetra-spanning membrane protein found mostly in the late Golgi, can suppress some sed5 alleles; may be part of the transport machinery, but precise function is unknown; similar to mammalian syntaxin 5

73;Non-essential protein of unknown function; promoter contains several Gcn4p binding elements

74;RNApolymerase II holoenzyme component

75;Methionine aminopeptidase, catalyzes the cotranslational removal of N-terminal methionine from nascent polypeptides; function is partially redundant with that of Map1p

76;Mitochondrial ribosomal protein of the large subunit; MRP21 exhibits genetic interactions with mutations in the COX2 and COX3 mRNA 5'-untranslated leader sequences

77;Protein kinase, primarily involved in telomere length regulation; contributes to cell cycle checkpoint control in response to DNA damage; functionally redundant with Meclp; homolog of human ataxia telangiectasia (ATM) gene

78;Subunit of the Anaphase-Promoting Complex/Cyclosome (APC/C), which is a ubiquitin-protein ligase required for degradation of anaphase inhibitors, including mitotic cyclins, during the metaphase/anaphase transition

79;Cytoplasmic isoleucine-tRNA synthetase, target of the G1-specific inhibitor reveromycin A

80;Protein component of the small (40S) ribosomal subunit; identical to Rps8Ap and has similarity to rat S8 ribosomal protein

81;Protein required, along with Dph1p, Dph2p, Jjj3p, and Dph5p, for synthesis of diphthamide, which is a modified histidine residue of translation elongation factor 2 (Eft1p or Eft2p); may act in a complex with Dph1p and Dph2p

82;Peripheral membrane protein that interacts with the plasma membrane ATPase Pmalp and has a role in its targeting to the plasma membrane, possibly by influencing its incorporation into lipid rafts

83;Mitochondrial peroxiredoxin (1-Cys Prx) with thioredoxin peroxidase activity, has a role in reduction of hydroperoxides; induced during respiratory growth and under conditions of oxidative stress

84;Kinesin-related motor protein required for mitotic spindle assembly and chromosome segregation; functionally redundant with Cin8p

85;Type 2C protein phosphatase; dephosphorylates Hoglp (see also Ptc2p) to limit maximal kinase activity induced by osmotic stress; dephosphorylates T169 phosphorylated Cdc28p (see also Ptc2p); role in DNA checkpoint inactivation

86;Hypothetical protein

87;Histone acetyltransferase catalytic subunit of NuA3 complex that acetylates histone H3, involved in transcriptional silencing; homolog of the mammalian MOZ proto-oncogene; sas3 gcn5 double mutation confers lethality

88;Protein involved in G2/M phase progression and response to DNA damage, interacts with Rad53p; contains an RNA recognition motif, a nuclear localization signal, and several SQ/TQ cluster domains; hyperphosphorylated in response to DNA damage

89;Protein of unknown function, has homology to kinase Snf7p; not required for growth on nonfermentable carbon sources; essential for viability in stationary phase

90;High affinity uridine permease, localized to the plasma membrane; not involved in uracil transport

91;20S proteasome beta-type subunit

92;Identified by homology to Ashbya gossypii

93;Major CTP synthase isozyme (see also URA8), catalyzes the ATP-dependent transfer of the amide nitrogen from glutamine to UTP, forming CTP, the final step in de novo biosynthesis of pyrimidines; involved in phospholipid biosynthesis

94;Mitochondrial ribosomal protein of the large subunit

95;Alpha-adaptin, large subunit of the clathrin associated protein complex (AP-2); involved in vesicle mediated transport

96;Single-domain racemase, possibly non-specific due to the lack of the second domain, which presumably determines specificity

97;B subunit of DNA polymerase alpha-primase complex, required for initiation of DNA replication during mitotic and premeiotic DNA synthesis; also functions in telomere capping and length regulation

98;GTP cyclohydrolase II; catalyzes the first step of the riboflavin biosynthesis pathway

99;Cytoskeletal protein of unknown function; overexpression causes growth arrest

100;Major ADP/ATP carrier of the mitochondrial inner membrane, exchanges cytosolic ADP for mitochondrially synthesized ATP; required for viability in many common lab strains carrying a mutation in the polymorphic SAL1 gene

101;Protein of unknown function; green fluorescent protein (GFP)-fusion protein localizes to the cell periphery; has potential orthologs in Saccharomyces species and in Yarrowia lipolytica

102;Hypothetical proteins

103;Hypothetical protein

104;Protein involved in promoting high level transcription of rDNA, subunit of UAF (upstream activation factor) for RNA polymerase I

105;protein involved in DNA replication; component of the Mcm2-7 hexameric complex that binds chromatin as a part of the pre-replicative complex

106;Subunit of the heme-activated, glucose-repressed Hap2p/3p/4p/5p CCAAT-binding complex, a transcriptional activator and global regulator of respiratory gene expression; contains sequences contributing to both complex assembly and DNA binding

107;Class II abasic (AP) endonuclease involved in repair of DNA damage; homolog of human HAP1 and E. coli exoIII

108;Hypothetical protein

109;haspin

110;Cytoskeletal protein binding protein required for assembly of the cortical actin cytoskeleton; contains 3 SH3 domains; interacts with proteins regulating actin dynamics and with proteins required for endocytosis

111;Transcriptional activator of the pleiotropic drug resistance network, regulates expression of ATP-binding cassette (ABC) transporters through binding to cis-acting sites known as PDREs (PDR responsive elements)

112;Component of the small-subunit (SSU) processome, which is involved in the biogenesis of the 18S rRNA

113;One of two nearly identical (see also HTA1) histone H2A subtypes; core histone required for chromatin assembly and chromosome function; DNA damage-dependent phosphorylation by Mec1p facilitates DNA repair; acetylated by Nat4p

114;One of two nearly identical (see HTB1) histone H2B subtypes required for chromatin assembly and chromosome function; Rad6p-Brelp-Lgelp mediated ubiquitination regulates transcriptional activation, meiotic DSB formation and H3 methylation

115;Non-essential protein of unknown function, likely exists as tetramer, may be regulated by the binding of small-molecule ligands (possibly sulfate ions), may have a role in yeast cell-wall biogenesis

116;Putative neutral trehalase, required for thermotolerance and may mediate resistance to other cellular stresses

117;Cis-prenyltransferase involved in dolichol synthesis; participates in endoplasmic reticulum (ER) protein sorting

118;Functional ortholog of human PIG-V, which is a mannosyltransferase that transfers the second mannose in glycosylphosphatidylinositol biosynthesis; the authentic, non-tagged protein was localized to mitochondria

119;Succinate semialdehyde dehydrogenase involved in the utilization of gamma-aminobutyrate (GABA) as a nitrogen source; part of the 4-aminobutyrate and glutamate degradation pathways; localized to the cytoplasm

120;Deletion suppressor of mpt5 mutation

121;One of two identical histone H4 proteins (see also HHF2); core histone required for chromatin assembly and chromosome function; contributes to telomeric silencing; N-terminal domain involved in maintaining genomic integrity

122;One of two identical histone H3 proteins (see also HHT2); core histone required for chromatin assembly, involved in heterochromatin-mediated telomeric and HM silencing; regulated by acetylation, methylation, and mitotic phosphorylation

123;Cytoplasmic inorganic pyrophosphatase (PPase), catalyzes the rapid exchange of oxygens from Pi with water, highly expressed and essential for viability, active-site residues show identity to those from E. coli PPase

124;Transportin, cytosolic karyopherin beta 2 involved in delivery of heterogeneous nuclear ribonucleoproteins to the nucleoplasm, binds rg-nuclear localization signals on Nab2p and Hrp1p, plays a role in cell-cycle progression

125;Galactose-1-phosphate uridyl transferase, synthesizes glucose-1-phosphate and UDP-galactose from UDP-D-glucose and alpha-D-galactose-1-phosphate in the second step of galactose catabolism

126;UDP-glucose-4-epimerase, catalyzes the interconversion of UDP-galactose and UDP-D-glucose in galactose metabolism; also catalyzes the conversion of alpha-D-glucose or alpha-D-galactose to their beta-anomers

127;Uracil permease, localized to the plasma membrane; expression is tightly regulated by uracil levels and environmental cues

128;Phosphatase that is highly specific for ADP-ribose 1 "-phosphate, a tRNA splicing metabolite; may have a role in regulation of tRNA splicing

129;Chitin synthase III, catalyzes the transfer of N-acetylglucosamine (GlcNAc) to chitin; required for synthesis of the majority of cell wall chitin, the chitin ring during bud emergence, and spore wall chitosan

130;Hypothetical protein

131;2-enoyl thioester reductase, member of the medium chain dehydrogenase/reductase family; localized to in mitochondria, where it has a probable role in fatty acid synthesis

132;Hypothetical protein

133;Phosphatidate cytidylyltransferase (CDP-diglyceride synthetase); an enzyme that catalyzes that conversion of CTP + phosphate into diphosphate + CDP-diaclglyerol, a critical step in the synthesis of all major yeast phospholipids

134;Nuclear SAM-dependent mono- and asymmetric arginine dimethylating methyltransferase that modifies hnRNPs, including Np13p and Hrp1p, thus facilitating nuclear export of these proteins; required for viability of np13 mutants

135;Pyridoxine (pyridoxamine) phosphate oxidase, has homologs in E. coli and Myxococcus xanthus; transcription is under the general control of nitrogen metabolism

136;Endoplasmic reticulum membrane protein, required for mannosylation of inositolphosphorylceramide and for growth at high calcium concentrations

137;Copper-binding protein of the mitochondrial inner membrane, required for cytochrome c oxidase activity and respiration; may function to deliver copper to cytochrome c oxidase; has similarity to thioredoxins

138;Gamma subunit of the F1 sector of mitochondrial F1F0 ATP synthase, which is a large, evolutionarily conserved enzyme complex required for ATP synthesis

139;Integral membrane protein required for efficient mating; may participate in or regulate the low affinity Ca2+ influx system, which affects intracellular signaling and cell-cell fusion during mating

140;Zeta-crystallin homolog, found in the cytoplasm and nucleus; has similarity to E. coli quinone oxidoreductase and to human zeta-crystallin, which has quinone oxidoreductase activity

141;The authentic, non-tagged protein was localized to the mitochondria

142;Regulatory subunit of the Glc7p type-1 protein phosphatase; involved with Reglp, Glc7p, and Snf1p in regulation of glucose-repressible genes, also involved in glucose-induced proteolysis of maltose permease

143;Protein of unknown function; member of a flavodoxin-like fold protein family that includes Pst2p and Ycp4p; green fluorescent protein (GFP)-fusion protein localizes to the cytoplasm in a punctate pattern

144;Hypothetical proteinj 145;Putative plasma membrane protein of unknown function, transcriptionally regulated by Haalp; green fluorescent protein (GFP)-fusion protein localizes to the cell periphery and bud

146;Hypothetical protein

147;Protein that stimulates the activity of serine palmitoyltransferase (Lcblp, Lcb2p) several-fold; involved in sphingolipid biosynthesis

148;Subunit of the origin recognition complex, which directs DNA replication by binding to replication origins and is also involved in transcriptional silencing; may be phosphorylated by Cdc28p

149;2'-O-ribose methyltransferase, methylates the 2'-O-ribose of nucleotides at positions 32 and 34 of the tRNA anticodon loop

150;Pre-mRNA splicing factor, facilitates the cooperative formation of U2/U6 helix II in association with stem II in the spliceosome, function may be regulated by Slu7p

151;Transcriptional repressor that mediates glucose repression and negatively regulates filamentous growth; has similarity to Nrglp

152;Major cell wall mannoprotein with possible lipase activity; transcription is induced by heat- and cold-shock; member of the Srp1p/Tip1p family of serine-alanine-rich proteins

153;Amino acid transport protein for valine, leucine, isoleucine, and tyrosine, low-affinity tryptophan and histidine transporter; overexpression confers FK506 resistance

154;Hypothetical protein

155;Small heat shock protein with chaperone activity that is regulated by a heat induced transition from an inactive oligomeric (24-mer) complex to an active dimer; induced by heat, upon entry into stationary phase, and during sporulation

156;Subunit of the core complex of translation initiation factor 3(eIF3), essential for translation; part of a subcomplex (Prt1p-Rpg1p-Nip1p) that stimulates binding of mRNA and tRNA(i)Met to ribosomes

157;ATPase required for the release of Sec17p during the 'priming' step in homotypic vacuole fusion and for ER to Golgi transport; homolog of the mammalian NSF

158;Subunit of the SAGA transcriptional regulatory complex, involved in proper assembly of the complex; also present as a C-terminally truncated form in the SLIK/SALSA transcriptional regulatory complex

159;Transcription factor required for full Ty1 epxression, Ty1-mediated gene activation, and haploid invasive and diploid pseudohyphal growth; TEA/ATTS DNA-binding domain family member

160;Mitochondrial inner membrane ADP/ATP translocator, exchanges cytosolic ADP for mitochondrially synthesized ATP; expressed under anaerobic conditions; similar to Pet9p and Aaclp; has roles in maintenance of viability and in respiration

161;Hypothetical protein

162;Plasma membrane protein that may be involved in osmotolerance, localizes to the mother cell in small-budded cells and to the bud in medium- and large-budded cells; mRNA is transported to the bud tip by an actomyosin-driven process

163;Subunit of heteropentameric Replication factor C (RF-C), which is a DNA binding protein and ATPase that acts as a clamp loader of the proliferating cell nuclear antigen (PCNA) processivity factor for DNA polymerases delta and epsilon

164;Proliferating cell nuclear antigen (PCNA), functions as the sliding clamp for DNA polymerase delta; may function as a docking site for other proteins required for mitotic and meiotic chromosomal DNA replication and for DNA repair

165;High-mobility group non-histone chromatin protein, functionally redundant with Nhp6Ap; homologous to mammalian high mobility group proteins 1 and 2; acts to recruit transcription factor Rcs1p to certain promoters

166;Constitutively expressed acid phosphatase similar to Pho5p; brought to the cell surface by transport vesicles; hydrolyzes thiamin phosphates in the periplasmic space, increasing cellular thiamin uptake; expression is repressed by thiamin

167;Hypothetical protein

168;Hypothetical protein

169;Subunit of the structure-specific Mms4p-Mus81p endonuclease that cleaves branched DNA; involved in recombination and DNA repair

170;Essential protein with dual roles in spliceosome assembly and exocytosis; the exocyst complex (Sec3p, Sec5p, Sec6p, Sec8p, Sec10p, Sec15p, Exo70p, and Exo84p) mediates polarized targeting of secretory vesicles to active sites of exocytosis

171;Putative mitochondrial inner membrane transporter, member of the mitochondrial carrier (MCF) family

172;Peripheral membrane protein located at Vid (vacuole import and degradation) vesicles; regulates fructose-1,6-bisphosphatase (FBPase) targeting to the vacuole; involved in proteasome-dependent catabolite degradation of FBPase

173;Probable membrane protein, involved in phosphate transport; pho88 pho86 double null mutant exhibits enhanced synthesis of repressible acid phosphatase at high inorganic phosphate concentrations

174;Protein with a role in kinetochore function, localizes to the outer kinetochore in a Ctf19p-dependent manner, interacts with Chl4p and Ctf19p

175;Mannosyltransferase, involved in asparagine-linked glycosylation in the endoplasmic reticulum (ER); essential for viability, mutation is functionally complemented by human ortholog

176;Alpha aminoadipate reductase, catalyzes the reduction of alpha-aminoadipate to alpha-aminoadipate 6-semialdehyde, which is the fifth step in biosynthesis of lysine; activation requires posttranslational phosphopantetheinylation by Lys5p177;Transketolase, similar to Tkl1p; catalyzes conversion of xylulose-5-phosphate and ribose-5-phosphate to sedoheptulose-7-phosphate and glyceraldehyde-3-phosphate in the pentose phosphate pathway; needed for synthesis of aromatic amino acids

178;Protein required for translation of the mitochondrial COB mRNA

179;Cytoplasmic and mitochondrial glycyl-tRNA synthase that ligates glycine to the cognate anticodon bearing tRNA; transcription termination factor that may interact with the 3'-end of pre-mRNA to promote 3'-end formation

180;Subunit B of the eight-subunit V1 peripheral membrane domain of the vacuolar H+-ATPase (V-ATPase), an electrogenic proton pump found throughout the endomembrane system; contains nucleotide binding sites; also detected in the cytoplasm

181;Subunit of an autophagy-specific phosphatidylinositol 3-kinase complex (with Vps34p, Vps15p, and Vps30p) required for organization of a pre-autophagosomal structure; ATG14 transcription is activated by Gln3p during nitrogen starvation

182;High affinity polyamine permease, preferentially uses spermidine over putrescine; expression is down-regulated by osmotic stress; plasma membrane carnitine transporter, also functions as a low-affinity amino acid permease

183;Protein arginine N-methyltransferase that exhibits septin and Hsl1p-dependent bud neck localization and periodic Fisl1p-dependent phosphorylation; required along with Hsl1p for bud neck recruitment, phosphorylation, and degradation of Swelp

184;Subunit of the Cdc28 protein kinase, required for mitotic proteolysis, may also be involved in the proteolysis of the G1 cyclin

185;Protein of unknown function; localized to the cytoplasm; binds to Replication Protein A (RPA); YBR137W is not an essential gene

186;GTPase-activating protein that negatively regulates RAS by converting it from the GTP- to the GDP-bound inactive form, required for reducing cAMP levels under nutrient limiting conditions, mediates membrane association of adenylate cyclase

187;Putative protein of unknown function; green fluorescent protein (GFP)-fusion protein localizes to the nucleolus; YBR141C is not an essential gene

188;Dubious ORF unlikely to encode a protein, based on available experimental and comparative sequence data; overlaps the uncharacterized ORF YBR141C; identified by gene-trapping, microarray analysis, and genome-wide homology searching

189;Essential nucleolar protein, putative DEAD-box RNA helicase required for maintenance of M1 dsRNA virus; involved in biogenesis of large (60S) ribosomal subunits

190;Polypeptide release factor involved in translation termination; mutant form acts as a recessive omnipotent suppressor

191;Alcohol dehydrogenase isoenzyme V; involved in ethanol production

192;Mitochondrial ribosomal protein of the small subunit

193;Putative protein of unknown function; YBR147W is not an essential gene; resistant to fluconazole

194;Protein expressed specifically in spores

195;Large subunit of NADP+ dependent arabinose dehydrogenase, involved in carbohydrate metabolism; small subunit is unidentified

196;Protein of unknown function, required for normal localization of actin patches and for normal tolerance of sodium ions and hydrogen peroxide; localizes to both cytoplasm and nucleus

197;mRNA splicing factor, component of U4/U6.U5 tri-snRNP; interacts genetically and physically with Prp38p

198;Diaminohydroxyphoshoribosylaminopyrimidine deaminase; catalyzes the second step of the riboflavin biosynthesis pathway

199;RNA polymerase subunit ABC27, common to RNA polymerases I, II, and III; contacts DNA and affects transactivation

200;Catalytic subunit of the main cell cycle cyclin-dependent kinase (CDK); alternately associates with G1 cyclins (CLNs) and G2/M cyclins (CLBs) which direct the CDK to specific substrates

201;Covalently-bound cell wall protein of unknown function; identified as a cell cycle regulated SBF target gene; deletion mutants are highly resistant to treatment with beta-1,3-glucanase; has sequence similarity to YJL171C

202;Protein whose expression suppresses a secretory pathway mutation in E. coli; has similarity to the mammalian RAMP4 protein involved in secretion

203;Soluble GTPase with a role in regulation of membrane traffic; regulates potassium influx; G protein of the Ras superfamily, similar to ADP-ribosylation factor

204;Ubiquitin-conjugating enzyme suppressor that functions as a general positive regulator of Cdc34p activity; nuclear protein that may represent a link between nucleocytoplasmic transport and ubiquitin ligase activity

205;Subunit of both RNase MRP, which cleaves pre-rRNA, and nuclear RNase P, which cleaves tRNA precursors to generate mature 5' ends

206;Peroxisomal integral membrane protein, involved in negative regulation of peroxisome size; partially functionally redundant with Pex31p; genetic interactions suggest action at a step downstream of steps mediated by Pex28p and Pex29p

207;Endoplasmic reticulum and nuclear membrane protein, forms a complex with Cdc48p and Ufd1p that recognizes ubiquitinated proteins in the endoplasmic reticulum and delivers them to the proteasome for degradation

208;Short-lived chaperone required for correct maturation of the 20S proteasome; degraded by proteasome upon completion of its assembly; involved in ubiquitin-mediated proteolysis; mutant defective in degradation of short-lived proteins

209;Ketopantoate hydroxymethyltransferase, required for pantothenic acid biosynthesis, converts 2-oxoisovalerate into 2-dehydropantoate

210;Multidrug resistance dityrosine transporter of the major facilitator superfamily, essential for spore wall synthesis, facilitates the translocation of bisformyl dityrosine through the prospore membrane

211;Putative protein of unknown function; green fluorescent protein (GFP)-fusion protein localizes to the vacuole; YBR187W is an essential gene

212;Mitochondrial pyrimidine nucleotide transporter; imports pyrimidine nucleoside triphosphates and exports pyrimidine nucleoside monophosphates; member of the mitochondrial carrier family

213;Glycolytic enzyme phosphoglucose isomerase, catalyzes the interconversion of glucose-6-phosphate and fructose-6-phosphate; required for cell cycle progression and completion of the gluconeogenic events of sporulation

214;Subunit (90 kDa) of TFIID and SAGA complexes, involved in RNA polymerase II transcription initiation and in chromatin modification

215;Putative mannosyltransferase involved in protein glycosylation; member of the KRE2/MNT1 mannosyltransferase family

216;Protein containing SH3-domains, involved in establishing cell polarity and morphogenesis; functions as a scaffold protein for complexes that include Cdc24p, Ste5p, Ste20p, and Rsr1p

217;Endoplasmic reticulum membrane protein, required for ER-associated protein degradation, involved in the retrograde transport of misfolded or unassembled proteins; N- and C- termini protrude into the cytoplasm, has similarity to Dfm1p

218;Component of the hexameric MCM complex, which is important for priming origins of DNA replication in G1 and becomes an active ATP-dependent helicase that promotes DNA melting and elongation when activated by Cdc7p-Dbf4p in S-phase

219;Protein required for wild-type resistance to the antifungal drug ciclopirox olamine; not related to the COS family of subtelomerically-encoded proteins

220;Putative alpha-1,2-mannosyltransferase involved in O- and N-linked protein glycosylation; member of the KRE2/MNT1 mannosyltransferase family

221;Putative high affinity iron transporter involved in transport of intravacuolar stores of iron; forms complex with Fet5p; expression is regulated by iron; proposed to play indirect role in endocytosis

222;Bifunctional dehydrogenase and ferrochelatase, involved in the biosynthesis of siroheme; also involved in the expression of PAPS reductase and sulfite reductase

223;One of two S. cerevisiae homologs (Sds23p and Sds24p) of the Schizosaccharomyces pombe Sds23 protein, which genetic studies have implicated in APC/cyclosome regulation; may play an indirect role in fluid-phase endocytosis

224;Ubiquitin-like modifier, conjugated via an isopeptide bond to a lysine residue of Atg5p by the E1 enzyme, Atg7p, and the E2 enzyme, Atg10p, a step that is essential for autophagy

225;Putative protein of unknown function; YBR220C is not an essential gene

226;E1 beta subunit of the pyruvate dehydrogenase (PDH) complex, which is an evolutionarily-conserved multi-protein complex found in mitochondria

227;Peroxisomal AMP-binding protein, localizes to both the peroxisomal peripheral membrane and matrix, expression is highly inducible by oleic acid, similar to E. coli long chain acyl-CoA synthetase

228;Hypothetical protein

229;RNA binding protein with similarity to mammalian heterogeneous nuclear RNP K protein, involved in the regulation of telomere position effect and telomere length

230;Essential protein, component of the DASH complex; involved in spindle integrity and kinetochore function; interacts with Duolp and Dam1p; localizes to intranuclear spindles and kinetochore

231;Essential subunit of the ARP2/3 complex, which is required for the motility and integrity of cortical actin patches

232;Putative ion transporter, similar to mammalian electroneutral Na(+)-(K+)-C1-cotransporter family; YBR235W is not an essential gene

233;RNA helicase in the DEAD-box family, necessary for prespliceosome formation, bridges U1 and U2 snRNPs and enables stable U2 snRNP association with intron RNA

234;Putative protein of unknown function; green fluorescent protein (GFP)-fusion protein localizes to the cytoplasm and nucleus; YBR239C is not an essential gene

235;Zinc finger protein of the Zn(II)2Cys6 type, probable transcriptional activator of thiamine biosynthetic genes

236;Putative protein of unknown function; green fluorescent protein (GFP)-fusion protein localizes to the vacuolar membrane; YBR241C is not an essential gene

237;Phospholipid hydroperoxide glutathione peroxidase induced by glucose starvation that protects cells from phospholipid hydroperoxides and nonphospholipid peroxides during oxidative stress

238;Hypothetical protein

239;Protein associated with U3 and U14 snoRNAs, required for pre-rRNA processing and 40S ribosomal subunit synthesis; localized in the nucleus and concentrated in the nucleolus

240;3-deoxy-D-arabino-heptulosonate-7-phosphate (DAHP) synthase, catalyzes the first step in aromatic amino acid biosynthesis and is feedback-inhibited by tyrosine

241;Mitochondrial ribosomal protein of the small subunit

242;One of 10 subunits of the transport protein particle (TRAPP) complex of the cis-Golgi which mediates vesicle docking and fusion; mutations in the human homolog cause the spondyloepiphyseal dysplasia tarda (SEDL) disorder

243;Protein of unknown function, required for normal growth rate at 15 degrees C; green fluorescent protein (GFP)-fusion protein localizes to the cytoplasm in a punctate pattern

244;Riboflavin synthase; catalyzes the last step of the riboflavin biosynthesis pathway

245;Subunit of the COMPASS (Set1C) complex, which methylates histone H3 on lysine 4 and is required in transcriptional silencing near telomeres

246;Protein of unknown function involved in bud growth in the mitotic signaling network; proposed negative regulator of Swelp and Gin4p; contains dispersed C2H2 zinc finger domains

247;Mitochondrial ribosomal protein of the large subunit

248;Putative S-adenosylmethionine-dependent methyltransferase of the seven beta-strand family; green fluorescent protein (GFP)-fusion protein localizes to the cytoplasm; YBR271W is not as essential gene

249;Protein that binds to the Rap1p C-terminus and acts synergistically with Rif2p to help control telomere length and establish telomeric silencing; deletion results in telomere elongation

250;Protein phosphatase with specificity for serine, threonine, and tyrosine residues; has a role in the DNA synthesis phase of the cell cycle

251;Putative protein of unknown function; green fluorescent protein (GFP)-fusion protein localizes to the cytoplasm and nucleus; YBR281C is not an essential gene

252;Mitochondrial ribosomal protein of the large subunit

253;Subunit of the Ssh1 translocon complex; Sec61p homolog involved in cotranslational pathway of protein translocation; not essential

254;Putative protein of unknown function; YBR284W is not an essential gene; null mutant exhibits decreased resistance to rapamycin and wortmannin

255;Putative protein of unknown function; YBR287W is not an essential gene and deletion of the YBR287W leads to poor growth on glucose-minimal medium at 15C

256;Protein of unknown function; mutation results in a zinc sensitive phenotype

257;Mu3-like subunit of the clathrin associated protein complex (AP-3); functions in transport of alkaline phosphatase to the vacuole via the alternate pathway

258;Heavy metal ion homeostasis protein, facilitates trafficking of Smf1p and Smf2p metal transporters to the vacuole where they are degraded, controls metal ion transport, prevents metal hyperaccumulation, functions in copper detoxification

259;Na+/Pi cotransporter, active in early growth phase; similar to phosphate transporters of Neurospora crassa; transcription regulated by inorganic phosphate concentrations and Pho4p

260;Catabolic L-serine (L-threonine) deaminase, catalyzes the degradation of both L-serine and L-threonine; required to use serine or threonine as the sole nitrogen source, transcriptionally induced by serine and threonine

261;S-phase checkpoint protein found at replication forks, required for DNA replication; also required for Rad53p activation during DNA replication stress, where it forms a replication-pausing complex with Toflp and is phosphorylated by Meclp; protein involved in replication checkpoint

262;Essential nucleolar protein required for the synthesis of 18S rRNA and for the assembly of 40S ribosomal subunit

263;Hypothetical protein, has similarity to proteins in S. pombe, C. elegans, D. melanogaster.

264;Zinc metalloendopeptidase, found in the cytoplasm and intermembrane space of mitochondria; with Cymlp, involved in degradation of mitochondrial proteins and of presequence peptides cleaved from imported proteins

265;Protein of unknown function; green fluorescent protein (GFP)-fusion protein localizes to the cytoplasm in a punctate pattern

266;Transcription factor required for induction of KAR3 and CIK1 during mating, also required during meiosis; exists in two forms, a slower-migrating form more abundant during vegetative growth and a faster-migrating form induced by pheromone

267;Essential component of glycosylphosphatidylinositol-mannosyltransferase I, required for the autocatalytic post-translational processing of the protease B precursor Prblp, localizes to ER in lumenal orientation; homolog of mammalian PIG-X

268;Protein involved in control of cell wall structure and stress response; inhibits Cbk1p protein kinase activity; overproduction confers resistance to cell-wall degrading enzymes

269;Diadenosine 5',5"-P1,P4-tetraphosphate phosphorylase I (AP4A phosphorylase), involved in catabolism of bis(5'-nucleosidyl) tetraphosphates; has similarity to Apa2p

270;Hypothetical protein

271;Hypothetical protein

272;Subunit, with Ymelp, of the mitochondrial inner membrane i-AAA protease complex, which is responsible for degradation of unfolded or misfolded mitochondrial gene products; required for growth of cells lacking the mitochondrial genome

273;Glucokinase, catalyzes the phosphorylation of glucose at C6 in the first irreversible step of glucose metabolism; one of three glucose phosphorylating enzymes; expression regulated by non-fermentable carbon sources

274;Protein of unknown function, involved in proteasome-dependent catabolite inactivation of fructose-1,6-bisphosphatase; contains six WD40 repeats; computational analysis suggests that Gid7p and Moh1p have similar functions

275;Protein required for the breakdown of autophagic vesicles in the vacuole during autophagy, putative integral membrane protein that localizes to vacuolar membranes and punctate structures attached to the vacuole

276;Protein of unknown function; binds Las17p, which is a homolog of human Wiskott-Aldrich Syndrome protein involved in actin patch assembly and actin polymerization

277;Protein involved in mating response, invasive/filamentous growth, and osmotolerance, acts as an adaptor that links G protein-associated Cdc42p-Ste20p complex to the effector Ste11p to modulate signal transduction

278;Essential protein involved in rRNAprocessing and ribosome biogenesis

279;Multifunctional enzyme containing phosphoribosyl-ATP pyrophosphatase, phosphoribosyl-AMP cyclohydrolase, and histidinol dehydrogenase activities; catalyzes the second, third, ninth and tenth steps in histidine biosynthesis

280;Microtubule-associated protein, component of the interface between microtubules and kinetochore, involved in sister chromatid separation; essential in polyploid cells but not in haploid or diploid cells; ortholog of mammalian CLIP-170

281;[PIN(+)] prion, an infectious protein conformation that is generally an ordered protein aggregate

282;Protein of unknown function, involved in the integration of lipid signaling pathways with cellular homeostasis

283;Beta-isopropylmalate dehydrogenase, catalyzes the third step in the leucine biosynthesis pathway

284;Cysteine desulfurase involved in iron-sulfur cluster (Fe/S) biogenesis; required for the post-transcriptional thio-modification of mitochondrial and cytoplasmic tRNAs; essential protein located predominantly in mitochondria

285;Subunit of a complex with Ctf8p and Ctf18p that shares some components with Replication Factor C, required for sister chromatid cohesion

286;Protein involved in bud-site selection and required for axial budding pattern; localizes with septins to bud neck in mitosis and may constitute an axial landmark for next round of budding

287;Poly(A+) RNA-binding protein, involved in the export of mRNAs from the nucleus to the cytoplasm; similar to Hrblp and Np13p; also binds single-stranded telomeric repeat sequence in vitro

288;SaGa associated Factor 29kDa; Probable 29kKDa Subunit of SAGA histone acetyltransferase complex

289;Regulatory subunit of acetolactate synthase, which catalyzes the first step of branched-chain amino acid biosynthesis; enhances activity of the Ilv2p catalytic subunit, localizes to mitochondria

290;Protein of unknown function; null mutant shows a reduced affinity for the alcian blue dye suggesting a decreased net negative charge of the cell surface

291;Phosphatidylglycerolphosphate synthase, catalyzes the synthesis of phosphatidylglycerolphosphate from CDP-diacylglycerol and sn-glycerol 3-phosphate in the first committed and rate-limiting step of cardiolipin biosynthesis

292;Protein involved in retention of membrane proteins, including Sec12p, in the ER; localized to Golgi; functions as a retrieval receptor in returning membrane proteins to the ER

293;Component of the septin ring of the mother-bud neck that is required for cytokinesis; septins recruit proteins to the neck and can act as a barrier to diffusion at the membrane, and they comprise the 10nm filaments seen with EM

294;Mitochondrial ribosomal protein of the large subunit

295;Protein of unknown function, has sequence and structural similarity to flavodoxins; green fluorescent protein (GFP)-fusion protein localizes to the cytoplasm in a punctate pattern

296;Citrate synthase, catalyzes the condensation of acetyl coenzyme A and oxaloacetate to form citrate, peroxisomal isozyme involved in glyoxylate cycle; expression is controlled by Rtg1p and Rtg2p transcription factors

297;Ser/Thr protein kinase involved in salt tolerance; funtions in regulation of Trklp-Trk2p potassium transporter; partially redundant with Hal5p; has similarity to Npr1p

298;Amphiphysin-like lipid raft protein; subunit of a complex (Rvs161p-Rvs167p) that regulates polarization of the actin cytoskeleton, endocytosis, cell polarity, cell fusion and viability following starvation or osmotic stress

299;Acetate transporter required for normal sporulation

300;Putative ATP-dependent permease of the ABC transporter family of proteins

301;Protein required for respiratory growth and stability of the mitochondrial genome

302;Non-catalytic subunit of N-terminal acetyltransferase of the NatC type; required for replication of dsRNA virus; member of the Sm protein family

303;Hydrophobic plasma membrane localized, stress-responsive protein that negatively regulates the H(+)-ATPase Pmalp; induced by heat shock, ethanol treatment, weak organic acid, glucose limitation, and entry into stationary phase

304;Mitochondrial asparaginyl-tRNA synthetase

305;Small single-membrane span proteolipid that functions as a regulatory subunit of the plasma membrane H(+)-ATPase Pmalp, forms unique helix and positively charged cytoplasmic domain that is able to specifically segregate phosphatidylserines

306;Nucleotide pyrophosphatase/phosphodiesterase family member; mediates extracellular nucleotide phosphate hydrolysis along with Npp2p and Pho5p; activity and expression enhanced during conditions of phosphate starvation

307;Putative Rheb-related GTPase involved in regulating canavanine resistance and arginine uptake; member of the Ras superfamily of G-proteins

308;Protein with a potential role in actin cytoskeletal organization; overexpression suppresses a pfy1 (profilin) null mutation

309;Subunit of the Set3C deacetylase complex; putative DNA-binding protein

310;Fatty acid elongase, involved in sphingolipid biosynthesis; acts on fatty acids of up to 24 carbons in length; mutations have regulatory effects on 1,3-beta-glucan synthase, vacuolar ATPase, and the secretory pathway

311 hypothetical protein

312;Hypothetical protein

313;Phosphorelay intermediate protein, phosphorylated by the plasma membrane sensor Sln1p in response to osmotic stress and then in turn phosphorylates the response regulators Ssk1p in the cytosol and Skn7p in the nucleus

314;Subunit of the oligosaccharyltransferase complex of the ER lumen, which catalyzes protein asparagine-linked glycosylation; type I membrane protein required for incorporation of Ost3p or Ost6p into the OST complex

315;Cytoplasmic ATPase that is a ribosome-associated molecular chaperone; may be involved in the folding of newly-synthesized polypeptide chains; member of the heat shock protein 70 (HSP70) family; interacts with the phosphatase subunit Reglp

316;Site-specific endonuclease required for gene conversion at the MAT locus (homothallic switching) through the generation of a ds DNA break; expression restricted to mother cells in late G1 as controlled by Swi4p-Swi6p, Swi5p and Ash1p

317;ADP-ribosylation factor GTPase activating protein (ARF GAP), involved in ER-Golgi transport; shares functional similarity with Glo3p

318;One of five related septins (Cdc3p, Cdc10p, Cdc11p, Cdc12p, Shs1p) that form a cortical filamentous collar at the mother-bud neck which is necessary for normal morphogenesis and cytokinesis

319;Putative RNA binding protein and partially redundant Whi3p homolog that regulates the cell size requirement for passage through Start and commitment to cell division

320;Hypothetical protein

321;Component of the mitochondrial Tim54p-Tim22p complex involved in insertion of polytopic proteins into the inner membrane

322;Protein kinase with a possible role in MAP kinase signaling in the pheromone response pathway

323;Protein with similarity to hydrophilins, which are involved in the adaptive response to hyperosmotic conditions; computational analysis of large-scale protein-protein interaction data suggests a possible role in rRNA processing

324;Endoplasmic reticulum packaging chaperone, required for incorporation of amino acid permeases into COPII coated vesicles for transport to the cell surface

325;Hypothetical protein

326;Cytoplasmic nucleoporin required for polyadenylated RNA export but not for protein import; component of Nup82p nuclear pore subcomplex; contains a nuclear export signal

327;Phorphobilinogen deaminase, catalyzes the conversion of 4-porphobilinogen to hydroxymethylbilane, the third step in the heme biosynthetic pathway; localizes to both the cytoplasm and nucleus; expression is regulated by Hap2p-Hap3p

328;Protein of unknown function; has similarity to mammalian reticulon proteins; member of the RTNLA (reticulon-like A) subfamily

329;Mitochondrial ribosomal protein of the large subunit

330;Subunit of a tRNA methyltransferase complex composed of Trm8p and Trm82p that catalyzes 7-methylguanosine modification of tRNA

331;Prenyltransferase, required for cell viability

332;ADP-ribosylation factor, GTPase of the Ras superfamily involved in regulation of coated formation vesicles in intracellular trafficking within the Golgi; functionally interchangeable with Arf2p

333;Catalytic subunit of protein phosphatase 2A, functionally redundant with Pph21p; methylated at C terminus; forms alternate complexes with several regulatory subunits; involved in signal transduction and regulation of mitosis

334;Ribosomal protein L47 of the large (60S) ribosomal subunit, identical to Rp141Bp and has similarity to rat L41 ribosomal protein; comprised of only 25 amino acids; rp141a rp141b double null mutant is viable

335;Protein that inhibits ATP hydrolysis by the F1F0-ATP synthase, inhibitory function is enhanced by stabilizing proteins Stf1p and Stf2p; has similarity to Stf1p and both Inh1p and Stf1p exhibit the potential to form coiled-coil structures

336;Hypothetical protein

337;Hypothetical protein

338;Hypothetical protein

339;RING finger protein that interacts with the arginine methyltransferase Hmt1p; may regulate methylation of Np13p, which modulates Np13p function in mRNA processing and export; has similarity to Air1p

340;D-lactate dehydrogenase, oxidizes D-lactate to pyruvate, transcription is heme-dependent, repressed by glucose, and derepressed in ethanol or lactate; located in the mitochondrial inner membrane

341;Transcriptional activator necessary for gamma-aminobutyrate (GABA)-dependent induction of GABA genes (such as UGA1, UGA2, UGA4); zinc-finger transcription factor of the Zn(2)-Cys(6) binuclear cluster domain type; localized to the nucleus

342;Essential nuclear protein, involved in the oxidative stress response

343;Component of the CCR4-NOT complex, which has multiple roles in regulating mRNA levels including regulation of transcription and destabilizing mRNAs by deadenylation; basal transcription factor

344;DNA ligase found in the nucleus and mitochondria, an essential enzyme that joins Okazaki fragments during DNA replication; also acts in nucleotide excision repair, base excision repair, and recombination

345;Epsin-like protein involved in endocytosis and actin patch assembly and functionally redundant with Ent2p; binds clathrin via a clathrin-binding domain motif at C-terminus

346;Cytoplasmic DExD/H-box helicase, stimulates mRNA decapping, coordinates distinct steps in mRNA function and decay, interacts with both the decapping and deadenylase complexes, may have a role in mRNA export and translation

347;B-type cyclin involved in cell cycle progression; activates Cdc28p to promote the G2/M transition; may be involved in DNA replication and spindle assembly; accumulates during S phase and G2, then targeted for ubiquitin-mediated degradation

348;Component of the small (ribosomal) subunit (SSU) processosome required for pre-18S rRNa processing; essential nucleolar protein that, when overproduced, disrupts silencing

349;RNA polymerase III subunit C53

350;Essential, non-ATPase regulatory subunit of the 26S proteasome lid, similar to mammalian p55 subunit and to another S. cerevisiae regulatory subunit, Rpn7p

351;protein of unknown function; GFP-fusion protein localizes to the cell periphery, cytoplasm, bud, and bud neck; null mutant shows a reduced affinity for the alcian blue dye suggesting a decreased net negative charge of the cell surface

352;Biotin:apoprotein ligase, covalently modifies proteins with the addition of biotin, required for acetyl-CoA carboxylase (Acclp) holoenzyme formation

353;RNA polymerase II largest subunit B220, part of central core; phosphorylation of C-terminal heptapeptide repeat domain regulates association with transcription and splicing factors; similar to bacterial beta-prime

354;ADP-ribosylation factor, GTPase of the Ras superfamily involved in regulation of coated formation vesicles in intracellular trafficking within the Golgi; functionally interchangeable with Arf1p

355;Rho GDP dissociation inhibitor involved in the localization and regulation of Cdc42p

356;Ribosomal protein L47 of the large (60S) ribosomal subunit, identical to Rp141Ap and has similarity to rat L41 ribosomal protein; comprised of only 25 amino acids; rp141a rp141b double null mutant is viable

357;Hypothetical protein

358;Cullin, structural protein of SCF complexes (which also contain Skp1p, Cdc34p, and an F-box protein) involved in ubiquitination; SCF promotes the G1-S transition by targeting G1 cyclins and the Cln-CDK inhibitor Sic1p for degradation

359;Protein involved in regulation of the mitochondrial F1F0-ATP synthase; Stf1p and Stf2p act as stabilizing factors that enhance inhibitory action of the Inh1p protein

360;ATPase in ER, nuclear membrane and cytosol with homology to mammalian p97; in a complex with Np14p and Ufd1p participates in retrotranslocation of ubiquitinated proteins from the ER into the cytosol for degradation by the proteasome

361;Protein of unknown function, localized to the vacuolar outer membrane

362;Ubiquitin-specific protease that removes ubiquitin from ubiquitinated proteins; cleaves at the C terminus of ubiquitin fusions irrespective of their size; capable of cleaving polyubiquitin chains

363;Hypothetical protein

364;Frataxin, regulates mitochondrial iron accumulation; interacts with Isu1p which promotes Fe-S cluster assembly; interacts with electron transport chain components and may influence respiration; human homolog involved in Friedrich's ataxia

365;Hypothetical protein

366;Protein required for transport of aminopeptidase I (Lap4p) through the cytoplasm-to-vacuole targeting pathway; binds phosphatidylinositol-3-phosphate, involved in localization of membranes to the preautophagosome, potential Cdc28p substrate

367;2'-O-ribose methyltransferase, catalyzes the ribose methylation of the guanosine nucleotide at position 18 of tRNAsj 368;Protein involved in rRNA processing; component of the exosome 3->5 exonuclea se complex with Rrp4p, Rrp41p, Rrp43p and Dis3p

369;Homeobox transcription factor; regulatory targets include genes involved in phosphate metabolism; binds cooperatively with Pho4p to the PHO5 promoter; phosphorylation of Pho2p facilitates interaction with Pho4p

370;Nuclear protein that plays a role in the function of the Smc5p-Rhc18p complex

371;Catalytic subunit of DNA polymerase delta; required for chromosomal DNA replication during mitosis and meiosis, intragenic recombination, repair of double strand DNA breaks, and DNA replication during nucleotide excision repair (NER)

372;Cell-cycle checkpoint serine-threonine kinase required for DNA damage-induced transcription of certain target genes, phosphorylation of Rad55p and Sml1p, and transient G2/M arrest after DNA damage; also regulates postreplicative DNA repair

373;ATPase, subunit of the GET complex; required for the retrieval of HDEL proteins from the Golgi to the ER in an ERD2 dependent fashion; involved in resistance to heat and metal stress

374;Protein of unknown function; green fluorescent protein (GFP)-fusion protein localizes to the cytoplasm in a punctate pattern

375;Protein O-mannosyltransferase, transfers mannose residues from dolichyl phosphate-D-mannose to protein serine/threonine residues; acts in a complex with Pmt2p, can instead interact with Pmt3p in some conditions; target for new antifungals

376;Signal recognition particle (SRP) subunit, interacts with the RNA component or SRP to form the Alu domain, which is the region of SRP responsible for arrest of nascent chain elongation during membrane targeting; homolog of mammalian SRP14

377;UBX (ubiquitin regulatory X) domain-containing protein that interacts with Cdc48p, green fluorescent protein (GFP)-fusion protein localizes to the cytoplasm in a punctate pattern

378;Protein of unknown function; interacts with meiotic division protein Csm1p; green fluorescent protein (GFP)-fusion protein localizes to the nuclear periphery, potential Cdc28p substrate

379;Nuclear pore complex subunit, part of a subcomplex also containing Nup53p, Nup170p, and Pselp

380;Hypothetical protein

381;Mitochondrial external NADH dehydrogenase, catalyzes the oxidation of cytosolic NADH; Nde1p and Nde2p are involved in providing the cytosolic NADH to the mitochondrial respiratory chain

382;Component of the TREX complex required for nuclear mRNA export; DEAD-box RNA helicase involved in early and late steps of spliceosome assembly; homolog of the human splicing factor hUAP56

383;Ribosomal protein P1 alpha, a component of the ribosomal stalk, which is involved in the interaction between translational elongation factors and the ribosome; accumulation of P1 in the cytoplasm is regulated by phosphorylation and interaction with the P2 stalk component

384;Cytoplasmic malate dehydrogenase, catalyzes interconversion of malate and oxaloacetate; involved in the glyoxylate cycle

385;Vacuolar protein that plays a critical role in the tethering steps of vacuolar membrane fusion by facilitating guanine nucleotide exchange on small guanosine triphosphatase Ypt7p

386;Protein of unknown function, may be involved in chromatin silencing

387;E3 ubiquitin ligase for Rad6p, required for the ubiquitination of histone H2B, recruitment of Rad6p to promoter chromatin and subsequent methylation of histone H3 (on L4 and L79), contains RING finger domain

388;Endoplasmic reticulum transmembrane protein, homolog of human BAP31 protein

389;Protein involved in transcription initiation at TATA-containing promoters; associates with the basal transcription factor TFIID; contains two bromodomains; corresponds to the C-terminal region of mammalian.TAF1; redundant with Bdf1p

390;Subunit VIIa of cytochrome c oxidase, which is the terminal member of the mitochondrial inner membrane electron transport chain

391;Mitochondrial NADP-specific isocitrate dehydrogenase, catalyzes the oxidation of isocitrate to alpha-ketoglutarate; not required for mitochondrial respiration and may function to divert alpha-ketoglutarate to biosynthetic processes

392;Protein component of the small (40S) ribosomal subunit; nearly identical to Rps29Ap and has similarity to rat S29 and E. coli S 14 ribosomal proteins

393;Protein required for processing of 20S pre-rRNA in the cytoplasm, associates with pre-40S ribosomal particles

394;Hypothetical protein

395;GDP-mannose pyrophosphorylase (mannose-1-phosphate guanyltransferase), synthesizes GDP-mannose from GTP and mannose-1-phosphate in cell wall biosynthesis; required for normal cell wall structure

396;Protein with similarity to mammalian monocarboxylate permeases, which are involved in transport of monocarboxylic acids across the plasma membrane; mutant is not deficient in monocarboxylate transport

397;1-acyl-sn-gylcerol-3-phosphate acyltransferase, catalyzes the acylation of lysophosphatidic acid to form phosphatidic acid, a key intermediate in lipid metabolism; located in lipid particles and endoplasmic reticulum

398;Protein containing a Kruppel-type zinc-finger domain; has similarity to Stp1p, Stp2p, and Stp3p

399;Type 2A-related serine-threonine phosphatase that functions in the G1/S transition of the mitotic cycle; cytoplasmic and nuclear protein that modulates functions mediated by Pkc1p including cell wall and actin cytoskeleton organization

400;Functional homolog of human NPC2/He1, which is a cholesterol-binding protein whose deficiency causes Niemann-Pick type C2 disease involving retention of cholesterol in lysosomes

401;Mitochondrial ribosomal protein of the small subunit

402;Flavin adenine dinucleotide (FAD) synthetase, performs the second step in synthesis of FAD from riboflavin

403;Mitochondrial matrix protein that interacts with an N-terminal region of mitochondrial RNA polymerase (Rpo41p) and couples RNA processing and translation to transcription

404;Subunit of the N-terminal acetyltransferase NatA (Nat1p, Ard1p, Nat5p); N-terminally acetylates many proteins, which influences multiple processes such as the cell cycle, heat-shock resistance, mating, sporulation, and telomeric silencing

405;Dual-specificity kinase required for spindle pole body (SPB) duplication and spindle checkpoint function; substrates include SPB proteins Spc42p, Spc110p, and Spc98p, mitotic exit network protein Mob1p, and checkpoint protein Mad1p

406;Hypothetical protein

407;Protein of unknown function, potentially phosphorylated by Cdc28p

408;Homolog of Gpm1p phosphoglycerate mutase which converts 3-phosphoglycerate to 2-phosphoglycerate in glycolysis; may be non-functional derivative of a gene duplication event

409;Transcription factor that stimulates expression of proteasome genes; Rpn4p levels are in turn regulated by the 26S proteasome in a negative feedback control mechanism; RPN4 is transcriptionally regulated by various stress responses

410;Member of an oxysterol-binding protein family with seven members in S. cerevisiae; family members have overlapping, redundant functions in sterol metabolism and collectively perform a function essential for viability

411;Protein with similarity to Emp24p and Erv25p, member of the p24 family involved in ER to Golgi transport

412;DDK (Dbf4-dependent kinase) catalytic subunit required for firing origins and replication fork progression in mitosis through phosphorylation of Mcm2-7p complexes and Cdc45p; kinase activity correlates with cyclical DBF4 expression

413;Enoyl reductase that catalyzes the last step in each cycle of very long chain fatty acid elongation, localizes to the ER, highly enriched in a structure marking nuclear-vacuolar junctions, coimmunoprecipitates with elongases Fen1p and Sur4p

414;Nucleolar protein, component of the small subunit processome complex, which is required for processing of pre-18S rRNA; has similarity to mammalian fibrillarin

415;Ring finger protein involved in the DNA damage response with possible recombination role; genetically identified by synthetic lethality with SGS1 (DNA helicase) and TOP3 (DNA topoisomerase); sporulation role; interacts with Slx8p and Lin1p

416;Hypothetical protein

417;One of six ATPases of the 19S regulatory particle of the 26S proteasome involved in the degradation of ubiquitinated substrates; required for normal peptide hydrolysis by the core 20S particle

418;Type 2C protein phosphatase (PP2C); inactivates the osmosensing MAPK cascade by dephosphorylating Hoglp; mutation delays mitochondrial inheritance; deletion reveals defects in precursor tRNA splicing, sporulation and cell separation

419;Subunit of the RNA polymerase II mediator complex; associates with core polymerase subunits to form the RNA polymerase II holoenzyme; essential for transcriptional regulation

420;Delta subunit of the central stalk of mitochondrial F1F0 ATP synthase, which is a large, evolutionarily conserved enzyme complex required for ATP synthesis

421;Essential protein required for sister chromatid cohesion in mitosis and meiosis; subunit of the cohesin complex; expression is cell cycle regulated and peaks in S phase

422;Protein related to mammalian high mobility group proteins; likely component of the INO80 complex, which is an ATP-dependent chromatin-remodeling complex

423;Neutral trehalase, degrades trehalose; required for thermotolerance and may mediate resistance to other cellular stresses; may be phosphorylated by Cdc28p

424;Protein whose overexpression suppresses the growth defect of mutants lacking protein kinase A activity; involved in cAMP-mediated signaling; localized to the nucleus; similar to the mouse testis-specific protein PBS 13

425;ABC transporter protein involved in multidrug resistance and resistance to singlet oxygen species

426;Subunit of the GINS complex (S1d5p, Psf1p, Psf2p, Psf3p), which is localized to DNA replication origins and implicated in assembly of the DNA replication machinery

427;Inositol hexaphosphate kinase, phosphorylates inositol hexakisphosphate (InsP6) to diphosphoinositol polyphosphates, required for proper vacuole morphology and involved in salt stress response, contains two leucine heptad repeats

428;Hypothetical protein

429;Nucleolar protein required for maturation of 18S rRNA, member of the eIF4A subfamily of DEAD-box ATP-dependent RNA helicases

430;Protein of unknown function that may be involved in microtubule organization; high-copy suppressor of CIK1 deletion

431;Hypothetical protein

432;Component of the GARP (Golgi-associated retrograde protein) complex, Vps51p-Vps52p-Vps53p-Vps54p, which is required for the recycling of proteins from endosomes to the late Golgi; potentially phosphorylated by Cdc28p

433;Protein involved in transcription-coupled repair nucleotide excision repair of UV-induced DNA lesions; homolog of human CSA protein

434;Protein of unknown function with similarity to members of a family of flavodoxin-like proteins; induced by oxidative stress in a Yap1p dependent manner; GFP-fusion protein localizes to the cytoplasm in a punctate pattern

435;Protein that localizes primarily to the plasma membrane, also found at the nuclear envelope; has similarity to Hsp30p and Yro2p, which are induced during heat shock

436;Transcriptional activator involved in regulation of genes of the lysine biosynthesis pathway; requires 2-aminoadipate semialdehyde as co-inducer

437;Protein of unknown function; green fluorescent protein (GFP)-fusion protein localizes to the cell periphery

438;3-deoxy-D-arabino-heptulosonate-7-phosphate (DAHP) synthase, catalyzes the first step in aromatic amino acid biosynthesis and is feedback-inhibited by phenylalanine

439;Protein of unconfirmed function, plays an indirect role in endocytic membrane trafficking, member of a family of enoyl-CoA hydratase/isomerases

440;Mitochondrial ribosomal protein of the small subunit, has similarity to E. coli S10 ribosomal protein; essential for viability, unlike most other mitoribosomal proteins

441;Transcriptional repressor that recruits the Cyc8p-Tuplp complex to promoters; mediates glucose repression and negatively regulates a variety of processes including filamentous growth and alkaline pH response

442;Coproporphyrinogen III oxidase, an oxygen requiring enzyme that catalyzes the sixth step in the heme biosynthetic pathway; localizes to the mitochondrial inner membrane; transcription is repressed by oxygen and heme (via Roxlp and Hap1p)

443;RNA polymerase III subunit C11; mediates pol III RNA cleavage activity and is important for termination of transcription

444;Amino acid permease involved in the uptake of cysteine, leucine, isoleucine and valine

445;Uroporphyrinogen decarboxylase, catalyzes the fifth step in the heme biosynthetic pathway; localizes to both the cytoplasm and nucleus; activity inhibited by Cu2+, Zn2+, Fe2+, Fe3+ and sulfhydryl-specific reagents

446;Hypothetical protein

447;Ubiquitin-conjugating enzyme or E2; together with Skp1p, Rbxlp, Cdc53p, and an F-box protein, forms a ubiquitin-protein ligase called the SCF complex which regulates cell cycle progression by targeting key substrates for degradation

448;Cell wall protein that contains a putative GPI-attachment site; secreted by regenerating protoplasts; up-regulated by activation of the cell integrity pathway, as mediated by R1m1p; upregulated by cell wall damage via disruption of FKS1

449;Lectin; soluble lumenal ER protein; member of the OS-9 protein family; similar to mannose-6-phosphate receptors (MPRs); serves as a receptor that recognizes misfolded N-glycosylated proteins and participates in their targeting to ERAD

450;Protein with lipolytic activity towards triacylglycerols and diacylglycerols when expressed in E. coli; role in yeast lipid degradation is unclear

451;Component of serine palmitoyltransferase, responsible along with Lcblp for the first committed step in sphingolipid synthesis, which is the condensation of serine with palmitoyl-CoA to form 3-ketosphinganine

452;Hypothetical protein

453;Hypothetical protein

454;Cytoplasmic protein required for replication of Brome mosaic virus in S. cerevisiae, which is a model system for studying replication of positive-strand RNA viruses in their natural hosts

455;Protein of unknown function, green fluorescent protein (GFP)-fusion protein localizes to the cytoplasm

456;Ubiquitin hydrolase, required for recycling ubiquitin from proteasome-bound ubiquitinated intermediates, acts at the late endosome/prevacuolar compartment to recover ubiquitin from ubiquitinated membrane proteins en route to the vacuole457;The authentic, non-tagged protein was localized to the mitochondria

458;Polyamine acetyltransferase; acetylates polyamines such as putrescine, spermidine and spermine; may be involved in transcription and/or DNA replication via regulation of levels of polyamines bound to chromosomal DNA

459;One of 11 subunits of the SWI/SNF chromatin remodeling complex involved in transcriptional regulation; interacts with a highly conserved 40-residue sequence of Snf2p

460;Phosphatase subunit of the trehalose-6-phosphate synthase/phosphatase complex, which synthesizes the storage carbohydrate trehalose; expression is induced by stress conditions and repressed by the Ras-cAMP pathway

461;Protein that stimulates strand exchange by stabilizing the binding of Rad51p to single-stranded DNA; involved in the recombinational repair of double-strand breaks in DNA during vegetative growth and meiosis; forms heterodimer with Rad57p

462;Protein of unassigned function involved in mutation suppression, important for error-free repair of spontaneous and induced DNA lesions to protect the genome from mutation; associates with Shulp, Psy3p, and Csm2p

463;Chaperone that specifically facilitates the assembly of cytochrome c oxidase, located in the mitochondrial inner membrane

464;Component of the RNA polymerase II general transcription and DNA repairfactor TFIIH; involved in transcription initiation; homolog of the Chlamydomonas reinhardtii REX1-S protein which is involved in DNA repair

465;Transcription factor required for the synthesis of the glycolytic enzyme pyruvate decarboxylase, required for high level expression of both the THI and the PDC genes

466;Integral membrane protein localized to late Golgi vesicles along with the v-SNARE Tlg2p; green fluorescent protein (GFP)-fusion protein localizes to the cytoplasm in a punctate pattern

467;Subunit of the Sec61p translocation complex (Sec61p-Sss1p-Sbh1p) that forms a channel for passage of secretory proteins through the endoplasmic reticulum membrane, and of the Sshlp complex (Ssh1p-Sbh2p-Sss1p); interacts with Ost4p and Wbp1p

468;RNA splicing factor, required for ATP-independent portion of 2nd catalytic step of spliceosomal RNA splicing; interacts with Prp18p; contains zinc knuckle domain

469;Hypothetical protein

470;Hypothetical protein

471;Essential iron-sulfur protein required for ribosome biogenesis and translation initiation; facilitates binding of a multifactor complex (MFC) of translation initiation factors to the small ribosomal subunit; predicted ABC family ATPase

472;Transcriptional factor, involved in the expression of genes during nutrient limitation; also involved in the negative regulation of DPP1 and PHR1

473;Protein required for mismatch repair in mitosis and meiosis, forms a complex with Msh2p to repair both single-base & insertion-deletion mispairs; potentially phosphorylated by Cdc28p

474;14-3-3 protein, minor isoform; binds proteins and DNA, involved in regulation of many processes including exocytosis and vesicle transport, Ras/MAPK signaling during pseudohyphal development, rapamycin-sensitive signaling, and others

475;Integral membrane protein localized to late Golgi vesicles along with the v-SNARE T1g2p

476;Scaffold protein that, in response to pheromone, shuttles from the nucleus to the plasma membrane and assembles kinases Ste11p, Ste7p, and Fus3p into a specific signaling complex; active oligomeric form interacts with Ste4p-Ste18p complex

477;Vacuolar membrane protein of unknown function that is conserved in mammals; predicted to contain eleven transmembrane helices; interacts with Pdr5p, a protein involved in multidrug resistance

478;multispanning membrane protein

479;Putative alanine transaminase (glutamic pyruvic transaminase)

480;Putative mitochondrial ribosomal protein of the large subunit, has similarity to E. coli L34 ribosomal protein; required for respiratory growth, as are most mitochondrial ribosomal proteins

481;Mitochondrial ribosomal protein of the large subunit

482;Hypothetical protein

483;Palmitoyltransferase that acts on the SNAREs Snc1p, Syn8p, Tlglp and likely on all SNAREs; member of a family of putative palmitoyltransferases containing an Asp-His-His-Cys-cysteine rich (DHHC-CRD) domain; may have a role in vacuole fusion

484;Vacuolar glutathione S-conjugate transporter of the ATP-binding cassette family, has a role in detoxifying metals such as cadmium, mercury, and arsenite; also transports unconjugated bilirubin; similar to human cystic fibrosis protein CFTR

485;Subunit of a Golgi membrane exchange factor (Ric1p-Rgp1p) that catalyzes nucleotide exchange on Ypt6p

486;Peroxisomal signal receptor for the N-terminal nonapeptide signal (PTS2) of peroxisomal matrix proteins; WD repeat protein; defects in human homolog cause lethal rhizomelic chondrodysplasia punctata (RCDP)

487;GPI-anchored aspartyl protease (yapsin) involved in protein processing; shares functions with Yap3p and Kex2p

488;Subunit (61/68 kDa) of TFIID and SAGA complexes, involved in RNA polymerase II transcription initiation and in chromatin modification, similar to histone H2A

489;Dihydrolipoyl transsuccinylase, a component of the mitochondrial alpha-ketoglutarate dehydrogenase complex, which catalyzes a step in the tricarboxylic acid (TCA) cycle, the oxidative decarboxylation of alpha-ketoglutarate to succinyl-CoA

490;Member of the CCCH zinc finger family; has similarity to mammalian Tis11 protein, which activates transcription and also has a role in mRNA degradation; may function with Tis11p in iron homeostasis

491;Highly-acidic cytoplasmic RWD domain-containing protein of unknown function, sensitive to proteolysis, N-terminal region has high content of acidic amino acid residues, putative IUP (intrinsically unstructured protein)

492;Protein containing an N-terminal epsin-like domain involved in clathrin recruitment and traffic between the Golgi and endosomes; associates with the clathrin adaptor Gga2p, clathrin adaptor complex AP-1, and clathrin

493;Cytoplasmic peptidyl-prolyl cis-trans isomerase (cyclophilin), catalyzes the cis-trans isomerization of peptide bonds N-terminal to proline residues; binds the drug cyclosporin A

494;Aspartic beta semi-aldehyde dehydrogenase, catalyzes the second step in the common pathway for methionine and threonine biosynthesis; expression regulated by Gcn4p and the general control of amino acid synthesis

495;Nuclear pore-associated protein, forms a complex with Thplp that is involved in transcription and in mRNA export from the nucleus

496;Component of the SPS plasma membrane amino acid sensor system (Ssylp-Ptr3p-Ssy5p), which senses external amino acid concentration and transmits intracellular signals that result in regulation of expression of amino acid permease genes

497;interacts with PP2C

498;Protein involved in the HOG (high osmolarity glycerol) pathway, negatively regulates Hoglp by recruitment of phosphatase Ptclp the Pbs2p-Hoglp complex, found in the nucleus and cytoplasm, contains an SH3 domain that binds Pbs2p

499;Non-essential protein involved in pre-mRNA splicing, component of a complex containing Ceflp; has similarity to S. pombe Cwf15p

500;Sm-like protein involved in docking and fusion of exocytic vesicles through binding to assembled SNARE complexes at the membrane; localization to sites of secretion (bud neck and bud tip) is dependent on SNARE function

501;Subunit of a tRNA methyltransferase complex composed of Trm8p and Trm82p that catalyzes 7-methylguanosine modification of tRNA

502;Subunit (145 kDa) of TFIID and SAGA complexes, involved in RNA polymerase II transcription initiation and in chromatin modification

503;Essential Hsp90p co-chaperone; necessary for passage through the START phase of the cell cycle

504;Protein that binds Sin3p in a two-hybrid assay

505;Guanine nucleotide exchange factor (GEF) for ADP ribosylation factors involved in proliferation of the Golgi, intra-Golgi transport and ER-to-Golgi transport; found in the cytoplasm and on Golgi-associated coated vesicles

506;Small cytosolic stress-induced chaperone that forms barrel-shaped oligomers and suppresses the aggregation of non-native proteins; oligomer dissociation is not required for function; involved in cytoskeleton reorganization after heat shock

507;Translation termination factor eRF3; altered protein conformation creates the [PSI(+)] prion, a dominant cytoplasmically inherited protein aggregate that alters translational fidelity and creates a nonsense suppressor phenotype

508;Protein involved in regulation of arginine-responsive and Mcmlp-dependent genes; has a dual-specificity inositol polyphosphate kinase activity required for regulation of phosphate- and nitrogen-responsive genes

509;Chromatin associated high mobility group (HMG) family member involved in genome maintenance; rDNA-binding component of the Pol I transcription system; associates with a 5'-3' DNA helicase and Fpr1p, a prolyl isomerase

510;Ubiquitin-conjugating enzyme that mediates selective degradation of short-lived and abnormal proteins; plays a role in vesicle biogenesis and ER-associated protein degradation (ERAD); component of the cellular stress response

511;Putative protein kinase and subunit of cohesin loading factor (Scc2p-Scc4p), a complex required for the loading of cohesin complexes onto chromosomes; involved in establishing sister chromatid cohesion during DSB repair via histone H2AX

512;Putative membrane protein of unknown function involved in Mn2+ homeostasis; mutants display actin and general growth defects, heterogeneous cell cycle arrests, and pleiotropic defects in cell cycle progression and organelle distribution

513;Hypothetical protein

514;Hypothetical protein

515;Hydrophilic protein involved in vesicle trafficking between the ER and Golgi; SM (Sec1/Munc-18) family protein that binds the tSNARE Sed5p and stimulates its assembly into a trans-SNARE membrane-protein complex

516;Essential protein involved in transcription regulation; component of chromatin remodeling complexes; required for assembly and function of the INO80 complex;member of the RUVB-like protein family

517;Subunit of the nuclear pore complex (NPC) that localizes exclusively to the cytoplasmic side; involved in RNA export, most likely at a terminal step; interacts with Glelp

518;DEAD-box protein required for efficient splicing of mitochondrial Group I and II introns; presumed RNA helicase due to DEAD-box motif

519;Mitochondrial translational activator of the COB mRNA; interacts with translating ribosomes, acts on the COB mRNA 5'-untranslated leader

520;Component of Dam1p complex, important for spindle and kinetochore integrity; localized to nuclear side of spindle pole body and along mitotic spindle

521;Protein of unknown function, contains a putative RNA recognition motif, deletion results in short telomeres; similar to Est1p, may be partially redundant with Est1p for telomere maintenance

522;key transcriptional regulator of early meiotic genes, binds URS1 upstream regulatory sequence, couples metabolic responses to nutritional cues with initiation and progression of meiosis, forms complex with Ime1p, and also with Sin3p-Rpd3p

523;Phosphatidylinositol-4-phosphate 5-kinase, involved in actin cytoskeleton organization and cell morphogenesis; multicopy suppressor of stt4 mutation

524;Protein of unknown function; green fluorescent protein (GFP)-fusion protein localizes to the cell periphery

525;Catalytic epsilon subunit of the translation initiation factor eIF2B, the guanine-nucleotide exchange factor for eIF2; activity subsequently regulated by phosphorylated eIF2; first identified as a negative regulator of GCN4 expression

526;Alpha subunit of chaperonin-containing T-complex, which mediates protein folding in the cytosol; involved in maintenance of actin cytoskeleton; homolog to Drosophila melanogaster and mouse tailless complex polypeptide

527;Sterol regulatory element binding protein, induces transcription of sterol transport and biosynthetic genes; involved in the anaerobic induction of DAN/TIR mannoproteins and seripauperins; binucleate zinc cluster protein; Ecm22p homolog528;Co-chaperone that binds to Hsp82p and activates its ATPase activity; similar to Hch1p; expression is regulated by stresses such as heat shock

529;Glucosidase II beta subunit, forms a complex with alpha subunit Rot2p, involved in removal of two glucose residues from N-linked glycans during glycoprotein biogenesis in the ER

530;Protein of unknown function; green fluorescent protein (GFP)-fusion protein localizes to the cytoplasm in a punctate pattern

531;One of two nearly identical (see HTB2) histone H2B subtypes required for chromatin assembly and chromosome function; Rad6p-Brelp-Lgelp mediated ubiquitination regulates transcriptional activation, meiotic DSB formation and H3 methylation

532;One of two nearly identical (see also HTA2) histone H2A subtypes; core histone required for chromatin assembly and chromosome function; DNA damage-dependent phosphorylation by Mec1p facilitates DNA repair; acetylated by Nat4p

533;Adenylate kinase, required for purine metabolism; localized to the cytoplasm and the mitochondria; lacks cleavable signal sequence

534;mRNA 3' end processing factor, essential component of cleavage and polyadenylation factor IA (CF IA), involved in pre-mRNA 3' end processing and in transcription termination; binds C-terminal domain of largest subunit of RNA pol II (Rpo21p)

535;Phospholipid-binding protein that interacts with both Ypt7p and Vps33p, may partially counteract the action of Vps33p and vice versa, localizes to the rim of the vacuole as cells approach stationary phase

536;Mitochondrial inner membrane protein, required for proteolytic processing of Cox2p and its assembly into cytochrome c oxidase

537;Protein of unknown function; has similarity to mammalian reticulon proteins; member of the RTNLA (reticulon-like A) subfamily

538;Homoaconitase, catalyzes the conversion of homocitrate to homoisocitrate, which is a step in the lysine biosynthesis pathway

539;Riboflavin kinase, phosphorylates riboflavin to form riboflavin monophosphate (FMN), which is a necessary cofactor for many enzymes; localizes to microsomes and to the mitochondrial inner membrane

540;Mitochondrial ribosomal protein of the large subunit

541;Putative amidase

542;RNA helicase in the DEAD-box family, involved in RNA isomerization at the 5' splice site

543;Peroxisomal membrane signal receptor for C-terminal tripeptide signal sequence (PTS1) of peroxisomal matrix proteins, required for peroxisomal matrix protein import, tetratricopeptide repeat protein, also involved in PTS1-independent import

544;One of 10 subunits of the transport protein particle (TRAPP) complex of the cis-Golgi which mediates vesicle docking and fusion; involved in endoplasmic reticulum (ER) to Golgi membrane traffic; human homolog is TRAPPC4

545;Hypothetical protein

546;Essential protein of unknown function; exhibits variable expression during colony morphogenesis; overexpression permits survival without protein phosphatase 2A, inhibits growth, and induces a filamentous phenotype

547;Zinc-finger DNA-binding protein, involved in regulating expression of the methionine biosynthetic genes, similar to Met31p

548;Cytosolic protein required for sporulation; also required for the ubiquitination of the gluconeogenetic enzyme fructose-1,6-bisphosphatase, which is degraded rapidly after the switch from gluconeogenesis to glycolysis

549;Oligomeric mitochondrial matrix chaperone that cooperates with Ssclp in mitochondrial thermotolerance after heat shock; prevents the aggregation of misfolded matrix proteins; component of the mitochondrial proteolysis system

550;Subunit of the anaphase-promoting complex, which is an E3 ubiquitin ligase that regulates the metaphase-anaphase transition and exit from mitosis; required for activation of the daughter-specific gene expression and spore wall maturation

551;Exo-1,3-beta-glucanase, involved in cell wall beta-glucan assembly; may be anchored to the plasma membrane via a glycosylphosphatidylinositol (GPI) anchor

552;Palmitoyl transferase involved in protein palmitoylation; acts as a negative regulator of pheromone response pathway; required for endocytosis of pheromone receptors; involved in cell shape control; contains ankyrin repeats

553;RING finger peroxisomal membrane peroxin required for peroxisomal matrix protein import, interacts with Pex12p, links ubiquitin-conjugating Pex4p to protein import machinery; mutations in human homolog cause a variety of peroxisomal disorders

554;Hypothetical protein

555;Mitochondrial tryptophanyl-tRNA synthetase

556;Protein of unknown function, ORF exhibits genomic organization compatible with a translational readthrough-dependent mode of expression

557;Small plasma membrane protein related to a family of plant polypeptides that are overexpressed under high salt concentration or low temperature, not essential for viability, deletion causes hyperpolarization of the plasma membrane potential

558;Negative regulator of the glucose-sensing signal transduction pathway, required for repression of transcription by Rgt1p; interacts with Rgt1p and the Snf3p and Rgt2p glucose sensors; phosphorylated by Yck1p, triggering Mth1p degradation

559;Ribonuclease H2 subunit, required for RNase H2 activity

560;Transverse filament protein of the synaptonemal complex; required for normal levels of meiotic recombination and pairing between homologous chromosome during meiosis; potential Cdc28p substrate

561;inositol monophosphatase

562;Essential protein of unknown function; interacts with Nse4p, which is a component of the Smc5/6 DNA repair complex

563;Protein that interacts with exonuclease Ratlp and Railp and plays a role in transcription termination by RNA polymerase II, has an RPR domain (carboxy-terminal domain interacting domain); also involved in regulation of Ty1 transposition564;Signal recognition particle (SRP) receptor - alpha subunit; contain GTPase domains; involved in SRP-dependent protein targeting; interacts with SRP102p

565;Subunit of a possibly tetrameric trichostatin A-sensitive class II histone deacetylase complex that contains an Hda1p homodimer and an Hda2p-Hda3p heterodimer; required for the activity of the complex; has similarity to Hda3p; Ploidy-related

566;Protein involved in homologous recombination in mitochondria and in transcription regulation in nucleus; binds to activation domains of acidic activators; required for recombination-dependent mtDNA partitioning

567;Subunit 5 of the stator stalk of mitochondrial F1F0 ATP synthase, which is a large, evolutionarily conserved enzyme complex required for ATP synthesis; homologous to bovine subunit OSCP (oligomycin sensitivity-conferring protein)

568;Essential protein possibly involved in secretion; multicopy suppressor of sensitivity to Brefeldin A

569;RNA-binding subunit of the mRNA cleavage and polyadenylation factor; involved in poly(A) site recognition and required for both pre-mRNA cleavage and polyadenylation, 51% sequence similarity with mammalian AAUAA-binding subunit of CPSF

570;ER membrane protein involved in a late step of glycosylphosphatidylinositol (GPI) anchor assembly; involved in the addition of phosphoethanolamine to the multiply mannosylated GPI intermediate; human PIG-Fp is a functional homolog

571;One of 15 subunits of the 'Remodel the Structure of Chromatin' (RSC) complex; essential gene required for regulation of ribosomal protein genes and the cell wall/stress response; highly similar to Rsc30p

572;Peptidyl-prolyl cis-trans isomerase (cyclophilin) of the endoplasmic reticulum, catalyzes the cis-trans isomerization of peptide bonds N-terminal to proline residues; transcriptionally induced in response to unfolded proteins in the ER

573;Hypothetical protein

574;RING-type ubiquitin ligase of the endosomal and vacuolar membranes, binds phosphatidylinositol(3)-phosphate; contains a FYVE finger domain

575;Hypothetical protein

576;Protein integral to the mitochondrial membrane; has a conserved methyltransferase motif; multicopy suppressor of respiratory defects caused by OXA1 mutations

577;Hypothetical protein

578;Auxilin-like protein involved in vesicular transport; clathrin-binding protein required for uncoating of clathrin-coated vesicles

579;Essential protein, component of the DASH complex; involved in spindle integrity and kinetochore function; interacts with Duolp and Dam1p; localizes to intranuclear spindles and kinetochore

580;Subunit e of mitochondrial F1F0-ATPase, which is a large, evolutionarily conserved enzyme complex required for ATP synthesis; essential for the dimeric state of ATP synthase

581;Evolutionarily conserved kinetochore protein that is part of multiple protein complexes, including the SCF ubiquitin ligase complex, the CBF3 complex that binds centromeric DNA, and the RAVE complex that regulates assembly of the V-ATPase

582;ER membrane glycoprotein subunit of the glycosylphosphatidylinositol transamidase complex that adds glycosylphosphatidylinositol (GPI) anchors to newly synthesized proteins; human PIG-K protein is a functional homolog

583;Karyopherin involved in nuclear import and export; shown to be responsible for nuclear import of replication protein A and for export of several proteins including Swi6p, Far1p, and Pho4p; cargo dissociation involves binding to RanGTP

584;Hypothetical protein

585;Mitochondrial ribosomal protein of the small subunit

586;Hypothetical protein

587;Protein required for cell viability

588;Cytoplasmic arginyl-tRNA synthetase

589;Low affinity glucose transporter of the major facilitator superfamily, expression is induced in low or high glucose conditions

590;Protein with a potential role in cell survival pathways, required for the diauxic growth shift; expression in mammalian cells increases survival under conditions inducing apoptosis

591;Mitochondrial ribosomal protein of the small subunit; MRP1 exhibits genetic interactions with PET122, encoding a COX3-specific translational activator, and with PET123, encoding a small subunit mitochondrial ribosomal protein

592;Protein of unknown function; green fluorescent protein (GFP)-fusion protein localizes to the cell periphery and bud neck; potential Cdc28p substrate

593;Putative GPI-anchored aspartic protease, located in the cytoplasm and endoplasmic reticulum

594;Hypothetical protein

595;Cytoplasmic thioredoxin reductase, key regulatory enzyme that determines the redox state of the thioredoxin system, which acts as a disulfide reductase system and protects cells against both oxidative and reductive stress

596;Protein of unknown function; green fluorescent protein (GFP)-fusion protein localizes to the cytoplasm in a punctate pattern

597;Protein involved in mating-type locus silencing, interacts with Sir2p; probably functions to recruit or stabilize Sir proteins

598;Pre-mRNA splicing factor, important for catalytic step II of pre-mRNA splicing and plays a role in cell cycle progression; required for DNA synthesis during mitosis and meiosis; has WD repeats

599;Nucleolar protein involved in pre-rRNA processing; depletion causes severely decreased 18S rRNA levels

600;2-methylbutyraldehyde reductase, may be involved in isoleucine catabolism

601;Protein required for DNA repair; component of the Mrell complex, which is involved in double strand breaks, meiotic recombination, telomere maintenance, and checkpoint signaling

602;Non-essential protein of unknown function involved in vacuolar protein sorting; belongs to a family of cytosolic Golgi-associated proteins suggesting that it may play a role in secretion; also detected in the nucleus

603;N-myristoylated calcium-binding protein that may have a role in intracellular signaling through its regulation of the phosphatidylinositol 4-kinase Pik1p; member of the recoverin/frequenin branch of the EF-hand superfamily

604;Hypothetical protein

605;Oxidoreductase of the mitochondrial inner membrane, involved in cytoplasmic and mitochondrial iron homeostasis and required for activity of Fe-S cluster-containing enzymes; one of the few mitochondrial proteins essential for viability

606;Subunit f of the F0 sector of mitochondrial F1F0 ATP synthase, which is a large, evolutionarily conserved enzyme complex required for ATP synthesis

607;GTPase-activating protein for the polarity-establishment protein Cdc42p; implicated in control of septin organization, pheromone response, and haploid invasive growth

608;Hypothetical protein identified by homology. See FEBS Letters [2000] 487:31-36.

609;Phenylpyruvate decarboxylase, catalyzes decarboxylation of phenylpyruvate to phenylacetaldehyde, which is the first specific step in the Ehrlich pathway

610;Ribosomal protein P2 beta, a component of the ribosomal stalk, which is involved in the interaction between translational elongation factors and the ribosome; regulates the accumulation of P1 (Rpp1Ap and Rpp1Bp) in the cytoplasm

611;Plasma membrane protein, regulation pattern suggests a possible role in export of ammonia from the cell; member of the TC 9.B.33 YaaH family of putative transporters

612;Elongation factor 2 (EF-2), also encoded by EFT1; catalyzes ribosomal translocation during protein synthesis; contains diphthamide, the unique posttranslationally modified histidine residue specifically ADP-ribosylated by diphtheria toxin

613;Helix-hairpin-helix protein, involved in DNA repair and replication fork stability; functions as an endonuclease in complex with Mms4p; interacts with Rad54p

614;Subunit of the SAGA and SAGA-like transcriptional regulatory complexes, interacts with Spt15p to activate transcription of some RNA polymerase II-dependent genes, also functions to inhibit transcription at some promoters

615;One of six ATPases of the 19S regulatory particle of the 26S proteasome involved in the degradation of ubiquitinated substrates; substrate of N-acetyltransferase B

616;Nuclear transport factor (karyopherin) involved in protein transport between the cytoplasm and nucleoplasm; similar to Nmd5p, Cse1p, Lph2p, and the human cellular apoptosis susceptibility protein, CAS1

617;Nucleolar protein, component of the small subunit (SSU) processome containing the U3 snoRNA that is involved in processing of pre-18S rRNA

618;Sporulation-specific enzyme required for spore wall maturation, involved in the production of a soluble LL-dityrosine-containing precursor of the spore wall; transcripts accumulate at the time of prospore enclosure

619;RNA polymerase II subunit B16; forms two subunit dissociable complex with Rpb4p

620;Phosphoribosyl-glycinamide transformylase, catalyzes a step in the 'de novo' purine nucleotide biosynthetic pathway

621;Protein required for cell viability

622;Predicted membrane protein required for the retention of lumenal endoplasmic reticulum proteins; mutants secrete the endogenous ER protein, BiP (Kar2p)

623;Hypothetical protein

624;Protein component of the large (60S) ribosomal subunit, nearly identical to Rp112Ap; rp112a rp112b double mutant exhibits slow growth and slow translation; has similarity to E. coli L11 and rat L12 ribosomal proteins

625;Sorting nexin, involved in the retrieval of late-Golgi SNAREs from the post-Golgi endosome to the trans-Golgi network; forms a complex with Snx4p and Atg20p626;Hypothetical protein

627;Subunit of the core complex of translation initiation factor 3(eIF3), which is essential for translation

628;Carboxyl methyl transferase, methylates the C terminus of the protein phosphatase 2A catalytic subunit (Pph21p or Pph22p), which is important for complex formation with regulatory subunits

629;Subunit of GPI-GlcNAc transferase involved in synthesis of N-acetylglucosaminyl phosphatidylinositol (GlcNAc-PI), which is the first intermediate in glycosylphosphatidylinositol (GPI) anchor synthesis, shares similarity with mammalian PIG-P

630;Protein involved in rDNA silencing; positively charged coiled-coil protein with limited similarity to myosin

631;Apparent pseudogene, not transcribed or translated under normal conditions; encodes a protein with similarity to adenine phosphoribosyltransferase, but artificially expressed protein exhibits no enzymatic activity

632;Non-essential protein apparently involved in meiosis, GFP fusion protein is present in discrete clusters in the nucleus throughout mitosis; may be involved in maintaining chromatin structure

633;One of two homeobox transcriptional repressors (see also Yoxlp), that bind to Mcm1p and to early cell cycle box (ECB) elements of cell cycle regulated genes, thereby restricting ECB-mediated transcription to the M/G1 interval

634;Vacuolar endopolyphosphatase with a role in phosphate metabolism; functions as a homodimer

635;Endosomal Na+/H+ exchanger, required for intracellular sequestration of Na+; required for osmotolerance to acute hypertonic shock

636;E3 ubiquitin ligase of the hect-domain class; has a role in mRNA export from the nucleus and may regulate transcriptional coactivators

637;Mitochondrial ribosomal protein of the large subunit

638;Essential t-SNARE that forms a complex with Tlg2p and Vtilp and mediates fusion of endosome-derived vesicles with the late Golgi; binds the docking complex VFT (Vps fifty-three) through interaction with Vps51p

639;Protein of unknown function; outer membrane component of the mitochondrial fusion machinery; Ugolp bind directly to Fzolp and Mgmlp and thereby link these two GTPases during mitochondrial fusion

640;Splicing factor, component of the U4/U6-U5 snRNP complex

641;AMP-activated serine/threonine protein kinase found in a complex containing Snf4p and members of the Sip1p/Sip2p/Gal83p family; required for transcription of glucose-repressed genes, thermotolerance, sporulation, and peroxisome biogenesis

642;Alphal,2-mannosyltransferase of the Golgi involved in protein mannosylation

643;Component of the GARP (Golgi-associated retrograde protein) complex, Vps51p-Vps52p-Vps53p-Vps54p, which is required for the recycling of proteins from endosomes to the late Golgi; involved in localization of actin and chitin

644;Subunit of the GINS complex (Sld5p, Psf1p, Psf2p, Psf3p), which is localized to DNA replication origins and implicated in assembly of the DNA replication machinery

645;Serine/threonine protein kinase involved in sphingolipid-mediated signaling pathway that controls endocytosis; activates Ypklp and Ykr2p, components of signaling cascade required for maintenance of cell wall integrity; redundant with Pkh2p

646;Mitochondrial ribosomal protein of the small subunit; genetic interactions suggest a possible role in promoting translation initiation

647;Pumilio-homology domain protein that binds ASH1 mRNA at PUF consensus sequences in the 3' UTR and represses its translation, resulting in proper asymmetric localization of ASH1 mRNA

648;Myo-inositol transporter with strong similarity to the minor myo-inositol transporter Itr2p, member of the sugar transporter superfamily; expression is repressed by inositol and choline via Opilp and derepressed via Ino2p and Ino4p

649;Essential protein required for the DNA integrity checkpoint pathways; interacts physically with Meclp; putative homolog of S. pombe Rad26 and human ATRIP

650;Protein required for partitioning of the 2-micron plasmid

651;Asn and gln rich protein of unknown function; high-copy suppressor of POL1 (DNA polymerase alpha) and partial suppressor of CDC2 (polymerase delta) and CDC6 (pre-RC loading factor) mutations; overexpression results in growth inhibition

652;Protein kinase involved in bud growth and assembly of the septin ring, proposed to have kinase-dependent and kinase-independent activities; undergoes autophosphorylation; similar to Kcc4p and Hsl1p

653;High-affinity glutamine permease, also transports Leu, Ser, Thr, Cys, Met and Asn; expression is fully dependent on Grr1p and modulated by the Ssy1p-Ptr3p-Ssy5p (SPS) sensor of extracellular amino acids

654;Ubiquitin-like protein of the SUMO family, conjugated to lysine residues of target proteins; regulates chromatid cohesion, chromosome segregation, APC-mediated proteolysis, DNA replication and septin ring dynamics

655;Hypothetical protein

656;RNA binding protein that associates with polysomes; proposed to be involved in regulating mRNA translation; involved in the copper-dependent mineralization of copper sulfide complexes on cell surface in cells cultured in copper salts

657;Non-essential protein of unknown function required for transcriptional induction of the early meiotic-specific transcription factor IME1, also required for sporulation

658;Protein disulfide isomerase of the endoplasmic reticulum lumen, function overlaps with that of Pdi1p; may interact with nascent polypeptides in the ER

659;Membrane-bound peptidyl-prolyl cis-trans isomerase (PPIase), binds to the drugs FK506 and rapamycin; expression pattern suggests possible involvement in ER protein trafficking

660;ADP-ribosylation factor (ARF) GTPase activating protein (GAP) effector, involved in the secretory and endocytic pathways; contains C2C2H2 cysteine/histidine motif

661;ORF, Uncharacterized

662;Protein of unknown function, has similarity to Pmp3p, which is involved in cation transport; green fluorescent protein (GFP)-fusion protein localizes to the cytoplasm in a punctate pattern

663;Protein involved in transcription; interacts with RNA polymerase II subunits Rpb2p, Rpb3, and Rpb11p; has similarity to human RPAP1

664;Subunit 7 of the ubiquinol cytochrome-c reductase complex, which is a component of the mitochondrial inner membrane electron transport chain; oriented facing the mitochondrial matrix; N-terminus appears to play a role in complex assembly

665;Possible chaperone and cysteine protease with similarity to E. coli Hsp31 and S. cerevisiae Hsp32p, Hsp33p, and Sno4p; member of the DJ-1/ThiJ/PfpI superfamily, which includes human DJ-1 involved in Parkinson's disease; exists as a dimer

666;D-lactate dehydrogenase, part of the retrograde regulon which consists of genes whose expression is stimulated by damage to mitochondria and reduced in cells grown with glutamate as the sole nitrogen source, located in the cytoplasm

667;Ferrioxamine B transporter, member of the ARN family of transporters that specifically recognize siderophore-iron chelates; transcription is induced during iron deprivation and diauxic shift; potentially phosphorylated by Cdc28p

668;Putative transporter, member of a family of seven S. cerevisiae genes (AVT1-7) related to vesicular GABA-glycine transporters

669;Vacuolar proteinase B (yscB), a serine protease of the subtilisin family; involved in protein degradation in the vacuole and required for full protein degradation during sporulation

670;Subunit of the mitochondrial inner membrane peptidase, which is required for maturation of mitochondrial proteins of the intermembrane space; Somlp facilitates cleavage of a subset of substrates

671;Hypothetical protein

672;Subunit of the Hatlp-Hat2p histone acetyltransferase complex; required for high affinity binding of the complex to free histone H4, thereby enhancing Hat1p activity; similar to human RbAp46 and 48; has a role in telomeric silencing

673;Protein component of the large (60S) ribosomal subunit, nearly identical to Rpl12Bp; rpl12a rpl12b double mutant exhibits slow growth and slow translation; has similarity to E. coli L11 and rat L12 ribosomal proteins

674;Subunit D of the eight-subunit V1 peripheral membrane domain of the vacuolar H+-ATPase (V-ATPase), an electrogenic proton pump found throughout the endomembrane system; plays a role in the coupling of proton transport and ATP hydrolysis

675;Mitochondrial ribosomal protein of the large subunit, has similarity to E. coli L2 ribosomal protein; fat21 mutant allele causes inability to utilize oleate and may interfere with activity of the Adr1p transcription factor

676;Hypothetical protein

677;Threonine aldolase, catalyzes the cleavage of L-allo-threonine and L-threonine to glycine; involved in glycine biosynthesis

678;Hypothetical protein

679;Cytochrome c isoform 2, expressed under hypoxic conditions; electron carrier of the mitochondrial intermembrane space that transfers electrons from ubiquinone-cytochrome c oxidoreductase to cytochrome c oxidase during cellular respiration

680;Protein with ubiquitin-like N terminus, recognizes and binds damaged DNA (with Rad4p) during nucleotide excision repair; regulates Rad4p levels, subunit of Nuclear Excision Repair Factor 2 (NEF2); homolog of human HR23A and HR23B proteins

681;Subunit of the alpha-1,6 mannosyltransferase complex; type II membrane protein; has a role in retention of glycosyltransferases in the Golgi; involved in osmotic sensitivity and resistance to aminonitrophenyl propanediol

682;Translation initiation factor eIF-5A, promotes formation of the first peptide bond; similar to and functionally redundant with Anblp; undergoes an essential hypusination modification; expressed under aerobic conditions

683;Protein involved in DNA replication; component of the Mcm2-7 hexameric complex that binds chromatin as a part of the pre-replicative complex

684;P-type ATPase, ion transporter of the ER membrane involved in ER function and Ca2+ homeostasis; required for regulating Hmg2p degradation; confers sensitivity to a killer toxin (SMKT) produced by Pichia farinosa KK1

685;Protein involved in bud-site selection; diploid mutants display a random budding pattern instead of the wild-type bipolar pattern; has similarity to pyridoxal kinases

686;Proteolipid subunit of the vacuolar H(+)-ATPase V0 sector (subunit c; dicyclohexylcarbodiimide binding subunit); required for vacuolar acidification and important for copper and iron metal ion homeostasis

687;RNA binding protein, part of U3 snoRNP involved in rRNA processing, part of U4/U6-U5 tri-snRNP involved in mRNA splicing, similar to human 15.5K protein

688;Ubiquinol-cytochrome-c reductase, a Rieske iron-sulfur protein of the mitochondrial cytochrome bc1 complex; transfers electrons from ubiquinol to cytochrome c1 during respiration

689;Orotidine-5'-phosphate (OMP) decarboxylase, catalyzes the sixth enzymatic step in the de novo biosynthesis of pyrimidines, converting OMP into uridine monophosphate (UMP); converts 5-FOA into 5-fluorouracil, a toxic compound

690;Mitochondrial intermembrane space protein, forms a complex with Mrs11p/Tim10p that mediates import and insertion of a subset of polytopic inner membrane proteins; may prevent aggregation of incoming proteins in a chaperone-like manner

691;Hypothetical protein with low sequence identity to Pdclp

692;SUM0 ligase involved in chromosomal organization and DNA repair; forms a complex with Smc5p, Rhc18p, and Nse1p; mutants are sensitive to methyl methanesulfonate and show increased spontaneous mutation and mitotic recombination

693;Esalp-associated factor, subunit of the NuA4 acetyltransferase complex

694;Proteolipid associated with plasma membrane H(+)-ATPase (Pma1p); regulates plasma membrane H(+)-ATPase activity; nearly identical to PMP1

695;Protein of unknown function with a possible role in glutathione metabolism, as suggested by computational analysis of large-scale protein-protein interaction data; GFP-fusion protein localizes to the nuclear periphery

696;Non-essential conserved protein of unknown function, plays a role in mRNA decapping by specifically affecting the function of the decapping enzyme Dcp1p; localizes to cytoplasmic mRNA processing bodies

697;Phosphorylated vacuolar membrane protein that interacts with Atg13p, required for the cytoplasm-to-vacuole targeting (Cvt) pathway; interacts with Nvj1p to form nucleus-vacuole junctions

698;Glycogen branching enzyme, involved in glycogen accumulation; green fluorescent protein (GFP)-fusion protein localizes to the cytoplasm in a punctate pattern

699;Protein with a potential role in vacuolar function, as suggested by its ability to bind Vac8p; green fluorescent protein (GFP)-fusion protein localizes to the cytoplasm in a punctate pattern

700;Uridine diphosphate-N-acetylglucosamine (UDP-GlcNAc) transporter required for cell wall chitin synthesis; localized to the ER

701;Beta subunit of the oligosaccharyl transferase (OST) glycoprotein complex; required for N-linked glycosylation of proteins in the endoplasmic reticulum

702;Putative protein of unknown function; green fluorescent protein (GFP)-fusion localizes to the ER; YEL001C is non-essential

703;Nuclear envelope protein, interacts with GDP-bound Gsplp and with proteins of the nuclear pore to transport Gsp1p into the nucleus where it is an essential player in nucleocytoplasmic transport

704;Cell wall mannoprotein of the Srp1p/Tip1p family of serine-alanine-rich proteins; expression is downregulated at acidic pH and induced by cold shock and anaerobiosis; abundance is increased in cells cultured without shaking

705;20S proteasome beta-type subunit; localizes to the nucleus throughout the cell cycle

706;Protoporphyrinogen oxidase, a mitochondrial enzyme that catalyzes the seventh step in the heme biosynthetic pathway, converting protoporphyrinogen IX to protoporphyrin IX

707;Long chain fatty acyl-CoA synthetase; accepts a wider range of acyl chain lengths than Faa1p, preferring C9:0-C13:0; involved in the activation of endogenous pools of fatty acids

708;Microtubule-binding protein that together with Kar9p makes up the cortical microtubule capture site and delays the exit from mitosis when the spindle is oriented abnormally

709;Component, with Yta12p, of the mitochondrial inner membrane m-AAA protease that mediates degradation of misfolded or unassembled proteins and is also required for correct assembly of mitochondrial enzyme complexes

710;Component of the evolutionarily conserved kinetochore-associated Ndc80 complex (Ndc80p-Nuf2p-Spc24p-Spc25p); involved in chromosome segregation, spindle checkpoint activity and kinetochore clustering

711;Sshlp-Ssslp-Sbh2p complex component, involved in protein translocation into the endoplasmic reticulum; homologous to Sbh1p

712;Nucleotide binding alpha subunit of the heterotrimeric G protein that interacts with the receptor Gpr1p, has signaling role in response to nutrients; green fluorescent protein (GFP)-fusion protein localizes to the cell periphery

713;Essential, non-ATPase regulatory subunit of the 26S proteasome lid, similar to the p58 subunit of the human 26S proteasome; temperature-sensitive alleles cause metaphase arrest, suggesting a role for the proteasome in cell cycle control

714;Subunit of the RNA polymerase II mediator complex; associates with core polymerase subunits to form the RNA polymerase II holoenzyme; essential for transcriptional regulation

715;Delta 1-pyrroline-5-carboxylate reductase, catalyzes the last step in proline biosynthesis

716;Carnitine acetyltransferase; has similarity to Yatlp, which is a carnitine acetyltransferase associated with the mitochondrial outer membrane

717;Gamma subunit of the translation initiation factor eIF2, involved in the identification of the start codon; binds GTP when forming the ternary complex with GTP and tRNAi-Met

718;Phosphatidylserine synthase, functions in phospholipid biosynthesis; catalyzes the reaction CDP-diaclyglycerol + L-serine = CMP + L-1-phosphatidylserine, transcriptionally repressed by myo-inositol and choline

719;One of three possible beta-subunits of the Snfl kinase complex, allows nuclear localization of the Snfl kinase complex in the presence of a nonfermentable carbon source; contains glycogen-binding domain

720;Core Sm protein Sm B; part of heteroheptameric complex (with Smd1p, Smd2p, Smd3p, Sme1p, Smx3p, and Smx2p) that is part of the spliceosomal U1, U2, U4, and U5 snRNPs; homolog of human Sm B and Sm B'

721;GTPase of the Ypt/Rab family, very similar to Ypt32p; involved in the exocytic pathway; mediates intra-Golgi traffic or the budding of post-Golgi vesicles from the trans-Golgi

722;Cytoplasmic protein of unknown function, transcription is induced under conditions of zinc deficiency

723;Hypothetical protein

724;ATPase of the ATP-binding cassette (ABC) family involved in 40S and 60S ribosome biogenesis, has similarity to Gcn20p; shuttles from nucleus to cytoplasm, physically interacts with Tif6p, Lsglp

725;Protein of unknown function, expression is induced by low phosphate levels and by inactivation of Pho85p

726;Essential protein of unknown function; heterozygous mutant shows haploinsufficiency in K1 killer toxin resistance

727;Transcriptional activator of genes regulated by nitrogen catabolite repression (NCR), localization and activity regulated by quality of nitrogen source

728;Protein of unknown function, has similarity to endonuclease Rthlp; potentially phosphorylated by Cdc28p

729;Peptide methionine sulfoxide reductase, reverses the oxidation of methionine residues; involved in oxidative damage repair, providing resistance to oxidative stress and regulation of lifespan

730;S-adenosyl-L-homocysteine hydrolase, catabolizes S-adenosyl-L-homocysteine which is formed after donation of the activated methyl group of S-adenosyl-L-methionine (AdoMet) to an acceptor

731;Endoplasmic reticulum membrane protein, may facilitate protein-protein interactions between the Erg26p dehydrogenase and the Erg27p 3-ketoreductase and/or tether these enzymes to the ER, also interacts with Erg6p

732;Meiosis-specific protein of unknown function, required for spore wall formation during sporulation; dispensible for both nuclear divisions during meiosis

733;Putative ATPase of the AAA family, interacts with the Sin1p transcriptional repressor in the two-hybrid system

734;Nuclear type II J heat shock protein of the E. coli dnaJ family, contains a leucine zipper-like motif, binds to non-native substrates for presentation to Ssa3p, may function during protein translocation, assembly and disassembly

735;Protein required for mitochondrial iron-sulfur cluster biosynthesis

736;Hypothetical protein

737;Mitochondrial phosphate carrier, imports inorganic phosphate into mitochondria; functionally redundant with Mir1p but less abundant than Mir1p under normal conditions; expression is induced at high temperature

738;Hypothetical protein

739;ATP phosphoribosyltransferase, a hexameric enzyme, catalyzes the first step in histidine biosynthesis; mutations cause histidine auxotrophy and sensitivity to Cu, Co, and Ni salts; transcription is regulated by general amino acid control

740;Member of the p14.5 protein family with similarity to Mmf1p, functionally complements Mmflp function when targeted to mitochondria; heat shock inducible; high-dosage growth inhibitor; forms a homotrimer in vitro

741;Pho85p cyclin of the Pho80p subfamily; forms the major Glc8p kinase together with Pcl7p and Pho85p; involved in the control of glycogen storage by Pho85p; stabilized by Elongin C binding

742;Putative purine-cytosine permease, very similar to Fcy2p but cannot substitute for its function

743;One of two redundant DL-glycerol-3-phosphatases (RHR2/GPP1 encodes the other) involved in glycerol biosynthesis; induced in response to hyperosmotic stress and oxidative stress, and during the diauxic transition

744;Protein of unknown function; overproduction suppresses the transcriptional defect caused by an hpr1 mutation

745;Isocitrate lyase, catalyzes the formation of succinate and glyoxylate from isocitrate, a key reaction of the glyoxylate cycle; expression of ICL1 is induced by growth on ethanol and repressed by growth on glucose

746;Hypothetical protein

747;Ribonucleotide-diphosphate reductase (RNR), large subunit; the RNR complex catalyzes the rate-limiting step in dNTP synthesis and is regulated by DNA replication and DNA damage checkpoint pathways via localization of the small subunits

748;Protein of unknown function; green fluorescent protein (GFP)-fusion protein localizes to the cytoplasm in a punctate pattern

749;Vacuolar transporter chaperon (VTC) involved in distributing V-ATPase and other membrane proteins; together with other VTC proteins, forms a heterotetrameric complex that associates with the SNARE Nyvlp and the V0 sector of the V-ATPase

750;Mitochondrial aldehyde dehydrogenase, involved in regulation or biosynthesis of electron transport chain components and acetate formation; activated by K+; utilizes NADP+ as the preferred coenzyme; constitutively expressed

751;Metallopeptidase, localized to the mitochondrial matrix

752;Hypothetical protein

753;The authentic, non-tagged protein was localized to the mitochondria

754;Threonine deaminase, catalyzes the first step in isoleucine biosynthesis; expression is under general amino acid control; ILV1 locus exhibits highly positioned nucleosomes whose organization is independent of known ILV1 regulation

755;Hypothetical protein

756;Beta subunit of the Sec61p ER translocation complex (Sec61p-Sss1p-Sbh1p); involved in protein translocation into the endoplasmic reticulum; interacts with the exocyst complex; homologous to Sbh2p

757;Cobalamin-independent methionine synthase, involved in amino acid biosynthesis; requires a minimum of two glutamates on the methyltetrahydrofolate substrate, similar to bacterial metE homologs

758;Protein that associates with the INO80 chromatin remodeling complex under low-salt conditions

759;Beta subunit of the 20S proteasome involved in ubiquitin-dependent catabolism; human homolog is subunit C10

760;Strand exchange protein, forms a helical filament with DNA that searches for homology; involved in the recombinational repair of double-strand breaks in DNA during vegetative growth and meiosis; homolog of Dmclp and bacterial RecA protein

761;Sporulation-specific activator of Chs3p (chitin synthase III), required for the synthesis of the chitosan layer of ascospores; has similarity to Skt5p, which activates Chs3p during vegetative growth; transcriptionally induced at alkaline pH

762;Ubiquitin-specific protease that cleaves ubiquitin-protein fusions

763;Ubiquitin-conjugating enzyme involved in ER-associated protein degradation; located at the cytosolic side of the ER membrane; tail region contains a transmembrane segment at the C-terminus; substrate of the ubiquitin-proteasome pathway

764;Protein that may have a role in targeting of plasma membrane [H+]ATPase (Pma1p) to the plasma membrane, as suggested by analysis of genetic interactions

765;Protein component of the small (40S) ribosomal subunit; identical to Rps8Bp and has similarity to rat S8 ribosomal protein

766;Heat shock protein that is highly induced upon stress; plays a role in SRP-dependent cotranslational protein-membrane targeting and translocation; member of the HSP70 family; cytoplasmic protein that concentrates in nuclei upon starvation

767;Protein with a role in regulation of Ty1 transposition

768;Abundant subunit of the nuclear pore complex (NPC), present on both sides of the NPC, has similarity to Nup170p

769;Component of the nuclear pore complex required for polyadenylated RNA export but not for protein import, homologous to S. pombe Raelp

770;Transcription factor required for flocculation, diploid filamentous growth, and haploid invasive growth; genome reference strain S288C and most laboratory strains have a mutation in this gene

771;Karyopherin beta, mediates nuclear import of ribosomal proteins prior to assembly into ribosomes and import of histones H3 and H4; localizes to the nuclear pore, nucleus, and cytoplasm; exhibits genetic interactions with RAI1

772;DNA binding component of the SBF complex (Swi4p-Swi6p), a transcriptional activator that in concert with MBF (Mbp1-Swi6p) regulates late G1-specific transcription of targets including cyclins and genes required for DNA synthesis and repair

773;Lsm (Like Sm) protein; part of heteroheptameric complexes (Lsm2p-7p and either Lsm1p or 8p): cytoplasmic Lsm1p complex involved in mRNA decay; nuclear Lsm8p complex part of U6 snRNP and possibly involved in processing tRNA, snoRNA, and rRNA

774;Protein implicated in polar growth, functionally redundant with Boi1p; interacts with bud-emergence protein Bemlp; contains an SH3 (src homology 3) domain and a PH (pleckstrin homology) domain

775;Protein of unknown function, expressed during sporulation; not required for sporulation, but gene exhibits genetic interactions with other genes required for sporulation

776;Protein containing a RING finger domain that forms a complex with Hex3p; mutant phenotypes and genetic interactions suggest a possible role in resolving recombination intermediates during DNA replication or repair

777;Vacuolar transporter, exports aspartate and glutamate from the vacuole; member of a family of seven S. cerevisiae genes (AVT1-7) related to vesicular GABA-glycine transporters

778;Integral ER membrane protein that regulates phospholipid metabolism via an interaction with the FFAT motif of Opilp, also involved in telomeric silencing, disruption causes inositol auxotrophy above 34 degrees C, VAP homolog

779;ADP-ribosylation factor GTPase activating protein (ARF GAP), involved in ER-Golgi transport; shares functional similarity with Gcs1p

780;Palmitoylated, vacuolar membrane-localized casein kinase I isoform; negatively regulates vacuole fusion during hypertonic stress via phosphorylation of the HOPS complex subunit, Vps41p; shares overlapping essential functions with Hrr25p

781;Daughter cell-specific protein, may participate in pathways regulating cell wall metabolism; deletion affects cell separation after division and sensitivity to drugs targeted against the cell wall

782;Ubiquitin-protein ligase involved in ubiquitin-mediated protein degradation; plays a role in heat shock element (HSE)-mediated gene expression and multivesicular body sorting; contains a hect (homologous to E6-AP carboxyl terminus) domain

783;Constituent of 66S pre-ribosomal particles, involved in 60S ribosomal subunit biogenesis

784;Hypothetical protein

785;Protein component of the small (40S) ribosomal subunit; nearly identical to Rps26Ap and has similarity to rat S26 ribosomal protein

786;Protein with an N-terminal kelch-like domain, putative negative regulator of early meiotic gene expression; required, with Mds3p, for growth under alkaline conditions

787;GDP dissociation inhibitor, regulates vesicle traffic in secretory pathways by regulating the dissociation of GDP from the Sec4/Ypt/rab family of GTP binding proteins

788;Hypothetical protein

789;Hypothetical protein

790;Hypothetical protein

791;Protein required for the hydroxylation of heme O to form heme A, which is an essential prosthetic group for cytochrome c oxidase

792;DNA damage-inducible v-SNARE binding protein, contains a ubiquitin-associated (UBA) domain, may act as a negative regulator of constitutive exocytosis, may play a role in S-phase checkpoint control

793;Putative ubiquitin-specific protease that does not associate with the proteasome

794;High affinity iron permease involved in the transport of iron across the plasma membrane; forms complex with Fet3p; expression is regulated by iron

795;TATA-binding protein, general transcription factor that interacts with other factors to form the preinitiation complex at promoters, essential for viability

796;TATA-binding protein, general transcription factor that interacts with other factors to form the preinitiation complex at promoters, essential for viability

797;TATA-binding protein, general transcription factor that interacts with other factors to form the preinitiation complex at promoters, essential for viability

798;Coiled-coil polarisome protein required for polarized morphogenesis, cell fusion, and low affinity Ca2+ influx; forms polarisome complex with Bni1p, Bud6p, and Spa2p; localizes to sites of polarized growth

799;GPI-anchored, serine/threonine rich cell wall protein of unknown function; basal expression requires Msn2p/Msn4p; expression is induced under conditions of stress and during the diauxic shift; similar to Sed1p

800;Specific translational activator for the COX3 mRNA that acts together with Pet54p and Pet494p; located in the mitochondrial inner membrane

801;Translocase of the mitochondrial inner membrane, mediates the insertion of both mitochondrial- and nuclear-encoded proteins from the matrix into the inner membrane, interacts with mitochondrial ribosomes; null is respiratory deficient

802;Rho GTPase activating protein (RhoGAP) involved in the control of cytoskeleton organization and cellular morphogenesis; required for bud emergence

803;Hypothetical protein

804;Protein involved in negative regulation of transcription, exhibits regulated interactions with both histones and SWI-SNF components, has similarity to mammalian HMG1 proteins

805;Hypothetical protein

806;Nucleosome remodeling factor that functions in regulation of transcription elongation; contains a chromo domain, a helicase domain and a DNA-binding domain; component of both the SAGA and SILK complexes

807;Poly(A) binding protein, part of the 3'-end RNA-processing complex, mediates interactions between the 5' cap structure and the 3' mRNA poly(A) tail, involved in control of poly(A) tail length, interacts with translation factor eIF-4G

808;Protein rich in serine and threonine residues involved in protein kinase C signaling pathway, which controls cell integrity; overproduction suppresses pkc1 mutations

809;ATP (CTP):tRNA-specific tRNA nucleotidyltransferase; different forms targeted to the nucleus, cytosol, and mitochondrion are generated via the use of multiple transcriptional and translational start sites

810;5' to 3' DNA helicase, involved in nucleotide excision repair and transcription; subunit of RNA polymerase II transcription initiation factor TFIIH; subunit of Nucleotide Excision Repair Factor 3 (NEF3); homolog of human XPD protein

811;RNA-dependent ATPase RNA helicase involved in the facilitation and disruption of snRNA interactions, required for disruption of U4/U6 base-pairing in native snRNPs to activate the spliceosome for catalysis

812;Hydroperoxide and superoxide-radical responsive glutathione-dependent oxidoreductase; monothiol glutaredoxin subfamily member along with Grx3p and Grx5p; protects cells from oxidative damage

813;Trans-aconitate methyltransferase, cytosolic enzyme that catalyzes the methyl esterification of 3-isopropylmalate, an intermediate of the leucine biosynthetic pathway, and trans-aconitate, which inhibits the citric acid cycle

814;DNA dependent ATPase/DNA helicase belonging to the Dna2p- and Nam7p-like family of helicases that is involved in modulating translation termination; interacts with the translation termination factors, localized to polysomes

815;14-3-3 protein, major isoform; binds proteins and DNA, involved in regulation of many processes including exocytosis and vesicle transport, Ras/MAPK signaling during pseudohyphal development, rapamycin-sensitive signaling, and others

816;Protein required for sporulation, transcript is induced 7.5 hours after induction of meiosis, expected to play significant role in the formation of reproductive cells

817;Protein interacting with Arl3p, which is a GTPase of the Ras superfamily involved in vesicle-tethering at the Golgi; putative ortholog of human SCOCO

818;Member of a stationary phase-induced gene family; transcription of SNZ2 is induced prior to diauxic shift, and also in the absence of thiamin in a Thi2p-dependent manner; forms a coregulated gene pair with SNO3

819;Protein involved in synthesis of the thiamine precursor hydroxymethylpyrimidine (HMP); member of a subtelomeric gene family including THI5, THI11, THI12, and THI13

820;Integral membrane component of endoplasmic reticulum-derived COPII-coated vesicles, which function in ER to Golgi transport

821;The authentic, non-tagged protein was localized to the mitochondria

822;Phosphomannomutase, involved in synthesis of GDP-mannose and dolichol-phosphate-mannose; required for folding and glycosylation of secretory proteins in the ER lumen

823;Putative protein of unknown function; YFL040W is not an essential gene

824;Beta-tubulin; associates with alpha-tubulin (Tub1p and Tub3p) to form tubulin dimer, which polymerizes to form microtubules

825;Mitochondrial RNA polymerase; single subunit enzyme similar to those of T3 and T7 bacteriophages; requires a specificity subunit encoded by MTF1 for promoter recognition

826;Putative integral membrane protein that interacts with Rpp0p, which is a component of the ribosomal stalk

827;Alanine : glyoxylate aminotransferase, catalyzes the synthesis of glycine from glyoxylate, which is one of three pathways for glycine biosynthesis in yeast; has similarity to mammalian and plant alanine : glyoxylate aminotransferases

828;Cyclin-dependent kinase-activating kinase required for passage through the cell cycle, phosphorylates and activates Cdc28p; nucleotide-binding pocket differs significantly from those of most other protein kinases

829;Part of the evolutionarily-conserved CCR4-NOT transcriptional regulatory complex involved in controlling mRNA initiation, elongation, and degradation; putative ABC ATPase; interacts with Ssn2p, Ssn3p, and Ssn8p

830;GTPase-activating protein for yeast Rab family members; Ypt1p is the preferred in vitro substrate but also acts on Sec4p, Ypt31p and Ypt32p; involved in the regulation of ER to Golgi vesicle transport

831;Receptor for alpha-factor pheromone; seven transmembrane-domain GPCR that interacts with both pheromone and a heterotrimeric G protein to initiate the signaling response that leads to mating between haploid a and alpha cells

832;GPI inositol deacylase of the ER that negatively regulates COPII vesicle formation, prevents production of vesicles with defective subunits, required for proper discrimination between resident ER proteins and Golgi-bound cargo molecules

833;Component of NuA4, which is an essential histone H4/H2A acetyltransferase complex; homologous to Drosophila Enhancer of Polycomb

834;Alpha subunit of cytoplasmic phenylalanyl-tRNA synthetase, forms a tetramer with Frs1p to form active enzyme; evolutionarily distant from mitochondrial phenylalanyl-tRNA synthetase based on protein sequence, but substrate binding is similar

835;Dihydrolipoamide dehydrogenase, the lipoamide dehydrogenase component (E3) of the pyruvate dehydrogenase and 2-oxoglutarate dehydrogenase multi-enzyme complexes

836;Core Sm protein Sm G; part of heteroheptameric complex (with Smblp, Smd1p, Smd2p, Smd3p, Sme1p, and Smx3p) that is part of the spliceosomal U1, U2, U4, and U5 snRNPs; homolog of human Sm G

837;Evolutionarily conserved glucosamine-6-phosphate acetyltransferase required for multiple cell cycle events including passage through START, DNA synthesis, and mitosis; involved in UDP-N-acetylglucosamine synthesis, forms GlcNAc6P from AcCoA

838;Protein involved in folding of mitochondrially synthesized proteins in the mitochondrial matrix; localizes to the mitochondrial inner membrane; member of the DnaJ family of molecular chaperones

839;Plasma membrane localized protein that protects membranes from desiccation; induced by heat shock, oxidative stress, osmostress, stationary phase entry, glucose depletion, oleate and alcohol; regulated by the HOG and Ras-Pka pathways

840;WW domain containing protein of unknown function; binds to Mcalp, a caspase-related protease that regulates H2O2-induced apoptosis; overexpression causes Gi phase growth arrest and clonal death that is suppressed by overexpression of MCA1

841;Subunit of the multiprotein cohesin complex, essential protein involved in chromosome segregation and in double-strand DNA break repair; SMC chromosomal ATPase family member, binds DNA with a preference for DNA with secondary structure

842;Secretory vesicle-associated Rab GTPase essential for exocytosis; associates with the exocyst component Sec15p and may regulate polarized delivery of transport vesicles to the exocyst at the plasma membrane

843;Putative ATP-dependent RNA helicase, nucleolar protein required for synthesis of 60S ribosomal subunits at a late step in the pathway; sediments with 66S pre-ribosomes in sucrose gradients

844;Non-essential tRNA:pseudouridine synthase, introduces pseudouridines at position 38 or 39 in tRNA, important for maintenance of translation efficiency and normal cell growth, localizes to both the nucleus and cytoplasm

845;Component of the nuclear pore complex, required for nuclear pore formation; forms a subcomplex with Nsp1p, Nup57p, and Nup49p

846;Inhibitor of the type I protein phosphatase Glc7p, which is involved in regulation of a variety of metabolic processes; overproduction causes decreased cellular content of glycogen

847;Metalloprotease subunit of the 19S regulatory particle of the 26S proteasome lid; couples the deubiquitination and degradation of proteasome substrates 848;Putative X-Pro aminopeptidase; green fluorescent protein (GFP)-fusion protein localizes to the cytoplasm; YFR006W is not an essential gene

849;Hypothetical protein

850;Positive regulator of the Gcn2p kinase activity, forms a complex with Gcn1p; proposed to stimulate Gcn2p activation by an uncharged tRNA

851;Dubious ORF unlikely to encode a protein, based on available experimental and comparative sequence data; completely overlaps the verified gene YFR009W; identified by expression profiling and mass spectrometry

852;Putative protein of unknown function; localizes to the mitochondrion; green fluorescent protein (GFP)-fusion protein localizes to the cytoplasm; YFR011C is not an essential gene

853;Member of a complex (Iswla) with Iswlp that has nucleosome-stimulated ATPase activity and represses transcription initiation by specific positioning of a promoter proximal dinucleosome; has homology to Esc8p, which is involved in silencing

854;Glycogen synthase with similarity to Gsy2p, the more highly expressed yeast homolog; expression induced by glucose limitation, nitrogen starvation, environmental stress, and entry into stationary phase

855;Putative protein of unknown function; green fluorescent protein (GFP)-fusion protein localizes to the cytoplasm; YFR017C is not an essential gene

856;1-phosphatidylinositol-3-phosphate 5-kinase; vacuolar membrane kinase that generates phosphatidylinositol (3,5)P2, which is involved in vacuolar sorting and homeostasis

857;Phosphatidylinositol 3,5-bisphosphate-binding protein of the vacuolar membrane, predicted to fold as a seven-bladed beta-propeller; required for recycling of Atg9p through the pre-autophagosomal structure

858;Protein that binds to Rsp5p, which is a hect-type ubiquitin ligase, via its 2 PY motifs; has similarity to Rodlp; mutation suppresses the temperature sensitivity of an mck1 rim11 double mutant

859;Poly(A) binding protein, suppressor of DNA polymerase epsilon mutation, similar to Mip6p

860;Histidinolphosphatase, catalyzes the eighth step in histidine biosynthesis; mutations cause histidine auxotrophy and sensitivity to Cu, Co, and Ni salts; transcription is regulated by general amino acid control

861;Acetyltransferase required for the establishment of sister chromatid cohesion during DNA replication, but not for its maintenance during G2 and M phases; also required for postreplicative double-strand break repair; interacts with Chl1p

862;Protein phosphatase required for mitotic exit; located in the nucleolus until liberated by the FEAR and Mitotic Exit Network in anaphase, enabling it to act on key substrates to effect a decrease in CDK/B-cyclin activity and mitotic exit

863;Component of the SPS plasma membrane amino acid sensor system (Ssylp-Ptr3p-Ssy5p), which senses external amino acid concentration and transmits intracellular signals that result in regulation of expression of amino acid permease genes

864;Subunit alpha of assimilatory sulfite reductase, which is responsible for the conversion of sulfite into sulfide

865;Component of the condensin complex, essential SMC chromosomal ATPase family member that forms a complex with Smc4p to form the active ATPase; Smc2p/Smc4p complex binds DNA, possibly in the cleft formed by the coiled-coil of the folded dimer

866;Protein component of the large (60S) ribosomal subunit, has similarity to rat L29 ribosomal protein; not essential for translation, but required for proper joining of the large and small ribosomal subunits and for normal translation rate

867;Subunit 6 of the ubiquinol cytochrome-c reductase complex, which is a component of the mitochondrial inner membrane electron transport chain; highly acidic protein; required for maturation of cytochrome c1

868;Subunit of the Anaphase-Promoting Complex/Cyclosome (APC/C), which is a ubiquitin-protein ligase required for degradation of anaphase inhibitors, including mitotic cyclins, during the metaphase/anaphase transition

869;One of 15 subunits of the 'Remodel the Structure of Chromatin' (RSC) complex; essential for viability and mitotic growth; homolog of SWI/SNF subunit Swi3p, but unlike Swi3p, does not activate transcription of reporters

870;Endoplasmic reticulum protein that may function as a cochaperone, as suggested by the presence of a DnaJ-like domain

871;Protein required for cell viability

872;Kinetochore protein of unknown function; associated with the essential kinetochore proteins Nnf11p and Spc24p; phosphorylated by both Clb5-Cdk1 and, to a lesser extent, Clb2-Cdk1.

873;Quinolinate phosphoribosyl transferase, required for biosynthesis of nicotinic acid from tryptophan via kynurenine pathway

874;Mitochondrial ribosomal protein of the small subunit, has similarity to human mitochondrial ribosomal protein MRP-S36

875;20S proteasome beta-type subunit

876;Delta subunit of the coatomer complex (COPI), which coats Golgi-derived transport vesicles; involved in retrograde transport between Golgi and ER

877;Subunit of the 19S regulatory particle of the 26S proteasome lid; synthetically lethal with RPT1, which is an ATPase component of the 19S regulatory particle; physically interacts with Noblp and Rpn3p

878;Hexokinase isoenzyme 1, a cytosolic protein that catalyzes phosphorylation of glucose during glucose metabolism; expression is highest during growth on non-glucose carbon sources; glucose-induced repression involves the hexokinase Hxk2p

879;Protein required for respiratory growth; null mutation suppresses the Cyclp translation defect caused by the presence of an aberrant ATG codon upstream of the correct start

880;High affinity Ca2+/Mn2+ P-type ATPase required for Ca2+ and Mn2+ transport into Golgi; involved in Ca2+ dependent protein sorting and processing; mutations in human homolog ATP2C1 cause acantholytic skin condition Hailey-Hailey disease

881;Copper-binding transcription factor; activates transcription of the metallothionein genes CUP 1-1 and CUP1-2 in response to elevated copper concentrations

882;RanGTP-binding protein, inhibits RanGAP1 (Rnalp)-mediated GTP hydrolysis of RanGTP (Gsp1p); shares similarity to proteins in other fungi but not in higher eukaryotes

883;DNA-dependent ATPase, stimulates strand exchange by modifying the topology of double-stranded DNA; involved in the recombinational repair of double-strand breaks in DNA during vegetative growth and meiosis; member of the SWI/SNF family

884;Protein that interacts with Rab GTPases; computational analysis of large-scale protein-protein interaction data suggests a possible role in vesicle-mediated transport

885;Vacuolar alpha mannosidase, involved in free oligosaccharide (fOS) degradation; delivered to the vacuole in a novel pathway separate from the secretory pathway

886;Beta subunit of geranylgeranyltransferase type I, catalyzes geranylgeranylation to the cysteine residue in proteins containing a C-terminal CaaX sequence ending in Leu or Phe; has substrates important for morphogenesis

887;Peroxisomal membrane protein that is a central component of the peroxisomal protein import machinery, interacts with PTS1 (Pex5p) and PTS2 (Pex7p) peroxisomal matrix protein signal recognition factors and membrane receptor Pex13p

888;Component of the RNA polymerase II mediator complex, which is required for transcriptional activation and also has a role in basal transcription

889;Mitochondrial polypeptide chain release factor, involved in stop codon recognition and hydrolysis of the peptidyl-tRNA bond during mitochondrial translation; lack of MRF1 causes mitochondrial genome instability

890;Hypothetical protein

891;N-terminally acetylated protein component of the large (60S) ribosomal subunit, nearly identical to Rpl1Bp and has similarity to E. coli L1 and rat L10a ribosomal proteins; rpl1a rpl1b double null mutation is lethal

892;Alpha (guanylyltransferase) subunit of the mRNA capping enzyme, a heterodimer (the other subunit is CET1, an RNA 5'-triphophatase) involved in adding the 5' cap to mRNA; the mammalian enzyme is a single bifunctional polypeptide

893;Protein required for fusion of cvt-vesicles and autophagosomes with the vacuole; associates, as a complex with Ccz1p, with a perivacuolar compartment; potential Cdc28p substrate

894;Proposed gamma subunit of the heterotrimeric G protein that interacts with the receptor Grp1p; involved in regulation of pseudohyphal growth; requires Gpb1p or Gpb2p to interact with Gpa2p

895;RNA helicase in the DEAH-box family, involved in release of the lariat-intron from the spliceosome

896;Hypothetical protein

897;Cell-cycle regulated activator of anaphase-promoting complex/cyclosome (APC/C), which is required for metaphase/anaphase transition; directs ubiquitination of mitotic cyclins, Pds1p, and other anaphase inhibitors; potential Cdc28p substrate

898;Protein kinase activator found in a complex containing Snf1p and members of the Sip1p/Sip2p/Ga183p family; activates the Snf1p protein kinase; involved in expression of glucose-repressed genes, sporulation, and peroxisome biogenesis

899;Putative member of the oligopeptide transporter (OPT) family of membrane transporters

900;Subunit (60 kDa) of TFIID and SAGA complexes, involved in transcription initiation of RNA polymerase II and in chromatin modification, similar to histone H4

901;Protein of unknown function; has a CUE domain that binds ubiquitin, which may facilitate intramolecular monoubiquitination

902;Protein of unknown function; green fluorescent protein (GFP)-fusion protein localizes to the cell periphery

903;Essential light chain for myosin Myo2p; may stabilize Myo2p by binding to the neck region; may interact with Myo1p, Iqglp, and Myo2p to coordinate formation and contraction of the actomyosin ring with targeted membrane deposition

904;Nuclear pore protein that is part of the evolutionarily conserved Nup84p complex (Nup84p, Nup85p, Nup120p, Nup145p, and Seh1p); homologous to Sec13p

905;Putative GTPase involved in 60S ribosomal subunit biogenesis; required for the release of Nmd3p from 60S subunits in the cytoplasm

906;Nucleotide exchange factor for Gsplp, localizes to the nucleus, required for nucleocytoplasmic trafficking of macromolecules; potentially phosphorylated by Cdc28p

907;Protein of the Seclp/Munc-18 family, essential for vacuolar protein sorting; required for the function of Pep12p and the early endosome/late Golgi SNARE Tlg2p; essential for fusion of Golgi-derived vesicles with the prevacuolar compartment

908;Hypothetical protein

909;Plasma membrane protein with a possible role in proton symport of glycerol; member of the MBOAT family of putative membrane-bound O-acyltransferases

910;Hypothetical protein

911;Putative ATP-dependent RNA helicase of the DEAD-box family involved in ribosomal biogenesis

912;Transcription factor involved in iron utilization and homeostasis; binds the consensus site PyPuCACCCPu and activates the expression of target genes in response to changes in iron availability

913;RNA polymerase II subunit B12.6; contacts DNA; mutations affect transcription start site

914;Protein associated with the mitochondrial nucleoid; putative mitochondrial ribosomal protein with similarity to E. coli L7/L12 ribosomal protein; required for normal respiratory growth

915;NADH diphosphatase (pyrophosphatase), hydrolyzes the pyrophosphate linkage in NADH and related nucleotides; localizes to peroxisomes

916;Essential mitotic spindle protein required to maintain spindle integrity and kinetochore function, part of the multisubunit DASH complex which binds microtubules and is transferred to the kinetochore prior to mitosis

917;Hypothetical protein

918;Ubiquitin-conjugating enzyme (E2), involved in postreplication repair (with Rad18p), sporulation, telomere silencing, and ubiquitin-mediated N-end rule protein degradation (with Ubr1p)

919;One of two S. cerevisiae homologs (Sds23p and · Sds24p) of the Schizosaccharomyces pombe Sds23 protein, which genetic studies have implicated in APC/cyclosome regulation

920;Fatty acid desaturase, required for monounsaturated fatty acid synthesis and for normal distribution of mitochondria

921;Protein localized to COPII-coated vesicles, involved in vesicle formation and incorporation of specific secretory cargo; required for the delivery of bud-site selection protein Ax12p to cell surface; related to Drosophila cornichon

922;Translation initiation factor eIF4G, subunit of the mRNA cap-binding protein complex (eIF4F) that also contains eIF4E (Cdc33p); associates with the poly(A)-binding protein Pablp, also interacts with eIF4A (Tif1p); homologous to Tif4631p

923;Cleavage and polyadenylation factor I (CF I) component involved in cleavage and polyadenylation of mRNA 3' ends; interacts with the A-rich polyadenylation signal in complex with Rna14p and Hrplp

924;General transcription elongation factor TFIIS, enables RNA polymerase II to read through blocks to elongation by stimulating cleavage of nascent transcripts stalled at transcription arrest sites

925;Delta-aminolevulinate dehydratase, a homo-octameric enzyme, catalyzes the conversion of delta-aminolevulinic acid to porphobilinogen, the second step in the heme biosynthetic pathway; localizes to both the cytoplasm and nucleus

926;Nicotinamidase that converts nicotinamide to nicotinic acid as part of the NAD(+) salvage pathway, required for life span extension by calorie restriction; PNC1 expression responds to all known stimuli that extend replicative life span

927;Mtf1 Two Hybrid Clone 2

928;Transcription factor involved in glucose repression; C2H2 zinc finger protein similar to mammalian Egr and Wilms tumor proteins

929;Adhesion subunit of a-agglutinin of a-cells, C-terminal sequence acts as a ligand for alpha-agglutinin (Saglp) during agglutination, modified with O-linked oligomannosyl chains, linked to anchorage subunit Aga1p via two disulfide bonds

930;Ribosomal protein L30 of the large (60S) ribosomal subunit, nearly identical to Rp124Bp and has similarity to rat L24 ribosomal protein; not essential for translation but may be required for normal translation rate

931;Protein involved in nucleolar integrity and processing of the pre-rRNA for the 60S ribosome subunit; transcript is induced in response to cytotoxic stress but not genotoxic stress

932;Subunit of the GET complex; required for the retrieval of HDEL proteins from the Golgi to the ER in an ERD2 dependent fashion and for normal mitochondrial morphology and inheritance

933;Beta regulatory subunit of casein kinase 2, a Ser/Thr protein kinase with roles in cell growth and proliferation; the holoenzyme also contains CKA1, CKA2 and CKB2, the many substrates include transcription factors and all RNA polymerases

934;Molecular chaperone involved, with partner Ssqlp, in assembly of Fe/S clusters and in mitochondrial iron metabolism; contains a J domain typical to J-type chaperones; localizes to the mitochondrial matrix

935;Arginyl-tRNA-protein transferase, catalyzes post-translational conjugation of arginine to the amino termini of acceptor proteins which are then subject to degradation via the N-end rule pathway

936;C-24(28) sterol reductase, catalyzes the final step in ergosterol biosynthesis; mutants are viable, but lack ergosterol

937;Alpha subunit of the 20S core complex of the 26S proteasome involved in the degradation of ubiquitinated substrates; essential for growth; detected in the mitochondria

938;Hypothetical protein

939;Isopropylmalate isomerase, catalyzes the second step in the leucine biosynthesis pathway

940;Vacuolar Ca2+ ATPase involved in depleting cytosol of Ca2+ ions; prevents growth inhibition by activation of calcineurin in the presence of elevated concentrations of calcium

941;Component of the conserved oligomeric Golgi complex (Coglp through Cog8p), a cytosolic tethering complex that functions in protein trafficking to mediate fusion of transport vesicles to Golgi compartments

942;Cell-cycle regulated activator of the anaphase-promoting complex/cyclosome (APC/C), which directs ubiquitination of mitotic cyclins resulting in exit from mitosis; targets the APC/C to specific substrates including CDC20, ASE1 and CIN8

943;Protein with similarity to Emp24p and Erv25p, member of the p24 family involved in ER to Golgi transport

944;C-3 sterol dehydrogenase, catalyzes the second of three steps required to remove two C-4 methyl groups from an intermediate in ergosterol biosynthesis

945;Protein of unknown; function, component of the Swr1p complex that incorporates Htz1p into chromatin; component of the NuA4 histone acetyltransferase complex

946;Peroxisomal integral membrane protein, involved in negative regulation of peroxisome size; partially functionally redundant with Pex30p and Pex32p; probably acts at a step downstream of steps mediated by Pex28p and Pex29p

947;TFIIF (Transcription Factor II) middle subunit; involved in both transcription initiation and elongation of RNA polymerase II; homologous to human RAP30

948;Choline phosphate cytidylyltransferase, catalyzes the second step of phosphatidylethanolamine biosynthesis; involved in the maintenance of plasma membrane; similar to mammalian CTP: phosphocholine cytidylyl-transferases

949;Protein involved in regulation of the mitochondrial F1F0-ATP synthase; Stf1p and Stf2p act as stabilizing factors that enhance inhibitory action of the Inh1p protein

950;t-SNARE protein important for fusion of secretory vesicles with the plasma membrane; similar to but not functionally redundant with Spo20p; SNAP-25 homolog

951;Nicotinic acid mononucleotide adenylyltransferase, involved in NAD(+) salvage pathway

952;Hypothetical protein

953;Putative protein of unknown function; green fluorescent protein (GFP)-fusion protein localizes to both the nucleus and the cytoplasm

954;Gamma-aminobutyrate (GABA) transaminase (4-aminobutyrate aminotransferase) involved in the 4-aminobutyrate and glutamate degradation pathways; required for normal oxidative stress tolerance and nitrogen utilization

955;Subunit F of the eight-subunit V1 peripheral membrane domain of vacuolar H+-ATPase (V-ATPase), an electrogenic proton pump found throughout the endomembrane system; required for the V1 domain to assemble onto the vacuolar membrane

956;Protein of unknown function; green fluorescent protein (GFP)-fusion protein localizes to the cell periphery

957;Protein component of the small (40S) ribosomal subunit; nearly identical to Rps25Bp and has similarity to rat S25 ribosomal protein

958;Mitochondrial protein involved in sorting of proteins in the mitochondria; putative membrane-spanning ATPase

959;Subunit of both RNase MRP, which cleaves pre-rRNA, and nuclear RNase P, which cleaves tRNA precursors to generate mature 5' ends

960;Constituent of the mitochondrial inner membrane presequence translocase (TIM23 complex); may regulate protein import by binding to both the translocase of the outer membrane (TOM) and presequence-associated motor (PAM) complexes

961;Protein of unknown function, potential Cdc28p substrate; transcription is activated by paralogous transcription factors Yrmlp and Yrr1p along with genes involved in multidrug resistance

962;Acyl-CoA-binding protein, transports newly synthesized acyl-CoA esters from fatty acid synthetase (Fas1p-Fas2p) to acyl-CoA-consuming processes

963;Evolutionarily conserved protein with similarity to Orm2p, required for resistance to agents that induce the unfolded protein response; human ortholog is located in the endoplasmic reticulum

964;Protein involved in bud-site selection; diploid mutants display a unipolar budding pattern instead of the wild-type bipolar pattern, and bud at the distal pole

965;Putative protein of unknown function; green fluorescent protein (GFP)-fusion protein localizes to both the cytoplasm and the nucleus

966;Putative protein of unknown function; transcription is repressed by MOT1/YPL082C and induced by alpha-factor and during diauxic shift; green fluorescent protein (GFP)-fusion protein localizes to the nucleus

967;Putative protein of unknown function; mRNA is targeted to the bud via the mRNA transport system involving She2p; deletion mutant is inviable

968;One of six subunits of the RNA polymerase III transcription initiation factor complex (TFIIIC); part of the TauA domain of TFIIIC that binds BoxA DNA promoter sites of tRNA and similar genes; has TPR motifs; human homolog is TFIIIC-102

969;Protein that interacts with Cdc48p and Np14p, involved in recognition of polyubiquitinated proteins and their presentation to the 26S proteasome for degradation; involved in transporting proteins from the ER to the cytosol

970;Hypothetical protein

971;High affinity methionine permease, integral membrane protein with 13 putative membrane-spanning regions; also involved in cysteine uptake

972;Putative protein of unknown function; green fluorescent protein (GFP)-fusion protein localizes to both the cytoplasm and the nucleus

973;Sporulation-specific homolog of the yeast CDC3/10/11/12 family of bud neck microfilament genes; septin protein involved in sporulation; regulated by ABFI

974;C-4 methyl sterol oxidase, catalyzes the first of three steps required to remove two C-4 methyl groups from an intermediate in ergosterol biosynthesis; mutants accumulate the sterol intermediate 4,4-dimethylzymosterol

975;Mitochondrial inner membrane protein, required for export of the Cox2p C terminus from the mitochondrial matrix to the intermembrane space during its assembly into cytochrome c oxidase; similar to Oxa2p ofN. crassa

976;Putative protein of unknown function

977;GDP/GTP exchange protein (GEP) for Rholp; mutations are synthetically lethal with mutations in rom2, which also encodes a GEP

978;Component of the nonsense-mediated mRNA decay (NMD) pathway, along with Nam7p and Nmd2p; involved in decay of mRNA containing nonsense codons

979;Core Sm protein Sm D1; part of heteroheptameric complex (with Smblp, Smd2p, Smd3p, Smelp, Smx3p, and Smx2p) that is part of the spliceosomal U1, U2, U4, and U5 snRNPs; homolog of human Sm D1

980;Unique component of the U4/U6.U5 tri-snRNP particle, dispensable for spliceosome assembly, but required for conformational changes which lead to catalytic activation of the spliceosome

981;Mitochondrial ribosomal protein of the large subunit

982;Twinfilin, highly conserved actin monomer-sequestering protein involved in regulation of the cortical actin cytoskeleton, composed of two cofilin-like regions, localizes actin monomers to sites of rapid filament assembly

983;Component of the TOM (translocase of outer membrane) complex responsible for recognition and initial import steps for all mitochondrially directed proteins; acts as a receptor for incoming precursor proteins

984;Delta subunit of the translation initiation factor eIF2B, the guanine-nucleotide exchange factor for eIF2; activity subsequently regulated by phosphorylated eIF2; first identified as a negative regulator of GCN4 expression

985;Long chain base-responsive inhibitor of protein kinases Pkh1p and Pkh2p, acts along with Lsp1p to down-regulate heat stress resistance via regulation of the Pkc1p and Ypk1p pathways; phosphorylated by Phk1p and Phk2p

986;Minor isoform of pyruvate decarboxylase, key enzyme in alcoholic fermentation, decarboxylates pyruvate to acetaldehyde, regulation is glucose- and ethanol-dependent, involved in amino acid catabolism

987;Ser/Thr kinase involved in transcription and stress response; functions as part of a network of genes in exit from mitosis; localization is cell cycle regulated; activated by Cdc15p during the exit from mitosis

988;Putative protein of unknown function; green fluorescent protein (GFP)-fusion protein localizes to the nucleus

989;Component of the RNA polymerase II holoenzyme, phosphorylated in response to oxidative stress; has a role in destruction of Ssn8p, which relieves repression of stress-response genes

990;Cytoplasmic GTPase-activating protein for Ypt/Rab transport GTPases Ypt6p, Ypt31p and Sec4p; involved in recycling of internalized proteins and regulation of Golgi secretory function

991;Mitochondrial serine protease required for the processing of various mitochondrial proteins and maintenance of mitochondrial DNA and morphology; belongs to the rhomboid-G1pG superfamily of intramembrane peptidases

992;Hypothetical protein

993;Nucleolar protein involved in rRNA processing and 60S ribosomal subunit biogenesis; constituent of several different pre-ribosomal particles

994;Subunit of the RNA polymerase II mediator complex; associates with core polymerase subunits to form the RNA polymerase II holoenzyme; essential for transcriptional regulation

995;Integral membrane protein that is required for vacuolar H+-ATPase (V-ATPase) function, although not an actual component of the V-ATPase complex; functions in the assembly of the V-ATPase; localized to the yeast endoplasmic reticulum (ER)

996;Hypothetical protein

997;B-type cyclin involved in cell cycle progression; activates Cdc28p to promote the transition from G2 to M phase; accumulates during G2 and M, then targeted via a destruction box motif for ubiquitin-mediated degradation by the proteasome998;Hypothetical protein

999;Mitochondrial inner membrane protein required for normal respiration, possible chaperone involved in assembly of cytochrome c oxidase; similar to SURF1 from mammals, chickens, and D. melanogaster

1000;Component of the DASH complex, localized to intranuclear spindles and spindle pole bodies; interacts with Duolp and Mps1p; key Ipllp target for regulating kinetochore-microtubule attachments

1001;Essential subunit of the nuclear pore complex (NPC), functions as the organizing center of an NPC subcomplex containing Nsp1p, Nup49p, Nup57p, and Nic96p

1002;Hypothetical protein

1003;Asparagine synthetase, isozyme of Asn1p; catalyzes the synthesis of L-asparagine from L-aspartate in the asparagine biosynthetic pathway

1004;Hypothetical protein

1005;Hypothetical protein

1006;Hypothetical protein

1007;Component of the spliceosome complex involved in pre-mRNA splicing; involved in regulation of cell cycle progression

1008;Protein of unknown function; green fluorescent protein (GFP)-fusion protein localizes to the cytoplasm in a punctate pattern

1009;Subunit of the prohibitin complex (Phb1p-Phb2p), a 1.2 MDa ring-shaped inner mitochondrial membrane chaperone that stabilizes newly synthesized proteins; determinant of replicative life span; involved in mitochondrial segregation

1010;20S proteasome beta-type subunit; the only nonessential 20S subunit

1011;Protein containing an N-terminal SH3 domain; binds Las17p, which is a homolog of human Wiskott-Aldrich Syndrome protein involved in actin patch assembly and actin polymerization

1012;Protein involved in sphingolipid biosynthesis; type II membrane protein with similarity to Kre6p

1013;Essential nucleolar protein of unknown function; contains WD repeats, interacts with Mpp10p and Bfr2p, and has homology to Spb1p

1014;Hypothetical protein

1015;N alpha-acetyl-transferase, transfers acetyl group from acetyl coenzyme A to the N-terminal methionine residues of proteins

1016;Ribosomal protein L30 of the large (60S) ribosomal subunit, nearly identical to Rp124Ap and has similarity to rat L24 ribosomal protein; not essential for translation but may be required for normal translation rate

1017;GTP-binding protein of the ras superfamily required for bud site selection, morphological changes in response to mating pheromone, and efficient cell fusion; localized to the plasma membrane; significantly similar to mammalian Rap GTPases

1018;Cystathionine beta-synthase, catalyzes the synthesis of cystathionine from serine and homocysteine, the first committed step in cysteine biosynthesis

1019;Ptalp Interacting protein

1020;Nucleolar protein that binds nuclear localization sequences, required for pre-rRNA processing and ribosome biogenesis

1021;Putative component of the protein phosphatase type 2A complex

1022;Translation initiation factor eIF4G, subunit of the mRNA cap-binding protein complex (eIF4F) that also contains eIF4E (Cdc33p); associates with the poly(A)-binding protein Pablp, also interacts with eIF4A (Tiflp); homologous to Tif4632p

1023;Pseudouridine synthase responsible for modification of cytoplasmic and mitochondrial tRNAs at position 31; mutation of Asp168 to Ala abolishes enzyme activity; not essential for viability

1024;Identified by homology to Ashbya gossypii

1025;Phosphatidylserine decarboxylase of the Golgi and vacuolar membranes, converts phosphatidylserine to phosphatidylethanolamine

1026;Protein with similarity to mammalian developmentally regulated GTP-binding protein

1027;Squalene epoxidase, catalyzes the epoxidation of squalene to 2,3-oxidosqualene; plays an essential role in the ergosterol-biosynthesis pathway and is the specific target of the antifungal drug terbinafine

1028;Protein interacting with poly(A)-binding protein Pablp; likely involved in control of the extent of mRNA polyadenylation; also interacts with Mkt1p to form a complex that may regulate translation of the HO mRNA

1029;Mitochondrial intermembrane space protein, forms a complex with TIm8p that medates import and insertion of a subset of polytopic inner membrane proteins; may prevent aggregation of incoming proteins in a chaperone-like manner

1030;Ubiquitin-protein ligase (E3) that interacts with Rad6p/LJbc2p to ubiquitinate substrates of the N-end rule pathway; binds to the Rpn2p, Rpt1p, and Rpt6p proteins of the 19S particle of the 26S proteasome

1031;Cytoplasmic tyrosyl-tRNA synthetase, class I aminoacyl-tRNA synthetase that aminoacylates tRNA(Tyr), required for cytoplasmic protein synthesis, interacts with positions 34 and 35 of the anticodon of tRNATyr

1032;TFIIF (Transcription Factor II) largest subunit; involved in both transcription initiation and elongation of RNA polymerase II; homologous to human RAP74

1033;Protein of unknown function with similarity to human HMG1 and HMG2; localizes to the cytoplasm

1034;High-affinity histidine permease, also involved in the transport of manganese ions

1035;Glyceraldehyde-3-phosphate dehydrogenase, isozyme 3, involved in glycolysis and gluconeogenesis; tetramer that catalyzes the reaction of glyceraldehyde-3-phosphate to 1,3 bis-phosphoglycerate; detected in the cytoplasm and cell-wall

1036;Dihydrolipoamide dehydrogenase (E3)-binding protein (E3BP) of the mitochondrial pyruvate dehydrogenase (PDH) complex, plays a structural role in the complex by binding and positioning E3 to the dihydrolipoamide acetyltransferase (E2) core

1037;3'-to-5' phosphorolytic exoribonuclease that is a subunit of the exosome; required for 3' processing of the 5.8S rRNA; involved in 3' to 5' mRNA degradation and translation inhibition ofnon-poly(A) mRNAs

1038;Protein of unknown function; homolog of mammalian electron transfer flavoprotein complex subunit ETF-beta; interacts with frataxin (Yfh1p)

1039;Phosphoserine phosphatase of the phosphoglycerate pathway, involved in serine and glycine biosynthesis, expression is regulated by the available nitrogen source

1040;N-acetyltransferase, confers resistance to the sphingolipid biosynthesis inhibitor myriocin (ISP-1) by converting it into N-acetyl-myriocin, co-operates with Ypklp in mediating resistance to myriocin

1041;Mitochondrial ribosomal protein of the small subunit

1042;Membrane protein involved in the synthesis of N-acetylglucosaminyl phosphatidylinositol (GlcNAc-PI), the first intermediate in the synthesis of glycosylphosphatidylinositol (GPI) anchors; human and mouse GPI1p are functional homologs

1043;Major karyopherin, involved in export of proteins, RNAs, and ribosomal subunits from the nucleus

1044;Dolichyl-phosphoglucose-dependent glucosyltransferase of the ER, functions in the dolichol pathway that synthesizes the dolichol-linked oligosaccharide precursor for N-linked protein glycosylation, has a role in regulation of ITR1 and INO1

1045;Protein involved in the regulation of cell wall synthesis; proposed to be involved in coordinating cell cycle progression with cell wall integrity

1046;Regulatory, non-ATPase subunit of the 26S proteasome; homolog of the human oncoprotein gankyrin, which interacts with the retinoblastoma tumor suppressor (Rb) and cyclin-dependent kinase 4/6

1047;Putative protein of unknown function; localizes to the mitochondrion 1048;Putative protein of unknown function; green fluorescent protein (GFP)-fusion protein localizes to the cytoplasm

1049;The authentic, non-tagged protein was localized to mitochondria

1050;cyclic nucleotide phosphodiesterase, hydrolyzes ADP-ribose 1", 2"-cyclic phosphate to ADP-ribose 1"-phosphate; no detectable phenotype is conferred by null mutation or by overexpression

1051;6-phosphogluconolactonase with similarity to Sol3p

1052;Protein similar to heat shock transcription factor; multicopy suppressor of pseudohyphal growth defects of ammonium permease mutants

1053;Histone acetyltransferase, acetylates lysine 14 on histone H3; catalytic subunit of the ADA and SAGA histone acetyltransferase complexes; founding member of the Gcn5p-related N-acetyltransferase superfamily

1054;Alpha subunit of the 20S proteasome involved in ubiquitin-dependent catabolism; human homolog is subunit zeta

1055;Enolase I, a phosphopyruvate hydratase that catalyzes the conversion of 2-phosphoglycerate to phosphoenolpyruvate during glycolysis and the reverse reaction during gluconeogenesis; expression is repressed in response to glucose

1056;Mitochondrial protein of the mitochondrial carrier family, involved in activating mitochondrial Sod2p probably by facilitating insertion of an essential manganese cofactor

1057;High affinity nicotinic acid plasma membrane permease, responsible for uptake of low levels of nicotinic acid; expression of the gene increases in the absence of extracellular nicotinic acid or para-aminobenzoate (PABA)

1058;Protein involved in bud-site selection; diploid mutants display a random budding pattern instead of the wild-type bipolar pattern

1059;Putative protein of unknown function; green fluorescent protein (GFP)-fusion protein localizes to the endoplasmic reticulum

1060;GTP-cyclohydrolase I, catalyzes the first step in the folic acid biosynthetic pathway

1061;Cytoplasmic protein containing a zinc finger domain with sequence similarity to that of Type I J-proteins; computational analysis of large-scale protein-protein interaction data suggests a possible role in actin patch assembly

1062;TFIID subunit (145 kDa), involved in RNA polymerase II transcription initiation, has histone acetyltransferase activity, involved in promoter binding and G1/S progression

1063;3' exoribonuclease, required for 5S and tRNA-Arg3 maturation

1064;cue11 wall protein with similarity to glucanases; scw4 scw10 double mutants exhibit defects in mating

1065;Essential protein involved in rRNA and snoRNA maturation; competes with TLC1 RNA for binding to Est2p, suggesting a role in regulation of telomerase; human homolog inhibits telomerase; contains a G-patch RNA interacting domain

1066;Endo-beta-1,3-glucanase, major protein of the cell wall, involved in cell wall maintenance

1067;Protein localized to COPII-coated vesicles, involved in vesicle formation and incorporation of specific secretory cargo

1068;Biotin synthase, catalyzes the conversion of dethiobiotin to biotin, which is the last step of the biotin biosynthesis pathway; complements E. coli bioB mutant

1069;Putative protein of unknown function; green fluorescent protein (GFP)-fusion protein localizes to the cytoplasm

1070;Nucleolar single-strand nucleic acid binding protein; associates with small nuclear RNAs

1071;Ribosomal protein L4 of the large (60S) ribosomal subunit, nearly identical to Rp18Bp and has similarity to rat L7a ribosomal protein; mutation results in decreased amounts of free 60S subunits

1072;Glycerol kinase, converts glycerol to glycerol-3-phosphate; glucose repression of expression is mediated by Adrlp and Ino2p-Ino4p; derepression of expression on non-fermentable carbon sources is mediated by Opilp and Rsflp

1073;v-SNARE protein involved in Golgi transport, homolog of the mammalian protein

1074;Major component of the proteasome; tethers the proteasome core particle to the regulatory particle, and enhances the stability of the proteasome

1075;Cytoplasmic protein required for replication of Brome mosaic virus in S. cerevisiae, which is a model system for studying replication of positive-strand RNA viruses in their natural hosts

1076;Transcriptional repressor involved in the response to pH; required for alkaline pH-stimulated differentiation pathways such as haploid invasive growth and sporulation; activated by proteolytic processing; has similarity to the A. nidulans transcription factor PacC

1077;One of 11 subunits of the SWI/SNF chromatin remodeling complex involved in transcriptional regulation; functions interdependently in transcriptional activation with Snf2p and Snf5p

1078;Putative RNA-binding protein required for the expression of early and middle sporulation genes

1079;Protein required for sporulation

1080;The authentic, non-tagged protein was localized to the mitochondria

1081;Protein of unknown function, homologous to the medium chain of mammalian clathrin-associated protein complex; involved in vesicular transport

1082;Plasma membrane urea transporter, expression is highly sensitive to nitrogen catabolite repression and induced by allophanate, the last intermediate of the allantoin degradative pathway

1083;Protein component of the small (40S) ribosomal subunit; overproduction suppresses mutations affecting RNA polymerase III-dependent transcription; has similarity to E. coli S10 and rat S20 ribosomal proteins

1084;Putative protein of unknown function; predicted to be a member of the ovarian tumor-like (OTU) superfamily of cysteine proteases; green fluorescent protein (GFP)-fusion protein localizes to the cytoplasm

1085;Putative protein of unknown function, has some homology to Ugplp, which encodes UDP-glucose pyrophosphorylase

1086;5-phospho-ribosyl-1(alpha)-pyrophosphate synthetase, involved in nucleotide, histidine, and tryptophan biosynthesis; one of a five related enzymes, which are active as heteromultimeric complexes

1087;Putative protein of unknown function, contains a zinc finger region and has homology to human BRAP2, which is a cytoplasmic protein that binds nuclear localization sequences

1088;Signal transducing kinase of the PAK (p21-activated kinase) family, involved in pheromone response and pseudohyphal/invasive growth pathways, activated by Cdc42p; binds Ste4p at a GBB motif present in noncatalytic domains of PAK kinases

1089;Ceramide synthase component, involved in synthesis of ceramide from C26(acyl)-coenzyme A and dihydrosphingosine or phytosphingosine, functionally equivalent to Lac1p

1090;Subunit of the endosomal Vps27p-Hselp complex required for sorting of ubiquitinated membrane proteins into intralumenal vesicles prior to vacuolar degradation, as well as for recycling of Golgi proteins and formation of lumenal membranes

1091;Mitochondrial carrier protein involved in the accumulation of CoA in the mitochondrial matrix; homolog of human Graves disease protein; does not encode an isozyme of Leu4p, as first hypothesized

1092;Putative protein of unknown function, localizes to the mitochondria

1093;Protein of the nuclear envelope required for the spherical shape of the nucleus; required for normal sporulation

1094;GTP-binding alpha subunit of the heterotrimeric G protein that couples to pheromone receptors; negatively regulates the mating pathway by sequestering G(beta)gamma and by triggering an adaptive response; activates the pathway via Scp160p

1095;Essential protein of the mitochondrial intermembrane space, forms a complex with Tim9p (TIM10 complex) that mediates insertion of hydrophobic proteins at the inner membrane, has homology to Mrs5p, which is also involved in this process

1096;Transcription factor, activated by proteolytic processing in response to signals from the SPS sensor system for external amino acids; activates transcription of amino acid permease genes

1097;Lanosterol 14-alpha-demethylase, catalyzes the C-14 demethylation of lanosterol to form 4,4"-dimethyl cholesta-8,14,24-triene-3-beta-ol in the ergosterol biosynthesis pathway; member of the cytochrome P450 family

1098;Manganese-containing superoxide dismutase; protects cells against oxygen toxicity

1099;Putative protein of unknown function; not an essential gene

1100;Probable mitochondrial seryl-tRNA synthetase, mutant displays increased invasive and pseudohyphal growth

1101;Subunit of the N-terminal acetyltransferase NatA (Nat1p, Ard1p, Nat5p); N-terminally acetylates many proteins, which influences multiple processes such as the cell cycle, heat-shock resistance, mating, sporulation, and telomeric silencing

1102;Meiosis-specific protein, involved in maintaining sister chromatid cohesion during meiosis I as well as promoting proper attachment of kinetochores to the spindle during meiosis I and meiosis II

1103;Putative protein of unknown function, has similarity to proline-tRNA ligase; YHR020W is an essential gene

1104;Larger subunit of the mitochondrial processing protease, essential processing enzyme that cleaves the N-terminal targeting sequences from mitochondrially imported proteins

1105;Homoserine kinase, conserved protein required for threonine biosynthesis; expression is regulated by the GCN4-mediated general amino acid control pathway

1106;Non-ATPase base subunit of the 19S regulatory particle of the 26S proteasome; may participate in the recognition of several ligands of the proteasome; contains a leucine-rich repeat (LRR) domain, a site for protein-protein interactions1107;Dipeptidyl aminopeptidase, synthesized as a glycosylated precursor; localizes to the vacuolar membrane; similar to Ste13p

1108;Protein of unknown function that is a member of the PhzF superfamily, although unlike its bacterial homolog, is most likely not involved in phenazine production; possibly involved in a membrane regulation metabolic pathway

1109;Protein of unknown function that is a member of the PhzF superfamily, although unlike its bacterial homolog, is most likely not involved in phenazine production; possibly involved in a membrane regulation metabolic pathway

1110;Serine/threonine MAP kinase involved in regulating the maintenance of cell wall integrity and progression through the cell cycle; regulated by the PKC1-mediated signaling pathway

1111;DNA helicase involved in rDNA replication and Ty1 transposition; structurally and functionally related to Piflp

1112;Protein of unresolved function; may function in protein folding and/or rRNA processing, interacts with a chaperone (Hsp82p), two chromatin remodeling factors (Rvb1p, Rvb2p) and two rRNA processing factors (Rrp43p, Nop58p)

1113;Essential nuclear envelope integral membrane protein identified as a suppressor of a cold-sensitive mutant of CRM1, a karyopherin; homologous to and interacts with Brr6p, which is a nuclear envelope protein involved in nuclear export

1114;Mitochondrial ribosome recycling factor, essential for mitochondrial protein synthesis and for the maintenance of the respiratory function of mitochondria

1115;Protein of unknown function, green fluorescent protein (GFP)-fusion protein localizes to the endoplasmic reticulum; msc7 mutants are defective in directing meiotic recombination events to homologous chromatids

1116;NADP-cytochrome P450 reductase; involved in ergosterol biosynthesis; associated and coordinately regulated with Erg11p

1117;Putative protein of unknown function; green fluorescent protein (GFP)-fusion protein localizes to the endoplasmic reticulum

1118;Inositol monophosphatase, involved in biosynthesis of inositol and in phosphoinositide second messenger signaling; INM1 expression increases in the presence of inositol and decreases upon exposure to antibipolar drugs lithium and valproate

1119;Arginine/alanine aminopeptidase, overproduction stimulates glycogen accumulation

1120;Serine hydrolase that localizes to both the nucleus and cytoplasm; sequence is similar to Fsh2p and Fsh3p

1121;Divalent metal ion transporter involved in manganese homeostasis; has broad specificity for di-valent and tri-valent metals; post-translationally regulated by levels of metal ions; member of the Nramp family of metal transport proteins

1122;Subunit VI of cytochrome c oxidase, which is the terminal member of the mitochondrial inner membrane electron transport chain; expression is regulated by oxygen levels

1123;Peptidyl-prolyl cis-trans isomerase (cyclophilin), catalyzes the cis-trans isomerization of peptide bonds N-terminal to proline residues; has a potential role in the secretory pathway

1124;Subunit of the RNA polymerase II mediator complex; associates with core polymerase subunits to form the RNA polymerase II holoenzyme; essential for transcriptional regulation

1125;Protein of unknown function, required for survival upon exposure to K1 killer toxin

1126;Constituent of 66S pre-ribosomal particles, required for ribosomal large subunit maturation; functionally redundant with Ssf2p; member of the Brix family

1127;Mitochondrial 3-hydroxyacyl-thioester dehydratase involved in fatty acid biosynthesis, required for respiratory growth and for normal mitochondrial morphology

1128;Deoxyhypusine synthase, catalyzes formation of deoxyhypusine, the first step in hypusine biosynthesis; triggers posttranslational hypusination of translation elongation factor eIF-5A and regulates its intracellular levels; tetrameric

1129;Protein involved in rRNA processing; component of the exosome 3->5 exonucle ase complex with Rrp41p, Rrp42p, Rrp43p and Dis3p

1130;tRNA(m(1)G37)methyltransferase, methylates a tRNA base adjacent to the anticodon that has a role in prevention of frameshifting; highly conserved across Archaea, Bacteria, and Eukarya

1131;Constituent of small nucleolar ribonucleoprotein particles containing H/ACA-type snoRNAs, which are required for pseudouridylation and processing of pre-18S rRNA

1132;Member of an oxysterol-binding protein family with seven members in S. cerevisiae; family members have overlapping, redundant functions in sterol metabolism and collectively perform a function essential for viability

1133;Glutamine-dependent NAD(+) synthetase, essential for the formation of NAD(+) from nicotinic acid adenine dinucleotide

1134;Serine-threonine kinase and endoribonuclease; transmembrane protein that initiates the unfolded protein response signal by regulating synthesis of Haclp through HAC1 mRNA splicing

1135;Nucleolar protein involved in the assembly of the large ribosomal subunit; constituent of 66S pre-ribosomal particles; contains a sigma(70)-like motif, which is thought to bind RNA

1136;Subunit of the NuA4 histone acetyltransferase complex that acetylates histone H4 and H2A; has similarity to the human tumor suppressor ING1

1137;Mitochondrial arginyl-tRNA synthetase

1138;Member of the Sec24p family; forms a complex, with Sec23p, that is involved in sorting of Pmalp into COPII vesicles; peripheral ER membrane protein; potential Cdc28p substrate

1139;Hypothetical protein

1140;Protein kinase of the PAK/Ste20 kinase family, required for cell integrity possibly through regulating 1,6-beta-glucan levels in the wall; physically interacts with Cdc31p (centrin), which is a component of the spindle pole body

1141;Protein involved in the transport of cell wall components from the Golgi to the cell surface; similar in structure and functionally redundant with Sbe2p; involved in bud growth

1142;Aldose reductase involved in methylglyoxal, d-xylose and arabinose metabolism; stress induced (osmotic, ionic, oxidative, heat shock, starvation and heavy metals); regulated by the HOG pathway

1143;Golgi-localized protein with homology to gamma-adaptin, interacts with and regulates Arf1p and Arf2p in a GTP-dependent manner in order to facilitate traffic through the late Golgi

1144;Protein with similarity to Emp24p and Erv25p, member of the p24 family involved in ER to Golgi transport

1145;Protein that activates Urmlp before its conjugation to proteins (urmylation); one target is the thioredoxin peroxidase Ahplp, suggesting a role of urmylation in the oxidative stress response

1146;Hypothetical protein

1147;Cytoplasmic aspartyl aminopeptidase; cleaves unblocked N-terminal acidic amino acid residues from peptide substrates; forms a 12 subunit homo-oligomeric complex; inhibited by EDTA and 1,10-phenanthroline; M18 metalloprotease family member

1148;Protein involved in regulating spindle position and orientation, functionally redundant with Dma2p; homolog of S. pombe Dma1 and H. sapiens Chfr

1149;Mitochondrial outer membrane protein with similarity to Tom70p; probable minor component of the TOM (translocase of outer membrane) complex responsible for recognition and import of mitochondrially directed proteins

1150;Histone methyltransferase, subunit of the COMPASS (Set1C) complex which methylates histone H3 on lysine 4; required in transcriptional silencing near telomeres and at the silent mating type loci; contains a SET domain

1151;DNA-binding protein of the mitochondria involved in repair of mitochondrial DNA, has ATPase activity and binds to DNA mismatches; has homology to E. coli MutS; transcription is induced during meiosis

1152;Protein containing an Lsm domain and an AD domain; may bind RNA and have a role in RNA processing

1153;Protein required for cell viability

1154;Meiosis-specific transcription factor required for exit from pachytene and for full meiotic recombination; activates middle sporulation genes; competes with Sum1p for binding to promoters containing middle sporulation elements (MSE)

1155;(H)igh copy (S)uppressor of (N)34 dominant negative allele of SEC4. Suppression is very specific to this allele. It has no affect on the analogous YPT1 allele. No homology or known function.

1156;Actin-related protein of the dynactin complex; required for spindle orientation and nuclear migration; putative ortholog of mammalian centractin

1157;Hypothetical protein

1158;Palmitoylated, plasma membrane-bound casein kinase I isoform; shares redundant functions with Yck2p in morphogenesis, proper septin assembly, endocytic trafficking;; provides an essential function overlapping with that of Yck2p

1159;Protein required for spore wall maturation; expressed during sporulation; may be a component of the spore wall

1160;Protein of unknown function, involved in chitin biosynthesis by regulating Chs3p export from the ER

1161;RNA polymerase subunit, found in RNA polymerase complexes I, II, and III 1162;Deaminase required for dCTP and dTTP synthesis; expression is cell cycle regulated

1163;Mitochondrial ribosomal protein of the large subunit

1164;Component of the SSU processome, which is required for pre-18S rRNA processing, essential protein that interacts with Mpp10p and mediates interactions of Imp4p and Mpp10p with U3 snoRNA

1165;Hypothetical protein

1166;Nucleolar protein of unknown function, positive regulator of exit from mitosis; involved in regulating the release of Cdc14p from the nucleolus in early anaphase; proposed to play similar role in meiosis

1167;Non-essential component of US snRNP; nuclear protein; physically interacts with Irr1p of cohesin complex; may link together proteins involved in chromosome segregation, RNA splicing and DNA replication

1168;Protein involved in early stages of meiotic recombination; required for meiotic crossing over; forms a complex with Rec102p and Spo11p necessary during the initiation of recombination

1169;Part of a two-member peroxin family (Pex18p and Pex21p) specifically required for peroxisomal targeting of the Pex7p peroxisomal signal recognition factor and PTS2 peroxisomal matrix proteins

1170;Protein involved in clathrin cage assembly; binds Panlp and clathrin; homologous to Yap1802p, member of the AP180 protein family

1171;Hypothetical protein

1172;Essential tripartite DNA replication factor with single-stranded DNA-dependent ATPase, ATP-dependent nuclease, and helicasc activities; required for Okazaki fragment processing; involved in DNA repair pathways; potential Cdc28p substrate

1173;Component of the U4/U6-U5 snRNP complex, involved in the second catalytic step of splicing

1174;Subunit of the anaphase-promoting complex/cyclosome (APC/C), which is a ubiquitin-protein ligase required for degradation of anaphase inhibitors, including mitotic-cyclins, during the metaphase/anaphase transition

1175;Protein involved in nuclear export of the large ribosomal subunit; acts as a Crm1p-dependent adapter protein for export of nascent ribosomal subunits through the nuclear pore complex

1176;Component of the microtubule-nucleating Tub4p (gamma-tubulin) complex; interacts with Spc110p at the spindle pole body (SPB) inner plaque and with Spc72p at the SPB outer plaque

1177;Activator of multidrug resistance genes, forms heterodimer with Pdr1p; contains a Zn(II)2Cys6 zinc finger domain that interacts with a PDRE (pleotropic drug resistance element) in vitro; binds Sin3p in a two-hybrid assay

1178;Integral membrane protein of the early Golgi apparatus, may function to promote retention of proteins in the early Golgi compartment; mutation affects protein N-glycosylation and cell wall integrity

1179;Protein of unknown function; green fluorescent protein (GFP)-fusion protein localizes to the cell periphery and cytoplasm

1180;6-phosphogluconate dehydrogenase (decarboxylating), catalyzes an NADPH regenerating reaction in the pentose phosphate pathway; required for growth on D-glucono-delta-lactone and adaptation to oxidative stress

1181;Protein involved in the control of meiotic nuclear division and coordination of meiosis with spore formation; transcription is induced midway through meiosis

1182;Subunit of TORC1, a rapamycin-sensitive complex involved in growth control that contains Tor1p or Tor2p, Lst8p and Tco89p; contains four HEAT repeats and seven WD-40 repeats; may act as a scaffold protein to couple TOR and its effectors

1183;Subunit of the Elp4p-Iki1p-Elp6p-subcomplex of RNA polymerase II elongator complex, which is a histone acetyltransferase; iki1 mutations confer resistance to the K. lactis toxin zymocin

1184;Transmembrane protein subunit of the glycosylphosphatidylinositol transamidase complex that adds GPIs to newly synthesized proteins; human PIG-Tp homolog

1185;One of two (see also PTH2) mitochondrially-localized peptidyl-tRNA hydrolases; dispensable for cell growth and for mitochondrial respiration

1186;Farnesyl-diphosphate farnesyl transferase (squalene synthase), joins two farnesyl pyrophosphate moieties to form squalene in the sterol biosynthesis pathway

1187;Subunit of a complex with Ctf18p that shares some subunits with Replication Factor C and is required for sister chromatid cohesion

1188;Mitochondrial inner membrane protein with similarity to Mdm32p, required for normal mitochondrial morphology and inheritance; interacts genetically with MMM1, MDM10, MDM12, and MDM34

1189;Nuclear envelope protein that interacts with the vacuolar membrane protein Vac8p to promote formation of nucleus-vacuole junctions during piecemeal microautophagy of the nucleus (PMN)

1190;Nucleolar protein, component of the small subunit (SSU) processome containing the U3 snoRNA that is involved in processing of pre-18S rRNA

1191;The authentic, non-tagged protein was localized to the mitochondria

1192;Non-ATPase base subunit of the 19S regulatory particle (RP) of the 26S proteasome; N-terminus plays a role in maintaining the structural integrity of the RP; binds selectively to polyubiquitin chains; homolog of the mammalian S5a protein

1193;Hypothetical protein

1194;Alpha mannosidase-like protein of the endoplasmic reticulum required for degradation of glycoproteins but not for processing of N-linked oligosaccharides

1195;Protein kinase that regulates signal transduction activity and G1 progression, controls cAPK activity, required for nitrogen activation of the FGM pathway, involved in life span regulation, homologous to mammalian Akt/PKB

1196;Nuclear response regulator and transcription factor, part of a branched two-component signaling system; required for optimal induction of heat-shock genes in response to oxidative stress; involved in osmoregulation

1197;Zinc-finger protein of unknown function, contains one canonical and two unusual fingers in unusual arrangements; deletion enhances replication of positive-strand RNA virus

1198;Mitochondrial branched-chain amino acid aminotransferase, homolog of murine ECA39; highly expressed during logarithmic phase and repressed during stationary phase

1199;Putative S-adenosylmethionine-dependent methyltransferase of the seven beta-strand family

1200;Putative protein with similarity to the allantoate permease (Dal5p) subfamily of the major facilitator superfamily; mRNA expression is elevated by sulfur limitation; YIL166C is a non-essential gene

1201;3-ketoacyl-CoA thiolase with broad chain length specificity, cleaves 3-ketoacyl-CoA into acyl-CoA and acetyl-CoA during beta-oxidation of fatty acids

1202;Formin, nucleates the formation of linear actin filaments, involved in cell processes such as budding and mitotic spindle orientation which require the formation of polarized actin cables, functionally redundant with BNI1

1203;Putative protein of unknown function; green fluorescent protein (GFP)-fusion protein localizes to the vacuole

1204;Transcriptional activator involved in maintenance of ion homeostasis and protection against DNA damage caused by bleomycin and other oxidants, contains a C-terminal leucine-rich repeat

1205;Hypothetical protein

1206;Hypothetical protein

1207;Essential, chromatin-associated protein involved in the initiation of DNA replication; required for the association of the MCM2-7 complex with replication origins

1208;Non-essential protein of unknown function

1209;Component of the holoenzyme form of RNA polymerase transcription factor TFIIH, has DNA-dependent ATPase/helicase activity and is required, with Rad3p, for unwinding promoter DNA; involved in DNA repair; homolog of human ERCC3

1210;Subunit beta of the cytosolic chaperonin Cct ring complex, related to Tcplp, required for the assembly of actin and tubulins in vivo

1211;Integral plasma membrane protein required for axial budding in haploid cells, localizes to the incipient bud site and bud neck; glycosylated by Pmt4p; potential Cdc28p substrate

1212;Minor isoform of tropomyosin, binds to and stabilizes actin cables and filaments, which direct polarized cell growth and the distribution of several organelles; appears to have distinct and also overlapping functions with Tpm1p

1213;Putative metalloprotease

1214;Protein of unknown function, major constituent of the mitochondrial outer membrane; located on the outer (cytosolic) face of the outer membrane

1215;Cytoplasmic protein of unknown function; identified as a high-copy suppressor of the synthetic lethality of a sis2 sit4 double mutant, suggesting a role in G1/S phase progression; similar to Mlf3p

1216;Protein required for transport of flavin adenine dinucleotide (FAD) from mitochondria, where it is synthesized from riboflavin, to the cytosol

1217;Proposed transcriptional activator, member of the Gal4p family of zinc cluster proteins

1218;DNA repair and TFIIH regulator, required for both nucleotide excision repair (NER) and RNA polymerase II (RNAP II) transcription; possible role in assembly of a multiprotein complex(es) required for NER and RNAP II transcription

1219;Putative protein of unknown function; green fluorescent protein (GFP)-fusion protein localizes to the nucleolus

1220;Component of the mitochondrial alpha-ketoglutarate dehydrogenase complex, which catalyzes a key step in the tricarboxylic acid (TCA) cycle, the oxidative decarboxylation of alpha-ketoglutarate to form succinyl-CoA

1221;NADPH-dependent 1-acyl dihydroxyacetone phosphate reductase found in lipid particles and ER; involved in phosphatidic acid biosynthesis and required for spore germination; capable of metabolizing mammalian steroid hormones

1222;Protein of the SUN family (Simlp, Uthlp, Nca3p, Sun4p) that may participate in DNA replication, promoter contains SCB regulation box at -300 bp indicating that expression may be cell cycle-regulated

1223;Putative transcriptional activator that promotes recovery from pheromone induced arrest; inhibits both alpha-factor induced G1 arrest and repression of CLN1 and CLN2 via SCB/MCB promoter elements; potential Cdc28p substrate; SBF regulated

1224;Putative transcriptional regulator; overexpression suppresses the heat shock sensitivity of wild-type RAS2 overexpression and also suppresses the cell lysis defect of an mpk1 mutation

1225;Non-essential small GTPase of the Rho/Rac subfamily of Ras-like proteins involved in the establishment of cell polarity; GTPase activity positively regulated by the GTPase activating protein (GAP) Rgdlp

1226;Stress-inducible dual-specificity MAP kinase phosphatase, negatively regulates Slt2p MAP kinase by direct dephosphorylation, diffuse localization under normal conditions shifts to punctate localization after heat shock

1227;subunit of the Set3 complex, which is a meiotic-specific repressor of sporulation specific genes that contains deacetylase activity; potential Cdc28p substrate

1228;Component of the Sec23p-Sec24p heterodimeric complex of the COPII vesicle coat; involved in ER to Golgi transport, cargo selection and autophagy; required for the binding of the Sec13 complex to ER membranes; homologous to Lst1p and Lss1p

1229;6-phosphofructo-2-kinase, inhibited by phosphoenolpyruvate and sn-glycerol 3-phosphate, has negligible fructose-2,6-bisphosphatase activity, transcriptional regulation involves protein kinase A

1230;Phosphoinositide PI4,5P(2) binding protein, forms a complex with Slm2p; acts downstream of Mss4p in a pathway regulating actin cytoskeleton organization in response to stress; subunit of and phosphorylated by the TORC2 complex

1231;Essential nuclear protein, required for accumulation of box H/ACA snoRNAs and for rRNA processing; interacts with Naflp

1232;Hypothetical protein

1233;Protein serine/threonine kinase; regulates the organization and function of the actin cytoskeleton through the phosphorylation of the Pan1p-Sla1p-End3p protein complex

1234;Homo-isocitrate dehydrogenase, an NAD-linked mitochondrial enzyme required for the fourth step in the biosynthesis of lysine, in which homo-isocitrate is oxidatively decarboxylated to alpha-ketoadipate

1235;Mitochondrial ribosomal protein of the small subunit

1236;Protein required for cell viability

1237;Integral ER membrane protein with type-III transmembrane domains; mutations cause defects in cortical ER morphology in both the mother and daughter cells

1238;Putative transporter, member of a family of seven S. cerevisiae genes (AVT1-7) related to vesicular GABA-glycine transporters

1239;Hypothetical protein

1240;Putative mannosyltransferase involved in protein glycosylation; member of the KRE2/MNT1 mannosyltransferase family

1241;Component of the Rpd3p/Sin3p deacetylase complex required for its structural integrity and catalytic activity, involved in transcriptional silencing and required for sporulation; cells defective in SDS3 display pleiotropic phenotypes

1242;Homolog to human PPCS

1243;RING finger protein that interacts with the arginine methyltransferase Hmt1p to regulate methylation of Np13p, which modulates Np13p function in mRNA processing and export; has similarity to Air2p

1244;Threonyl-tRNA synthetase, essential cytoplasmic protein

1245;Subunit of the 26S proteasome, substrate of the N-acetyltransferase Nat1p

1246;3-phosphoglycerate dehydrogenase, catalyzes the first step in serine and glycine biosynthesis; isozyme of Ser3p

1247;Meiosis-specific DNA binding protein that displays Red1p dependent localization to the unsynapsed axial-lateral elements of the synaptonemal complex; required for homologous chromosome synapsis and chiasma formation

1248;Acidic protein of the mitochondrial matrix involved in oxidative phosphorylation; related to the human complement receptor gC1q-R

1249;Hypothetical protein

1250;Mitochondrial outer membrane protein involved in membrane fission, required for localization of Dnm1p and Mdvlp during mitochondrial division

1251;Protein of unknown function involved in nuclear processes of the Ran-GTPase cycle; involved in nuclear protein export; contains Ran Binding Domain and FxFG repeats; interacts with Srmlp, GTP-Gsp1p, Rna1p and Crmlp; is not essential

1252;Subunit of the ARP2/3 complex, which is required for the motility and integrity of cortical actin patches

1253;Component of U1 snRNP required for mRNA splicing via spliceosome; may interact with poly(A) polymerase to regulate polyadenylation; homolog of human U1 70K protein

1254;Putative protein of unknown function; green fluorescent protein (GFP)-fusion protein localizes to the cytoplasm and nucleus

1255;Constitutively expressed isoform of DL-glycerol-3-phosphatase; involved in glycerol biosynthesis, induced in response to both anaerobic and, along with the Hor2p/Gpp2p isoform, osmotic stress

1256;Mitochondrial protein involved in maintenance of the mitochondrial genome

1257;Pho85p cyclin of the Pho80p subfamily, forms a functional kinase complex with Pho85p which phosphorylates Mmr1p and is regulated by Pho81p; involved in glycogen metabolism, expression is cell-cycle regulated

1258;Protein of unknown function, involved in filamentous growth

1259;Protein involved in the retrograde transport from the Golgi complex to the ER and the endosomal membrane traffic; putative aminophospholipid translocase; member of the highly conserved Drs2 family of P-type ATPases

1260;Plasma membrane protein of unknown function; truncation and overexpression suppresses lethality of G-alpha protein deficiency

1261;F-box protein containing five copies of the WD40 motif, controls cell cycle function, sulfur metabolism, and methionine biosynthesis as part of the ubiquitin ligase complex; interacts with and regulates Met4p, localizes within the nucleus

1262;Putative type-1 protein phosphatase targeting subunit that tethers Glc7p type-1 protein phosphatase to Gsy2p glycogen synthase

1263;ADP-ribosylation factor (ARF) GTPase activating protein (GAP) effector, involved in Trans-Golgi-Network (TGN) transport; contains C2C2H2 cysteine/histidine motif

1264;Hypothetical protein

1265;Golgi vesicle protein of unknown function; localizes to both early and late Golgi vesicles

1266;Protein involved in nucleocytoplasmic transport of mRNA

1267;Hypothetical protein

1268;Subunit of the CCR4-NOT complex, which is a global transcriptional regulator with roles in transcription initiation and elongation and in mRNA degradation

1269;Pheromone-regulated protein, predicted to have 4 transmembrane segments and a coiled coil domain; regulated by Ste12p

1270;Alpha catalytic subunit of casein kinase 2, a Ser/Thr protein kinase with roles in cell growth and proliferation; the holoenzyme also contains CKA2, CKB1 and CKB2, the many substrates include transcription factors and all RNA polymerases

1271;Beta subunit of the capping protein (CP) heterodimer (Caplp and Cap2p) which binds to the barbed ends of actin filaments preventing further polymerization; localized predominantly to cortical actin patches

1272;Regulatory subunit of the cyclic AMP-dependent protein kinase (PKA), a component of a signaling pathway that controls a variety of cellular processes, including metabolism, cell cycle, stress response, stationary phase, and sporulation

1273;Protein of unknown function; null mutant shows K1 killer toxin resistance

1274;Hypothetical protein

1275;Yeast KE4, yeast ortholog of the mouse KE4

1276;Peripheral mitochondrial membrane protein involved in mitochondrial protein import, tethers essential chaperone Ssclp to the translocon channel at the matrix side of the inner membrane

1277;RNA polymerase II third largest subunit B44, part of central core; similar to prokaryotic alpha subunit

1278;Protein required for pre-rRNA processing and 40S ribosomal subunit assembly1279;Protein involved in proteasome-dependent catabolite degradation of fructose-1,6-bisphosphatase (FBPase); localized to the nucleus and the cytoplasm

1280;Protein of unknown function proposed to be involved in nuclear pore complex biogenesis and maintenance as well as protein folding; has similarity to the mammalian BAG- protein

1281;Aspartyl protease secreted into the periplasmic space of mating type a cells, helps cells find mating partners, cleaves and inactivates alpha factor allowing cells to recover from alpha-factor-induced cell cycle arrest

1282:Cell wall mannoprotein of the Srp1p/Tip1p family of serine-alanine-rich proteins; expressed under anaerobic conditions and required for anaerobic growth

1283;Nuclear thiol peroxidase which functions as an allcyl-hydroperoxide reductase during post-diauxic growth

1284;Ubiquitin-like protein with only weak sequence similarity to ubiquitin; depends on the El-like activating enzyme Uba4p; molecular function of the Urmlp pathway is unknown, but it is required for normal growth, particularly at high temperature

1285;Protein with similarity to the p27 subunit of mammalian proteasome modulator; not essential; interacts with Rpn4p

1286;Pdilp (protein disulfide isomerase)-related protein involved in endoplasmic reticulum retention of resident ER proteins

1287;Phosphatidylinositol 4,5-bisphosphate 5-phosphatase, synaptojanin-like protein with an N-terminal Sac1 domain, plays a role in phosphatidylinositol 4,5-bisphosphate homeostasis and in endocytosis; null mutation confers cold-tolerant growth

1288;Member of the DEAH family of helicases, functions in an error-free DNA damage bypass pathway that involves homologous recombination, mutations confer a mutator phenotype

1289;Cytosolic J-domain-containing protein, required for peroxisomal protein import and involved in peroxisome assembly, homologous to E. coli DnaJ

1290;Part of actin cytoskeleton-regulatory complex Panlp-Slalp-End3p, associates with actin patches on the cell cortex; promotes protein-protein interactions essential for endocytosis; previously thought to be a subunit of poly(A) ribonuclease

1291;Putative protein of unknown function; green fluorescent protein (GFP)-fusion protein localizes to the cytoplasm; YIR007W is a non-essential gene

1292;Subunit of DNA primase, which is required for DNA synthesis and double-strand break repair

1293;U2B component of U2 snRNP, involved in splicing, binds the U2 snRNA stem-loop IV in vitro; does not contain the conserved C-terminal RNA binding domain found in other family members

1294;Essential component of the MIND kinetochore complex (Mtw1p Including Nnflp-Ns11p-Dsn1p) which joins kinetochore subunits contacting DNA to those contacting microtubules; important for chromosome segregation

1295;Protein containing GATA family zinc finger motifs

1296;18kDa catalytic subunit of the Signal Peptidase Complex (SPC; Spc1p, Spc2p, Spc3p, and Sec11p) which cleaves the signal sequence of proteins targeted to the endoplasmic reticulum

1297;Allantoin permease; expression sensitive to nitrogen catabolite repression and induced by allophanate, an intermediate in allantoin degradation

1298;Allantoicase, converts allantoate to urea and ureidoglycolate in the second step of allantoin degradation; expression sensitive to nitrogen catabolite repression and induced by allophanate, an intermediate in allantoin degradation

1299;Malate synthase, role in allantoin degradation unknown; expression sensitive to nitrogen catabolite repression and induced by allophanate, an intermediate in allantoin degradation

1300;Ureidoglycolate hydrolase, converts ureidoglycolate to glyoxylate and urea in the third step of allantoin degradation; expression sensitive to nitrogen catabolite repression

1301;ER membrane protein involved, with its homolog Spt23p, in regulation of OLE1 transcription; inactive ER form dimerizes and one subunit is then activated by ubiquitin/proteasome-dependent processing followed by nuclear targeting

1302;Saccharopine dehydrogenase (NAD+, L-lysine-forming), catalyzes the conversion of saccharopine to L-lysine, which is the final step in the lysine biosynthesis pathway

1303;Hypothetical protein

1304;Thiol peroxidase that functions as a hydroperoxide receptor to sense intracellular hydroperoxide levels and transduce a redox signal to the Yap1p transcription factor

1305;Putative GPI-anchored aspartic protease

1306;Putative protein of unknown function; YIR042C is a non-essential gene

1307;Putative protein of unknown function; YIR042C is a non-essential gene

1308;Hypothetical protein

1309;Plasma membrane transporter that transports tetra- and pentapeptides and glutathione; member of the OPT family

1310;RING-finger peroxin, peroxisomal membrane protein with a C-terminal zinc-binding RING domain, forms putative translocation subcomplex with Pex10p and Pex12p which functions in peroxisomal matrix protein import

1311;Mitochondrial protein that interacts with the 5'-untranslated region of the COB mRNA and has a role in its stability and translation; found in a complex at the inner membrane along with Pet309p

1312;Hypothetical protein

1313;F-box protein involved in recycling plasma membrane proteins internalized by endocytosis; localized to sites of polarized growth

1314;Subunit of the SF3a splicing factor complex, required for spliceosome assembly

1315;Hypothetical protein

1316;Ubiquitin-specific protease present in the nucleus and cytoplasm that cleaves ubiquitin from ubiquitinated proteins

1317;Hypothetical protein

1318;Protein kinase that regulates the G2/M transition by inhibition of Cdc28p kinase activity; localizes to the nucleus and to the daughter side of the mother-bud neck; homolog of S. pombe Weelp; potential Cdc28p substrate

1319;Alpha-1,2-mannosyltransferase, responsible for addition of the second alpha-1,2-linked mannose of the branches on the mannan backbone of oligosaccharides, localizes to an early Golgi compartment

1320;Hypothetical protein

1321;Subunit of a Golgi mannosyltransferase complex that also contains Anp1p, Mnn9p, Mnn10p, and Hoc1p, and mediates elongation of the polysaccharide mannan backbone; has homology to Mnn10p

1322;Molecular chaperone, required for the assembly of alpha and beta subunits into the P1 sector of mitochondrial F1F0 ATP synthase

1323;Subunit of heterohexameric prefoldin, which binds cytosolic chaperonin and transfers target proteins to it; involved in the biogenesis of actin and of alpha- and gamma-tubulin

1324;One of 11 subunits of the SWI/SNF chromatin remodeling complex that regulates transcription by remodeling chromosomes; required for transcription of many genes, including ADH1, ADH2, GAL1, HO, INO1 and SUC2

1325;Glycoprotein involved in cell wall beta-glucan assembly; null mutation leads to severe growth defects, aberrant multibudded morphology and mating defects

1326;Subunit of heterotrimeric Replication Factor A (RF-A), which is a highly conserved single-stranded DNA binding protein involved in DNA replication, repair, and recombination

1327;Vacuolar carboxypeptidase yscS; expression is induced under low-nitrogen conditions

1328;Farnesyl pyrophosphate synthetase, has both dimethylallyltranstransferase and geranyltranstransferase activities; catalyzes the formation of C15 farnesyl pyrophosphate units for isoprenoid and sterol biosynthesis

1329;Subunit 8 of ubiquinol cytochrome-c reductase complex, which is a component of the mitochondrial inner membrane electron transport chain; oriented facing the intermembrane space; expression is regulated by Abflp and Cpflp

1330;Putative protein kinase; overexpression increases sodium and lithium tolerance, whereas gene disruption increases cation and low pH sensitivity and impairs potassium uptake, suggesting a role in regulation of Trk1p and/or Trk2p transporters

1331;Subunit of cytoplasmic cAMP-dependent protein kinase, which contains redundant catalytic subunits Tpk1p, Tpk2p, and Tpk3p and regulatory subunit Bcy1p; promotes vegetative growth in response to nutrients; inhibits filamentous growth

1332;The authentic, non-tagged protein was localized to the mitochondria 1333;Mannose-containing glycoprotein constituent of the cell wall; member of the PIR (proteins with internal repeats) family

1334;Fructose-2,6-bisphosphatase, required for glucose metabolism

1335;Endosomal protein that is a subunit of the membrane-associated retromer complex essential for endosome-to-Golgi retrograde transport; forms a subcomplex with Vps26p and Vps29p that selects cargo proteins for endosome-to-Golgi retrieval1336;Hypothetical protein; has similarity to F-box proteins

1337;RNA polymerase I subunit A34.5

1338;Hypothetical protein

1339;Protein involved in modulation of Ime2p activity during meiosis, appears to act indirectly to promote Ime2p-mediated late meiotic functions; found in growing cells and degraded during sporulation

1340;Hypothetical protein

1341;Essential constituent of the mitochondrial inner membrane presequence translocase; interacts with Pam18p to recruit the presequence translocase-associated motor (PAM complex) and also required for protein sorting during import

1342;Serine-threonine protein kinase that is part of a glucose-sensing system involved in growth control in response to glucose availability; translocates from the cytoplasm to the nucleus and phosphorylates Pop2p in response to a glucose signal

1343;RNA polymerase II subunit B32; forms two subunit dissociable complex with Rpb7p; dispensable under some environmental conditions; involved in export of mRNA to cytoplasm under stress conditions 1344;Translation initiation factor eIF4A, identical to Tif1p; DEA(D/H)-box RNA

helicase that couples ATPase activity to RNA binding and unwinding; forms a dumbbell structure of two compact domains connected by a linker; interacts with eIF4G1345;Long-chain base-1-phosphate phosphatase, regulates ceramide and long-chain base phosphates levels, involved in incorporation of exogenous long chain bases in sphingolipids

1346;ORF, Uncharacterized

1347;Mitochondrial iron transporter of the mitochondrial carrier family (MCF), very similar to and functionally redundant with Mrs4p; functions under low-iron conditions; may transport other cations in addition to iron

1348;MAP kinase kinase that plays a pivotal role in the osmosensing signal-transduction pathway, activated under severe osmotic stress

1349;Identified by homology to Ashbya gossypii

1350;Nit protein, one of two proteins in S. cerevisiae with similarity to the Nit domain of NitFhit from fly and worm and to the mouse and human Nit protein which interacts with the Fhit tumor suppressor; nitrilase superfamily member

1351;Lsm (Like Sm) protein; forms heteroheptameric complex (with Lsm2p, Lsm3p, Lsm4p, Lsm5p, Lsm6p, and Lsm7p) involved in degradation of cytoplasmic mRNAs

1352;Hypothetical protein

1353;Endoplasmic reticulum (ER) resident protein required for ER exit of the high-affinity phosphate transporter Pho84p, specifically required for packaging of Pho84p into COPII vesicles

1354;Protein that functions with Nca2p to regulate mitochondrial expression of subunits 6 (Atp6p) and 8 (Atp8p) of the Fo-F1 ATP synthase; member of the SUN family

1355;Nucleosome assembly factor, involved in chromatin assembly after DNA replication, anti-silencing protein that causes derepression of silent loci when overexpressed

1356;Peripheral protein of the cytosolic face of the mitochondrial outer membrane, required for mitochondrial fission; interacts with Fis1p and with the dynamin-related GTPase Dnm1p; contains WD repeats

1357;Serine/threonine protein kinase involved in activation of meiosis, associates with Imelp and mediates its stability, activates Ndt80p; IME2 expression is positively regulated by Ime1p

1358;Constituent of the mitochondrial import motor associated with the presequence translocase, along with Ssclp, Tim44p, Mge1p, and Pam18p; has similarity to J-domain containing proteins

1359;Hypothetical protein

1360;Phosphatidylinositol 4-kinase that binds Las17p, which is a homolog of human wiskott-Aldrich Syndrome protein involved in actin patch assembly and actin polymerization

1361;Protein that forms a complex with the Sit4p protein phosphatase and is required for its function; member of a family of similar proteins including Sap4p, Sap155p, and Sap190p

1362;DNA helicase and DNA-dependent ATPase involved in DNA repair, required for proper timing of commitment to meiotic recombination and the transition from Meiosis I to Meiosis II; potential Cdc28p substratej 1363;Essential BRCT repeat protein, required on the prereplicative complex at replication origins for loading DNA polymerases to initiate DNA synthesis, also required for S/M checkpoint control

1364;C6 zinc cluster transcriptional activator that binds to the carbon source-responsive element (CSRE) of gluconeogenic genes; involved in the positive regulation of gluconeogenesis; regulated by Snf1p protein kinase; localized to the nucleus

1365;Ornithine carbamoyltransferase (carbamoylphosphate:L-ornithine carbamoyltransferase), catalyzes the sixth step in the biosynthesis of the arginine precursor ornithine

1366;tRNA ligase, required for tRNA splicing; composed of three essential domains containing the phosphodiesterase, polynucleotide kinase, and ligase activities required for ligation; localized at the inner membrane of the nuclear envelope

1367;Essential 70kDa subunit of the exocyst complex (Sec3p, Sec5p, Sec6p, Sec8p, Sec10p, Sec15p, Exo70p, and Exo84p), which has the essential function of mediating polarized targeting of secretory vesicles to active sites of exocytosis

1368;Nuclear actin-related protein involved in chromatin remodeling, component of chromatin-remodeling enzyme complexes

1369;Essential RNA-binding G protein effector of mating response pathway, predominantly associated with nuclear envelope and ER, interacts in mRNA-dependent manner with translating ribosomes via multiple KH domains, similar to vertebrate vigilins

1370;Protein of unknown function, has similarity to Pry2p and Pry3p and to the plant PR-1 class of pathogen related proteins

1371;subunit of the multiprotein cohesin complex required for sister chromatid cohesion in mitotic cells; also required, with Rec8p, for cohesion and recombination during meiosis; phylogenetically conserved SMC chromosomal ATPase family member

1372;Subunit of the GINS complex (Sld5p, Psf1p, Psf2p, Psf3p), which is localized to DNA replication origins and implicated in assembly of the DNA replication machinery

1373;Hypothetical protein

1374;Non-essential intracellular esterase that can function as an S-formylglutathione hydrolase; may be involved in the detoxification of formaldehyde, which can be metabolized to S-formylglutathione; similar to human esterase D

1375;Subunit of ISW2/yCHRAC chromatin accessibility complex along with Itclp, Isw2p, and Dpb4p; involved in inheritance of telomeric silencing

1376;Mitochondrial ribosomal protein of the large subunit

1377;Integral plasma membrane protein involved in the synthesis of the glycosylphosphatidylinositol (GPI) core structure; mutations affect cell wall integrity

1378;Subunit of the nuclear pore complex (NPC), forms a subcomplex with Nup159p and Nsp1p, interacts with Nup116p and is required for proper localization of Nup116p in the NPC

1379;Arylformamidase_{;} involved in biosynthesis of nicotinic acid from tryptophan via kynurenine pathway; potential Cdc28p substrate

1380;Vacuolar membrane protein involved in the ATP-dependent transport of arginine into the vacuole and possibly in balancing ion homeostasis; homolog of human CLN3 involved in Batten disease (juvenile onset neuronal ceroid lipofuscinosis)

1381;Subunit of TORC2 (Tor2p-Lst8p-Avol-Avo2-Tsc11p-Bit61p-Slm1p-Slm2p), a membrane-associated complex that regulates cell cycle-dependent actin cytoskeletal dynamics during polarized growth and cell wall integrity

1382;probable serine/threonine kinase

1383;Vacuolar protein sorting protein that forms part of the multimeric membrane-associated retromer complex along with Vps35p, Vps29p, Vps17p, and Vps5p; essential for endosome-to-Golgi retrograde protein transport

1384;Glyceraldehyde-3-phosphate dehydrogenase, isozyme 1, involved in glycolysis and gluconeogenesis; tetramer that catalyzes the reaction of glyceraldehyde-3-phosphate to 1,3 bis-phosphoglycerate; detected in the cytoplasm and cell-wall

1385;Dead-box family ATP dependent helicase required for mRNA export from the nucleus; co-factor of the exosome complex, required for 3' end formation of 5.8S rRNA

1386;Minor succinate dehydrogenase isozyme; homologous to Sdh1p, the major isozyme reponsible for the oxidation of succinate and transfer of electrons to ubiquinone; induced during the diauxic shift in a Cat8p-dependent manner

1387;Putative protein of unknown function; expression induced during sporulation and repressed during vegetative growth by Sum1p and Hstlp; similar to adjacent open reading frame, YJL037W

1388;Sorting nexin, involved in the retrieval of late-Golgi SNAREs from the post-Golgi endosome to the trans-Golgi network and in cytoplasm to vacuole transport; contains a PX domain; forms complex with Snx41p and Atg20p

1389;Subunit of tRNA-specific adenosine-34 deaminase, forms a heterodimer with Tad3p that converts adenosine to inosine at the wobble position of several tRNAs

1390;ATPase involved in protein import into the ER, also acts as a chaperone to mediate protein folding in the ER and may play a role in ER export of soluble proteins; regulates the unfolded protein response via interaction with Ire1p

1391;Component of the spindle-assembly checkpoint complex, which delays the onset of anaphase in cells with defects in mitotic spindle assembly; forms a complex with Mad1p

1392;Protein possibly involved in assembly of actin patches; interacts with an actin assembly factor Las17p and with the SH3 domains of Type I myosins Myo3p and Myo5p; localized predominantly to cortical actin patches

1393;Essential integral membrane protein required for spindle pole body duplication and for nuclear fusion, localizes to the spindle pole body half bridge, interacts with DnaJ-like chaperone Jem1p and with centrin homolog Cdc3 1 p

1394;RNA polymerase III subunit C17; physically interacts with C31, C11, and TFIIIB70; may be involved in the recruitment of pol III by the preinitiation complex

1395;Essential nucleolar protein required for 18S rRNA synthesis

1396;Subunit of the cytosolic chaperonin Cct ring complex, related to Tcplp, required for the assembly of actin and tubulins in vivo

1397;Hypothetical protein

1398;Beta subunit of C-terminal domain kinase I (CTDK-I), which phosphorylates the C-terminal repeated domain of the RNA polymerase II large subunit (Rpo21p) to affect both transcription and pre-mRNA3' end processing; has similarity to cyclins

1399;Adenylate cyclase, required for cAMP production and cAMP-dependent protein kinase signaling; involved in cell cycle control and glucose and nitrogen repression of sporulation

1400;Integral membrane protein of the Golgi required for targeting of the Arf-like GTPase Ar13p to the Golgi; multicopy suppressor of ypt6 null mutation

1401;Integral membrane protein of the Golgi required for targeting of the Arf-like GTPase Ar13p to the Golgi; multicopy suppressor of ypt6 null mutation

1402;Mitochondrial inner membrane protein, required for assembly of cytochrome c oxidase

1403;Mitochondrial inner membrane protein, required for assembly of cytochrome c oxidase

1404;Alpha subunit of the oligosaccharyltransferase complex of the ER lumen, which catalyzes asparagine-linked glycosylation of newly synthesized proteins

1405;Alpha subunit of the oligosaccharyltransferase complex of the ER lumen, which catalyzes asparagine-linked glycosylation of newly synthesized proteins

1406;Vacuolar transporter, imports large neutral amino acids into the vacuole; member of a family of seven S. cerevisiae genes (AVT1-7) related to vesicular GABA-glycine transporters

1407;Vacuolar transporter, imports large neutral amino acids into the vacuole; member of a family of seven S. cerevisiae genes (AVT1-7) related to vesicular GABA-glycine transporters

1408;Hypothetical protein

1409;Alpha-agglutinin of alpha-cells, binds to Aga1p during agglutination, N-terminal half is homologous to the immunoglobulin superfamily and contains binding site for a-agglutinin, C-terminal half is highly glycosylated and contains GPI anchor

1410;Beta-adaptin, large subunit of the clathrin associated protein complex (AP-2); involved in vesicle mediated transport; similar to mammalian beta-chain of the clathrin associated protein complex

1411;Beta-adaptin, large subunit of the clathrin associated protein complex (AP-2); involved in vesicle mediated transport; similar to mammalian beta-chain of the clathrin associated protein complex

1412;Identified by homology to Ashbya gossypii

1413;Identified by homology to Ashbya gossypii

1414;DNA polymerase III (delta) subunit, essential for cell viability; involved in DNA replication and DNA repair

1415;DNA polymerase III (delta) subunit, essential for cell viability; involved in DNA replication and DNA repair

1416;Alpha subunit of the translation initiation factor eIF2, involved in the identification of the start codon; phosphorylation of Ser51 is required for regulation of translation by inhibiting the exchange of GDP for GTP

1417;Alpha subunit of the translation initiation factor eIF2, involved in the identification of the start codon; phosphorylation of Ser51 is required for regulation of translation by inhibiting the exchange of GDP for GTP

1418;Hypothetical protein

1419;Subunit of the signal peptidase complex (SPC), which cleaves the signal sequence from proteins targeted to the endoplasmic reticulum (ER), homolog of the SPC12 subunit of mammalian signal peptidase complex

1420;Hypothetical protein

1421;Hypothetical protein

1422;Dihydroxyacid dehydratase, catalyzes third step in the common pathway leading to biosynthesis of branched-chain amino acids

1423;Peroxisomal acyl-CoA thioesterase likely to be involved in fatty acid oxidation rather than fatty acid synthesis; conserved protein also found in human peroxisomes; TES 1 mRNA levels increase during growth on fatty acids

1424;3-hydroxyanthranilic acid dioxygenase, required for biosynthesis of nicotinic acid from tryptophan via kynurenine pathway

1425;Peptidyl-prolyl cis-trans isomerase (cyclophilin), catalyzes the cis-trans isomerization of peptide bonds N-terminal to proline residues; binds to Hsp82p and contributes to chaperone activity

1426;Subunit of the RAVE complex (Ravlp, Rav2p, Skplp), which promotes assembly of the V-ATPase holoenzyme; required for transport between the early and late endosome/PVC and for localization of TGN membrane proteins; potential Cdc28p substrate

1427;Protein required for assembly of cytochrome c oxidase

1428;Protein with similarity to hect domain E3 ubiquitin-protein ligases, not essential for viability

1429;Chloride channel localized to late- or post-Golgi vesicles, involved in iron metabolism; highly homologous to voltage-gated chloride channels in vertebrates

1430;Nucleolar protein required for normal metabolism of the rRNA primary transcript, proposed to be involved in ribosome biogenesis

1431;Third subunit of DNA polymerase delta, involved in chromosomal DNA replication; required for error-prone DNA synthesis in the presence of DNA damage and processivity; interacts with Hys2p, PCNA (Pol30p), and Po11p

1432;Late endosomal protein involved in late endosome to vacuole trafficking; functional homolog of human obesity receptor gene-related protein (OB-RGRP)

1433;Mitochondrial matrix ATPase that is a subunit of the presequence translocase-associated protein import motor (PAM); involved in protein translocation into the matrix and protein folding; member of the heat shock protein 70 (HSP70) family

1434;Translation initiation factor eIF-5A, promotes formation of the first peptide bond; similar to and functionally redundant with Hyp2p; undergoes an essential hypusination modification; expressed under anaerobic conditions

1435;Cytochrome c, isoform 1; electron carrier of the mitochondrial intermembrane space that transfers electrons from ubiquinone-cytochrome c oxidoreductase to cytochrome c oxidase during cellular respiration

1436;NAD kinase, active as a hexamer; enhances the activity of ferric reductase (Fre1p) 1437;Component of the spliceosome complex involved in pre-mRNA splicing, auxiliary splicing factor that may modulate Syflp activity and help optimize splicing; isy1 syf2 double mutation activates the spindle checkpoint, causing cell cycle arrest

1438;Fumarate reductase, catalyzes the reduction of fumarate to succinate, required for the reoxidation of intracellular NADH under anaerobic conditions; mutations cause osmotic sensitivity

1439;Protein that recognizes and binds damaged DNA in an ATP-dependent manner (with Rad16p) during nucleotide excision repair; subunit of Nucleotide Excision Repair Factor 4 (NEF4)

1440;Component of the GTPase-activating Bfalp-Bub2p complex involved in multiple cell cycle checkpoint pathways that control exit from mitosis

1441;Vacuolar protein of unknown function; potential Cdc28p substrate 1442;Protein of unknown function, required for growth at high temperature

1443;Small subunit of the clathrin-associated adaptor complex AP-2, which is involved in protein sorting at the plasma membrane; related to the sigma subunit of the mammalian plasma membrane clathrin-associated protein (AP-2) complex

1444;Putative serine/threonine protein kinase involved in regulation of ion transport across plasma membrane; enhances spermine uptake

1445;RNA polymerase I subunit A12.2; contains two zinc binding domains, and the N terminal domain is responsible for anchoring to the RNA pol I complex

1446;Subunit of the cytosolic chaperonin Cct ring complex, related to Tcplp, required for the assembly of actin and tubulins in vivo

1447;Essential component of the Arp2/3 complex, which is a highly conserved actin nucleation center required for the motility and integrity of actin patches; involved in endocytosis and membrane growth and polarity

1448;PIK-related protein kinase and rapamycin target; subunit of TORC1, a complex that controls growth in response to nutrients by regulating translation, transcription, ribosome biogenesis, nutrient transport and autophagy; involved in meiosis

1449;Subunit of heteropentameric Replication factor C (RF-C), which is a DNA binding protein and ATPase that acts as a clamp loader of the proliferating cell nuclear antigen (PCNA) processivity factor for DNA polymerases delta and epsilon

1450;Protein of unknown function that is involved in DNA repair; mutant is sensitive to the base analog, 6-N-hydroxylaminopurine, while gene disruption does not increase the rate of spontaneous mutagenesis

1451;Cytoplasmic protein required for cell viability, identified by association with pre-ribosomal particles

1452;Phospholipid methyltransferase (methylene-fatty-acyl-phospholipid synthase), catalyzes the last two steps in phosphatidylcholine biosynthesis

1453;Component of the septin ring of the mother-bud neck that is required for cytokinesis; septins recruit proteins to the neck and can act as a barrier to diffusion at the membrane, and they comprise the 10nm filaments seen with EM

1454;Mitochondrial phosphate carrier, imports inorganic phosphate into mitochondria; functionally redundant with Pic2p but more abundant than Pic2 under normal conditions

1455;The authentic, non-tagged protein was localized to the mitochondria

1456;Esalp-associated factor, subunit of the NuA4 acetyltransferase complex

1457;Protein of unknown function, computational analysis of large-scale protein-protein interaction data suggests a possible role in actin cytoskeleton organization; potential Cdc28p substrate

1458;Putative protein of unknown function; detectable in highly purified mitochondria 1459;G protein gamma subunit, forms a dimer with Ste4p to activate the mating signaling pathway, forms a heterotrimer with Gpa1p and Ste4p to dampen signaling; C-terminus is palmitoylated and farnesylated, which are required for normal signaling

1460;Essential chromosomal passenger protein involved in coordinating cell cycle events for proper chromosome segregation; C-terminal region binds Sli15p, and the middle region, upon phosphorylation, localizes Cbf2p to the spindle at anaphase

1461;Member of the Puf family of RNA-binding proteins, interacts with mRNAs encoding membrane-associated proteins; overexpression suppresses a tub2-150 mutation and causes increased sensitivity to benomyl in wild-type cells

1462;Subunit of cleavage polyadenylation factor (CPF), interacts directly with poly(A) polymerase (Pap1p) to regulate its activity

1463;Master regulator of meiosis that is active only during meiotic events, activates transcription of early meiotic genes through interaction with Ume6p, degraded by the 26S proteasome following phosphorylation by Ime2p

1464;Mitochondrial succinate-fumarate transporter, transports succinate into and fumarate out of the mitochondrion; required for ethanol and acetate utilization1465;Putative xylose and arabinose reductase

1466;Ubiquitin C-terminal hydrolase that cleaves ubiquitin-protein fusions to generate monomeric ubiquitin; hydrolyzes the peptide bond at the C-terminus of ubiquitin; also the major processing enzyme for the ubiquitin-like protein Rublp

1467;Mitochondrial ribosomal protein of the small subunit

1468;Cu, Zn superoxide dismutase; some mutations are analogous to those that cause ALS (amyotrophic lateral sclerosis) in humans

1469;Adenosine kinase, required for the utilization of S-adenosylmethionine (AdoMet); may be involved in recycling adenosine produced through the methyl cycle

1470;Phosphatidylinositol 3-phosphate [PI(3)P] phosphatase, regulates the localization and levels of PI(3)P; involved in cytoplasm to vacuole (CVT) transport; has similarity to the conserved myotubularin dual specificity phosphatase family

1471;Putative protein of unknown function; green fluorescent protein (GFP)-fusion protein localizes to the mitochondria

1472;Essential component of the MIND kinetochore complex (Mtw1p Including Nnflp-Ns11p-Dsn1p) which joins kinetochore subunits contacting DNA to those contacting microtubules; required for accurate chromosome segregation

1473;Mitochondrial ribosomal protein of the small subunit, has similarity to E. coli S7 ribosomal protein

1474;Hypothetical protein

1475;Highly conserved zinc metalloprotease that functions in two steps of a-factor maturation, C-terminal CAAX proteolysis and the first step of N-terminal proteolytic processing; contains multiple transmembrane spans

1476;Putative protein of unknown function; green fluorescent protein (GFP)-fusion protein localizes to the cytoplasm and nucleus

1477;Beta subunit of the F1 sector of mitochondrial F1F0 ATP synthase, which is a large, evolutionarily conserved enzyme complex required for ATP synthesis

1478;Protein component of the small (40S) ribosomal subunit, the least basic of the non-acidic ribosomal proteins; phosphorylated in vivo; essential for viability; has similarity to E. coli S7 and rat S5 ribosomal proteins

1479;Protein containing an N-terminal epsin-like domain involved in clathrin recruitment and traffic between the Golgi and endosomes; associates with the clathrin adaptor Gga2p

1480;Putative protein of unknown function; predicted S-adenosylmethionine-dependent methyltransferase of the seven beta-strand family; green fluorescent protein (GFP)-fusion protein localizes to the cytoplasm

1481;Cystathionine gamma-synthase, converts cysteine into cystathionine

1482;Karyopherin, a carrier protein involved in nuclear import of proteins; importin beta homolog

1483;Xanthine-guanine phosphoribosyl transferase, required for xanthine utilization and for optimal utilization of guanine

1484;Protein of unknown function, required for wild-type growth rate on galactose and mannose; localizes to COPI coated vesicles and the Golgi apparatus

1485;Protein involved in minichromosome maintenance; component of the kinetochore; binds to centromeric DNA in a Ctf19p-dependent manner

1486;Mitochondrial intermembrane space protein mediating import and insertion of polytopic inner membrane proteins; homolog of human DDP1 (deafness dystonia peptide 1) which is mutated in the X-linked Mohr-Tranebjaerg syndrome

1487;Sulfite reductase beta subunit, involved in amino acid biosynthesis, transcription repressed by methionine

1488;Protein of unknown function, green fluorescent protein (GFP)-fusion protein localizes to the vacuolar membrane

1489;Homoserine dehydrogenase (L-homoserine:NADP oxidoreductase), dimeric enzyme that catalyzes the third step in the common pathway for methionine and threonine biosynthesis; enzyme has nucleotide-binding, dimerization and catalytic regions

1490;Transcriptional corepressor involved in the cell cycle-regulated transcription of histone genes HTA1, HTB1, HHT1, and HHT2; involved in position-dependent gene silencing and nucleosome reassembly

1491;Putative protein of unknown function

1492;Protein O-mannosyltransferase, transfers mannose residues from dolichyl phosphate-D-mannose to protein serine/threonine residues; appears to form homodimers in vivo and does not complex with other Pmt proteins; target for new antifungals

1493;Cytosolic branched-chain amino acid aminotransferase, homolog of murine ECA39; highly expressed during stationary phase and repressed during logarithmic phase

1494;Cell wall mannoprotein with similarity to Tir1p, Tir2p, Tir3p, and Tir4p; expressed under anaerobic conditions, completely repressed during aerobic growth

1495;Allantoin permease; ureidosuccinate permease; expression is constitutive but sensitive to nitrogen catabolite repression

1496;Endo-polygalacturonase, pectolytic enzyme that hydrolyzes the alpha-1,4-glycosidic bonds in the rhamnogalacturonan chains in pectins

1497;Endo-polygalacturonase, pectolytic enzyme that hydrolyzes the alpha-1,4-glycosidic bonds in the rhamnogalacturonan chains in pectins

1498;Dihydroorotate dehydrogenase, catalyzes the fourth enzymatic step in the de novo biosynthesis of pyrimidines, converting dihydroorotic acid into orotic acid

1499;Member of the REF (RNA and export factor binding proteins) family; when overexpressed, can substitute for the function of Yralp in export of poly(A)+ mRNA from the nucleus

1500;WD repeat protein required for ubiquitin-mediated protein degradation, forms complex with Cdc48p, plays a role in controlling cellular ubiquitin concentration; also promotes efficient NHEJ in postdiauxic/stationary phase

1501;Lipid phosphoinositide phosphatase of the ER and Golgi, involved in protein trafficking and secretion

1502;Bifunctional enzyme exhibiting both indole-3-glycerol-phosphate synthase and anthranilate synthase activities, forms multifunctional hetero-oligomeric anthranilate synthase:indole-3-glycerol phosphate synthase enzyme complex with Trp2p1503;Ubiquitin activating enzyme, involved in ubiquitin-mediated protein degradation and essential for viability

1504;Putative protein of unknown function; green fluorescent protein (GFP)-fusion protein localizes to the cytoplasm

1505;Nuclear pore protein involved in nuclear export of pre-tRNA

1506;PIK-related protein kinase and rapamycin target; subunit of TORC1, a complex that regulates growth in response to nutrients and TORC2, a complex that regulates cell-cycle dependent polarization of the actin cytoskeleton; involved in meiosis

1507;Vesicle membrane protein (v-SNARE) with acyltransferase activity; involved in trafficking to and within the Golgi, endocytic trafficking to the vacuole, and vacuolar fusion; membrane localization due to prenylation at the carboxy-terminus

1508;Mitochondrial threonyl-tRNA synthetase

1509;Conserved nuclear regulatory subunit of Gle7p type 1 protein serine-threonine phosphatase (PP1), functions positively with Glc7p to promote dephosphorylation of nuclear substrates required for chromosome transmission during mitosis

1510;Mitochondrial matrix acyl carrier protein, involved in biosynthesis of octanoate, which is a precursor to lipoic acid; activated by phosphopantetheinylation catalyzed by Ppt2p

1511;Protein required, along with Dph1p, Kti11p, Jjj3p, and Dph5p, for synthesis of diphthamide, which is a modified histidine residue of translation elongation factor 2 (Eft1p or Eft2p); may act in a complex with Dph1p and Kti11p

1512;Component of the RAM signaling network that is involved in regulation of Ace2p activity and cellular morphogenesis, interacts with Kiclp and Sog2p, localizes to sites of polarized growth during budding and during the mating response

1513;Ornithine decarboxylase, catalyzes the first step in polyamine biosynthesis; degraded in a proteasome-dependent manner in the presence of excess polyamines1514;5-phospho-ribosyl-1(alpha)-pyrophosphate synthetase, involved in nucleotide, histidine, and tryptophan biosynthesis; one of five related enzymes, which are active as heteromultimeric complexes

1515;Protein involved in excretion of putrescine and spermidine; putativepolyamine transporter in the Golgi or post-Golgi vesicles

1516;Mitochondrial ribosomal protein of the large subunit; appears as two protein spcts (YmL34 and YmL38) on two-dimensional SDS gels

1517;Subunit of cytoplasmic cAMP-dependent protein kinase, which contains redundant catalytic subunits Tpk1p, Tpk2p, and Tpk3p and regulatory subunit Bcy1p; promotes vegetative growth in response to nutrients; inhibits filamentous growth

1518;Putative protein of unknown function; green fluorescent protein (GFP)-fusion protein localizes to the mitochondrion

1519;Transcription elongation factor that contains a conserved zinc finger domain; implicated in the maintenance of proper chromatin structure in actively transcribed regions; deletion inhibits Brome mosaic virus (BMV) gene expression

1520;Merged open reading frame, does not encode a discrete protein; YKL158W was originally annotated as an independent ORF, but was later demonstrated to be an exon of an adjacent ORF, YKL157W

1521;Mitochondrial ribosomal protein of the small subunit

1522;Tetrameric phosphoglycerate mutase, mediates the conversion of 3-phosphoglycerate to 2-phosphoglycerate during glycolysis and the reverse reaction during gluconeogenesis

1523;Mitochondrial NADH-cytochrome b5 reductase, involved in ergosterol biosynthesis

1524;RNA polymerase III subunit C25

1525;Putative mitochondrial ribosomal protein, has similarity to E. coli ribosomal protein S2

1526;Cytochrome b subunit of succinate dehydrogenase (Sdhlp, Sdh2p, Sdh3p, Sdh4p), which couples the oxidation of succinate to the transfer of electrons to ubiquinone

1527;Component of the DASH complex which is found at kinetochores and regulates spindle assembly; in vitro DASH forms rings on microtubules and promotes microtubule stability

1528;Mitochondrial ribosomal protein of the large subunit

1529;Putative dihydrofolate synthetase; has similarity to Fo13p; similar to E. coli folylpolyglutamate synthetase/dihydrofolate synthetase

1530;RNA-binding protein that binds specific mRNAs and interacts with She3p; part of the mRNA localization machinery that restricts accumulation of certain proteins to the bud

1531;Protein required for transcription of rDNA by RNA polymerase I; transcription factor independent of DNA template; involved in recruitment of RNA polymerase I to rDNA

1532;Subunit of the signal recognition particle (SRP), which functions in protein targeting to the endoplasmic reticulum membrane; not found in mammalian SRP; forms a pre-SRP structure in the nucleolus that is translocated to the cytoplasm

1533;Putative protein of unknown function

1534;Mitochondrial inner membrane transporter, transports oxaloacetate, sulfate, and thiosulfate; member of the mitochondrial carrier family

1535;Integral membrane protein required for vacuolar H+-ATPase (V-ATPase) function, although not an actual component of the V-ATPase complex; functions in the assembly of the V-ATPase; localized to the endoplasmic reticulum (ER)

1536;Co-chaperone that binds to and regulates Hsp90 family chaperones; important for pp60v-src activity in yeast; homologous to the mammalian p23 proteins

1537;Protein kinase with a possible role in MAP kinase signaling in the pheromone response pathway

1538;5' to 3' exonuclease, 5' flap endonuclease, required for Okazaki fragment processing and maturation as well as for long-patch base-excision repair; member of the S. pombe RAD2/FEN1 family

1539;Protein associated with the RNA polymerase II Elongator complex; involved in sensitivity to G1 arrest induced by Kluyveromyces lactis toxin, zymocin

1540;Subunit of the heme-activated, glucose-repressed Hap2p/3p/4p/5p CCAAT-binding complex, a transcriptional activator and global regulator of respiratory gene expression; provides the principal activation function of the complex

1541;Mitochondrial aspartate aminotransferase, catalyzes the conversion of oxaloacetate to aspartate in aspartate and asparagine biosynthesis

1542;Glutamine-fructose-6-phosphate amidotransferase, catalyzes the formation of glucosamine-6-P and glutamate from fructose-6-P and glutamine in the first step of chitin biosynthesis

1543;Covalently linked cell wall mannoprotein, major constituent of the cell wall; plays a role in stabilizing the cell wall; involved in low pH resistance; precursor is GPI-anchored

1544;Cell wall mannoprotein, linked to a beta-1,3- and beta-1,6-glucan heteropolymer through a phosphodiester bond; involved in cell wall organization

1545;Protein of unknown function, localizes to lipid particles

1546;Protein involved in mitochondrial functions and stress response; overexpression suppresses growth defects of hap2, hap3, and hap4 mutants

1547;Protein of unknown function; has two CUE domains that bind ubiquitin, which may facilitate intramolecular monoubiquitination

1548;Kinetochore protein with homology to human CENP-C, required for structural integrity of the spindle during anaphase spindle elongation, interacts with histones H2A, H2B, and H4, phosphorylated by Ip11p

1549;Cytochrome c1 heme lyase, involved in maturation of cytochrome c1, which is a subunit of the mitochondrial ubiquinol-cytochrome-c reductase; links heme covalently to apocytochrome c1

1550;Mitochondrial malate dehydrogenase, catalyzes interconversion of malate and oxaloacetate; involved in the tricarboxylic acid (TCA) cycle

1551;Mitochondrial intermembrane space protein, first component of a pathway mediating assembly of small TIM (Translocase of the Inner Membrane) complexes which escort hydrophobic inner membrane proteins en route to the TIM22 complex

1552;Putative protein of unknown function; green fluorescent protein (GFP)-fusion protein localizes to the cytoplasm

1553;Putative protein of unknown function; green fluorescent protein (GFP)-fusion protein localizes to the cytoplasm and nucleus

1554;Nucleoside diphosphate kinase, catalyzes the transfer of gamma phosphates from nucleoside triphosphates, usually ATP, to nucleoside diphosphates by a mechanism that involves formation of an autophosphorylated enzyme intermediate

1555;Endoplasmic reticulum transmembrane protein, homolog of human BAP31 protein

1556;Putative protein of unknown function; green fluorescent protein (GFP)-fusion protein localizes to the Golgi

1557;Transcriptional activator related to Msn2p; activated in stress conditions, which results in translocation from the cytoplasm to the nucleus; binds DNA at stress response elements of responsive genes, inducing gene expression

1558;Putative protein of unknown function; green fluorescent protein (GFP)-fusion protein localizes to the endosome

1559;Fructose 1,6-bisphosphate aldolase, a cytosolic enzyme required for glycolysis and gluconeogenesis; catalyzes the conversion of fructose 1,6 bisphosphate into two 3-carbon products: glyceraldehyde-3-phosphate and dihydroxyacetone phosphate

1560;TFIIA small subunit; involved in transcriptional activation, acts as antirepressor or as coactivator; homologous to smallest subunit of human and Drosophila TFIIA

1561;Protein of unknown function; putative ribosomal protein; homolog of translationally controlled tumor protein; green fluorescent protein (GFP)-fusion protein localizes to the cytoplasm; YKL056C is not an essential gene

1562;Protein of unknown function; mutation affects mitochondrial distribution and morphology

1563;Putative mannosidase, GPI-anchored membrane protein required for cell wall biosynthesis in bud formation;homologous to Dfg5p

1564;Transcriptional activator that enhances pseudohyphal growth; regulates expression of FLO11, an adhesin required for pseudohyphal filament formation; similar to StuA, an A. nidulans developmental regulator; potential Cdc28p substrate

1565;Central plaque component of spindle pole body (SPB); involved in SPB duplication, may facilitate attachment of the SPB to the nuclear membrane

1566;One of four subunits of the endosomal sorting complex required for transport III (ESCRT-III); forms an ESCRT-III subcomplex with Vps2p; involved in the sorting of transmembrane proteins into the multivesicular body (MVB) pathway

1567;Glucose-responsive transcription factor that regulates expression of several glucose transporter (HXT) genes in response to glucose; binds to promoters and acts both as a transcriptional activator and repressor

1568;UDP-glucose pyrophosphorylase (LTGPase), catalyses the reversible formation of UDP-Glc from glucose 1-phosphate and UTP, involved in a wide variety of metabolic pathways, expression modulated by Pho85p through Pho4p

1569;Golgi-localized RING-finger ubiquitin ligase (E3), involved in ubiquitinating and sorting membrane proteins that contain polar transmembrane domains to multivesicular bodies for delivery to the vacuole for quality control purposes

1570;Protein that binds DNA containing intrastrand cross-links formed by cisplatin, contains two HMG (high mobility group box) domains, which confer the ability to bend cisplatin-modified DNA; mediates aerobic transcriptional repression of COX5b

1571;Mitochondrial malic enzyme, catalyzes the oxidative decarboxylation of malate to pyruvate, which is a key intermediate in sugar metabolism and a precursor for synthesis of several amino acids

1572;TFIIE large subunit, involved in recruitment of RNA polymerase II to the promoter, activation of TFIIH, and promoter opening

1573;Essential subunit of the Pan2p-Pan3p poly(A)-ribonuclease complex, which acts to control poly(A) tail length and regulate the stoichiometry and activity of postreplication repair complexes

1574;Uridylate kinase, catalyzes the seventh enzymatic step in the de novo biosynthesis of pyrimidines, converting uridine monophosphate (UMP) into uridine-5'-diphosphate (UDP)

1575;Putative protein of unknown function; green fluorescent protein (GFP)-fusion protein localizes to the cytoplasm

1576;ER membrane protein involved, with its homolog Mga2p, in regulation of OLE1 transcription; inactive ER form dimerizes and one subunit is then activated by ubiquitin/proteasome-dependent processing followed by nuclear targeting

1577;Alpha subunit of both the farnesyltransferase and type I geranylgeranyltransferase that catalyze prenylation of proteins containing a CAAX consensus motif; essential protein required for membrane localization of Ras proteins and a-factor

1578;Putative protein of unknown function; identified by homology; green fluorescent protein (GFP)-fusion protein localizes to the cytoplasm

1579;Subunit of the COMPASS (Set1C) complex, which methylates histone H3 on lys 4 and is involved in telomeric silencing; subunit of CPF (cleavage and polyadenylation factor), a complex involved in RNAP II transcription termination

1580;Hexameric DNA polymerase alpha-associated DNA helicase A involved in lagging strand DNA synthesis; contains single-stranded DNA stimulated ATPase and dATPase activities; replication protein A stimulates helicase and ATPase activities

1581;Subunit d of the stator stalk of mitochondrial F1F0 ATP synthase, which is a large, evolutionarily conserved enzyme complex required for ATP synthesis

1582;Transcriptional activator of proline utilization genes, constitutively binds PUT1 and PUT2 promoter sequences and undergoes a conformational change to form the active state; has a Zn(2)-Cys(6) binuclear cluster domain

1583;Nucleolar protein required for the normal accumulation of 25S and 5.8S rRNAs, associated with the 27SA2 pre-ribosomal particle; proposed to be involved in the biogenesis of the 60S ribosomal subunit

1584;Subunit of the ARP2/3 complex, which is required for the motility and integrity of cortical actin patches

1585;Mitochondrial cruciform cutting endonuclease, cleaves Holliday junctions formed during recombination of mitochondrial DNA

1586;Ubiquitin-protein ligase (E3) that interacts with Rpt4p and Rpt6p, two subunits of the 19S particle of the 26S proteasome; cytoplasmic E3 involved in the degradation of ubiquitin fusion proteins

1587;Protein involved in mRNA turnover and ribosome assembly, localizes to the nucleolus

1588;Ceramide synthase component, involved in synthesis of ceramide from C26(acyl)-coenzyme A and dihydrosphingosine or phytosphingosine, functionally equivalent to Laglp

1589;Alpha subunit of the capping protein (CP) heterodimer (Caplp and Cap2p) which binds to the barbed ends of actin filaments preventing further polymerization; localized predominantly to cortical actin patches

1590;Negative regulator of transcription elongation, contains a TFIIS-like domain and a PHD finger, multicopy suppressor of temperature-sensitive ess1 mutations, probably binds RNA polymerase II large subunit

1591;Phosphatidylinositol:ceramide phosphoinositol transferase (IPC synthase), required for sphingolipid synthesis; can mutate to confer aureobasidin A resistance

1592;Mitochondrial ribosomal protein of the small subunit; MRP17 exhibits genetic interactions with PET122, encoding a COX3-specific translational activator

1593;Adenylylsulfate kinase, required for sulfate assimilation and involved in methionine metabolism

1594;GTPase required for vacuolar protein sorting, functions in actin cytoskeleton organization via its interaction with Slalp; required for late Golgi-retention of some proteins including Kex2p; involved in regulating peroxisome biogenesis1595;Poly(A) polymerase, one of three factors required for mRNA 3'-end polyadenylation, forms multiprotein complex with polyadenylation factor I (PF I), also required for mRNA nuclear export; may also polyadenylate rRNAs

1596;Member of an oxysterol-binding protein family with overlapping, redundant functions in sterol metabolism and which collectively perform a function essential for viability; GFP-fusion protein localizes to the cell periphery

1597;Non-essential protein of unknown function

1598;Mitochondrial ribosomal protein of the large subunit, not essential for mitochondrial translation

1599;Component of the EGO complex, which is involved in the regulation of microautophagy; localizes to the vacuolar membrane, loss results in a defect in vacuolar acidification

1600;One of 15 subunits of the 'Remodel the Structure of Chromatin' (RSC) complex; found in close proximity to nucleosomal DNA; displaced from the surface of nucleosomal DNA after chromatin remodeling

1601;Multifunctional enzyme of the peroxisomal fatty acid beta-oxidation pathway; has 3-hydroxyacyl-CoA dehydrogenase and enoyl-CoA hydratase activities

1602;Putative protein of unknown function; green fluorescent protein (GFP)-fusion protein localizes to the nucleus

1603;Protein of unknown function, has similarity to Pry1p and Pry3p and to the plant PR-1 class of pathogen related proteins

1604;Putative protein of unknown function; contains a RING finger motif

1605;Putative protein of unknown function; green fluorescent protein (GFP)-fusion protein localizes to the cytoplasm and nucleus

1606;Protein involved in regulation of phosphatidylinositol 4,5-bisphosphate concentrations; Irs4p and Tax4p bind and activate the phosphatase Inp51p; mutation confers an increase in rDNA silencing

1607;Component of the GARP (Golgi-associated retrograde protein) complex, Vps51p-Vps52p-Vps53p-Vps54p, which is required for the recycling of proteins from endosomes to the late Golgi; links the (VFT/GARP) complex to the SNARE Tlg1p

1608;Putative protein of unknown function; green fluorescent protein (GFP)-fusion protein localizes to the cytoplasm and mitochondrion

1609;Putative ATP-dependent RNA helicase of the DEAD-box family involved in ribosomal biogenesis

1610;Alpha subunit of the translation initiation factor eIF2B, the guanine-nucleotide exchange factor for eIF2; activity subsequently regulated by phosphorylated eIF2; first identified as a positive regulator of GCN4 expression

1611;The authentic, non-tagged protein was localized to the mitochondria

1612;Defining member of the SET3 histone deacetylase complex which is a meiosis-specific repressor of sporulation genes; necessary for efficient transcription by RNAPII; one of two yeast proteins that contains both SET and PHD domains

1613;Golgi membrane protein of unknown function, interacts with Gea1p and Gea2p; required for localization of Gea2p; computational analysis suggests a possible role in either cell wall synthesis or protein-vacuolar targeting

1614;Class E protein of the vacuolar protein-sorting (Vps) pathway, associates reversibly with the late endosome, has human ortholog that may be altered in breast tumors

1615;General amino acid permease; localization to the plasma membrane is regulated by nitrogen source

1616;Putative protein of unknown function; green fluorescent protein (GFP)-fusion protein localizes to the cytoplasm and nucleus

1617;Protein of unknown function that interacts with Ulp1p, a Ub1 (ubiquitin-like protein)-specific protease for Smt3p protein conjugates

1618;Protein of unknown function that co-purifies with lipid particles; expression pattern suggests a role in respiratory growth; computational analysis of large-scale protein-protein interaction data suggests a role in ATP/ADP exchange

1619;Mitochondrial protein of unknown function; putative redox protein containing a thioredoxin fold

1620;Hypothetical protein

1621;Mitochondrial iron transporter of the mitochondrial carrier family (MCF), very similar to and functionally redundant with Mrs3p; functions under low-iron conditions; may transport other cations in addition to iron

1622;Non-essential small GTPase of the Rho/Rac subfamily of Ras-like proteins, likely to be involved in the establishment of cell polarity

1623;Translation initiation factor eIF4A, identical to Tif2p; DEA(D/H)-box RNA helicase that couples ATPase activity to RNA binding and unwinding; forms a dumbbell structure of two compact domains connected by a linker; interacts with eIF4G

1624;Possible U3 snoRNP protein involved in maturation of pre-18S rRNA, based on computational analysis of large-scale protein-protein interaction data

1625;Mannosyltransferase involved in N-linked protein glycosylation; member of the KRE2/MNT1 mannosyltransferase family

1626;TFIIE small subunit, involved in RNA polymerase II transcription initiation1627;Essential nuclear protein possibly involved in bud formation and morphogenesis; mutants require the SSD1-v allele for viability

1628;Putative protein of unknown function; green fluorescent protein (GFP)-fusion protein localizes to the cytoplasm and nucleus; detectable in highly purified mitochondria

1629;Presequence translocase-associated motor subunit, required for stable complex formation between cochaperones Pam16p and Pam18p, promotes association of Pam16p-Pam18p with the presequence translocase

1630;Mitochondrial cytochrome-c peroxidase; degrades reactive oxygen species in mitochondria, involved in the response to oxidative stress

1631;Hydrophilic protein that acts in conjunction with SNARE proteins in targeting and fusion of ER to Golgi transport vesicles; component of the TRAPP (transport protein particle) complex

1632;Putative protein of unknown function; green fluorescent protein (GFP)-fusion protein localizes to the mitochondrion

1633;Protein of unknown function; mutation displays synthetic lethal interaction with the po13-13 allele of CDC2

1634;Putative protein of unknown function; epitope-tagged protein localizes to the cytoplasm

1635;Protein of unknown function; similar to YOR062Cp and Reglp; expression regulated by glucose and Rgt1p

1636;Non-essential protein of unknown function; similar to Ygr154cp

1637;Hypothetical protein

1638;tRNase Z, involved in RNA processing, has two putative nucleotide triphosphate-binding motifs (P-loop) and a conserved histidine motif, homolog of the human candidate prostate cancer susceptibility gene ELAC2

1639;NAD-dependent 5,10-methylenetetrahydrafolate dehydrogenase, plays a catalytic role in oxidation of cytoplasmic one-carbon units; expression is regulated by Bas1p and Bas2p, repressed by adenine, and may be induced by inosito1 and choline

1640;Essential protein involved in the processing of pre-rRNA and the assembly of the 60S ribosomal subunit; interacts with ribosomal protein L11; localizes predominantly to the nucleolus; constituent of 66S pre-ribosomal particles

1641;Subunit of the Nup84p subcomplex of the nuclear pore complex (NPC), localizes to both sides of the NPC, required to establish a normal nucleocytoplasmic concentration gradient of the GTPase Gsp1p

1642;Essential protein, component of the DASH complex; involved in spindle integrity and kinetochore function; interacts with Duolp and Dam1p; localizes to intranuclear spindles and kinetochore

1643;GTP binding protein with sequence similarity to the elongation factor class of G proteins, EF-1alpha and Sup35p; associates with Dom34p, and shares a similar genetic relationship with genes that encode ribosomal protein componentsj 1644;Mitochondrial ribosomal protein of the large subunit

1645;RNA helicase in the DEAH-box family involved in the second catalytic step of splicing, exhibits ATP-dependent RNA unwinding activity

1646;Integral membrane protein localized to late Golgi vesicles along with the v-SNARE Tlg2p; green fluorescent protein (GFP)-fusion protein localizes to the cytoplasm in a punctate pattern

1647;Triacylglycerol lipase involved in TAG mobilization; found in lipid particles; potential Cdc28p substrate

1648;Nucleolar, serine-rich protein with a role in preribosome assembly or transport; may function as a chaperone of small nucleolar ribonucleoprotein particles (snoRNPs); immunologically and structurally to rat Nopp140

1649;Integral membrane peptide transporter, mediates transport of di- and tri-peptides; conserved protein that contains 12 transmembrane domains; PTR2 expression is regulated by the N-end rule pathway via repression by Cup9p

1650;Hypothetical protein

1651;Ubiquitin-specific protease that cleaves ubiquitin from ubiquitinated proteins

1652;Myb-related transcription factor involved in regulating basal and induced expression of genes of the purine and histidine biosynthesis pathways

1653;5-methylmethionine-homocysteine methyltransferase, functions along with Sam4p in the conversion of S-adenosylmethionine (AdoMet) to methionine to control the methionine/AdoMet ratio

1654;High-affinity S-methylmethionine permease, required for utilization of S-methylmethionine as a sulfur source; has similarity to S-adenosylmethionine permease Sam3p

1655;Fe(II)-dependent sulfonate/alpha-ketoglutarate dioxygenase, involved in sulfonate catabolism for use as a sulfur source, contains sequence that closely resembles a J domain (typified by the E. coli DnaJ protein)

1656;Hypothetical protein

1657;putative ferric reductase with similarity to Fre2p; expression induced by low iron levels

1658;Transporter of the ATP-binding cassette (ABC) family involved in bile acid transport; similar to mammalian bile transporters

1659;Ribosomal protein L4 of the large (60S) ribosomal subunit, nearly identical to Rp18Ap and has similarity to rat L7a ribosomal protein; mutation results in decreased amounts of free 60S subunits

1660;Plasma membrane glycerol channel, member of the major intrinsic protein (MIP) family of channel proteins; involved in efflux of glycerol and in uptake of the trivalent metalloids arsenite and antimonite

1661;Iron-sulfur protein subunit of succinate dehydrogenase (Sdh1p, Sdh2p, Sdh3p, Sdh4p), which couples the oxidation of succinate to the transfer of electrons to ubiquinone

1662;Protein of unknown function; heterooligomeric or homooligomeric complex; peripherally associated with membranes; homologous to human COH1; involved in sporulation, vacuolar protein sorting and protein-Golgi retention

1663;Ubiquitin, becomes conjugated to proteins, marking them for selective degradation via the ubiquitin-26S proteasome system; essential for the cellular stress response

1664;Protein of unknown function, contains an N-terminal epsin-like domain

1665;Protein of unknown function, required for cell growth and possibly involved in rRNA processing; mRNA is cell cycle regulated

1666;Hypothetical protein

1667;ER membrane localized phosphoryltransferase that adds phosphoethanolamine onto the third mannose residue of the glycosylphosphatidylinositol (GPI) anchor precursor; similar to human PIG-O protein

1668;Hypothetical protein

1669;Polyamine transporter that recognizes spermine, putrescine, and spermidine; catalyzes uptake of polyamines at alkaline pH and excretion at acidic pH; phosphorylation enhances activity and sorting to the plasma membrane

1670;Heat shock protein that cooperates with Ydj1p (Hsp40) and Ssa1p (Hsp70) to refold and reactivate previously denatured, aggregated proteins; responsive to stresses including: heat, ethanol, and sodium arsenite; involved in [PSI+] propagation

1671;Hypothetical protein

1672;Cytoplasmic aspartyl-tRNA synthetase, homodimeric enzyme that catalyzes the specific aspartylation of tRNA(Asp); class II aminoacyl tRNA synthetase; binding to its own mRNA may confer autoregulation

1673;Hypothetical protein

1674;Plasma membrane associated protein phosphatase involved in the general stress response; required along with binding partner Whi2p for full activation of STRE-mediated gene expression, possibly through dephosphorylation of Msn2p

1675;Copper metallochaperone that transfers copper to Scolp and Cox11p for eventual delivery to cytochrome c oxidase

1676;Nucleolar DEAD-box protein required for ribosome assembly and function, including synthesis of 60S ribosomal subunits; constituent of 66S pre-ribosomal particles

1677;Hypothetical protein

1678;Subunit of the origin recognition complex, which directs DNA replication by binding to replication origins and is also involved in transcriptional silencing

1679;Dynamin-related GTPase required for mitochondrial fission and the maintenance of mitochondrial morphology, assembles on the cytoplasmic face of mitochondrial tubules at sites at which division will occur; also participates in endocytosis

1680;Protein that forms a nuclear complex with Noc2p that binds to 66S ribosomal precursors to mediate their intranuclear transport; also binds to chromatin to promote the association of DNA replication factors and replication initiation

1681;Hypothetical protein

1682;Component of the core form of RNA polymerase transcription factor TFIIH, which has both protein kinase and DNA-dependent ATPase/helicase activities and is essential for transcription and nucleotide excision repair; interacts with Tf64p

1683;Essential nuclear protein required for DNA repair; forms a complex with Smc5p and Smc6p

1684;Ribosomal Like Protein 24

1685;Protein that regulates telomeric length; protects telomeric ends in a complex with Cdc13p and Stnlp

1686;Subunit of the RES complex, which is required for nuclear retention of unspliced pre-mRNAs; acts in the same pathway as Pml39p and Mlp1p

1687;Methylthioadenosine phosphorylase (MTAP), catalyzes the initial step in the methionine salvage pathway; affects polyamine biosynthesis through regulation of ornithine decarboxylase (Spe1p) activity; regulates NADH2 gene expression

1688;Integral membrane protein of the nuclear pore complex, localizes adjacent to the nuclear membrane

1689;Functionally redundant Psr1p homolog, a plasma membrane phosphatase involved in the general stress response; required with Psrlp and Whi2p for full activation of STRE-mediated gene expression, possibly through dephosphorylation of Msn2p

1690;Hypothetical proteinj 1691;Essential protein involved in RNA metabolism, one of two yeast homologs (with Yhr087wp) of the human protein SBDS responsible for autosomal recessive Shwachman-Bodian-Diamond Syndrome, also conserved in Archaea

1692;One of four subunits of the endosomal sorting complex required for transport III (ESCRT-III); involved in the sorting of transmembrane proteins into the multivesicular body (MVB) pathway; recruited from the cytoplasm to endosomal membranes

1693;cis-Golgi t-SNARE syntaxin required for vesicular transport between the ER and the Golgi complex, binds at least 9 SNARE proteins

1694;Enzyme of 'de novo' purine biosynthesis containing both 5-aminoimidazole-4-carboxamide ribonucleotide transformylase and inosine monophosphate cyclohydrolase activities, isozyme of Ade17p; ade16 ade17 mutants require adenine and histidine

1695;Protein component of the large (60S) ribosomal subunit, nearly identical to Rpl15Bp and has similarity to rat L15 ribosomal protein; binds to 5.8 S rRNA

1696;Single-stranded DNA-dependent ATPase, involved in postreplication repair; contains RING finger domain

1697;Remodels the structure of chromatin complex 58KDa subunit; Chromatin Remodeling Complex subunit

1698;Putative divalent metal ion transporter involved in iron homeostasis; transcriptionally regulated by metal ions; member of the Nramp family of metal transport proteins

1699;Subunit VIb of cytochrome c oxidase, which is the terminal member of the mitochondrial inner membrane electron transport chain; required for assembly of fully active cytochrome c oxidase but not required for activity after assembly

1700;Hypothetical protein

1701;Hypothetical protein

1702;Cytoplasmic thioredoxin isoenzyme of the thioredoxin system which protects cells against both oxidative and reductive stress, forms LMA1 complex with Pbi2p, acts as a cofactor for Tsalp, required for ER-Golgi transport and vacuole inheritance

1703;Major of three pyruvate decarboxylase isozymes, key enzyme in alcoholic fermentation, decarboxylates pyruvate to acetaldehyde; subject to glucose-, ethanol-, and autoregulation; involved in amino acid catabolism

1704;Hypothetical protein

1705;Protein required for cell viability

1706;Subunit of the INO80 chromatin remodeling complex

1707;Subunit of the SAGA transcriptional regulatory complex but not present in SAGA-like complex SLIK/SALSA, required for SAGA-mediated inhibition at some promoters

1708;C-5 sterol desaturase, catalyzes the introduction of a C-5(6) double bond into episterol, a precursor in ergosterol biosynthesis; mutants are viable, but cannot grow on non-fermentable carbon sources

1709;Hypothetical protein

1710;Cytosolic serine hydroxymethyltransferase, involved in one-carbon metabolism

1711;RNA exonuclease, required for U4 snRNA maturation; functions redundantly with Rnh70p in 5.85 rRNA maturation, and with Rnh70p and Rex3p in processing of U5 snRNA and RNase P RNA; member of RNase D family of exonucleases

1712;Beta subunit of cytoplasmic phenylalanyl-tRNA synthetase, forms a tetramer with Frs2p to generate the active enzyme; evolutionarily distant from mitochondrial phenylalanyl-tRNA synthetase based on protein sequence, but substrate binding is similar

1713;Hypothetical protein

1714;Hypothetical protein

1715;Subunit of signal peptidase complex (Spclp, Spc2p, Spc3p, Sec11p) which catalyzes cleavage of N-terminal signal sequences of proteins targeted to the secretory pathway; homologous to mammalian SPC22/23

1716;Specific translational activator for the COX1 mRNA, also influences stability of intron-containing COX1 primary transcripts; located in the mitochondrial inner membrane

1717;Protein of unknown function, required for survival upon exposure to K1 killer toxin; involved in processing the 35S rRNA primary transcript to generate the 20S and 27SA2 pre-rRNA transcripts

1718;Xylitol dehydrogenase, converts xylitol to D-xylulose in the endogenous xylose utilization pathway

1719;Component of RNA polymerase II holoenzyme/mediator complex; affects chromatin structure and transcriptional regulation of diverse genes; required for glucose repression, HO repression, RME1 repression and sporulation

1720;Hypothetical protein

1721;Protein component of the large (60S) ribosomal subunit, responsible for joining the 40S and 60S subunits; regulates translation initiation; has similarity to rat L10 ribosomal protein and to members of the QM gene family

1722;The authentic, non-tagged protein was localized to the mitochondria

1723;N-glycosylated protein involved in the maintenance of bud site selection during bipolar budding; localization requires Raxlp

1724;Nuclear actin-related protein involved in chromatin remodeling, component of chromatin-remodeling enzyme complexes

1725;Putative chaperone, homolog of E. coli DnaJ, closely related to Ydj1p

1726;High affinity sulfate permease; sulfate uptake is mediated by specific sulfate transporters Sullp and Sul2p, which control the concentration of endogenous activated sulfate intermediates

1727;Protein of unknown function

1728;DNA binding transcriptional activator, mediates serine/threonine activation of the catabolic L-serine (L-threonine) deaminase (CHA1); Zinc-finger protein with Zn[2]-Cys[6] fungal-type binuclear cluster domain

1729;Protein of unknown function, null mutation leads to an increase in sensitivity to Calcofluor white; expression of the gene is induced in the presence of isooctane

1730;Hypothetical protein identified by homology. See FEBS Letters [2000] 487:31-36.

1731;3-keto sterol reductase, catalyzes the last of three steps required to remove two C-4 methyl groups from an intermediate in ergosterol biosynthesis; mutants are sterol auxotrophs

1732;Subunit of the Anaphase-Promoting Complex/Cyclosome (APC/C), which is a ubiquitin-protein ligase required for degradation of anaphase inhibitors, including mitotic cyclins, during the metaphase/anaphase transition

1733;DNA replication initiation factor; recruited to MCM pre-RC complexes at replication origins; promotes release of MCM from Mcm10p, recruits elongation machinery; mutants in human homolog may cause velocardiofacial and DiGeorge syndromes1734;Subunit of the tRNA splicing endonuclease, which is composed of Sen2p, Sen15p, Sen34p, and Sen54p; Sen2p contains the active site for tRNA 5' splice site cleavage and has similarity to Sen34p and to Archaeal tRNA splicing endonuclease

1735;Hypothetical protein

1736;Thiol-specific peroxiredoxin, reduces hydroperoxides to protect against oxidative damage; function in vivo requires covalent conjugation to Urmlp

1737;Aspartic protease, attached to the plasma membrane via a glycosylphosphatidylinositol (GPI) anchor

1738;Hypothetical protein

1739;Subunit of the Anaphase-Promoting Complex/Cyclosome (APC/C), which is a ubiquitin-protein ligase required for degradation of anaphase inhibitors, including mitotic cyclins, during the metaphase/anaphase transition; similar to cullin Cdc53p

1740;Nucleolar protein, specifically associated with the U3 snoRNA, part of the large ribonucleoprotein complex known as the small subunit (SSU) processome, required for 18 S rRNA biogenesis, part of the active pre-rRNA processing complex

1741;Low-affinity zinc transporter of the plasma membrane; transcription is induced under low-zinc conditions by the Zap1p transcription factor

1742;Choline kinase, catalyzes the first step in the CDP-choline pathway phosphatidylcholine synthesis (Kennedy pathway); mRNA expression is regulated by inositol and choline, enzyme activity is stimulated by phosphorylation by protein kinase,

1743;Minor isoform of pyruvate decarboxylase, key enzyme in alcoholic fermentation, decarboxylates pyruvate to acetaldehyde, regulation is glucose- and ethanol-dependent, repressed by thiamine, involved in amino acid catabolism

1744;mRNA-binding protein expressed during iron starvation; binds to a sequence element in the 3'-untranslated regions of specific mRNAs to mediate their degradation; involved in iron homeostasis

1745;Na+/H+ antiporter involved in sodium and potassium efflux through the plasma membrane; required for alkali cation tolerance at acidic pH

1746;Mitochondrial membrane protein required for assembly of respiratory-chain enzyme complexes III and IV; coordinates expression of mitochondrially-encoded genes; may facilitate delivery of mRNA to membrane-bound translation machinery

1747;Hypothetical protein

1748;Protein required for cell viability, component of RNase MRP, which is involved in RNA processing in mitochondria

1749;Spermine synthase, required for the biosynthesis of spermine and also involved in biosynthesis of pantothenic acid

1750;Vacuolar peripheral membrane protein that promotes vesicular docking/fusion reactions in conjunction with SNARE proteins, required for vacuolar biogenesis, forms complex with Pep5p that mediates protein transport to the vacuole

1751 ;Hypothetical protein

1752;Protein that binds G4 quadruplex and purine motif triplex nucleic acid; acts with Cdc13p to maintain telomere structure; interacts with ribosomes and subtelomeric Y' DNA; multicopy suppressor of tom 1 and pop2 mutations

1753;Hypothetical protein

1754;Ribonuclease H2 subunit, required for RNase H2 activity

1755;Smaller subunit of the mitochondrial processing protease, essential processing enzyme that cleaves the N-terminal targeting sequences from mitochondrially imported proteins

1756;Fusion protein that is cleaved to yield a ribosomal protein of the small (40S) subunit and ubiquitin; ubiquitin may facilitate assembly of the ribosomal protein into ribosomes; interacts genetically with translation factor eIF2B

1757;possibly involved in intramitochondrial sorting

1758;Small subunit of the clathrin-associated adaptor complex AP-1, which is involved in protein sorting at the trans-Golgi network; homolog of the sigma subunit of the mammalian clathrin AP-1 complex

1759;Methyltransferase required for synthesis of diphthamide, which is a modified histidine residue of translation elongation factor 2 (Eft1p or Eft2p); not essential for viability; GFP-Dph5p fusion protein localizes to the cytoplasm

1760;Component of box H/ACA small nucleolar ribonucleoprotein particles (snoRNPs), probable rRNA pseudouridine synthase, binds to snoRNP Nop10p and also interacts with ribosomal biogenesis protein Nop53p

1761;Hypothetical protein

1762;Carboxypeptidase Y inhibitor, function requires acetylation by the NatB N-terminal acetyltransferase; phosphatidylethanolamine-binding protein involved in protein kinase A signaling pathway

1763;Protein of unknown function, transcription is activated by paralogous transcription factors Yrm1p and Yrr1p along with genes involved in multidrug resistance

1764;S-adenosylmethionine synthetase, catalyzes transfer of the adenosyl group of ATP to the sulfur atom of methionine; one of two differentially regulated isozymes (Samlp and Sam2p)

1765;Transcription factor that binds to a number of promoter regions, particularly promoters of some genes involved in pheromone response and cell cycle; potential Cdc28p substrate; expression is induced in G1 by bound SBF

1766;Protein required for the maturation of the 18S rRNA and for 40S ribosome production; associated with spindle/microtubules; nuclear localization depends on physical interaction with Nop14p; may bind snoRNAs

1767;UDP-glucose:sterol glucosyltransferase, conserved enzyme involved in synthesis of sterol glucoside membrane lipids, involved in autophagy

1768;Phosphorylated protein of the mitochondrial outer membrane, localizes only to mitochondria of the bud; interacts with Myo2p to mediate mitochondrial distribution to buds; mRNA is targeted to the bud via the transport system involving She2p

1769;Dual function protein involved in translation initiation as a substoichiometric component of eukaryotic translation initiation factor 3 (eIF3) and required for processing of 20S pre-rRNA; binds to eIF3 subunits Rpglp and Prt1p and 18S rRNA

1770;Hypothetical protein

1771;N-myristoyl transferase, catalyzes the cotranslational, covalent attachment of myristic acid to the N-terminal glycine residue of several proteins involved in cellular growth and signal transduction

1772;Protein with WD-40 repeats involved in rRNA processing; associates with transacting ribosome biogenesis factors; similar to beta-transducin superfamily

1773;Essential evolutionarily-conserved nucleolar protein component of the box C/D snoRNP complexes that direct 2'-O-methylation of pre-rRNA during its maturation; overexpression causes spindle orientation defects

1774;Hypothetical protein

1775;Subunit of the heterohexameric Gim/prefoldin protein complex involved in the folding of alpha-tubulin, beta-tubulin, and actin

1776;Nuclear encoded protein required for translation of COX1 mRNA; binds to Cox1 protein

1777;Resident protein of the ER membrane that plays a central role in ER-associated protein degradation (ERAD), forms HRD complex with Hrd1p and ERAD determinants that engages in lumen to cytosol communication and coordination of ERAD events

1778;Component of both the Nup84 nuclear pore sub-complex and of the COPII complex (Sarlp, Sec13p, Sec16p, Sec23p, Sec24p, Sec31p, Sfb2p, and Sfb3p) which is important for the formation of ER to Golgi transport vesicles

1779;Purine nucleoside phosphorylase, specifically metabolizes inosine and guanosine nucleosides

1780;Ferric reductase and cupric reductase, reduces siderophore-bound iron and oxidized copper prior to uptake by transporters; expression induced by low copper and iron levels

1781;Protein of unknown function, green fluorescent protein (GFP)-fusion protein localizes to the cell periphery; msc3 mutants are defective in directing meiotic recombination events to homologous chromatids; potential Cdc28p substrate

1782;putative vacuolar Fe2+/Mn2+ transporter; suppresses respiratory deficit of yfh1 mutants, which lack the ortholog of mammalian frataxin, by preventing mitochondrial iron accumulation

1783;Nucleolar protein, component of the small subunit (SSU) processome containing the U3 snoRNA that is involved in processing of pre-18S rRNA

1784;Hypothetical protein

1785;Hypothetical protein

1786;Cyclin for the Sgvlp (Bur1p) protein kinase; Sgvlp and Bur2p comprise a CDK-cyclin complex involved in transcriptional regulation through its phosphorylation of the carboxy-terminal domain of the largest subunit ofRNApolymerase II

1787;Small rho-like GTPase, essential for establishment and maintenance of cell polarity; mutants have defects in the organization of actin and septins

1788;Kynureninase, required for biosynthesis of nicotinic acid from tryptophan via kynurenine pathway

1789;DNA Topoisomerase III, conserved protein that functions in a complex with Sgs1p and Rmilp to relax single-stranded negatively-supercoiled DNA preferentially, involved in telomere stability and regulation of mitotic recombination

1790;Protein involved in G1 cell cycle arrest in response to pheromone, in a pathway different from the Farlp-dependent pathway; interacts with Far3p, Far7p, Far8p, Far9p, and Far11p; potential Cdc28p substrate

1791;Protein required for normal intracellular sterol distribution and for sphingolipid metabolism; similar to Nup120p and C. elegans R05H5.5 protein

1792;Methionine aminopeptidase, catalyzes the cotranslational removal of N-terminal methionine from nascent polypeptides; function is partially redundant with that of Map2p

1793;Protein kinase involved in the response to oxidative and osmotic stress; identified as suppressor of S. pombe cell cycle checkpoint mutations

1794;Translational elongation factor, stimulates the binding of aminoacyl-tRNA (AA-tRNA) to ribosomes by releasing EF-1 alpha from the ribosomal complex; contains two ABC cassettes; binds and hydrolyses ATP

1795;Protein of unknown function; green fluorescent protein (GFP)-fusion protein localizes to the cytoplasm in a punctate pattern

1796;Protein required for ethanol metabolism; induced by heat shock and localized to the inner mitochondrial membrane; homologous to mammalian peroxisomal membrane protein Mpv17

1797;Hypothetical protein

1798;homolog of nuclear distribution factor NudE, NUDEL

1799;GTPase, Ras-like GTP binding protein involved in the secretory pathway, required for fusion of endosome-derived vesicles with the late Golgi, maturation of the vacuolar carboxypeptidase Y; has similarity to the human GTPase, Rab6

1800;Similar to C. elegans protein

1801;Protein component of the small (40S) ribosomal subunit; nearly identical to Rps28Bp and has similarity to rat S28 ribosomal protein

1802;R-SNARE protein; assembles into SNARE complex with Bet1p, Bos1p and Sed5p; cycles between the ER and Golgi complex; involved in anterograde and retrograde transport between the ER and Golgi; synaptobrevin homolog

1803;Non-essential hydrolase involved in mRNA decapping, may function in a feedback mechanism to regulate deadenylation, contains pyrophosphatase activity and a HIT (histidine triad) motif; interacts with neutral trehalase Nth1p

1804;Non-SMC subunit of the condensin complex (Smc2p-Smc4p-Ycs4p-Brn1p-Ycglp); required for establishment and maintenance of chromosome condensation, chromosome segregation, chromatin binding of condensin and silencing at the mating type locus

1805;Component of the hexameric MCM complex, which is important for priming origins of DNA replication in G1 and becomes an active ATP-dependent helicase that promotes DNA melting and elongation when activated by Cdc7p-Dbf4p in S-phase

1806;ATP-dependent RNA helicase of the DEAD-box family involved in biogenesis of the 60S ribosomal subunit

1807;Putative endonuclease, subunit of the mRNA cleavage and polyadenylation specificity complex required for 3' processing of mRNAs

1808;Putative nicotinamide N-methyltransferase, has a role in rDNA silencing and in lifespan determinationj 1809;Endochitinase, required for cell separation after mitosis; transcriptional activation during late G and early M cell cycle phases is mediated by transcription factor Ace2p

1810;DNA damage and meiotic pachytene checkpoint protein; subunit of a heterotrimeric complex (Rad17p-Mec3p-Ddc1p) that forms a sliding clamp, loaded onto partial duplex DNA by a clamp loader complex; homolog of human and S. pombe Hus1

1811;Mitochondrial GTPase of unknown function, similar to E. coli elongation factor-type GTP-binding protein LepA and to LK1236.1 from Caenorhabditis elegans

1812;Non-essential subunit of Sec63 complex (Sec63p, Sec62p, Sec66p and Sec72p); with Sec61 complex, Kar2p/BiP and Lhslp forms a channel competent for SRP-dependent and post-translational SRP-independent protein targeting and import into the ER

1813;GTP binding protein (mammalian Ranp homolog) involved in the maintenance of nuclear organization, RNA processing and transport; regulated by Prp20p, Rna1p, Yrb1p, Yrb2p, Yrp4p, Yrb30p, Cselp and Kap95p; yeast Gsp2p homolog

1814;Subunit h of the F0 sector of mitochondrial F1F0 ATP synthase, which is a large, evolutionarily conserved enzyme complex required for ATP synthesis

1815;Component of the U1 snRNP complex required for pre-mRNA splicing; putative ortholog of human U1C protein, which is involved in formation of a complex between U1 snRNP and the pre-mRNA 5' splice site

1816;Major exo-1,3-beta-glucanase of the cell wall, involved in cell wall beta-glucan assembly; exists as three differentially glycosylated isoenzymes

1817;O-acetyl homoserine-O-acetyl serine sulfhydrylase, required for sulfur amino acid synthesis

1818;Phosphatidylinositol-4-kinase that functions in the Pkc1p protein kinase pathway; required for normal vacuole morphology, cell wall integrity, and actin cytoskeleton organizationj 1819;Chitin deacetylase, together with Cda2p involved in the biosynthesis ascospore wall component, chitosan; required for proper rigidity of the ascospore wall 1820;Identified by homology to Ashbya gossypii

1821;Membrane bound guanine nucleotide exchange factor (GEF or GDP-release factor); indirectly regulates adenylate cyclase through activation of Ras1p and Ras2p by stimulating the exchange of GDP for GTP; required for progression through G1

1822;Component of the septin ring of the mother-bud neck that is required for cytokinesis; septins recruit proteins to the neck and can act as a barrier to diffusion at the membrane, and they comprise the 10nm filaments seen with EM

1823;Subunit of the RSC chromatin remodeling complex required for kinetochore function in chromosome segregation; essential gene required for cell cycle progression; phosphorylated in the G1 phase of the cell cycle; Snf5p paralog

1824;Hypothetical protein

1825;Hypothetical protein

1826;Protein involved in early stages of meiotic recombination; required for chromosome synapsis; forms a complex with Rec104p and Spo11p necessary during the initiation of recombination

1827;Protein of unknown function, involved in chitin biosynthesis by regulating Chs3p localization, also involved in cell fusion during mating

1828;O-glycosylated plasma membrane protein that acts as a sensor for cell wall integrity signaling and activates the pathway; interacts with Rom2p, a guanine nucleotide exchange factor for Rho1p, and with cell integrity pathway protein Zeo1p

1829;Protein component of the small (40S) ribosomal subunit; nearly identical to Rps25Ap and has similarity to rat S25 ribosomal protein

1830;Conserved ribosomal protein P0 similar to rat P0, human P0, and E. coli L10e; shown to be phosphorylated on serine 302

1831;Protein of unknown function; expression regulated by PDR1

1832;Karyopherin beta, forms a dimeric complex with Srplp (Kap60p) that mediates nuclear import of cargo proteins via a nuclear localization signal (NLS), interacts with nucleoporins to guide transport across the nuclear pore complex

1833;Nit protein, one of two proteins in S. cerevisiae with similarity to the Nit domain of NitFhit from fly and worm and to the mouse and human Nit protein which interacts with the Fhit tumor suppressor; nitrilase superfamily member

1834;Hypothetical protein

1835;Protein involved in bud-site selection; diploid mutants display a unipolar budding pattern instead of the wild-type bipolar pattern, and bud at the proximal pole

1836;Transaldolase, enzyme in the non-oxidative pentose phosphate pathway; converts sedoheptulose 7-phosphate and glyceraldehyde 3-phosphate to erythrose 4-phosphate and fructose 6-phosphate

1837;Hypothetical protein

1838;One of 15 subunits of the 'Remodel the Structure of Chromatin' (RSC) complex; required for expression of mid-late sporulation-specific genes; contains two essential bromodomains, a bromo-adjacent homology (BAH) domain, and an AT hook

1839;Adenylosuccinate lyase, catalyzes two steps in the 'de novo' purine nucleotide biosynthetic pathway

1840;Part of a Vps34p phosphatidylinositol 3-kinase complex that functions in carboxypeptidase Y (CPY) sorting; binds Vps30p and Vps34p to promote production of phosphatidylinositol 3-phosphate (PtdIns3P) which stimulates kinase activity

1841;Putative phosphoesterase that functions as a dosage-dependent positive regulator of the G1/S phase transition by controlling the timing of START

1842;Identified by expression profiling and mass spectrometry

1843;Protein interacting with Nam7p, may be involved in the nonsense-mediated mRNA decay pathway

1844;Hypothetical protein

1845;Subunit of the ARP2/3 complex, which is required for the motility and integrity of cortical actin patches

1846;GDP/GTP exchange protein (GEP) for Rholp and Rho2p; mutations are synthetically lethal with mutations in rom1, which also encodes a GEP

1847;Glycosylated integral membrane protein localized to the plasma membrane; plays a role in fructose-1,6-bisphosphatase (FBPase) degradation; involved in FBPase transport from the cytosol to Vid (vacuole import and degradation) vesicles

1848;essential subunit of Sec61 complex (Sec61p, Sbh1p, and Sss1p); forms a channel for SRP-dependent protein import and retrograde transport of misfolded proteins out of the ER; with Sec63 complex allows SRP-independent protein import into ER

1849;Phosphatidylinositol transfer protein with a potential role in lipid turnover; interacts specifically with thioredoxin peroxidase (Tsa2p) and may have a role in oxidative stress resistance

1850;Mitochondrial leucyl-tRNA synthetase, also has a direct role in splicing of several mitochondrial group I introns; indirectly required for mitochondrial genome maintenance

1851;Protein involved in structural maintenance of chromosomes; required for interchromosomal and sister chromatid recombination; homologous to S. pombe rad18

1852;Protein involved in regulated synthesis of PtdIns(3,5)P(2), in control of trafficking of some proteins to the vacuole lumen via the MVB, and in maintenance of vacuole size and acidity; activator of Fab1p

1853;Protein of unknown function, similar to Reilp but not involved in bud growth; contains dispersed C2H2 zinc finger domains

1854;Protein component of the small (40S) ribosomal subunit; nearly identical to Rps29Bp and has similarity to rat S29 and E. coli S14 ribosomal proteins

1855;Covalently linked cell wall glycoprotein, present in the inner layer of the cell wall

1856;Mitochondrial inner membrane protein required for assembly of the F0 sector of mitochondrial F1F0 ATP synthase, interacts genetically withATP6

1857;Subunit VIII of cytochrome c oxidase, which is the terminal member of the mitochondrial inner membrane electron transport chain

1858;ATP-binding protein that is a subunit of the homotypic vacuole fusion and vacuole protein sorting (HOPS) complex; essential for membrane docking and fusion at both the Golgi-to-endosome and endosome-to-vacuole stages of protein transport

1859;ATPase of the CDC48/PAS1/SEC18 (AAA) family, forms a hexameric complex; may be involved in degradation of aberrant mRNAs

1860;Protein involved in transcription initiation at TATA-containing promoters; associates with the basal transcription factor TFIID; contains two bromodomains; corresponds to the C-terminal region of mammalian TAF1; redundant with Bdf2p

1861;Hypothetical protein

1862;Protein of unknown function probably involved in the function of the cortical actin cytoskeleton; putative ortholog of S. pombe asp1+

1863;High-affinity copper transporter of the plasma membrane, acts as a trimer; gene is disrupted by a Ty2 transposon insertion in many laboratory strains of S. cerevisiae

1864;Hypothetical protein

1865;Protein of unknown function; green fluorescent protein (GFP)-fusion protein localizes to the cell periphery, cytoplasm, and bud

1866;Hypothetical protein

1867;Protein that interacts with and is required for activation of Apglp protein kinase; involved in autophagy but not in the Cvt (cytoplasm to vacuole targeting) pathway

1868;Essential protein that forms a dimer with Ntr2p; also forms a trimer, with Ntr2p and Prp43p, that is involved in spliceosome disassembly; found also in a multisubunit complex with the splicing factor Clflp; suppressor of prp38-1 mutation

1869;Guanine nucleotide exchange factor (GEF) that functions to modulate Rhop1 activity as part of the cell integrity signaling pathway; multicopy suppressor of tor2 mutation and ypk1 ypk2 double mutation; potential Cdc28p substrate

1870;DNA-3-methyladenine glycosidase II that catalyzes of the hydrolysis of alkylated DNA

1871;Coronin, cortical actin cytoskeletal component that associates with the Arp2p/Arp3p complex to regulate its activity

1872;Nuclear protein, putative helicase required for processing of tRNAs, rRNAs, and small nuclear RNAs; potential Cdc28p substrate

1873;Inosine monophosphate dehydrogenase, catalyzes the first step of GMP biosynthesis, member of a four-gene family in S. cerevisiae, constitutively expressed

1874;Non-essential protein of unknown function

1875;Hypothetical protein

1876;Lsm (Like Sm) protein; part of heteroheptameric complexes (Lsm2p-7p and either Lsm1p or 8p): cytoplasmic Lsm1p complex involved in mRNA decay; nuclear Lsm8p complex part of U6 snRNP and possibly involved in processing tRNA, snoRNA, and rRNA

1877;Mitochondrial ribosomal protein of the large subunit

1878;Protein of unknown function proposed to be involved in protein secretion

1879;Ribosomal protein 10 (rp10) of the small (40S) subunit; nearly identical to Rps1Bp and has similarity to rat S3a ribosomal protein

1880;Subunit D of the five-subunit V0 integral membrane domain of vacuolar H+-ATPase (V-ATPase), an electrogenic proton pump found in the endomembrane system; stabilizes VO subunits; required for V1 domain assembly on the vacuolar membrane

1881;Nuclear protein, putative peptidyl-prolyl cis-trans isomerase (PPIase) with similarity to Fpr3p; overproduction suppresses the growth defect resulting from the absence of E3 ubiquitin-protein ligase Tom1p

1882;Zinc-finger transcription factor that regulates genes involved in branched chain amino acid biosynthesis and ammonia assimilation; positively regulated by alpha-isopropylmalate, an intermediate in leucine biosynthesis

1883;GTPase-activating protein for Gpa1p, regulates desensitization to alpha factor pheromone; also required to prevent receptor-independent signaling of the mating pathway; member of the RGS (regulator of G-protein signaling) family

1884;Hypothetical protein

1885;Component of the mitotic apparatus containing a coiled-coil domain, essential for the G2/M transition

1886;GPI transamidase subunit, involved in attachment of glycosylphosphatidylinositol (GPI) anchors to proteins; may have a role in recognition of the attachment signal or of the lipid portion of GPI

1887;Glycoprotein required for oxidative protein folding in the endoplasmic reticulum

1888;Protein of unknown function, green fluorescent protein (GFP)-fusion protein localizes to the endoplasmic reticulum; msc1 mutants are defective in directing meiotic recombination events to homologous chromatids

1889;One of 15 subunits of the 'Remodel the Structure of Chromatin' (RSC) complex; DNA-binding protein involved in the synthesis of rRNA and in transcriptional repression and activation of genes regulated by the Target of Rapamycin (TOR) pathway

1890;Essential protein required for maturation of Gas1p and Pho8p; GFP-fusion protein localizes to the endoplasmic reticulum; null mutants have a cell separation defect

1891;Cytoplasmic GTP binding protein and negative regulator of the Ran/Tc4 GTPase cycle, through its homolog and binding partner, Gtr2p; involved in phosphate transport and invasive growth; human RagA and RagB proteins are functional homologs

1892;NADH:ubiquinone oxidoreductase, transfers electrons from NADH to ubiquinone in the respiratory chain but does not pump protons, in contrast to the higher eukaryotic multisubunit respiratory complex I which is absent in S. cerevisiae

1893;Protein of unknown function, potential Cdc28p substrate

1894;Putative RNA-binding protein, based on computational analysis of large-scale protein-protein interaction data

1895;DNA binding protein that recognizes oligo(dA).oligo(dT) tracts; Arg side chain in its N-terminal pentad Gly-Arg-Lys-Pro-Gly repeat is required for DNA-binding; not essential for viability

1896;gamma subunit of C-terminal domain kinase I (CTDK-1), which phosphorylates the C-terminal repeated domain of the RNA polymerase II large subunit (Rpo21p) to affect both transcription and pre-mRNA 3' end processing

1897;Component of the Rsp5p E3-ubiquitin ligase complex, involved in intracellular amino acid permease sorting, functions in heat shock element mediated gene expression, essential for growth in stress conditions, functional homolog of BUL1

1898;2-hexaprenyl-6-methoxy-1,4-benzoquinone methyltransferase, involved in ubiquinone (Coenzyme Q) biosynthesis; located in mitochondria

1899;Protein that interacts with silencing proteins at the telomere, involved in transcriptional silencing; paralog of Zds1p

1900;Large subunit of trehalose 6-phosphate synthase (Tpslp)/phosphatase (Tps2p) complex, which converts uridine-5'-diphosphoglucose and glucose 6 phosphate to trehalose, homologous to Tps3p and may share function

1901;Zinc-finger transcription factor of the Zn(2)-Cys(6) binuclear cluster domain type, involved in the regulation of arginine-responsive genes; acts with Arg80p and Arg82p

1902;A guanine nucleotide exchange factor involved in vesicle-mediated vacuolar protein transport; specifically stimulates the intrinsic guanine nucleotide exchange activity of Vps21p/Rab5: similar to mammalian ras inhibitors; binds ubiquitin

1903;Single-stranded DNA endonuclease (with Rad1p), cleaves single-stranded DNA during nucleotide excision repair and double-strand break repair; subunit of Nucleotide Excision Repair Factor 1 (NEF1); homolog of human ERCC1 protein

1904;Nucleolar protein, component of the small subunit (SSU) processome containing the U3 snoRNA that is involved in processing of pre-18S rRNA

1905;Hypothetical protein

1906;D-Arabinono-1,4-lactone oxidase, catalyzes the final step in biosynthesis of D-erythroascorbic acid, which is protective against oxidative stress

1907;Hypothetical protein

1908;Subunit of the mitochondrial F1F0 ATP synthase, which is a large enzyme complex required for ATP synthesis; termed subunit I or subunit j; does not correspond to known ATP synthase subunits in other organisms

1909;Dihydrouridine synthase, member of a widespread family of conserved proteins including Smm1p, Dus3p, and Dus4p; modifies pre-tRNA(Phe) at U17

1910;Mitochondrial peptidyl-prolyl cis-trans isomerase (cyclophilin), catalyzes the cis-trans isomerization of peptide bonds N-terminal to proline residues; involved in protein refolding after import into mitochondria

1911;Component of the TRAPP (transport protein particle) complex, which plays an essential role in the vesicular transport from endoplasmic reticulum to Golgi

1912;Component of the conserved oligomeric Golgi complex (Coglp through Cog8p), a cytosolic tethering complex that functions in protein trafficking to mediate fusion of transport vesicles to Golgi compartments

1913;Subunit of the heterodimeric FACT complex (Spt16p-Pob3p), which facilitates RNA Polymerase II transcription elongation through nucleosomes by destabilizing and then reassembling nucleosome structure

1914;Protein that modulates the efficiency of translation termination, interacts with translation release factors eRF1 (Sup45p) and eRF3 (Sup35p) in vitro, contains a zinc finger domain characteristic of the TRIAD class of proteins

1915;Protein of unknown function involved in the assembly of the prospore membrane during sporulation

1916;Largest subunit of the origin recognition complex, which directs DNA replication by binding to replication origins and is also involved in transcriptional silencing; may be phosphorylated by Cdc28p

1917;GTP-binding protein of the ras superfamily involved in termination of M-phase; controls actomyosin and septin dynamics during cytokinesis

1918;Ribosomal protein 10 (rp10) of the small (40S) subunit; nearly identical to Rps1Ap and has similarity to rat S3a ribosomal protein

1919;Mitochondrial glycosylase/lyase that specifically excises 7,8-dihydro-8-oxoguanine residues located opposite cytosine or thymine residues in DNA, repairs oxidative damage to mitochondrial DNA

1920;Ribonucleotide reductase inhibitor involved in regulating dNTP production; regulated by Mec1p and Rad53p during DNA damage and S phase

1921;Cytochrome b2 (L-lactate cytochrome-c oxidoreductase), component of the mitochondrial intermembrane space, required for lactate utilization; expression is repressed by glucose and anaerobic conditions

1922;Hypothetical protein

1923;Integral membrane protein localized to cortical patch structures; multicopy suppressor of rvs167 mutation; mutants show defects in sporulation and altered sphingolipid content in plasma membrane

1924;Transcriptional regulator involved in the repression of GAL genes in the absence of galactose; inhibits transcriptional activation by Gal4p; inhibition relieved by Gal3p or Gallp binding

1925;U1 snRNP protein involved in splicing, contains multiple tetriatricopeptide repeats

1926;Protein required for rDNA transcription by RNA polymerase I, component of the core factor (CF) of rDNA transcription factor, which also contains Rrn6p and Rm7p

1927;Carnitine acetyl-CoA transferase present in both mitochondria and peroxisomes, transfers activated acetyl groups to carnitine to form acetylcarnitine which can be shuttled across membranes

1928;Protein of unknown function, component of the Swr1p complex that incorporates Htz1p into chromatin; required for vacuolar protein sorting

1929;Putative nucleotide sugar transporter, has similarity to Vrg4p

1930;Protein that stimulates strand exchange by facilitating Rad51p binding to single-stranded DNA; anneals complementary single-stranded DNA; involved in the repair of double-strand breaks in DNA during vegetative growth and meiosis

1931 hypothetical protein

1932;Homeodomain-containing transcriptional repressor, binds to Mcm1p and to early cell cycle boxes (ECBs) in the promoters of cell cycle-regulated genes expressed in M/G1 phase; expression is cell cycle-regulated; potential Cdc28p substrate

1933;Adenine phosphoribosyltransferase, catalyzes the formation of AMP from adenine and 5-phosphoribosylpyrophosphate; involved in the salvage pathway of purine nucleotide biosynthesis

1934;Hypothetical protein

1935;Subunit of the oligosaccharyltransferase complex of the ER lumen, which catalyzes asparagine-linked glycosylation of newly synthesized proteins; similar to and partially functionally redundant with Ost3p

1936;Hypothetical protein

1937;TFIID subunit (40 kDa), involved in RNA polymerase II transcription initiation, similar to histone H3 with atypical histone fold motif of Spt3-like transcription factors

1938;UBX (ubiquitin regulatory X) domain-containing protein that interacts with Cdc48p, has a ubiquitin-associated (UBA) domain, interacts with ubiquitylated proteins in vivo, and is required for degradation of a ubiquitylated model substrate

1939;Protein that forms a heterotrimeric complex with Erp1, Erp2p, and Emp24, member of the p24 family involved in endoplasmic reticulum to Golgi transport

1940;Hypothetical protein

1941;Hypothetical protein identified by homology. See FEBS Letters [2000] 487:31-36.

1942;Basic leucine zipper (bZIP) transcription factor required for oxidative stress tolerance; mediates pleiotropic drug and metal resistance; localized to the nucleus in response to the presence of oxidants

1943;CAAX box containing protein of unknown function, proposed to be involved in the RAS/cAMP signaling pathway

1944;GTPase; GTP-binding protein of the rab family; required for homotypic fusion event in vacuole inheritance, for endosome-endosome fusion, similar to mammalian Rab7

1945;Polo-like kinase with similarity to Xenopus Plx1 and S. pombe Plo1p; found at bud neck, nucleus and SPBs; has multiple functions in mitosis and cytokinesis through phosphorylation of substrates; may be a Cdc28p substrate

1946;Hypothetical protein

1947;Hypothetical protein

1948;Protein required for sorting proteins to the vacuole; overproduction of Mvp1p suppresses several dominant VPS1 mutations; Mvp1p and Vps1p act in concert to promote membrane traffic to the vacuole

1949;TFIID subunit (48 kDa), involved in RNA polymerase II transcription initiation; potential Cdc28p substrate

1950;Acireductone dioacygenease involved in the methionine salvage pathway; ortholog of human MTCBP-1

1951;eIF3 component of unknown function; deletion causes defects in mitochondrial organization but not in growth or translation initiation, can rescue cytokinesis; and mitochondrial organization defects of the Dictyostelium cluA-mutant

1952;Dolichol kinase, catalyzes the terminal step in dolichyl monophosphate (Dol-P) biosynthesis; required for viability and for normal rates of lipid intermediate synthesis and protein N-glycosylation

1953;Protein involved in bud-site selection; diploid mutants display a random budding pattern instead of the wild-type bipolar pattern

1954;C-22 sterol desaturase, a cytochrome P450 enzyme that catalyzes the formation of the C-22(23) double bond in the sterol side chain in ergosterol biosynthesis; may be a target of azole antifungal drugs

1955;Copper-sensing transcription factor involved in regulation of genes required for high affinity copper transport

1956;Ubiquitin conjugating enzyme, involved in the ER-associated protein degradation pathway; requires Cuelp for recruitment to the ER membrane; proposed to be involved in chromatin assembly

1957;RING-finger peroxisomal membrane peroxin that plays an essential role in peroxisome biogenesis and peroxisomal matrix protein import, forms translocation subcomplex with Pex2p and Pex10p

1958;Essential protein involved in the TOR signaling pathway; physically associates with the protein phosphatase 2A and the SIT4 protein phosphatase catalytic subunits

1959;Protein involved in G1 cell cycle arrest in response to pheromone, in a pathway different from the Farlp-dependent pathway; interacts with Far3p, Far7p, Far9p, Far10p, and Far11p

1960;Protein required for respiratory growth; localized to both the nucleus and mitochondrion; mutant displays decreased transcription of specific nuclear and mitochondrial genes whose products are involved in respiratory growth

1961;Protein of unknown function; green fluorescent protein (GFP)-fusion protein localizes to the cytoplasm in a punctate pattern

1962;Catalytic subunit of the mitochondrial inner membrane peptidase complex, required for maturation of mitochondrial proteins of the intermembrane space; complex contains Imp1p and Imp2p (both catalytic subunits), and Somlp

1963;Transcriptional activator related to Msn4p; activated in stress conditions, which results in translocation from the cytoplasm to the nucleus; binds DNA at stress response elements of responsive genes, inducing gene expression

1964;Copper chaperone for superoxide dismutase Sod1p, involved in oxidative stress protection; Met-X-Cys-X2-Cys motif within the N-terminal portion is involved in insertion of copper into Sod1p under conditions of copper deprivation

1965;Transcriptional coactivator, facilitates elongation by influencing enzymes that modify RNAP II, acts in a peroxide resistance pathway involving Rad2p; suppressor of TFIIB mutations

1966;Transcription factor involved in cell-type-specific transcription and pheromone response; plays a central role in the formation of both repressor and activator complexes

1967;Member of a complex (Isw1b) with Iswlp and Ioc2p that exhibits nucleosome-stimulated ATPase activity and acts within coding regions to coordinate transcription elongation with termination and processing, contains a PWWP motif

1968;Protein involved in G1 cell cycle arrest in response to pheromone, in a pathway different from the Farlp-dependent pathway; interacts with Far7p, Far8p, Far9p, Far10p, and Far11p

1969;Component of the mitochondrial outer membrane sorting and assembly machinery (SAM) complex; required for the sorting of some proteins to the outer membrane after import by the TOM complex

1970;Mitochondrial ornithine acetyltransferase, catalyzes the fifth step in arginine biosynthesis; also possesses acetylglutamate synthase activity, regenerates acetylglutamate while forming ornithine

1971;Protein of unknown function, involved in the proteolytic activation of Rim101p in response to alkaline pH; has similarity to A. nidulans PalI; putative membrane protein

1972;UBX (ubiquitin regulatory X) domain-containing protein that interacts with Cdc48p

1973;Integral membrane protein localized to late Golgi vesicles along with the v-SNARE Tlg2p

1974;Putative protein of unknown function; proposed to be involved in resistance to carboplatin and cisplatin; shares similarity to a human cytochrome oxidoreductase

1975;Myristoylated subunit of ESCRTIII, the endosomal sorting complex required for transport of transmembrane proteins into the multivesicular body pathway to the lysosomal/vacuolar lumen; cytoplasmic protein recruited to endosomal membranes

1976;Subunit of a complex with Ctf8p that shares some subunits with Replication Factor C and is required for sister chromatid cohesion; may have overlapping functions with Rad24p in the DNA damage replication checkpoint

1977;Mitochondrial alcohol dehydrogenase isozyme III; involved in the shuttling of mitochondrial NADH to the cytosol under anaerobic conditions and ethanol production

1978;Hypothetical protein

1979;Hypothetical protein

1980;Actin cortical patch component, interacts with the actin depolymerizing factor cofilin; required to restrict cofilin localization to cortical patches; contains WD repeats

1981;Subunit of the CBF3 complex, which binds to the CDE III element of centromeres, bending the DNA upon binding, and may be involved in sister chromatid cohesion during mitosis

1982;Protein involved in vitamin B6 biosynthesis; member of a stationary phase-induced gene family; coregulated with SNO1 interacts with Sno1p and with Yhr198p, perhaps as a multiprotein complex containing other Snz and Sno proteins

1983;Peripheral GTPase of the mitochondrial inner membrane, essential for respiratory competence, likely functions in assembly of the large ribosomal subunit, has homologs in plants and animals

1984;Hypothetical protein

1985;Protein of unknown function, deletion causes multi-budding phenotype; has similarity to Aspergillus nidulans samB gene

1986;Phosphoglucomutase, catalyzes the conversion from glucose-1-phosphate to glucose-6-phosphate, which is a key step in hexose metabolism; functions as the acceptor for a Glc-phosphotransferase

1987;Protein required for survival at high temperature during stationary phase; not required for growth on nonfermentable carbon sources

1988;Acetolactate synthase, catalyses the first common step in isoleucine and valine biosynthesis and is the target of several classes of inhibitors, localizes to the mitochondria; expression of the gene is under general amino acid control

1989;Hypothetical protein

1990;Component of the evolutionarily conserved kinetochore-associated Ndc80 complex (Ndc80p-Nuf2p-Spc24p-Spc25p); involved in chromosome segregation, spindle checkpoint activity and kinetochore clustering

1991;Hypothetical protein

1992;Putative integral membrane E3 ubiquitin ligase; genetic interactions suggest a role in negative regulation of amino acid uptake

1993;Enzyme of 'de novo' purine biosynthesis containing both 5-aminoimidazole-4-carboxamide ribonucleotide transformylase and inosine monophosphate cyclohydrolase activities, isozyme of Ade16p; ade16 ade17 mutants require adenine and histidine

1994;Protein component of the large (60S) ribosomal subunit, nearly identical to Rp115Ap and has similarity to rat L15 ribosomal protein; binds to 5.8 S rRNA

1995;Hypothetical protein

1996;Protein of unknown function; green fluorescent protein (GFP)-fusion protein localizes to the cell periphery, cytoplasm, bud, and bud neck

1997;Protein of unknown function, deletion causes sensitivity to thermal stress

1998;Histone acetyltransferase (HAT) catalytic subunit of the SAS complex (Sas2p-Sas4p-Sas5p), which acetylates free histones and nucleosomes and regulates transcriptional silencing; member of the MYSTacetyltransferase family

1999;Hypothetical protein

2000;Essential nuclear protein involved in early steps of ribosome biogenesis; physically interacts with the ribosomal protein Rp13p

2001;Protein required for cell viability

2002;Protein of unknown function, involved in proteasome-dependent catabolite inactivation of fructose-1,6-bisphosphatase; contains LisH and CTLH domains, like Vid30p; dosage-dependent regulator of START

2003;Protein containing GATA family zinc finger motifs; similar to Gln3p and Da180p; expression repressed by leucine

2004;Required for a post-incision step in the repair of DNA single and double-strand breaks that result from interstrand crosslinks produced by a variety of mono- and bifunctional psoralen derivatives; induced by UV-irradiation

2005;Protein kinase required for signal transduction during entry into meiosis; promotes the formation of the Imelp-Ume6p complex by phosphorylating Ime1p and Ume6p; shares similarity with mammalian glycogen synthase kinase 3-beta

2006;Protein of unknown function; interacts with both the Reglp/Glc7p phosphatase and the Snf1p kinase

2007;Mitochondrial external NADH dehydrogenase, catalyzes the oxidation of cytosolic NADH; Nde1p and Nde2p are involved in providing the cytosolic NADH to the mitochondrial respiratory chain

2008;Subunit of the core complex of translation initiation factor 3(eIF3), which is essential for translation

2009;Hypothetical protein

2010;Delta subunit of the oligosaccharyl transferase glycoprotein complex, which is required for N-linked glycosylation of proteins in the endoplasmic reticulum

2011;Catalytic subunit of the mitochondrial inner membrane peptidase complex, required for maturation of mitochondrial proteins of the intermembrane space; complex contains Imp1p and Imp2p (both catalytic subunits), and Somlp

2012;Protein of unknown function; proposed to be involved in responding to DNA damaging agents

2013;Subunit of the nuclear pore complex (NPC), interacts with karyopherin Kap121p or with Nup170p via overlapping regions of Nup53p, involved in activation of the spindle checkpoint mediated by the Madlp-Mad2p complex

2014;The authentic, non-tagged protein was localized to the mitochondria

2015;Mitochondrial ribosomal protein of the small subunit

2016;Non-essential P-type ATPase that is a potential aminophospholipid translocase, localizes to the trans-Golgi, likely involved in protein transport

2017;Protein of unknown function; green fluorescent protein (GFP)-fusion protein localizes to the cytoplasm in a punctate pattern

2018;Protein required for mismatch repair in mitosis and meiosis, postmeiotic segregation, and spore viability; forms a complex with Pmslp and Msh2p to repair mismatched DNA; human homolog is associated with hereditary non-polyposis colon cancer

2019;Essential kinetochore protein, component of the CBF3 complex that binds the CDEIII region of the centromere; contains an N-terminal Zn2Cys6 type zinc finger domain, a C-terminal acidic domain, and a putative coiled coil dimerization domain

2020;Cytoplasmic aldehyde dehydrogenase, involved in beta-alanine synthesis; uses NAD+ as the preferred coenzyme; very similar to Ald2p; expression is induced by stress and repressed by glucose

2021;Cytoplasmic proteinase A inhibitor, dependent on Pbs2p and Hoglp protein kinases for osmotic induction; intrinsically unstructured, N-terminal half becomes ordered in the active site of proteinase A upon contact

2022;Protein of unknown function whose expression is induced by osmotic stress

2023;Non-essential protein of unknown function, contains ATP/GTP-binding site motif A; null mutant exhibits cellular volume up to four times greater than wild-type, also large drooping buds with elongated necks

2024;Protein of unknown function; open reading frame may be part of a bicistronic transcript with RGM1

2025;Plasma membrane t-SNARE involved in fusion of secretory vesicles at the plasma membrane; syntaxin homolog that is functionally redundant with Sso1p

2026;Cytoplasmic chaperone of the Hsp90 family, redundant in function and nearly identical with Hsp82p, and together they are essential; expressed constitutively at 10-fold higher basal levels that HSP82 and induced 2-3 fold by heat shock

2027;P subunit of the mitochondrial glycine decarboxylase complex, required for the catabolism of glycine to 5,10-methylene-THF; expression is regulated by levels of levels of 5,10-methylene-THF in the cytoplasm

2028;Mitochondrial ribosomal protein of the large subunit

2029;Protein that interacts with the cytoskeleton and is involved in chromatin organization and nuclear transport, interacts genetically with TCP1 and ICY2, required for viability in rich media of cells lacking mitochondrial DNA

2030;Hypothetical protein

2031;Protein involved in cis-Golgi membrane traffic; v-SNARE that interacts with two t-SNARES, Sed5p and Pep12p; required for multiple vacuolar sorting pathways

2032;Kinesin-associated protein required for both karyogamy and mitotic spindle organization, interacts stably and specifically with Kar3p and may function to target this kinesin to a specific cellular role; has similarity to Vik1p

2033;Protein that may be involved in cell wall function; mutations in rot1 cause cell wall defects, suppress tor2 mutations, and are synthetically lethal with rot2 mutations

2034;C-8 sterol isomerase, catalyzes the isomerization of the delta-8 double bond to the delta-7 position at an intermediate step in ergosterol biosynthesis

2035;Component of the TOM (translocase of outer membrane) complex responsible for recognition and initial import steps for all mitochondrially directed proteins; constitutes the core element of the protein conducting pore

2036;Peripheral membrane protein of peroxisomes involved in peroxisomal inheritance

2037;Beta subunit of heterooctameric phosphofructokinase involved in glycolysis, indispensable for anaerobic growth, activated by fructose-2,6-bisphosphate and AMP, mutation inhibits glucose induction of cell cycle-related genes

2038;Mevalonate kinase, acts in the biosynthesis of isoprenoids and sterols, including ergosterol, from mevalonate

2039;Essential protein involved in mtDNA inheritance, may also function in the partitioning of the mitochondrial organelle or in the segregation of chromosomes, exhibits regions similar to members of a GTPase family

2040;Non-essential protein of unknown function; exhibits synthetic lethal genetic interactions with PHO85; green fluorescent protein (GFP)-fusion protein localizes to the cell periphery

2041;Putative 1,3-beta-glucanosyltransferase, has similarity to Gaslp; localizes to the cell wall

2042;SR protein kinase (SRPK) involved in regulating proteins involved in mRNA metabolism and cation homeostasis; similar to human SRPK1

2043;GMP synthase, an enzyme that catalyzes the second step in the biosynthesis of GMP from inosine 5'-phosphate (IMP); transcription is not subject to regulation by guanine but is negatively regulated by nutrient starvation

2044;One of 10 subunits of the transport protein particle (TRAPP) complex of the cis-Golgi which mediates vesicle docking and fusion; involved in ER to Golgi membrane traffic; mutation activates transcription of OCH1

2045;Ubiquitin-specific protease that is a component of the SAGA (Spt-Ada-Gen5-Acetyltransferase) acetylation complex; required for SAGA-mediated deubiquitination of histone H2B

2046;Subunit of a complex with Rad50p and Xrs2p (RMX complex) that functions in repair of DNA double-strand breaks and in telomere stability, exhibits nuclease activity that appears to be required for RMX function; widely conserved

2047;NADP(+)-dependent dehydrogenase; acts on serine, L-allo-threonine, and other 3-hydroxy acids

2048;TFIID subunit (67 kDa), involved in RNA polymerase II transcription initiation

2049;Mitochondrial RNA polymerase sigma-like specificity factor required for promoter recognition, interacts with mitochondrial core polymerase Rpo41p, imported into mitochondria via a novel process requiring most of the Mtflp sequence

2050;Protein required for the synthesis of both 18S and 5.8S rRNA; C-terminal region is crucial for the formation of 18S rRNA and N-terminal region is required for the 5.8S rRNA; component of small ribosomal subunit (SSU) processosome

2051;Ribonuclease H1, removes RNA primers during Okazaki fragment synthesis; degrades RNA attached to the 5'-end of a DNA strand

2052;GTPase activating protein (GAP) for Gsp1p, involved in nuclear transport

2053;Protein that colocalizes with clathrin-coated vesicles; involved in transport at the trans-Golgi

2054;Putative mannosidase, essential glycosylphosphatidylinositol (GPI)-anchored membrane protein required for cell wall biogenesis in bud formation, involved in filamentous growth, homologous to Dcwlp

2055;RNAase III; cleaves a stem-loop structure at the 3' end of U2 snRNA to ensure formation of the correct U2 3' end

2056;Protein required for assembly of U2 snRNP into the spliceosome, forms a complex with Hsh49p and Hsh155p

2057;Vacuolar membrane zinc transporter, transports zinc from the cytosol into the vacuole for storage; also has a role in resistance to zinc shock resulting from a sudden influx of zinc into the cytoplasm

2058;Glutamate decarboxylase, converts glutamate into gamma-aminobutyric acid (GABA) during glutamate catabolism; involved in response to oxidative stress

2059;Protein of unknown function; overexpression suppresses Ca2+ sensitivity of mutants lacking inosito1 phosphorylceramide mannosyltransferases Csglp and Cshlp; transcription is induced under hyperosmotic stress and repressed by alpha factor

2060;Hypothetical protein

2061;Protein of unknown function; green fluorescent protein (GFP)-fusion protein localizes to the cytoplasm in a punctate pattern

2062;Coiled-coiled protein of unknown function, identified as a high-copy suppressor of a dbp5 mutation

2063;Subunit VII of cytochrome c oxidase, which is the terminal member of the mitochondrial inner membrane electron transport chain

2064;Translation initiation factor eIF1 essential protein that forms a complex with Suilp (eIF1) and the 40S ribosomal subunit and scans for the start codon; C-terminus associates with Fun12p (eIF5B); N terminus interacts with eIF2 and eIF3

2065;Regulatory subunit of trehalose-6-phosphate synthase/phosphatase complex, which synthesizes the storage carbohydrate trehalose; expression is induced by stress conditions and repressed by the Ras-cAMP pathway

2066;Mitochondrial inorganic pyrophosphatase, required for mitochondrial function and possibly involved in energy generation from inorganic pyrophosphate

2067;Splicing factor that reanneals U4 and U6 snRNPs during spliceosome recycling

2068;One of two orotate phosphoribosyltransferase isozymes (see also URA5) that catalyze the fifth enzymatic step in the de novo biosynthesis of pyrimidines, converting orotate into orotidine-5'-phosphate

2069;Sphingolipid alpha-hydroxylase, functions in the alpha-hydroxylation of sphingolipid-associated very long chain fatty acids, has both cytochrome b5-like and hydroxylase/desaturase domains, not essential for growth

2070;Type II CAAX prenyl protease involved in the proteolysis and maturation of Ras and the a-factor mating pheromone

2071;Nuclear-enriched ubiquitin-like polyubiquitin-binding protein, required for spindle pole body (SPB) duplication and for transit through the G2/M phase of the cell cycle, involved in proteolysis, interacts with the proteasome

2072;Hypothetical protein

2073;Mitochondrial protein, likely involved in translation of the mitochondrial OLI1 mRNA; exhibits genetic interaction with the OLI1 mRNA 5'-untranslated leader

2074;Subunit of the telomeric Ku complex (Yku70p-Yku80p), involved in telomere length maintenance, structure and telomere position effect; relocates to sites of double-strand cleavage to promote nonhomologous end joining during DSB repair

2075;ATP-dependent RNA helicase; localizes to both the nuclear periphery and nucleolus; highly enriched in nuclear pore complex fractions; constituent of 66S pre-ribosomal particles

2076;Protein of unknown function; green fluorescent protein (GFP)-fusion protein localizes to the cell periphery and bud

2077;Ceramide synthase subunit; single-span ER membrane protein associated with Laglp and Lac1p and required for ceramide synthase activity, null mutant grows extremely slowly and is defective in ceramide synthesis

2078;Phosphoribosylpylphosphate amidotransferase (PRPPAT; amidophosphoribosyltransferase), catalyzes first step of the 'de novo' purine nucleotide biosynthetic pathway

2079;Mitochondrial inner membrane transporter, exports mitochondrially synthesized precursors of iron-sulfur (Fe/S) clusters to the cytosol; member of the ATP-binding cassette (ABC) transporter family

2080;Histone deacetylase; regulates transcription and silencingj 2081;Protein required for efficient mating, member of a family of eukaryotic proteins that contain a domain homologous to Sac1p

2082;Protein of unknown function, potential Cdc28p substrate

2083;Hypothetical protein

2084;Protein of unknown function with similarity to hexose transporter family members, expression is induced by low levels of glucose and repressed by high levels of glucose

2085;Integral subunit of the pre-mRNA cleavage and polyadenylation factor (CPF) complex; plays an essential role in mRNA 3'-end formation by bridging different processing factors and thereby promoting the assembly of the processing complex

2086;Molecular chaperone, required for the assembly of alpha and beta subunits into the F1 sector of mitochondrial F1F0 ATP synthase

2087;Positive regulator of allophanate inducible genes; binds a dodecanucleotide sequence upstream of all genes that are induced by allophanate; contains an UISALL DNA-binding, a transcriptional activation, and a coiled-coil domain

2088;Protein required for cell viability

2089;F-box protein

2090;Mitochondrial ribosomal protein of the small subunit; essential for viability, unlike most other mitoribosomal proteins

2091;Hypothetical protein

2092;Hypothetical protein

2093;Cdc42p activated signal transducing kinase of the PAK (p21-activated kinase) family, involved in septin ring assembly and cytokinesis; directly phosphorylates septins Cdc3p and Cdc10p; other yeast PAK family members are Ste20p and Skm1p

2094;Pseudouridine synthase, catalyzes only the formation of pseudouridine-55 (Psi55), a highly conserved tRNA modification, in mitochondrial and cytoplasmic tRNAs; PUS4 overexpression leads to translational derepression of GCN4 (Gcd- phenotype)

2095;N-glycosylated integral membrane protein of the ER membrane and plasma membrane, required for Ca2+ influx stimulated by pheromone, functions as a stretch-activated Ca2+-permeable cation channel in mammals; forms an oligomer

2096;Pho85 cyclin of the Pcl1,2-like subfamily, involved in entry into the mitotic cell cycle and regulation of morphogenesis, localizes to sites of polarized cell growth

2097;Evolutionarily conserved subunit of the CCR4-NOT complex involved in controlling mRNA initiation, elongation and degradation; binds Cdc39p

2098;Partially redundant sensor-transducer of the stress-activated PKC1-MPK1 signaling pathway involved in maintenance of cell wall integrity and recovery from heat shock; secretory pathway Wsc2p is required for the arrest of secretion response

2099;Heat shock protein regulator that binds to Hsp90p and may stimulate ATPase activity; originally identified as a high-copy number suppressor of a HSP90 loss-of-function mutation; GFP-fusion protein localizes to the cytoplasm and nucleus

2100;C-14 sterol reductase, acts in ergosterol biosynthesis; mutants accumulate the abnormal sterol ignosterol (ergosta-8,14 dienol), and are viable under anaerobic growth conditions but inviable on rich medium under aerobic conditions

2101;L-homoserine-O-acetyltransferase, catalyzes the conversion of homoserine to O-acetyl homoserine which is the first step of the methionine biosynthetic pathway

2102;Putative hydroxyisocaproate dehydrogenase

2103;Guanyl-nucleotide exchange factor for the small G-protein Sec4p, located on cytoplasmic vesicles; essential for post-Golgi vesicle transport

2104;Basic amino acid transporter, involved in uptake of cationic amino acids

2105;Lysine permease; one of three amino acid permeases (Alp1p, Canlp, Lyplp) responsible for uptake of cationic amino acids

2106;Phosphatidylinositol 4-kinase; catalyzes first step in the biosynthesis of phosphatidylinositol-4,5-biphosphate; may control cytokineses through the actin cytoskeleton

2107;Integral membrane protein required for the fusion of ER-derived COPII transport vesicles with the Golgi; interacts with Yip1p and Yos1p; localizes to the Golgi, the ER, and COPII vesicles

2108;Subunit of the origin recognition complex, which directs DNA replication by binding to replication origins and is also involved in transcriptional silencing

2109;Cytosolic copper metallochaperone that transports copper to the secretory vesicle copper transporter Ccc2p for eventual insertion into Fet3p, which is a multicopper oxidase required for high-affinity iron uptake

2110;Putative zinc finger protein with similarity to human CNBP, proposed to be involved in the RAS/cAMP signaling pathway

2111;Protein involved in mRNA export, component of the transcription export (TREX) complex

2112;Mitochondrial ribosomal protein of the large subunit

2113;RNA-binding protein that interacts with the C-terminal domain of the RNA polymerase II large subunit (Rpo21p), required for transcription termination and 3' end maturation of nonpolyadenylated RNAs

2114;rRNA polymerase I subunit A49

2115;Component of a complex containing Ceflp, involved in pre-mRNA splicing; has similarity to S. pombe Cwf25p

2116;Translation initiation factor eIF1; component of a complex involved in recognition of the initiator codon; modulates translation accuracy at the initiation phase

2117;Transmembrane actin-binding protein involved in membrane cytoskeleton assembly and cell polarization; adaptor protein that links actin to clathrin and endocytosis; present in the actin cortical patch of the emerging bud tip; dimer in vivo

2118;Probable type-III integral membrane protein of unknown function, has regions of similarity to mitochondrial electron transport proteins

2119;Similar to globins and has a functional heme-binding domain; involved in glucose signaling or metabolism; regulated by Rgt1p

2120;Targeting subunit for Glc7p protein phosphatase, localized to the bud neck, required for localization of chitin synthase III to the bud neck via interaction with the chitin synthase III regulatory subunit Skt5p

2121;Elongin A, F-box protein that forms a heterodimer with Elc1p and participates in transcription elongation

2122;Component of the spindle pole body outer plaque; required for spindle orientation and mitotic nuclear migration

2123;Hypothetical protein

2124;Transcription/RNA-processing factor that associates with TFIIB and cleavage/polyadenylation factor Ptalp; exhibits phosphatase activity on serine-5 of the RNA polymerase II C-terminal domain; affects start site selection in vivo

2125;Subunit of both RNase MRP, which cleaves pre-rRNA, and nuclear RNase P, which cleaves tRNA precursors to generate mature 5' ends; binds to the RPR1 RNA subunit in Rnase P

2126;Mannosyltransferase, involved in N-linked glycosylation; catalyzes the transfer of mannose from Dol-P-Man to lipid-linked oligosaccharides; mutation of the human ortholog causes type 1 congenital disorders of glycosylation

2127;Hypothetical protein

2128;DNA-binding protein involved in either activation or repression of transcription, depending on binding site context; also binds telomere sequences and plays a role in telomeric position effect (silencing) and telomere structure

2129;Protein that associates with the INO80 chromatin remodeling complex under low-salt conditions

2130;Hypothetical protein

2131;Cytoplasmic protein of unknown function; possibly involved in vacuolar protein degradation; not essential for proteasome-dependent degradation of fructose-1,6-bisphosphatase (FBPase); null mutants exhibit normal growth

2132;Essential serine kinase involved in the processing of the 20S pre-rRNA into mature 18S rRNA; has similarity to Riolp

2133;Protein with a role in regulation of Ty1 transposition

2134;Hypothetical protein; similarity to human TGR-CL10C; thyroidal receptor for N-acetylglucosamine

2135;Hypothetical protein

2136;Hypothetical protein

2137;Ubiquitin-specific protease that deubiquitinates ubiquitin-protein moieties; may regulate silencing by acting on Sir4p; involved in posttranscriptionally regulating Gaplp and possibly other transporters; primarily located in the nucleus

2138;Mitochondrial ribosomal protein of the large subunit

2139;Protein required for cell viability

2140;Non-essential small GTPase of the Rho/Rac subfamily of Ras-like proteins, likely involved in protein kinase C (Pkclp)-dependent signal transduction pathway that controls cell integrity

2141; Hypothetical protein

2142;Protein of unknown function, localizes to the nucleolus and nucleoplasm; contains an RNA recognition motif (RRM) and has similarity to Nop12p, which is required for processing of pre-18S rRNA

2143;Plasma membrane protein involved in G-protein mediated pheromone signaling pathway; overproduction suppresses bem1 mutations

2144;Phosphatidylserine decarboxylase of the mitochondrial inner membrane, converts phosphatidylserine to phosphatidylethanolamine

2145;The authentic, non-tagged protein was localized to mitochondria

2146;Basic leucine zipper (bZIP) transcription factor of the ATF/CREB family that forms a complex with Tup1p and Ssn6p to both activate and repress transcription; cytosolic and nuclear protein involved in the osmotic and oxidative stress responses

2147;Protein involved in organization of septins at the mother-bud neck, may interact directly with the Cdc11p septin, localizes to bud neck in a septin-dependent manner

2148;Hypothetical protein

2149;Hypothetical protein identified by homology. See FEBS Letters [2000] 487:31-36.

2150;Serine/threonine protein kinases that regulates cell morphogenesis pathways; involved in cell wall biosynthesis, apical growth, proper mating projection morphology, bipolar bud site selection in diploid cells, and cell separation

2151;Cell wall-related secretory glycoprotein; induced by nutrient deprivation-associated growth arrest and upon entry into stationary phase; may be involved in adaptation prior to stationary phase entry; has similarity to Sps100p

2152;Predicted membrane protein; genetic interactions suggest a role in negative regulation of amino acid uptake

2153;Hypothetical protein

2154;Protein of unknown function, potential homolog of mammalian Insig 1

2155;Palmitoylated, plasma membrane-bound casein kinase I isoform; shares redundant functions with Yck1p in morphogenesis, proper septin assembly, endocytic trafficking; provides an essential function overlapping with that of Yck1p

2156;Subunit of the heterohexameric cochaperone prefoldin complex which binds specifically to cytosolic chaperonin and transfers target proteins to it

2157;Essential protein required for maturation of Gas1p and Pho8p; GFP-fusion protein localizes to the endoplasmic reticulum (ER) and YFP-fusion protein to the nuclear envelope-ER network; null mutants have a cell separation defect

2158;Alpha-tubulin folding protein, similar to mammalian cofactor B; Alflp-GFP localizes to cytoplasmic microtubules; required for the folding of alpha-tubulin and may play an additional role in microtubule maintenance

2159;Hypothetical protein

2160;Mating pheromone a-factor, made by a cells; interacts with alpha cells to induce cell cycle arrest and other responses leading to mating; biogenesis involves C-terminal modification, N-terminal proteolysis, and export; also encoded by MFA1

2161;Adenine deaminase (adenine aminohydrolase), involved in purine salvage and nitrogen catabolism

2162;CAP (cyclase-associated protein) subunit of adenylyl cyclase complex; N-terminus binds adenylyl cyclase and facilitates activation by RAS; C-terminus binds ADP-actin monomers, facilitating regulation of actin dynamics and cell morphogenesis

2163;Subunit of the NuA4 histone acetyltransferase complex, which acetylates the N-terminal tails of histones H4 and H2A

2164;Peptidyl-prolyl cis-trans isomerase (PPIase), binds to the drugs FK506 and rapamycin; also binds to the nonhistone chromatin binding protein Hmo1p and may regulate its assembly or function

2165;Essential protein of unknown function; heterozygous mutant shows haploinsufficiency in K1 killer toxin resistance

2166;component of the TOM (translocase of outer membrane) complex responsible for initial import of mitochondrially directed proteins; acts as a receptor for precursor proteins and mediates interaction between the TOM and TIM complexes

2167;Nicotinamide riboside kinase, catalyzes the synthesis of nicotinamide nucleotide (NMN) from nicotinamide riboside; involved in a salvage pathway for NAD+ biosynthesis

2168;Protein involved in G1 cell cycle arrest in response to pheromone, in a pathway different from the Farlp-dependent pathway; interacts with Far3p, Far7p, Far8p, Far9p, and Far10p

2169;Protein with similarity to monocarboxylate permeases, appears not to be involved in transport of monocarboxylates such as lactate, pyruvate or acetate across the plasma membrane

2170;shows protein sequence similarity to the mammalian Omi/HtrA2 family of serine proteases

2171;Component of the TOM (translocase of outer membrane) complex responsible for recognition and initial import steps for all mitochondrially directed proteins; acts as a receptor for incoming precursor proteins

2172;Catalytic subunit of the Dcplp-Dcp2p decapping enzyme complex, which removes the 5' cap structure from mRNAs prior to their degradation; member of the Nudix hydrolase family

2173;Malate synthase, enzyme of the glyoxylate cycle, involved in utilization of non-fermentable carbon sources; expression is subject to carbon catabolite repression; localizes in peroxisomes during growth in oleic acid medium

2174;Protein involved in regulating spindle position and orientation, functionally redundant with Dma1p; homolog of S. pombe Dma1 and H. sapiens Chfr 2175;Hypothetical protein

2176;RNA polymerase subunit, common to RNA polymerases I and III

2177;Cytochrome b5, involved in the sterol and lipid biosynthesis pathways; required for sterol C5-6 and fatty acid desaturation

2178;Constituent of 66S pre-ribosomal particles, involved in 60S ribosomal subunit biogenesis; localizes to both nucleolus and cytoplasm

2179;Hypothetical protein

2180;Phosphatidylinositol 4,5-bisphosphate 5-phosphatase, synaptojanin-like protein with an N-terminal Sac1 domain, plays a role in endocytosis; hyperosmotic stress causes translocation to actin patches

2181;Vacuolar transporter, exports large neutral amino acids from the vacuole; member of a family of seven S. cerevisiae genes (AVT1-7) related to vesicular GABA-glycine transporters

2182;Hypothetical protein

2183;Putative protein tyrosine phosphatase, required for cell cycle arrest in response to oxidative damage of DNA

2184;GTP-binding protein that regulates the nitrogen starvation response, sporulation, and filamentous growth; famesylation and palmitoylation required for activity and localization to plasma membrane; homolog of mammalian Ras proto-oncogenes

2185;Probable component of the Rpd3 histone deacetylase complex, involved in transcriptional regulation of HO5; C-terminus has similarity to human candidate tumor suppressor p33(ING1)

2186;Hypothetical protein

2187;Protein of unknown function, interacts with Rvs161p and Rvs167p; computational analysis of protein-protein interactions in large-scale studies suggests a possible role in actin filament organization

2188;GTPase, similar to Ypt51p and Ypt52p and to mammalian rab5; required for vacuolar protein sorting and endocytosis

2189;Protein of unknown function, mediates sensitivity to salt stress; interacts physically with the splicing factor Msllp and also displays genetic interaction with MSL1

2190;Non-essential small GTPase of the Rho/Rac subfamily of Ras-like proteins, involved in the establishment of cell polarity and in microtubule assemblyj 2191;Essential type II topoisomerase, relieves torsional strain in DNA by cleaving and re-sealing the phosphodiester backbone of both positively and negatively supercoiled DNA; cleaves complementary strands; localizes to axial cores in meiosis

2192;Bud-specific protein with a potential role in membrane trafficking; GFP-fusion protein migrates from the cell surface to intracellular vesicles near vacuole; contains 3 calcium and lipid binding domains; mRNA is targeted to the bud via the mRNA transport system involving She2p

2193;Protein that forms a complex with Pbplp that may mediate posttranscriptional regulation of HO endonuclease; involved in propagation of M2 dsRNA satellite of L-A virus

2194;EH domain-containing protein involved in endocytosis, actin cytoskeletal organization and cell wall morphogenesis; forms a complex with Slalp and Panlp

2195;Putative mitochondrial ribosomal protein of the small subunit, has similarity to E. coli S 13 ribosomal protein

2196;Major isoform of tropomyosin; binds to and stabilizes actin cables and filaments, which direct polarized cell growth and the distribution of several organelles; acetylated by the NatB complex and acetylated form binds actin most efficiently

2197;Component of the SSU processome, which is required for pre-18S rRNA processing; interacts with Mpp10p; member of a superfamily of proteins that contain a sigma(70)-like motif and associate with RNAs

2198;Mitochondrial lysine-tRNA synthetase, required for import of both aminoacylated and deacylated forms of tRNTA(Lys) into mitochondria

2199;Ribonuclease H2 catalytic subunit, removes RNA primers during Okazaki fragment synthesis; cooperates with Rad27p nuclease

2200;Dihydrolipoamide acetyltransferase component (E2) of pyruvate dehydrogenase complex, which catalyzes the oxidative decarboxylation of pyruvate to acetyl-CoA

2201;Component of the TOM (translocase of outer membrane) complex responsible for recognition and initial import steps for all mitochondrially directed proteins; promotes assembly and stability of the TOM complexj 2202;Transcription factor of the forkhead family that regulates the cell cycle and pseudohyphal growth; also involved in chromatin silencing at HML and HMR; potential Cdc28p substrate

2203;Protein component of the large (60S) ribosomal subunit, nearly identical to Rp19Ap and has similarity to E. coli L6 and rat L9 ribosomal proteins

2204;Protein chaperone involved in regulation of the HSP90 and HSP70 functions; involved in protein translocation across membranes; member of the DnaJ family

2205;Cytoplasmic protein required for replication of Brome mosaic virus in S. cerevisiae, which is a model system for studying replication of positive-strand RNA viruses in their natural hosts

2206;Integral vacuolar membrane protein involved in vacuole inheritance and morphology; may function to regulate Fablp kinase activity

2207;Subunit Va of cytochrome c oxidase, which is the terminal member of the mitochondrial inner membrane electron transport chain; predominantly expressed during aerobic growth while its isoform Vb (Cox5Bp) is expressed during anaerobic growth

2208;Probable component of COPII coated vesicles that binds to Sec23p; similar to and functionally redundant with Sec24p, but expressed at low levels; involved in ER to Golgi transport and in autophagy

2209;Component of the conserved oligomeric Golgi complex (Coglp through Cog8p), a cytosolic tethering complex that functions in protein trafficking to mediate fusion of transport vesicles to Golgi compartments

2210;Subunit of mitochondrial NAD(+)-dependent isocitrate dehydrogenase, which catalyzes the oxidation of isocitrate to alpha-ketoglutarate in the TCA cycle

2211; Hypothetical protein

2212;Tyrosine phosphatase that plays a role in actin filament organization and endocytosis; localized to the cytoplasm

2213;One of two identical histone H3 proteins (see also HHT1); core histone required for chromatin assembly, involved in heterochromatin-mediated telomeric and HM silencing; regulated by acetylation, methylation, and mitotic phosphorylation2214;One of two identical histone H4 proteins (see also HHF1); core histone required for chromatin assembly and chromosome function; contributes to telomeric silencing; N-terminal domain involved in maintaining genomic integrity

2215;Putative mannosyltransferase involved in protein glycosylation; member of the KRE2/MNT1 mannosyltransferase family

2216;Transcription factor that activates transcription of genes involved in stress response; nuclear localization is positively regulated by calcineurin-mediated dephosphorylation

2217;Essential component of the Sorting and Assembly Machinery (SAM or Tob complex) of the mitochondrial outer membrane, which binds precursors of beta-barrel proteins and facilitates their outer membrane insertion; homologous to bacterial Omp85

2218;Cyclin-like component of the RNA polymerase II holoenzyme, involved in phosphorylation of the RNA polymerase II C-terminal domain; involved in glucose repression

2219;Identified by gene-trapping, microarray-based expression analysis, and genome-wide homology searching

2220;Putative catalytic subunit of a class II histone deacetylase complex that also contains Hda2p and Hda3p; Hda1p interacts with the Hda2p-Hda3p subcomplex to form an active tetramer; deletion increases histone H2B, H3 and H4 acetylation

2221;Serine/threonine protein kinase involved in regulation of the cortical actin cytoskeleton; involved in control of endocytosis

2222;Cytosolic inhibitor of vacuolar proteinase B, required for efficient vacuole inheritance; with thioredoxin forms protein complex LMA1, which assists in priming SNARE molecules and promotes vacuole fusion

2223;Hypothetical protein

2224;Hypothetical protein

2225;Peroxisomal NADP-dependent isocitrate dehydrogenase, catalyzes oxidation of isocitrate to alpha-ketoglutarate with the formation of NADP(H+), required for growth on unsaturated fatty acids

2226;Type II HSP40 co-chaperone that interacts with the HSP70 protein Ssa1p; not functionally redundant with Ydj1p due to due to substrate specificity; shares similarity with bacterial DnaJ proteins

2227;Protein required for the transport of amino acid permease Gap1p from the Golgi to the cell surface; component of the TOR signaling pathway; associates with both Tor1p and Tor2p; contains a WD-repeat

2228;Mitochondrial ribosomal protein of the large subunit

2229;Poly(A+) RNA-binding protein, involved in the export of mRNAs from the nucleus to the cytoplasm; similar to Gbp2p and Np13p

2230;Citrate synthase, catalyzes the condensation of acetyl coenzyme A and oxaloacetate to form citrate; the rate-limiting enzyme of the TCA cycle; nuclear encoded mitochondrial protein

2231;Putative transmembrane protein, involved in the export of ammonia, a starvation signal that promotes cell death in the center of aging colonies; member of the TC 9.B.33 YaaH family; homolog of Ady2p and Y. lipolytica Gpr1p

2232;RNA polymerase III subunit C34; interacts with TFIIIB70 and is a key determinant in pol III recruitment by the preinitiation complex

2233; Hypothetical protein

2234;Endosomal protein that forms a complex with Hselp; required for recycling Golgi proteins, forming lumenal membranes and sorting ubiquitinated proteins destined for degradation; has Ubiquitin Interaction Motifs which bind ubiquitin (Ubi4p)

2235;Protein involved in autophagy; E2-like enzyme that plays a role in formation of Atg8p-phosphatidylethanolamine conjugates, which are involved in membrane dynamics during autophagy

2236;Acyltransferase that catalyzes diacylglycerol esterification; one of several acyltransferases that contribute to triglyceride synthesis; putative homolog of human lecithin cholesterol acyltransferase

2237;Putative transcriptional repressor of MBF (MCB binding factor) target genes

2238;Component of the Med9/10 module, which is a subcomplex within the RNA polymerase II Mediator complex; required for regulation of RNA polymerase II activity

2239;Uridine/cytidine kinase, component of the pyrimidine ribonucleotide salvage pathway that converts uridine into UMP and cytidine into CMP; involved in the pyrimidine deoxyribonucleotide salvage pathway, converting deoxycytidine into dCMP

2240;Dihydrouridine synthase, member of a family of dihydrouridine synthases including Dus1p, Smmlp, Dus3p, and Dus4p; modifies uridine residues at position 20 of cytoplasmic tRNAs

2241;Essential protein of the mitochondrial inner membrane, component of the mitochondrial import system

2242;Hypothetical protein

2243;Acyl-CoA:sterol acyltransferase, isozyme of Are1p; endoplasmic reticulum enzyme that contributes the major sterol esterification activity in the presence of oxygen

2244;Mitochondrial ribosomal protein of the large subunit, not essential for mitochondrial translation

2245;73 kDa subunit of the 11-subunit SWI/SNF chromatin remodeling complex involved in transcriptional regulation; homolog of Rsc6p subunit of the RSC chromatin remodeling complex; deletion mutants are temperature-sensitive

2246;Protein involved in bud-site selection; diploid mutants display a random budding pattern instead of the wild-type bipolar pattern

2247;Peptidyl-prolyl cis-trans isomerase (cyclophilin), catalyzes the cis-trans isomerization of peptide bonds N-terminal to proline residues; similarity to Cpr4p suggests a potential role in the secretory pathway

2248;Alpha-1,6-mannosyltransferase localized to the ER; responsible for the addition of the alpha-1,6 mannose to dolichol-linked Man7GlcNAc2, acts in the dolichol pathway for N-glycosylation

2249;Putative serine/threonine protein phosphatase, required for glycogen accumulation; interacts with Tap42p, which binds to and regulates other protein phosphatases

2250;Ubiquitin-like protein modifier, may function in modification of Sph1p and Hbt1p, functionally complemented by the human or S. pombe ortholog; mechanism of Hublp adduct formation not yet clear

2251;Para-aminobenzoate (PABA) synthase, has similarity to Escherichia coli PABA synthase components PabA and PabB

2252;Protein with a possible role in tRNA export; shows similarity to 6-phosphogluconolactonase non-catalytic domains but does not exhibit this enzymatic activity; homologous to Sol2p, Sol3p, and Sol4p

2253;Hypothetical protein

2254;Subunit of the ARP2/3 complex, which is required for the motility and integrity of cortical actin patches; required for cortical localization of calmodulin2255;Mitochondrial ribosomal protein of the small subunit, has similarity to E. coli S 19 ribosomal protein

2256;Essential protein involved in ribosome biogenesis; putative ATP-dependent RNA helicase of the DEAD-box protein family

2257;Endoplasmic reticulum protein of unknown function, transcription is induced under conditions of zinc deficiency; mutant phenotype suggests a role in uptake of zinc

2258;Para hydroxybenzoate: polyprenyl transferase, catalyzes the second step in ubiquinone (coenzyme Q) biosynthesis

2259;Mevalonate pyrophosphate decarboxylase, essential enzyme involved in the biosynthesis of isoprenoids and sterols, including ergosterol; acts as a homodimer

2260;Specific translational activator for the COX3 mRNA that acts together with Pet54p and Pet122p; located in the mitochondrial inner membrane

2261;Subunit of an adoMet-dependent tRNA methyltransferase (MTase) complex (Trm11p-Trm112p), required for the methylation of the guanosine nucleotide at position 10 (m2G10) in tRNAs; putative zinc binding subunit of other MTase-related proteins

2262;Putative protein kinase that, when overexpressed, interferes with pheromone-induced growth arrest; localizes to the cytoplasm; potential Cdc28p substrate

2263;Protein proposed to interact with phospholipid translocases, shares similarity to Cdc50p

2264;Ubiquitin protease cofactor, forms deubiquitination complex with Ubp3p that coregulates anterograde and retrograde transport between the endoplasmic reticulum and Golgi compartments; null is sensitive to brefeldin A

2265;RNase of the DEDD superfamily, subunit of the Ccr4-Not complex that mediates 3' to 5' mRNA deadenylation

2266;Essential nucleolar protein involved in pre-18S rRNA processing; component of the small subunit (SSU) processome; has sequence similarity to mABT1, a mouse transcription activator

2267;Putative ion transporter similar to the major facilitator superfamily of transporters; mutations in membrane-spanning domains permit nonselective cation uptake

2268;7,8-diamino-pelargonic acid aminotransferase (DAPA), catalyzes the second step in the biotin biosynthesis pathway; BIO3 is in a cluster of 3 genes (BIO3 BIO4 and BIOS) that mediate biotin synthesis

2269;Putative alpha-1,3-mannosyltransferase, not required for protein O-glycosylation

2270;Daughter cell-specific secreted protein with similarity to glucanases, degrades cell wall from the daughter side causing daughter to separate from mother

2271;Hypothetical protein identified by homology. See FEBS Letters [2000] 487:31-36.

2272;Endosomal ferric enterobactin transporter, expressed under conditions of iron deprivation; member of the major facilitator superfamily; expression is regulated by Rcs1p and affected by chloroquine treatment

2273;Putative GPI-anchored protein; transcription is induced under low-zinc conditions, as mediated by the Zap1p transcription factor, and at alkaline pH

2274;Lumazine synthase (6,7-dimethyl-8-ribityllumazine synthase, also known as DMRL synthase); catalyzes synthesis of immediate precursor to riboflavin

2275;Protein involved in rRNA processing; component of the exosome 3->5 exonucle ase complex

2276;Acetylornithine aminotransferase, catalyzes the fourth step in the biosynthesis of the arginine precursor ornithine

2277;Cytoplasmic mRNA cap binding protein; the eIF4E-cap complex is responsible for mediating cap-dependent mRNA translation via interactions with the translation initiation factor eIF4G (Tif4631p or Tif4632p)

2278;Protein of unknown function containing 8 putative transmembrane seqments; ORF exhibits genomic organization compatible with a translational readthrough-dependent mode of expression

2279;RING finger containing subunit of Skp1-Cullin-F-box ubiquitin protein ligases (SCF); required for Gic2p, Far1p, Sic1p and Cln2p degradation; may tether Cdc34p (a ubiquitin conjugating enzyme or E2) and Cdc53p (a cullin) subunits of SCF

2280;Hypothetical protein

2281;Plasma membrane Mg(2+) transporter, expression and turnover are regulated by Mg(2+) concentration; overexpression confers increased tolerance to Al(3+) and Ga(3+) ions

2282;Vacuolar membrane protein of unknown function involved in vacuolar protein sorting; also detected in the mitochondria

2283;Protein kinase related to mammalian glycogen synthase kinases of the GSK-3 family; GSK-3 homologs (Mck1p, Rim11p, Mrk1p, Ygk3p) are involved in control of Msn2p-dependent transcription of stress responsive genes and in protein degradation

2284;Hypothetical protein

2285;Catalytic subunit of an adoMet-dependent tRNA methyltransferase complex (Trm11p-Trm112p), required for the methylation of the guanosine nucleotide at position 10 (m2G10) in tRNAs; contains a THUMP domain and a methyltransferase domain

2286;Subunit of cleavage factor I, a five-subunit complex required for the cleavage and polyadenylation of pre-mRNA 3' ends; RRM-containing heteronuclear RNA binding protein and hnRNPAB family member that binds to poly (A) signal sequences

2287;Divalent metal ion transporter with a broad specificity for di-valent and tri-valent metals; post-translationally regulated by levels of metal ions; member of the Nramp family of metal transport proteins

2288;Protein with similarity to mammalian monocarboxylate permeases, which are involved in transport of monocarboxylic acids across the plasma membrane; mutant is not deficient in monocarboxylate transport

2289;Transcriptional activator involved in regulation of invertase and glucoamylase expression, invasive growth and pseudohyphal differentiation, iron uptake, chromium accumulation, and response to osmotic stress; localizes to the nucleus

2290;Catalytic subunit of TRAMP (Trf4/Pap2p-Mtr4p-Air1p/2p), a nuclear poly (A) polymerase complex involved in RNA quality control; catalyzes polyadenylation of unmodified tRNAs, and snoRNA and rRNA precursors; disputed role as a DNA polymerase

2291;Hypothetical protein

2292;Member of the PAK family of serine/threonine protein kinases with similarity to Ste20p and Cla4p; proposed to be a downstream effector of Cdc42p during polarized growth

2293;Nuclear protein required for efficient mating, involved in shmoo formation and nuclear migration in the pre-zygote, contains a ubiquitin-like (UBL) domain

2294;Peripheral membrane protein of the plasma membrane that interacts with Mid2p; regulates the cell integrity pathway mediated by Pkc1p and Slt2p

2295;Transcription factor required for derepression of inositol-choline-regulated genes involved in phospholipid synthesis; forms a complex, with Ino2p, that binds the inositol-choline-responsive element through a basic helix-loop-helix domain

2296;Hypothetical protein

2297;Meiosis-specific telomere protein, required for bouquet formation, effective homolog pairing, ordered cross-over distribution (interference), sister chromatid cohesion at meiotic telomeres, and segregation of small chromosomes

2298;Serine/threonine protein kinase involved in sphingolipid-mediated signaling pathway that controls endocytosis; activates Ypk1p and Ykr2p, components of signaling cascade required for maintenance of cell wall integrity; redundant with Pkh1p

2299;Putative metalloprotease

2300;Mitochondrial inner membrane localized ATP-dependent DNA helicase, required for the maintenance of the mitochondrial genome; not required for mitochondrial transcription

2301;Component of the meiotic outer plaque of the spindle pole body, involved in modifying the meiotic outer plaque that is required prior to prospore membrane formationj 2302;Protein that forms heterodimers, with Msh3p and Msh6p, that bind to DNA mismatches to initiate the mismatch repair process; contains a Walker ATP-binding motif required for repair activity; Msh2p-Msh6p binds to and hydrolyzes ATP

2303;Putative transcription factor containing a zinc finger; overexpression increases salt tolerance through increased expression of the ENA1 (Na+/Li+ extrusion pump) gene while gene disruption decreases both salt tolerance and ENA1 expression

2304;Member of the protein disulfide isomerase (PDI) family, exhibits chaperone activity; overexpression suppresses the lethality of a pdi1 deletion but does not complement all Pdilp functions; undergoes oxidation by Erolp

2305;Hypothetical protein

2306;Protein of unknown function, expression is regulated by phosphate levels; green fluorescent protein (GFP)-fusion protein localizes to the cell periphery and vacuole

2307;Putative RNA exonuclease possibly involved in pre-rRNA processing and ribosome assembly

2308;Subunit k of the mitochondrial F1F0 ATP synthase, which is a large enzyme complex required for ATP synthesis; associated only with the dimeric form of ATP synthase

2309;Nucleolar protein, constituent of 66S pre-ribosomal particles; depletion leads to defects in rRNA processing and a block in the assembly of large ribosomal subunits; possesses a sigma(70)-like RNA-binding motif

2310;Non-catalytic subunit of the NatB N-terminal acetyltransferase, which catalyzes acetylation of the amino-terminal methionine residues of all proteins beginning with Met-Asp or Met-Glu and of some proteins beginning with Met-Asn or Met-Met; involved in mitochondrial inheritance and actin assembly

2311;hypothetical protein

2312;Hypothetical protein

2313;Component of the evolutionarily conserved kinetochore-associated Ndc80 complex (Ndc80p-Nuf2p-Spc24p-Spc25p); involved in chromosome segregation, spindle checkpoint activity and kinetochore clustering

2314;Transcription factor (bHLH) involved in interorganelle communication between mitochondria, peroxisomes, and nucleus

2315;Phosphatidylinositol 4,5-bisphosphate 5-phosphatase with a role in secretion, localizes to the endoplasmic reticulum via the C-terminal tail; lacks the Sac1 domain and proline-rich region found in the other 3 INP proteins

2316;Bisphosphate-3'-nucleotidase, involved in salt tolerance and methionine biogenesis; dephosphorylates 3'-phosphoadenosine-5'-phosphate and 3'-phosphoadenosine-5'-phosphosulfate, intermediates of the sulfate assimilation pathway

2317;Mu2-like subunit of the clathrin associated protein complex (AP-2); involved in vesicle transport

2318;Mu2-like subunit of the clathrin associated protein complex (AP-2); involved in vesicle transport

2319;5-phospho-ribosyl-1(alpha)-pyrophosphate synthetase, involved in nucleotide, histidine, and tryptophan biosynthesis; one of a five related enzymes, which are active as heteromultimeric complexes

2320;Protein required for normal mitochondrial morphology, has similarity to hemolysins

2321;NAD-dependent glycerol 3-phosphate dehydrogenase, homolog of Gpd1p, expression is controlled by an oxygen-independent signaling pathway required to regulate metabolism under anoxic conditions; located in cytosol and mitochondria

2322;Arginosuccinate synthetase, catalyzes the formation of L-argininosuccinate from citrulline and L-aspartate in the arginine biosynthesis pathway; potential Cdc28p substrate

2323;Putative metalloprotease

2324;Hydroxymethylpyrimidine phosphate kinase, involved in the last steps in thiamine biosynthesis; member of a gene family with THI21 and THI22; functionally redundant with Thi21p

2325;Nuclear protein, putative RNA polymerase II elongation factor; isolated as Pob3p/Spt16p-binding protein

2326;Multistress response protein, expression is activated by a variety of xenobiotic agents and environmental or physiological stresses

2327;Phosphorylated tail-anchored type II integral peroxisomal membrane protein required for peroxisome biogenesis, cells lacking Pex15p mislocalize peroxisomal matrix proteins to cytosol, overexpression results in impaired peroxisome assembly

2328;DNAN-glycosylase and apurinic/apyrimidinic (AP) lyase involved in base excision repair, localizes to the nucleus

2329;Putative endonuclease, has a domain similar to a magnesium-dependent endonuclease motif in mRNA deadenylase Ccr4p

2330;Protein component of the small (40S) ribosomal subunit; has similarity to E. coli S 19 and rat S 15 ribosomal proteins

2331;Ribosomal protein P2 alpha, a component of the ribosomal stalk, which is involved in the interaction between translational elongation factors and the ribosome; regulates the accumulation of P1 (Rpp1Ap and Rpp1Bp) in the cytoplasm

2332;20S proteasome alpha-type subunit

2333;Protein of unknown function; potential Cdc28p substrate

2334;Mitochondrial glutamyl-tRNA synthetase, encoded by a nuclear gene

2335;ORF, Uncharacterized

2336;Nucleotide exchange factor for the endoplasmic reticulum (ER) lumenal Hsp70 chaperone Kar2p, required for protein translocation into the ER; homolog of Yarrowia lipolytica SLS1; GrpE-like protein

2337;Putative 1,3-beta-glucanosyltransferase, has similarity to Gaslp; localizes to the cell wall

2338;Mitochondrial inner membrane protein, required for K+/H+ exchange and for normal mitochondrial morphology and inheritance; human ortholog Letm1 is implicated in Wolf-Hirschhorn syndrome

2339;Mitochondrial outer membrane protein, required for assembly of the translocase of the outer membrane (TOM) complex and thereby for mitochondrial protein import; N terminus is exposed to the cytosol: transmembrane segment is highly conserved

2340;Mitochondrial translation initiation factor 2

2341;Protein required for cell viability

2342;High affinity tryptophan and tyrosine permease, overexpression confers FK506 resistance

2343;Syntaxin-like t-SNARE that forms a complex with Tlglp and Vtilp and mediates fusion of endosome-derived vesicles with the late Golgi; binds Vps45p, which prevents Tlg2p degradation and also facilitates t-SNARE complex formation

2344;Hypothetical protein

2345;Ubiquitin-protein ligase required for endoplasmic reticulum-associated degradation (ERAD) of misfolded proteins; genetically linked to the unfolded protein response (UPR); regulated through association with Hrd3p; contains an H2 ring finger

2346;Histone variant H2AZ, exchanged for histone H2A in nucleosomes by the SWR1 complex; involved in transcriptional regulation through prevention of the spread of silent heterochromatin

2347;Phospholipase B (lysophospholipase) involved in phospholipid metabolism; hydrolyzes phosphatidylinositol and phosphatidylserine and displays transacylase activity in vitro

2348;RNA terminal phosphate cyclase-like protein involved in rRNA processing at sites A0, A1, and A2; does not possess detectable RNA cyclase activity

2349;Appears to be a structural component of the chitin synthase 3 complex

2350;Palmitoyltransferase with autoacylation activity; member of a family of putative palmitoyltransferases containing an Asp-His-His-Cys-cysteine rich (DHHC-CRD) domain

2351;Cyclin, negatively regulates phosphate metabolism; Pho80p-Pho85p (cyclin-CDK complex) phosphorylates Pho4p and Swi5p; deletion of PHO80 leads to aminoglycoside supersensitivity; truncated form of PHO80 affects vacuole inheritance

2352;Glucosyltransferase, involved in transfer of oligosaccharides from dolichyl pyrophosphate to asparagine residues of proteins during N-linked protein glycosylation; mutations in human ortholog are associated with disease

2353;Putative precursor to the subtilisin-like protease III

2354;Glutamine-rich cytoplasmic protein of unknown function, contains tetratricopeptide (TPR) repeats, which often mediate protein-protein interactions; conserved in human and C. elegans

2355;Putative cell wall mannoprotein of the Srplp/Tiplp family of serine-alanine-rich proteins; transcription is induced by cold shock and anaerobiosis

2356;B-type regulatory subunit of protein phosphatase 2A (PP2A); homolog of the mammalian B' subunit of PP2A

2357;Identified by homology to Ashbya gossypii

2358;Hypothetical protein

2359;Subunit of the Ada histone acetyltransferase complex, required for structural integrity of the complex

2360;Kinetochore checkpoint WD40 repeat protein that localizes to kinetochores during prophase and metaphase, delays anaphase in the presence of unattached kinetochores; forms complexes with Madlp-Bublp and with Cdc20p, binds Mad2p and Mad3p

2361;Hsp90 cochaperone, interacts with the Ssa group of the cytosolic Hsp70 chaperones; activates the ATPase activity of Ssa1p; homolog of mammalian Hop protein

2362;Probable multiple transmembrane protein, involved in invasive growth upon nitrogen starvation; required for accumulation of processed Rim101p

2363;Protein containing a UCS (UNC-45/CRO1/SHE4) domain, binds to myosin motor domains to regulate myosin function; involved in endocytosis, polarization of the actin cytoskeleton, and asymmetric mRNA localization

2364;Target membrane receptor (t-SNARE) for vesicular intermediates traveling between the Golgi apparatus and the vacuole; controls entry of biosynthetic, endocytic, and retrograde traffic into the prevacuolar compartment; syntaxin

2365;Beta' regulatory subunit of casein kinase 2, a Ser/Thr protein kinase with roles in cell growth and proliferation; the holoenzyme also contains CKA1, CKA2 and CKB1, the many substrates include transcription factors and all RNA polymerases

2366;Protein required, with binding partner Psrlp, for full activation of the general stress response, possibly through Msn2p dephosphorylation; regulates growth during the diauxic shift; negative regulator of G1 cyclin expression

2367;Hypothetical protein

2368;Component of the TOM (translocase of outer membrane) complex responsible for recognition and initial import steps for all mitochondrially directed proteins; promotes assembly and stability of the TOM complex

2369;Cytoplasmic ATP-dependent RNA helicase of the DEAD-box family involved in mRNA export from the nucleus

2370;Protein involved in control of glucose-regulated gene expression; interacts with protein kinase Snf1p, glucose sensors Snf3p and Rgt2p, and TATA-binding protein Spt15p; acts as a regulator of the transcription factor Rgt1p

2371;Hypothetical protein

2372;Essential nuclear protein involved in proteasome maturation and synthesis of 40S ribosomal subunits; required for cleavage of the 20S pre-rRNA to generate the mature 18S rRNA

2373;Probable cochaperone, regulates activity of Cyrlp (adenylyl cyclase); involved in assembly of the kinetochore complex, associates with the SCF (Skplp/Cdc53p/F box protein) ubiquitin ligase complex

2374;Protein required for cell viabilityj 2375;Alpha' catalytic subunit of casein kinase 2, a Ser/Thr protein kinase with roles in cell growth and proliferation; the holoenzyme also contains CKA1, CKB1 and CKB2, the many substrates include transcription factors and all RNA polymerases

2376;Protein of unknown function; similar to YKR075Cp and Reglp; expression regulated by glucose and Rgt1p

2377;Protein component of the large (60S) ribosomal subunit, has similarity to E. coli L3 and rat L3 ribosomal proteins; involved in the replication and maintenance of killer double stranded RNA virus

2378;Subunit of the NuA3 histone acetyltransferase complex that acetylates histone H3; has similarity to the human tumor suppressor ING1

2379;Cytochrome c1, component of the mitochondrial respiratory chain; expression is regulated by the heme-activated, glucose-repressed Hap2p/3p/4p/Sp CCAAT-binding complex

2380;Component of the spindle checkpoint, involved in sensing lack of tension on mitotic chromosomes; protects centromeric Rec8p at meiosis I; required for accurate chromosomal segregation at meiosis II and for mitotic chromosome stability

2381;Thymidylate synthase, required for de novo biosynthesis of pyrimidine deoxyribonucleotides; expression is induced at G1/S

2382;Basic zinc-finger protein, similar to human and mouse Kin17 proteins which are chromatin-associated proteins involved in UV response and DNA replication

2383;Golgi membrane protein involved in manganese homeostasis; overproduction suppresses the sod1 (copper, zinc superoxide dismutase) null mutation

2384;GTPase required for transport during endocytosis and for correct sorting of vacuolar hydrolases; localized in endocytic intermediates; detected in mitochondria; geranylgeranylation required for membrane association; mammalian Rab5 homolog

2385;Mitochondrially localized type 2C protein phosphatase; contains Mg2+/Mn2+-dependent casein phosphatase activity in vitro but in vivo substrates are unknown2386;Glucose-repressible ADP-ribosylation factor, GTPase of the Ras superfamily involved in development of polarity

2387;Ribose-5-phosphate ketol-isomerase, catalyzes the interconversion of ribose 5-phosphate and ribulose 5-phosphate in the pentose phosphate pathway; participates in pyridoxine biosynthesis

2388;Ribose-5-phosphate ketol-isomerase, catalyzes the interconversion of ribose 5-phosphate and ribulose 5-phosphate in the pentose phosphate pathway; participates in pyridoxine biosynthesis

2389;Hypothetical protein

2390;Nuclear pore complex (NPC) subunit, involved in protein import/export and in export of RNAs, possible karyopherin release factor that accelerates release of karyopherin-cargo complexes after transport across NPC; potential Cdc28p substrate

2391;Alpha-1,2-mannosyltransferase involved in O- and N-linked protein glycosylation; member of the KRE2/MNT1 mannosyltransferase family

2392;Mitochondrial inner membrane carnitine transporter, required for carnitine-dependent transport of acetyl-CoA from peroxisomes to mitochondria during fatty acid beta-oxidation

2393;GTPase involved in G-protein signaling in the adenylate cyclase activating pathway, plays a role in cell proliferation; localized to the plasma membrane; homolog of mammalian RAS proto-oncogenes

2394;Protein that induces appearance of [PIN+] prion when overproduced

>

2395;Phosphatidylinositol 4,5-bisphosphate 5-phosphatase, synaptojanin-like protein with an N-terminal Sac1 domain, plays a role in a TGN (trans Golgi network)-to-early endosome pathway; hyperosmotic stress causes translocation to actin patches

2396;One of six subunits of the RNA polymerase III transcription initiation factor complex (TFIIIC); part of the TauA globular domain of TFIIIC that binds DNA at the BoxA promoter sites of tRNA and similar genes

2397;Hypothetical protein

2398;Hypothetical protein

2399;One of 10 subunits of the transport protein particle (TRAPP) complex of the cis-Golgi which mediates vesicle docking and fusion; involved in endoplasmic reticulum (ER) to Golgi membrane traffic

2400;RNA polymerase III subunit C160, part of core enzyme; similar to bacterial beta-prime subunit

2401;One of six ATPases of the 19S regulatory particle of the 26S proteasome involved in the degradation of ubiquitinated substrates; recruited to the GAL1-10 promoter region upon induction of transcription

2402;Hypothetical protein

2403;Essential serine kinase involved in cell cycle progression and processing of the 20S pre-rRNA into mature 18S rRNA

2404;Putative NADP(+) coupled glycerol dehydrogenase, proposed to be involved in an alternative pathway for glycerol catabolism

2405;Isoamyl acetate-hydrolyzing esterase, required in balance with alcohol acetyltransferase to maintain optimal amounts of isoamyl acetate, which is particularly important in sake brewing

2406;Phosphoribosylaminoimidazole carboxylase, catalyzes a step in the 'de novo' purine nucleotide biosynthetic pathway; red pigment accumulates in mutant cells deprived of adenine

2407;Ornithine transporter of the mitochondrial inner membrane, exports ornithine from mitochondria as part of arginine biosynthesis; human ortholog is associated with hyperammonaemia-hyperornithinaemia-homocitrullinuria (HHH) syndrome

2408;Subunit of the membrane-associated retromer complex essential for endosome-to-Golgi retrograde protein transport; peripheral membrane protein that assembles onto the membrane with Vps5p to promote vesicle formation

2409;Subunit of mitochondrial NAD(+)-dependent isocitrate dehydrogenase, which catalyzes the oxidation of isocitrate to alpha-ketoglutarate in the TCA cycle

2410;Alpha subunit of succinyl-CoA ligase, which is a mitochondrial enzyme of the TCA cycle that catalyzes the nucleotide-dependent conversion of succinyl-CoA to succinate

2411;Protein required for S phase progression and telomere homeostasis, forms an alternative replication factor C complex important for DNA replication and genome integrity; mutants are sensitive to DNA damage

2412;Essential nucleolar protein required for pre-18S rRNA processing, interacts with Dimlp, an 18S rRNA dimethyltransferase, and also with Noblp, which is involved in proteasome biogenesis; contains a KH domain

2413;RNA polymerase II second largest subunit B150, part of central core; similar to bacterial beta subunit

2414;Hypothetical protein

2415;Short-lived membrane ABC (ATP-binding cassette) transporter, actively exports various drugs, expression regulated by Pdr1p; also involved in steroid transport, cation resistance, and cellular detoxification during exponential growth

2416;Hypothetical protein

2417;Inosine 5'-monophosphate (IMP)-specific 5'-nucleotidase, catalyzes the breakdown of IMP to inosine, does not show similarity to known 5'-nucleotidases from other organisms

2418; SUMO ligase, catalyzes the covalent attachment of SUMO (Smt3p) to proteins 2419;Endopeptidase with trypsin-like activity that cleaves after basic residues; beta-type subunit of 20S proteasome synthesized as a proprotein before being proteolytically processed for assembly into 20S particle; human homolog is subunit Z

2420;Core Sm protein Sm E; part of heteroheptameric complex (with Smblp, Smd1p, Smd2p, Smd3p, Smx3p, and Smx2p) that is part of the spliceosomal U1, U2, U4, and U5 snRNPs; homolog of human Sm E

2421;Protein of unknown function; has similarity to Torpedo californica tCTL1p, which is postulated to be a choline transporter, neither null mutation nor overexpression affects choline transport

2422;Hypothetical protein

2423;Hypothetical protein

2424;Protein component of the small (40S) ribosomal subunit; nearly identical to Rps28Ap and has similarity to rat S28 ribosomal protein

2425;Glutamine tRNA synthetase, monomeric class I tRNA synthetase that catalyzes the specific glutaminylation of tRNA(Glu); N-terminal domain proposed to be involved in enzyme-tRNA interactions

2426;Sphingoid long-chain base kinase, responsible for synthesis of long-chain base phosphates, which function as signaling molecules, regulates synthesis of ceramide from exogenous long-chain bases, localizes to the Golgi and late endosomes

2427;Subunit of the RNA polymerase II mediator complex; associates with core polymerase subunits to form the RNA polymerase II holoenzyme; essential for transcriptional regulation

2428;Member of the MBOAT family of putative membrane-bound O-acyltransferases

2429;Ferrochelatase, a mitochondrial inner membrane protein, catalyzes the insertion of ferrous iron into protoporphyrin IX, the eighth and final step in the heme biosynthetic pathway; Yfh1p mediates the use of iron by Hem15p

2430;Peroxisomal delta(3,5)-delta(2,4)-dienoyl-CoA isomerase, involved in fatty acid metabolism, contains peroxisome targeting signals at amino and carboxy termini

2431;Actin assembly factor, activates the Arp2/3 protein complex that nucleates branched actin filaments; localizes with the Arp2/3 complex to actin patches; homolog of the human Wiskott-Aldrich syndrome protein (WASP)

2432;3-phosphoserine aminotransferase, catalyzes the formation of phosphoserine from 3-phosphohydroxypyruvate, required for serine and glycine biosynthesis; regulated by the general control of amino acid biosynthesis mediated by Gcn4p

2433;GTP binding protein (mammalian Ranp homolog) involved in the maintenance of nuclear organization, RNA processing and transport; interacts with Kap121p, Kap123p and Pdr6p (karyophilin betas); Gsp1p homolog that is not required for viability

2434;Mitochondrial translation elongation factor Tu; comprises both GTPase and guanine nucleotide exchange factor activities, while these activities are found in separate proteins in S. pombe and humans

2435;Protein that associates with the INO80 chromatin remodeling complex under low-salt conditions

2436;Member of the SWI/SNF family of DNA-dependent ATPases, plays a role in antagonizing silencing during mating-type switching, contains an N-terminal domain that interacts with Sir4p and a C-terminal SNF2 domain

2437;TFIIA large subunit; involved in transcriptional activation, acts as antirepressor or as coactivator; homologous to largest and second largest subunits of human and Drosophila TFIIA

2438;Component of mRNP complexes associated with polyribosomes; implicated in secretion and nuclear segregation; multicopy suppressor of BFA (Brefeldin A) sensitivity

2439;Ribose methyltransferase that modifies a functionally critical, conserved nucleotide in mitochondrial 21 S rRNA

2440;Imidazoleglycerol-phosphate dehydratase, catalyzes the sixth step in histidine biosynthesis; mutations cause histidine auxotrophy and sensitivity to Cu, Co; and Ni salts; transcription is regulated by general amino acid control via Gcn4p

2441;ATP-dependent DEAD (Asp-Glu-Ala-Asp)-box RNA helicase, required for translation initiation of all yeast mRNAs; mutations in human DEAD-box DBY are a frequent cause of male infertility

2442;The authentic, non-tagged protein was localized to the mitochondria

2443;RNA polymerase subunit ABC10-beta, common to RNA polymerases I, II, and III2444;Hypothetical protein

2445;Golgi matrix protein involved in the structural organization of the cis-Golgi; interacts genetically with COG3 and USO1

2446;Hypothetical protein

2447;RNA polymerase subunit ABC14.5, common to RNA polymerases I, II, and III

2448;Conserved protein of the mitochondrial matrix, required for synthesis of mitochondrial and cytosolic iron-sulfur proteins, performs a scaffolding function in mitochondria during Fe/S cluster assembly; isu1 isu2 double mutant is inviable

2449;Hypothetical protein

2450;Transcriptional repressor involved in regulation of meiosis and silencing; contains WD repeats

2451;Mitogen-activated kinase kinase involved in protein kinase C signaling pathway that controls cell integrity; upon activation by Bck1p phosphorylates downstream target, Slt2p ; functionally redundant with Mkk2p

2452;Protein of the mitochondrial matrix involved in protein import into mitochondria; acts as a cochaperone and a nucleotide release factor for Ssclp; homolog of E. coli GrpE

2453;Nonessential protein kinase with unknown cellular role

2454;Dihydrofolate reductase, part of the dTTP biosynthetic pathway, involved in folate metabolism, possibly required for mitochondrial function

2455;Protein implicated in the regulation of ergosterol biosynthesis; one of a seven member gene family with a common essential function and non-essential unique functions; similar to human oxysterol binding protein (OSBP)

2456;Sporulation specific protein that localizes to the spore wall; required for sporulation at a point after meiosis II and during spore wall formation; SSP2 expression is induced midway in meiosis

2457;Pseudouridine synthase, catalyzes pseudouridylation at position 35 in U2 snRNA, position 13 in cytoplasmic tRNAs, and position 35 in pre-tRNA(Tyr); Asp(256) mutation abolishes activity; conserved in archaea, some bacteria, and vertebrates

2458;Histone acetyltransferase catalytic subunit of the native multisubunit complex (NuA4) that acetylates four conserved internal lysines of histone H4 N-terminal tail; required for cell cycle progression

2459;Diacylglycerol acyltransferase, catalyzes the terminal step of triacylglycerol (TAG) formation, acylates diacylglycerol using acyl-CoA as an acyl donor, localized to lipid particles

2460;Protein with similarity to oxidoreductases, found in lipid particles; required for replication of Brome mosaic virus in S. cerevisiae, which is a model system for studying replication of positive-strand RNA viruses in their natural hosts

2461;Subunit of the Anaphase-Promoting Complex/Cyclosome (APC/C), which is a ubiquitin-protein ligase required for degradation of anaphase inhibitors, including mitotic cyclins, during the metaphase/anaphase transition

2462;Subunit of cleavage factor I (CFI), involved in both the endonucleolyitc cleavage and polyadenylation steps of mRNA 3'-end maturation

2463;catalyzes transfer of the sulfane atom of thiosulfate to cyanide to form sulfite and thiocyanate

2464;Subunit of the N-terminal acetyltransferase NatA (Nat1p, Ard1p, Nat5p); N-terminally acetylates many proteins, which influences multiple processes such as the cell cycle, heat-shock resistance, mating, sporulation, and telomeric silencing

2465;Essential subunit of Sec63 complex (Sec63p, Sec62p, Sec66p and Sec72p); with Sec61 complex, Kar2p/BiP and Lhslp forms a channel competent for SRP-dependent and post-translational SRP-independent protein targeting and import into the ER

2466;Component of the spindle pole body (SPB) half-bridge, required for SPB duplication in mitosis and meiosis II; homolog of mammalian centrin; interacts with Kar1p

2467;Member of the third branch of the histidine triad (HIT) superfamily of nucleotide-binding proteins; similar to Aprataxin, a Hint related protein that is mutated in individuals with ataxia with oculomotor apraxia

2468;One of six ATPases of the 19S regulatory particle of the 26S proteasome involved in the degradation of ubiquitinated substrates; required for spindle pole body duplication; localized mainly to the nucleus throughout the cell cycle

2469;Protein required for cell viability

2470;Protein involved in microtubule morphogenesis, required for protection from excess free beta-tubulin; proposed to be involved the folding of beta-tubulin

2471;Protein kinase implicated in activation of the plasma membrane H(+)-ATPase Pmalp in response to glucose metabolism; plays a role in ion homeostasis

2472;Hypothetical protein

2473;Constituent of 66S pre-ribosomal particles, required for maturation of the large ribosomal subunit

2474;Delta 2-isopentenyl pyrophosphate:tRNA isopentenyl transferase, required for biosynthesis of the modified base isopentenyladenosine in mitochondrial and cytoplasmic tRNAs; gene is nuclear and encodes two isozymic forms

2475;Protein involved in proteolytic activation of Rim101p in response to alkaline pH; member of the PalA/AIP1/Alix family; interacts with the ESCRT-III subunits Snf7p, suggesting a relationship between the response to pH and multivesicular body formation

2476;DNA-binding protein required for vegetative repression of middle sporulation genes; specificity factor that directs the Hstlp histone deacetylase to some of the promoters regulated by Sum1p

2477;Essential protein with similarity to phosducins, which are GTPase inhibitors; lethality of null mutation is functionally complemented by expression of mouse phosducin-like protein MgcPhLP

2478;Hypothetical protein

2479;Cytoplasmic protein of unknown function; computational analysis of large-scale protein-protein interaction data suggests a possible role in actin patch assembly

2480;Hypothetical protein

2481;The authentic, non-tagged protein was localized to the mitochondria

2482;Member of the protein disulfide isomerase (PDI) family; overexpression suppresses the defect in maturation of carboxypeptidase Y, and defects in other essential Pdilp functions, caused by PDI1 deletion

2483;Catalytic subunit of the 11-subunit SWI/SNF chromatin remodeling complex involved in transcriptional regulation; contains DNA-stimulated ATPase activity; functions interdependently in transcriptional activation with Snf5p and Snf6p

2484;Hypothetical protein

2485;Hypothetical protein

2486;Essential protein that binds ribosomal protein L11 and is required for nuclear export of the 60S pre-ribosomal subunit during ribosome biogenesis; mouse homolog shows altered expression in Huntington's disease model mice

2487:Component of the mitochondrial Thn54p-Tim22p complex involved in insertion of polytopic proteins into the inner membrane; may function to stabilize the complex

2488;Protein of unknown function involved in the organization of the outer spore wall layers; has similarity to the tafazzins superfamily of acetyltransferases 2489;Protein involved in bud-site selection; diploid mutants display an axial-like budding pattern

2490;CPA1 uORF , Arginine attenuator peptide, regulates translation of the CPA1 mRNA

2491;Protein of unknown function; green fluorescent protein (GFP)-fusion protein localizes to the cell periphery, cytoplasm, bud, and bud neck

2492;Protein with similarity to mammalian monocarboxylate permeases, which are involved in transport of monocarboxylic acids across the plasma membrane; mutant is not deficient in monocarboxylate transport

2493;Protein involved in ER-to-Golgi transport

2494;U2-snRNP associated splicing factor with similarity to the mammalian splicing factor SAP49; proposed to function as a U2-snRNP assembly factor along with Hsh155p and binding partner Cus1p; contains two RNA recognition motifs (RRM)

2495;N-acetylglucosaminyltransferase capable of modification of N-linked glycans in the Golgi apparatus

2496;Protein of unknown function; null mutant shows a reduced affinity for the alcian blue dye suggesting a decreased net negative charge of the cell surface

2497;One of two type V myosins, involved in polarized distribution of mitochondria; required for mitochondrion and vacuole inheritance and nuclear spindle orientation; moves multiple cargo

2498;Vesicle membrane receptor protein (v-SNARE) involved in the fusion between Golgi-derived secretory vesicles with the plasma membrane; member of the synaptobrevin/VAMP family of R-type v-SNARE proteins

2499;Protein required for normal cortical actin organization and endocytosis; multicopy suppressor of clathrin deficiency; acts as a targeting subunit for protein phosphatase type 1

2500;Subunit E of the eight-subunit V1 peripheral membrane domain of the vacuolar H+-ATPase (V-ATPase), an electrogenic proton pump found throughout the endomembrane system; required for the V1 domain to assemble onto the vacuolar membrane

2501;Cytoplasmic alanyl-tRNA synthetase, required for protein synthesis; point mutation (cdc64-1 allele) causes cell cycle arrest at G1; lethality of null mutation is functionally complemented by human homolog

2502;Protein required for beta-1,6 glucan biosynthesis; mutations result in aberrant morphology and severe growth defects

2503;Ty1 enhancer activator required for full levels of Ty enhancer-mediated transcription; C6 zinc cluster DNA-binding protein

2504;RNA polymerase I subunit A43

2505;RNA polymerase I subunit; largest subunit of RNA polymerase I

2506;Hypothetical protein

2507;Serine-rich protein that contains a basic-helix-loop-helix (bHLH) DNA binding motif; binds E-boxes of glycolytic genes and contributes to their activation; may function as a transcriptional activator in Ty1-mediated gene expression

2508;Proline permease, required for high-affinity transport of proline; also transports the toxic proline analog azetidine-2-carboxylate (AzC); PUT4 transcription is repressed in ammonia-grown cells

2509;Tubulin folding factor D involved in beta-tubulin (Tub2p) folding; isolated as mutant with increased chromosome loss and sensitivity to benomyl

2510;Protein required for cell morphogenesis and cell separation after mitosis

2511;Protein of unknown function, required for growth on glycerol as a carbon source

2512;Hypothetical protein

2513;Subunit of the heme-activated, glucose-repressed Hasp2/3/4/5 CCAAT-binding complex, a transcriptional activator and global regulator of respiratory gene expression; required for assembly and DNA binding activity of the complex

2514;Protein of unknown function, shows genetic interactions with Vtilp, which is a v-SNARE involved in cis-Golgi membrane traffic

2515;20S proteasome alpha-type subunit

2516;Autoregulatory oleate-specific transcriptional activator of peroxisome proliferation, contains Zn(2)-Cys(6) cluster domain, forms heterodimer with Oaflp, binds oleate response elements (OREs), activates beta-oxidation genes

2517;Hypothetical protein

2518;Checkpoint protein, involved in the activation of the DNA damage and meiotic pachytene checkpoints; with Mec3p and Ddclp, forms a clamp that is loaded onto partial duplex DNA; homolog of human and S. pombe Rad1 and U. maydis Ree1 proteins

2519;Protein component of the small (40S) ribosomal subunit; has similarity to rat ribosomal protein S12

2520;Rab escort protein, forms a complex with the Ras-like small GTPase Ypt1p that is required for the prenylation of Ypt1p by protein geranylgeranyltransierase type II (Bet2p-Bet4p)

2521;Proposed beta subunit of the heterotrimeric G protein that interacts with the receptor Grp1p, has signaling role in response to nutrients; involved in regulation of pseudohyphal growth through cAMP levels; homolog of Gpb2p

2522;Transcriptional activator essential for nuclear division; localized to the nucleus; essential component of the mechanism that activates the expression of a set of late-S-phase-specific genes

2523;Mitochondrial aldehyde dehydrogenase, required for growth on ethanol and conversion of acetaldehyde to acetate; activity is K+ dependent; utilizes NADP+ or NAD+ equally as coenzymes; expression is glucose repressed

2524;NADP(+)-dependent glutamate dehydrogenase, synthesizes glutamate from ammonia and alpha-ketoglutarate; rate of alpha-ketoglutarate utilization differs from Gdh3p; expression regulated by nitrogen and carbon sources

2525;Hypothetical protein

2526;Transcriptional repressor involved in the control of multidrug resistance; negatively regulates expression of the PDR5 gene; member of the Gal4p family of zinc cluster proteins

2527;Mannoprotein that is incorporated into the cell wall via a glycosylphosphatidylinositol (GPI) anchor, involved in the retention of siderophore-iron in the cell wall

2528;Mannoprotein that is incorporated into the cell wall via a glycosylphosphatidylinositol (GPI) anchor, involved in the retention of siderophore-iron in the cell wall

2529;Putative ferric reductase with similarity to Fre2p; expression induced by low iron levels

2530;DNA photolyase involved in photoreactivation, repairs pyrimidine dimers in the presence of visible light; induced by DNA damage; regulated by transcriptional repressor Rph1p

2531;Beta subunit of the translation initiation factor eIF2, involved in the identification of the start codon; proposed to be involved in mRNA binding

2532;Hypothetical protein

2533;Essential protein involved in transcription regulation; component of chromatin remodeling complexes; required for assembly and function of the INO80 complex; member of the RUVB-like protein family

2534;Vacuolar ATPase V0 domain subunit c', involved in proton transport activity; hydrophobic integral membrane protein (proteolipid) containing four transmembrane segments; N and C termini are in the vacuolar lumen

2535;Essential component of the MIND kinetochore complex (Mtw1p Including Nnflp-Nsl1p-Dsn1p) which joins kinetochore subunits contacting DNA to those contacting microtubules; required for accurate chromosome segregation

2536;Plasma membrane t-SNARE involved in fusion of secretory vesicles at the plasma membrane; forms a complex, with t-SNARE Sec9p, that binds v-SNARE Snc2p; also required for sporulation; syntaxin homolog that is functionally redundant with Sso2p

2537;Alpha subunit of fatty acid synthetase, which catalyzes the synthesis of long-chain saturated fatty acids; contains beta-ketoacyl reductase and beta-ketoacyl synthase activities

2538;Hypothetical protein

2539;Beta (RNA 5'-triphosphatase) subunit of the mRNA capping enzyme, a heterodimer (the other subunit is CEG1, a guanylyltransferase) involved in adding the 5' cap to mRNA; the mammalian enzyme is a single bifunctional polypeptide

2540;UDP-glucose:dolichyl-phosphate glucosyltransferase, involved in asparagine-linked glycosylation in the endoplasmic reticulum

2541;ATP binding cassette family member; Asn/Gln-rich rich region supports [NU+] prion formation, susceptibility to [PSI+] prion induction and aggregation of a fragment of the human Machado-Joseph Disease protein

2542;Hypothetical protein

2543;Cyclin, interacts with Pho85p cyclin-dependent kinase (Cdk) to phosphorylate and regulate glycogen synthase, also activates Pho85p for Glc8p phosphorylation

2544;Essential conserved nucleolar GTP-binding protein required for synthesis of 40S ribosomal subunits and for processing of the 35S pre-rRNA at sites A0, A1, and A2; interacts with Rcl1p, has similarity to Tsr1p

2545;Component of U2 snRNP; disruption causes reduced U2 snRNP levels; physically interacts with Msl1p; putative homolog of human U2A' snRNP protein

2546;Nucleolar protein required for 60S ribosome subunit biogenesis, constituent of 66S pre-ribosomal particles; physically interacts with Nop8p and the exosome subunit Rrp43p

2547;SET-domain lysine-N-methyltransferase, catalyzes the formation of dimethyllysine residues on the large ribsomal subunit protein L23a (RPL23A and RPL23B)

2548;Endoplasmic reticulum protein of unknown function; contains glycerophosphodiester phosphodiesterase motifs but mutation does not detectably affect glycerophosphoinositol or glycerophosphocholine metabolism

2549;Protein kinase involved in regulating diverse events including vesicular trafficking, gene expression, DNA repair, and chromosome segregation; binds the CTD of RNA pol II; homolog of mammalian casein kinase 1delta (CK1delta)

2550;Subunit of cytoplasmic cAMP-dependent protein kinase, which contains redundant catalytic subunits Tpk1p, Tpk2p, and Tpk3p and regulatory subunit Bcy1p; promotes vegetative growth in response to nutrients; activates filamentous growth

2551;Protein that interacts with glycerol 3-phosphatase and plays a role in anaerobic glycerol production; localizes to the nucleus and cytosol

2552;Protein required for accurate chromosome segregation during meiosis

2553;Protein of unknown function required for normal levels of resistance to oxidative damage, null mutants are sensitive to hydrogen peroxide; member of a conserved family of proteins found in eukaryotes but not in prokaryotes

2554;DNA damage checkpoint protein, part of a PCNA-like complex required for DNA damage response, required for pachytene checkpoint to inhibit cell cycle in response to unrepaired recombination intermediates; potential Cdc28p substrate

2555;Hypothetical protein

2556;Identified by homology to Ashbya gossypii

2557;Probable membrane protein with a possible role in proton symport of glycerol; member of the MBOAT family of putative membrane-bound O-acyltransferases; Gup1p homolog

2558;Mating pheromone alpha-factor, made by alpha cells; interacts with mating type a cells to induce cell cycle arrest and other responses leading to mating; also encoded by MF(ALPHA)2, although MF(ALPHA)1 produces most alpha-factor

2559;Homolog of the prokaryotic ribosomal protein L36, likely to be a mitochondrial ribosomal protein coded in the nuclear genome

2560;Hypothetical protein

2561;Small subunit of the heterodimeric cap binding complex that also contains Sto1p, component of the spliceosomal commitment complex; interacts with Np13p, possibly to package mRNA for export from the nucleus; contains an RNA-binding motif

2562;Homeodomain-containing transcriptional repressor of PTR2, which encodes a major peptide transporter; imported peptides activate ubiquitin-dependent proteolysis, resulting in degradation of Cup9p and de-repression of PTR2 transcription

2563;Plasma membrane localized protein of unknown function; contains sequence similarity to the transferrin receptor

2564;Mitochondrial ribosomal protein of the large subunit

2565;Heme-binding protein involved in regulation of cytochrome P450 protein Erg11p; damage response protein, related to mammalian membrane progesterone receptors; mutations lead to defects in telomeres, mitochondria, and sterol synthesis

2566;Protein of unknown function; deletion heterozygote is sensitive to compounds that target ergosterol biosynthesis, may be involved in compound availability

2567;Cell wall and vacuolar protein, required for wild-type resistance to vanadate

2568;Hypothetical protein

2569;Cytosolic leucyl tRNA synthetase, ligates leucine to the appropriate tRNA

2570;Protein required for respiratory growth and stability of the mitochondrial genome

2571;Trimethyl guanosine synthase, conserved nucleolar methyl transferase responsible for conversion of the m(7)G cap structure of snRNAs and snoRNAs to m(2,2,7)G; also required for ribosome synthesis and nucleolar morphology

2572;Pheromone-regulated protein, predicted to have 1 transmembrane segment; transcriptionally regulated by Ste12p during mating and by Cat8p during the diauxic shift

2573;Vacuolar aspartyl protease (proteinase A), required for the posttranslational precursor maturation of vacuolar proteinases; synthesized as a zymogen, self-activates 2574;Protein kinase, required for cell-cycle arrest in response to DNA damage; activated by trans autophosphorylation when interacting with hyperphosphorylated Rad9p

2575;Activator of the phosphotyrosyl phosphatase activity of PP2A; regulates G1 phase progression, the osmoresponse and microtubule dynamics; implicated in the spindle assembly check; subunit of the Tap42p-Pph21p-Rrd2p complex

2576;Splicing factor that is found in the Ceflp subcomplex of the spliceosome

2577;Subunit of a heterodimeric peroxisomal ATP-binding cassette transporter complex (Pxa1p-Pxa2p), required for import of long-chain fatty acids into peroxisomes; similarity to human adrenoleukodystrophy transporter and ALD-related proteins

2578;Nucleolar protein; involved in biogenesis of the 60S subunit of the ribosome; interacts with rRNA processing factors Cbf5p and Nop2p; null mutant is viable but growth is severely impaired

2579;Member of the oxysterol binding protein family, which includes seven yeast homologs; involved in negative regulation of Sec14p-dependent Golgi complex secretory functions, peripheral membrane protein that localizes to the Golgi complex

2580;Subunit of COMPASS (Set1C), a complex which methylates histone H3 on lysine 4 and is required in telomeric transcriptional silencing; PHD finger domain protein similar to human CGBP, an unmethylated CpG binding protein

2581;Conserved protein of the mitochondrial matrix, performs a scaffolding function during assembly of iron-sulfur clusters, interacts physically and functionally with yeast frataxin (Yfh1p); isu1 isu2 double mutant is inviable

2582;Identified by gene-trapping, microarray-based expression analysis, and genome-wide homology searching

2583;Zinc cluster protein involved in conferring resistance to ketoconazole

2584;Protein component of the large (60S) ribosomal subunit with similarity to E. coli L18 and rat L5 ribosomal proteins; binds 5S rRNA and is required for 60S subunit assembly

2585;Meiosis-specific protein of unknown function, involved in completion of nuclear divisions; identified as a weak high-copy suppressor of the spo1-1 ts mutation; putative GPI-dependent cell-wall protein

2586;Histone H1, a linker histone required for nucleosome packaging at restricted sites; suppresses DNA repair involving homologous recombination; not required for telomeric silencing, basal transcriptional repression, or efficient sporulation

2587;U3 snoRNP protein, component of the small (ribosomal) subunit (SSU) processosome containing U3 snoRNA; required for the biogenesis of18S rRNA

2588;Inner plaque spindle pole body (SPB) component, links the central plaque component Spc42p to the inner plaque component Spc110p; required for SPB duplication

2589;Subunit of TFIIH and nucleotide excision repair factor 3 complexes, involved in transcription initiation, required for nucleotide excision repair, similar to 52 kDa subunit of human TFIIH

2590;Putative ATP-dependent RNA helicase of the DEAD-box protein family; mutants show reduced stability of the 40S ribosomal subunit scanning through 5' untranslated regions of mRNAs

2591;Mitochondrial ribosomal protein of the large subunit; MRP51 exhibits genetic interactions with mutations in the COX2 and COX3 mRNA 5'-untranslated leader sequences

2592;Isopentenyl diphosphate:dimethylallyl diphosphate isomerase (IPP isomerase), catalyzes an essential activation step in the isoprenoid biosynthetic pathway; required for viability

2593;Trichostatin A-insensitive homodimeric histone deacetylase (HDAC) with specificity in vitro for histones H3, H4, H2A, and H2B; similar to Hda1p, Rpd3p, Hos1p, and Hos2p; deletion results in increased histone acetylation at rDNA repeats

2594;Rho GTPase activating protein (RhoGAP) involved in control of the cytoskeleton organization; targets the essential Rho-GTPase Cdc42p, which controls establishment and maintenance of cell polarity, including bud-site assembly

2595;Putative dehydrogenase

2596;Peripheral peroxisomal membrane peroxin required for the regulation of peroxisome size and maintenance, recruits GTPase Rho1p to peroxisomes, induced by oleate, interacts with homologous protein Pex27p

2597;Arginase, responsible for arginine degradation, expression responds to both induction by arginine and nitrogen catabolite repression; disruption enhances freeze tolerance

2598;Glycerophosphocholine (GroPCho) phosphodiesterase; hydrolyzes GroPCho to choline and glycerolphosphate after its uptake by Gitlp permease, for use as a phosphate source and as a precursor for phosphocholine synthesis

2599;Hypothetical protein

2600;Mitochondrial aspartyl-tRNA synthetase, required for acylation of aspartyl-tRNA; yeast and bacterial aspartyl-, asparaginyl-, and lysyl-tRNA synthetases contain regions with high sequence similarity, suggesting a common ancestral gene

2601;The authentic, non-tagged protein was localized to the mitochondria

2602;Elongator protein, part of the HAP subcomplex of Elongator, which is a six-subunit component of the RNA polymerase II holoenzyme; required for Elongator structural integrity and histone acetyltransferase activity

2603;The authentic, non-tagged protein was localized to the mitochondria

2604;Protein required for growth of cells lacking the mitochondrial genome

2605;Endoplasmic reticulum membrane protein that binds to and inhibits GTP-bound Ras2p at the ER; component of the GPI-GnT complex which catalyzes the first step in GPI-anchor biosynthesis

2606;Putative GTPase that associates with free 60S ribosomal subunits in the nucleolus and is required for 60S ribosomal subunit biogenesis; constituent of 66S pre-ribosomal particles; member of the ODN family of nucleolar G-proteins

2607;Cytosolic and mitochondrial glutathione oxidoreductase, converts oxidized glutathione to reduced glutathione

2608;MADS-box transcription factor, component of the protein kinase C-mediated MAP kinase pathway involved in the maintenance of cell integrity; phosphorylated and activated by the MAP-kinase Slt2p

2609;Alkaline dihydroceramidase, involved in sphingolipid metabolism; preferentially hydrolyzes dihydroceramide to a free fatty acid and dihydrosphingosine; has a minor reverse activity

2610;Histone acetyltransferase subunit of the Elongator complex, which is a component of the RNA polymerase II holoenzyme; activity is directed specifically towards histones H3 and H4; disruption confers resistance to K. lactis zymotoxin

2611;Cytoplasmic class E vacuolar protein sorting (VPS) factor that coordinates deubiquitination in the multivesicular body (MVB) pathway by recruiting Doa4p to endosomes

2612;Subunit b of the stator stalk of mitochondrial F1F0 ATP synthase, which is a large, evolutionarily conserved enzyme complex required for ATP synthesis

2613;Protein of unknown function, localized to the cytoplasm; computational analysis of large-scale protein-protein interaction data suggests a possible role in transcriptional regulation

2614;Geranylgeranyl diphosphate synthase, increases the intracellular pool of geranylgeranyl diphosphate, suppressor of bet2 mutation that causes defective geranylgeranylation of small GTP-binding proteins that mediate vesicular traffic

2615;Hypothetical protein

2616;Hypothetical protein

2617;Hypothetical protein

2618;Component of the ESCRT-I complex, which is involved in ubiquitin-dependent sorting of proteins into the endosome; involved in transport of precursors for soluble vacuolar hydrolases from the late endosome to the vacuole

2619;Component of a complex containing Ceflp, putatively involved in pre-mRNA splicing; has similarity to S. pombe Cwf27p

2620;Constituent of the mitochondrial inner membrane presequence translocase (TIM23 complex); may promote binding of incoming precursor proteins to the intermembrane space domain of Tom22p during translocation

2621;Cytosolic aldehyde dehydrogenase that is activated by Mg2+ and utilizes NADP+ as the preferred coenzyme; required for the conversion of acetaldehyde to acetate; constitutively expressed

2622;Protein of unknown function; null mutant forms abnormally large cells

2623;GTPase of the Ras superfamily required to recruit Ar11p to the Golgi; similar to ADP-ribosylation factor

2624;Subunit of Golgi mannosyltransferase complex also containing Anp1p, Mnn10p, Mnn11p, and Hoc1p that mediates elongation of the polysaccharide mannan backbone; forms a separate complex with Van1p that is also involved in backbone elongation

2625;Translational cofactor elongation factor-1 gamma, participates in the regulation of GTP-binding protein EF-1 alpha, may play a redundant role in the regulation of protein synthesis or another GTP-dependent process

2626;Integral subunit of SAGA histone acetyltransferase complex, regulates transcription of a subset of SAGA-regulated genes, required for the Ubp8p association with SAGA and for H2B deubiquitylation

2627;Elongin C, forms heterodimer with Elalp that participates in transcription elongation; expression dramatically upregulated during sporulation; widely conserved among eukaryotes

2628;Subunit of the homotypic vacuole fusion and vacuole protein sorting (HOPS) complex; part of the Class C Vps complex essential for membrane docking and fusion at both the Golgi-to-endosome and endosome-to-vacuole stages of protein transport

2629;Nucleolar protein, essential for processing and maturation of 27S pre-rRNA and large ribosomal subunit biogenesis; constituent of 66S pre-ribosomal particles; contains four RNA recognition motifs (RRMs)

2630;Cyclin-dependent protein kinase, component of RNA polymerase II holoenzyme; involved in phosphorylation of the RNA polymerase II C-terminal domain; involved in glucose repression

2631;hypothetical protein

2632;Hypothetical protein

2633;Zinc-finger DNA-binding protein, involved in regulating expression of the methionine biosynthetic genes, similar to Met32p

2634;Subunit beta1 of the nascent polypeptide-associated complex (NAC) involved in protein targeting, associated with cytoplasmic ribosomes; enhances DNA binding of the Gal4p activator; homolog of human BTF3b

2635;Protein of unknown function, mutant phenotype suggests a potential role in vacuolar function; green fluorescent protein (GFP)-fusion protein localizes to the cell periphery, cytoplasm, bud, and bud neck

2636;Hypothetical protein

2637;Acetyl-CoA C-acetyltransferase (acetoacetyl-CoA thiolase), cytosolic enzyme that transfers an acetyl group from one acetyl-CoA molecule to another, forming acetoacetyl-CoA; involved in the first step in mevalonate biosynthesis

2638;Putative serine/threonine protein kinase; involved in the adaptation to low concentrations of glucose independent of the SNF3 regulated pathway

2639;Involved in response to DNA damage; null mutants have increased rates of recombination and delayed S phase; interacts physically and genetically with Sgs1p (RecQ family member) and Top3p (topoisomerase III)

2640;Non-essential protein of unconfirmed function; affects pre-rRNA processing, may act as a negative regulator of the transcription of genes involved in pseudohyphal growth; homologous to Srd1p

2641;Ub1 (ubiquitin-like protein)-specific protease that cleaves Smt3p protein conjugates; specifically required for cell cycle progression; associates with nucleoporins and may interact with septin rings during telophase

2642;Vacuolar membrane protein involved in vacuolar polyphosphate accumulation; functions as a regulator of vacuolar H+-ATPase activity and vacuolar transporter chaperones; involved in non-autophagic vacuolar fusion

2643;Outer kinetochore protein, required for accurate mitotic chromosome segregation; component of the kinetochore sub-complex COMA (Ctf19p, Okp1p, Mcm21p, Amelp) that functions as a platform for kinetochore assembly

2644;One of 11 subunits of the SWI/SNF chromatin remodeling complex that regulates transcription by remodeling chromosomes; required for transcription of many genes, including ADH1, ADH2, GAL1, HO, INO1 and SUC2

2645;Hypothetical protein

2646;Mitochondrial ribosomal protein of the small subunit

2647;Protein required for export of the ribosomal subunits; associates with the RNA components of the pre-ribosomes; contains HEAT-repeats

2648;Zeta subunit of the coatomer complex (COPI), which coats Golgi-derived transport vesicles; involved in retrograde transport between Golgi and ER

2649;Peripheral mitochondrial inner membrane protein, located on the matrix face of the membrane; stabilizes the bicistronic AAP1-ATP6 mRNA encoding subunits 6 and 8 of the ATP synthase complex

2650;Long chain base-responsive inhibitor of protein kinases Pkh1p and Pkh2p, acts along with Pil1p to down-regulate heat stress resistance via regulation of the Pkc1p and Ypk1p pathways; phosphorylated by Phk1p and Phk2p

2651;Protein that acts together with Uba3p to activate Rublp before its conjugation to proteins (neddylation), which may play a role in protein degradation

2652;Component of the ESCRT-II complex, which is involved in ubiquitin-dependent sorting of proteins into the endosome; appears to be functionally related to SNF7; involved in glucose derepression

2653;Hypothetical protein

2654;Meiosis-specific component of sister chromatid cohesion complex; maintains cohesion between sister chromatids during meiosis I; maintains cohesion between centromeres of sister chromatids until meiosis II; homolog of S. pombe Rec8p

2655;Transcriptional activator involved in the transcription of TPO2, HSP30 and other genes encoding membrane stress proteins; despite sequence similarity with the transcription factor Ace1p, it is not subject to metalloregulation

2656;Putative transcription factor; multicopy suppressor of mutations that cause low activity of the cAMP/protein kinase A pathway; highly similar to Sutlp

2657;Hypothetical protein

2658;Hypothetical protein

2659;Hypothetical protein

2660;Constituent of 66S pre-ribosomal particles, has similarity to human translation initiation factor 6 (eIF6); may be involved in the biogenesis and or stability of 60S ribosomal subunits

2661;Nucleotide release factor functioning in the post-Golgi secretory pathway, required for ER-to-Golgi transport, binds zinc, found both on membranes and in the cytosol; guanine nucleotide dissociation stimulator

2662;Essential helicase component of heterohexameric MCM2-7 complexes which bind pre-replication complexes on DNA and melt the DNA prior to replication; accumulates in the nucleus in G1; homolog of S. pombe Cdc21p

2663;Subunit g of the mitochondrial F1F0 ATP synthase, which is a large enzyme complex required for ATP synthesis; associated only with the dimeric form of ATP synthase

2664;Hypothetical protein

2665;Subunit, with Mgrlp, of the mitochondrial inner membrane i-AAA protease complex, which is responsible for degradation of unfolded or misfolded mitochondrial gene products; mutation causes an elevated rate of mitochondrial turnover

2666;Gamma-adaptin, large subunit of the clathrin-associated protein (AP-1) complex; binds clathrin; involved in vesicle mediated transport

2667;Protein with roles in exocytosis and cation homeostasis; functions in docking and fusion of post-Golgi vesicles with plasma membrane; homolog of Sro77p and Drosophila lethal giant larvae tumor suppressor; interacts with SNARE protein Sec9p

2668;Cytoplasmic and mitochondrial histidine tRNA synthetase; encoded by a single nuclear gene that specifies two messages; efficient mitochondrial localization requires both a presequence and an amino-terminal sequence

2669;Glutamine synthetase (GS), synthesizes glutamine from glutamate and ammonia; with Glt1p, forms the secondary pathway for glutamate biosynthesis from ammonia; expression regulated by nitrogen source and by amino acid limitation

2670;Protein that interacts physically and genetically with Tap42p, which regulates protein phosphatase 2A; component of the TOR (target of rapamycin) signaling pathway

2671;Member of the PUF protein family, which is defined by the presence of Pumilio homology domains that confer RNA binding activity; preferentially binds mRNAs encoding membrane-associated proteins

2672;Hypothetical protein

2673;Protein involved in kinetochore-microtubule mediated chromosome segregation; binds to centromere DNA

2674;Protein with a potential role in DNA replication; displays synthetic lethal genetic interaction with the pol3-13 allele of POL3, which encodes DNA polymerase delta

2675;Peripheral membrane protein required for delivery of aminopeptidase I (Lap4p) to the vacuole in the cytoplasm-to-vacuole targeting pathway; also required for peroxisomal degradation (pexophagy)

2676;High-mobility group non-histone chromatin protein, functionally redundant with Nhp6Bp; homologous to mammalian high mobility group proteins 1 and 2; acts to recruit transcription factor Rcs1p to certain promoters

2677;Subunit of TFIIH complex, involved in transcription initiation, similar to 34 kDa subunit of human TFIIH; interacts with Ssl1p

2678;Putative mitochondrial inner membrane transporter, member of the mitochondrial carrier (MCF) family

2679;Chorismate mutase, catalyzes the conversion of chorismate to prephenate to initiate the tyrosine/phenylalanine-specific branch of aromatic amino acid biosynthesis

2680;Probable Hsp40p co-chaperone, has a DnaJ-like domain and appears to be involved in ER-associated degradation of misfolded proteins containing a tightly folded cytoplasmic domain; inhibits replication of Brome mosaic virus in S. cerevisiae

2681;Heme-dependent repressor of hypoxic genes; contains an HMG domain that is responsible for DNA bending activity

2682;Putative class I histone deacetylase (HDAC) with sequence similarity to Hda1p, Rpd3p, Hos2p, and Hos3p; deletion results in increased histone acetylation at rDNA repeats; interacts with the Tup1p-Ssn6p corepressor complex

2683;Spermidine synthase, involved in biosynthesis of spermidine and also in biosynthesis of pantothenic acid; spermidine is required for growth of wild-type cells

2684;Subunit of the RNA polymerase II mediator complex; associates with core polymerase subunits to form the RNA polymerase II holoenzyme; essential for transcriptional regulation

2685;Subunit of the CCR4-NOT complex, which is a global transcriptional regulator with roles in transcription initiation and elongation and in mRNA degradation 2686;Protein phosphotyrosine phosphatase of unknown cellular role; activated by adenine

2687;Transketolase, similar to Tkl2p; catalyzes conversion of xylulose-5-phosphate and ribose-5-phosphate to sedoheptulose-7-phosphate and glyceraldehyde-3-phosphate in the pentose phosphate pathway; needed for synthesis of aromatic amino acids

2688;Translational elongation factor EF-1 alpha; also encoded by TEF2; functions in the binding reaction of aminoacyl-tR-NA (AA-tRNA) to ribosomes

2689;17-kDa component of the U4/U6aU5 tri-snRNP, plays an essential role in pre-mRNA splicing, orthologue of the human U5-specific 15-kDa protein

2690;Protein required for normal mitochondrial morphology and inheritance

2691;Hypothetical protein

2692;Transcription factor TFIIB, a general transcription factor required for transcription initiation and start site selection by RNA polymerase II

2693;Signal recognition particle (SRP) subunit (homolog of mammalian SRP54); contains the signal sequence-binding activity of SRP, interacts with the SRP RNA, and mediates binding of SRP to signal receptor; contains GTPase domain

2694;Guanine nucleotide exchange factor (GEF) for Arf proteins; involved in vesicular transport; suppressor of ypt3 mutations; member of the Sec7-domain family

2695;Hypothetical protein

2696;Hypothetical protein

2697;Mitochondrial ribosomal protein of the large subunit

2698;Protein component of the large (60S) ribosomal subunit, nearly identical to Rpl11Bp; involved in ribosomal assembly; depletion causes degradation of proteins and RNA of the 60S subunit; has similarity to E. coli L5 and rat L11

2699;20S proteasome beta-type subunit, responsible for the chymotryptic activity of the proteasome

2700;Essential component of the conserved oligomeric Golgi complex (Coglp through Cog8p), a cytosolic tethering complex that functions in protein trafficking to mediate fusion of transport vesicles to Golgi compartments

2701;Predicted protein kinase, overexpression causes sensitivity to staurosporine, which is a potent inhibitor of protein kinase C

2702;Essential, non-ATPase regulatory subunit of the 26S proteasome, similar to another S. cerevisiae regulatory subunit, Rpn5p, as well as to mammalian proteasome subunits

2703;Ser/Thr kinase involved in late nuclear division, one of the mitotic exit network (MEN) proteins; necessary for the execution of cytokinesis

2704;Phosphatidylinositol synthase, required for biosynthesis of phosphatidylinositol, which is a precursor for polyphosphoinositides, sphingolipids, and glycolipid anchors for some of the plasma membrane proteins

2705;Hypothetical protein

2706;B-type cyclin involved in cell cycle progression; activates Cdc28p to promote the transition from G2 to M phase; accumulates during G2 and M, then targeted via a destruction box motif for ubiquitin-mediated degradation by the proteasome

2707;Haploid specific endoprotease that performs one of two N-terminal cleavages during maturation of a-factor mating pheromone; required for axial budding pattern of haploid cells

2708;Hypothetical protein

2709;Protein containing an Lsm domain, may bind RNA and have a role in RNA processing; overproduction suppresses a null mutation in CHCl, which encodes the heavy chain of clathrin

2710;Catalytic subunit of the NatB N-terminal acetyltransferase, which catalyzes acetylation of the amino-terminal methionine residues of all proteins beginning with Met-Asp or Met-Glu and of some proteins beginning with Met-Asn or Met-Met

2711; Small mitochondrial outer membrane protein crucial to a binding relay for the import of proteins into mitochondria; subunit on the outer mouth of the TOM channel that accepts precursors from the receptors Tom20p and Tom22p

2712;Protein involved in pre-rRNA processing, associated with U3 snRNP; component of small ribosomal subunit (SSU) processosome; ortholog of the human U3-55k protein

2713;Ammonium permease of high capacity and low affinity; belongs to a ubiquitous family of cytoplasmic membrane proteins that transport only ammonium (NH4+); expression is under the nitrogen catabolite repression regulation ammonia permease

2714;Lyso-phosphatidylcholine acyltransferase, required for normal phospholipid content of mitochondrial membranes; may remodel acyl groups of cardiolipin in the inner membrane; similar to human tafazzin, which is implicated in Barth syndrome

2715;Nucleolar protein, constituent of pre-60S ribosomal particles; required for processing of the 27S pre-rRNA at the A2 site to yield 5.8S and 25S rRNA

2716;Asparagine synthetase, isozyme of Asn2p; catalyzes the synthesis of L-asparagine from L-aspartate in the asparagine biosynthetic pathway

2717;Hypothetical protein

2718;Protein of unknown function; contains transmembrane domains; involved in secretion of proteins that lack classical secretory signal sequences; component of the detergent-insoluble glycolipid-enriched complexes (DIGs)

2719;Protein that induces appearance of [PIN+] prion when overproduced

2720;Protein involved in regulation of mitochondrial expression of subunits 6 (Atp6p) and 8 (Atp8p) of the Fo-F1 ATP synthase; functions with Nca3p

2721;Polyamine transport protein specific for spermine; localizes to the plasma membrane; member of the major facilitator superfamily

2722;Hypothetical protein

2723;Protein required for beta-1,6 glucan biosynthesis; putative beta-glucan synthase; appears functionally redundant with Skn1p

2724;Non-essential glycogen phosphorylase required for the mobilization of glycogen, activity is regulated by cyclic AMP-mediated phosphorylation, expression is regulated by stress-response elements and by the HOG MAP kinase pathway

2725;Cyclin (Bur2p)-dependent protein kinase that functions in transcriptional regulation; phosphorylates the carboxy-terminal domain of Rpo21p, which is the largest subunit of RNA polymerase II; regulated by Cak1p

2726;Subunit of the origin recognition complex, which directs DNA replication by binding to replication origins and is also involved in transcriptional silencing

2727;Translation initiation factor eIF-4B, has RNA annealing activity; contains an RNA recognition motif and binds to single-stranded RNA

2728;Protein likely involved in protection against replication-dependent DNA damage; mutants are sensitive to methyl methanesulfonate (MMS); implicated in regulation of Ty1 transposition

2729;GTP-binding protein of the rho subfamily of Ras-like proteins, involved in establishment of cell polarity; regulates protein kinase C (Pkc1p) and the cell wall synthesizing enzyme 1,3-beta-glucan synthase (Fks1p and Gsc2p)

2730;Mitochondrial ribosomal protein of the small subunit

2731;3'-phosphoadenylsulfate reductase, reduces 3'-phosphoadenylyl sulfate to adenosine-3',5'-bisphosphate and free sulfite using reduced thioredoxin as cosubstrate, involved in sulfate assimilation and methionine metabolism

2732;Adapter that links synaptojanins Inp2p and Inp53p to the cortical actin cytoskeleton

2733;Protein of unknown function, transcriptionally activated by Yrmlp along with genes involved in multidrug resistance

2734;AAA-type ATPase required for efficient late endosome to vacuole transport; catalyzes the release of an endosomal membrane-associated class E VPS protein complex; cytoplasmic protein that is also associated with an endosomal compartment

2735;Protein of unknown function; green fluorescent protein (GFP)-fusion protein localizes to the nuclear periphery; potential Cdc28p substrate

2736;Beta subunit of Type II geranylgeranyltransferase required for vesicular transport between the endoplasmic reticulum and the Golgi; provides a membrane attachment moiety to Rab-like proteins Ypt1p and Sec4p

2737;Splicing factor, component of the U4/U6-U5 snRNP complex 2738;GTPase-activating protein; component of the Sec23p-Sec24p heterodimeric complex of the COPII vesicle coat, involved in ER to Golgi transport and autophagy; stimulates the GDP-bound form of Sar1p

2739;Core Sm protein Sm F; part of heteroheptameric complex (with Smblp, Smd1p, Smd2p, Smd3p, Sme1p, and Smx2p) that is part of the spliceosomal U1, U2, U4, and U5 snRNPs; homolog of human Sm F

2740;Dolichol phosphate mannose (DoI-P-Man) synthase of the ER membrane, catalyzes the formation of Dol-P-Man from Dol-P and GDP-Man; required for glycosyl phosphatidylinositol membrane anchoring, O mannosylation, and protein glycosylation

2741;Glycogen debranching enzyme containing glucanotranferase and alpha-1,6-amyloglucosidase activities, required for glycogen degradation

2742;Regulatory light chain for the type II myosin, Myo1p; binds to an IQ motif of Myo1p, localization to the bud neck depends on Myo1p; involved in the disassembly of the Myo1p ring

2743;Protein involved in exosome mediated 3' to 5' mRNA degradation and translation inhibition of non-poly(A) mRNAs; forms complex with Ski2p and Ski8p; required for repressing propagation of dsRNA viruses

2744;RNApolymerase III subunit C82

2745;Putative permease, member of the allantoate transporter subfamily of the major facilitator superfamily; mutation confers resistance to ethionine sulfoxide

2746;Hypothetical protein

2747;Guanine nucleotide exchange factor (GEF or GDP-release factor) for Cdc42p; required for polarity establishment and maintenance, and mutants have morphological defects in bud formation and shmooing

2748;Cytochrome c heme lyase (holocytochrome c synthase), attaches heme to apo-Cyc1p in the mitochondrial intermembrane space; human ortholog may have a role in microphthalmia with linear skin defects (MLS)

2749;Subunit of both RNase MRP, which cleaves pre-rRNA, and nuclear RNase P, which cleaves tRNA precursors to generate mature 5' ends

2750;Ortholog of human transcriptional coactivator SKIP, can activate transcription of a reporter gene; interacts with splicing factors Prp22p and Prp46p

2751;hypothetical protein

2752;Protein with a potential role in regulatory interactions between microtubules and the cell cycle, as suggested by genetic and physical interactions with Nap1p and genetic interactions with TUB1

2753;Protein whose overexpression affects chromosome stability, potential Cdc28p substrate; homolog of Snf2p

2754;Hypothetical protein

2755;Subunit of the COMPASS (Set1C) complex, which methylates histone H3 on lysine 4 and is required in transcriptional silencing near telomeres; WD40 beta propeller superfamily member with similarity to mammalian Rbbp7

2756;Basic helix-loop-helix-leucine zipper (bHLH/Zip) transcription factor that forms a complex with another bHLH/Zip protein, Rtg1p, to activate the retrograde (RTG) and TOR pathways

2757;Alpha subunit of the F1 sector of mitochondrial F1F0 ATP synthase, which is a large, evolutionarily conserved enzyme complex required for ATP synthesis

2758;Protein implicated in polar growth, functionally redundant with Boi2p; interacts with bud-emergence protein Bem1p; contains an SH3 (src homology 3) domain and a PH (pleckstrin homology) domain

2759;Protein required for autophagy; modified by the serial action of Atg4p, Atg7p, and Atg3p, and conjugated at the C terminus with phosphatidylethanolamine, to become the form essential for generation of autophagosomes

2760;UBX (ubiquitin regulatory X) domain-containing protein that regulates Glc7p phosphatase activity and interacts with Cdc48p; interacts with ubiquitylated proteins in vivo and is required for degradation of a ubiquitylated model substrate

2761;RNA binding protein with similarity to hnRNP-K that localizes to the cytoplasm and to subtelomeric DNA; required for the proper localization of ASH1 mRNA; involved in the regulation of telomere position effect and telomere length

2762;Type I transmembrane sorting receptor for multiple vacuolar hydrolases; cycles between the late-Golgi and prevacuolar endosome-like compartments

2763;Non-essential transcriptional corepressor involved in the cell cycle-regulated transcription of histone H2A, H2B, H3 and H4 genes; contributes to nucleosome formation, heterochromatic gene silencing, and formation of functional kinetochores

2764;Protein of the endoplasmic reticulum membrane involved in chitin deposition in the cell wall; overproduction confers resistance to Congo Red

2765;Plasma membrane multidrug transporter, member of the major facilitator superfamily; involved in efflux of fluconazole, diazaborine, benomyl, methotrexate, and other drugs

2766;Hypothetical protein

2767;Alpha-1,2-mannosyltransferase, responsible for addition of the first alpha-1,2-linked mannose to form the branches on the mannan backbone of oligosaccharides, localizes to an early Golgi compartment

2768;Protein anchored to the mitochondrial inner membrane, similar to Scolp and may have a redundant function with Scolp in delivery of copper to cytochrome c oxidase; interacts with Cox2p

2769;RNA polymerase I enhancer binding protein; DNA binding protein which binds to genes transcribed by both RNA polymerase I and RNA polymerase II; required for termination of RNA polymerase I transcription

2770;ORF, Dubious

2771;Hypothetical protein

2772;Essential protein of the inner mitochondrial membrane, peripherally localized; component of the TIM22 complex, which is a twin-pore translocase that mediates insertion of numerous multispanning inner membrane proteins

2773;Hsp70 (Ssalp) nucleotide exchange factor, cytosolic homolog of Sil1p, which is the nucleotide exchange factor for BiP (Kar2p) in the endoplasmic reticulum

2774;Protein that acts as an adaptor between Myo4p and the She2p-mRNA complex; part of the mRNA localization machinery that restricts accumulation of certain proteins to the bud; also required for cortical ER inheritance

2775;Protein involved in vacuolar assembly, essential for autophagy and the cytoplasm-to-vacuole pathway

2776;Cytoplasmic protein of unknown function, potentially phosphorylated by Cdc28p; YBR138C is not an essential gene

2777;TPR-containing co-chaperone; binds both Hsp82p (Hsp90) and Ssa1p (Hsp70) and stimulates the ATPase activity of SSA1, ts mutants reduce Hsp82p function while over expression suppresses the phenotypes of an HSP82 ts allele and a cpr7 deletion

2778;Subunit of the Ipl1p-Sli15p-Bir1p complex that regulates kinetochore-microtubule attachments, activation of the spindle tension checkpoint, and mitotic spindle disassembly; regulates the activity and localization of the Ipllp aurora kinase

2779;Soluble GTPase with a role in regulation of membrane traffic; regulates potassium influx; G protein of the Ras superfamily, similar to ADP-ribosylation factor

2780;Non-essential subunit of Sec63 complex (Sec63p, Sec62p, Sec66p and Sec72p); with Sec61 complex, Kar2p/BiP and Lhslp forms a channel competent for SRP-dependent and post-translational SRP-independent protein targeting and import into the ER

2781;Essential subunit of the COMPASS (Set1C) complex, which methylates histone H3 on lysine 4 and is required in transcriptional silencing near telomeres; WD40 beta propeller superfamily member and ortholog of mammalian WDR5

2782;Putative transcription factor involved in regulating the response to osmotic stress; member of the MADS-box family of transcription factors

2783;Putative protein of unknown function; YBR184W is not an essential gene

2784;Protein of unknown function, has similarity to Erv14p

2785;RNA binding protein that negatively regulates growth rate; interacts with the 3' UTR of the mitochondrial porin (POR1) mRNA and enhances its degradation; overexpression impairs mitochondrial function; expressed in stationary phase

2786;Mitochondrial ATP-binding protein, possibly a mitochondrial chaperone with non-proteolytic function; similar to bacterial ClpX proteins

2787;Methyltransferase, catalyzes the transfer of a methyl group from S-adenosylmethionine to the GpppN terminus of capped mRNA

2788;Subunit of both RNase MRP, which cleaves pre-rRNA, and nuclear RNase P, which cleaves tRNA precursors to generate mature 5' ends; binds to the RPR1 RNA subunit in Rnase P

2789;Putative S-adenosylmethionine-dependent methyltransferase of the seven beta-strand family; green fluorescent protein (GFP)-fusion protein localizes to the cytoplasm; YBR261C is not an essential gene

2790;The authentic, non-tagged protein was localized to the mitochondria

2791;3-ketosphinganine reductase, catalyzes the second step in phytosphingosine synthesis, essential for growth in the absence of exogenous dihydrosphingosine or phytosphingosine, member of short chain dehydrogenase/reductase protein family2792;Hypothetical protein

2793;UBX (ubiquitin regulatory X) domain-containing protein that interacts with Cdc48p

2794;Third-largest subunit of DNA polymerase II (DNA polymerase epsilon), required to maintain fidelity of chromosomal replication and also for inheritance of telomeric silencing; mRNA abundance peaks at the G1/S boundary of the cell cycle

2795;RNA polymerase II-associated protein, defines a large complex that is biochemically and functionally distinct from the Srb-Mediator form of Pol II holoenzyme and is required for full expression of a subset of cell cycle-regulated genes

2796;Mitochondrial inner membrane citrate transporter, member of the mitochondrial carrier family

2797;Protein involved in vacuole inheritance; acts as a vacuole-specific receptor for myosin Myo2p

2798;AdoMet-dependent methyltransferase involved in rRNA processing and 60S ribosomal subunit maturation; methylates G2922 in the putative tRNA docking site of the large subunit rRNA and in the absence of snR52, U2921; suppressor of PAB1 mutants

2799;Protein of unknown function, redundant with Sps2p for the organization of the beta-glucan layer of the spore wall

2800;DNA polymerase IV, undergoes pair-wise interactions with Dnl4p-Liflp and Rad27p to mediate repair of DNA double-strand breaks by non-homologous end joining (NHEJ); homologous to mammalian DNA polymerase beta

2801;Putative sensor/transporter protein involved in cell wall biogenesis; contains 14-16 transmembrane segments and several putative glycosylation and phosphorylation sites; null mutation is synthetically lethal with pkc1 deletion

2802;Protein necessary for structural stability of L-A double-stranded RNA-containing particles

2803;Subunit of the RSC chromatin remodeling complex, a multisubunit complex that functions in transcriptional regulation, chromosome stability and establishing sister chromatid cohesion

2804;Hypothetical protein

2805;Plasma membrane H+-pantothenate symporter; confers sensitivity to the antifungal agent fenpropimorph

2806;Protein involved in rRNA processing; component of the exosome 3->5 exonucle ase complex with Rrp41p, Rrp42p, Rrp4p and Dis3p; required for efficient maturation of 5.85, 18S and 25S rRNA

2807;Putative ribokinase

2808;Low-affinity inorganic phosphate (Pi) transporter, involved in activation of PHO pathway; expression is independent of Pi concentration and Pho4p activity; contains 12 membrane-spanning segments

2809;Plasma membrane permease, mediates uptake of glycerophosphoinositol and glycerophosphocholine as sources of the nutrients inositol and phosphate; expression and transport rate are regulated by phosphate and inositol availability

2810;Guanine deaminase, a catabolic enzyme of the guanine salvage pathway producing xanthine and ammonia from guanine; activity is low in exponentially-growing cultures but expression is increased in post-diauxic and stationary-phase cultures

2811;The authentic, non-tagged protein was localized to the mitochondria; cell cortex protein; not required for growth on nonfermentable carbon sources; required for viability in stationary phase

2812;Hypothetical protein

2813;Hypothetical protein

2814;Ubiquitin chain assembly factor (E4) that cooperates with a ubiquitin-activating enzyme (E1), a ubiquitin-conjugating enzyme (E2), and a ubiquitin protein ligase (E3) to conjugate ubiquitin to substrates; also functions as an E3

2815;Hypothetical protein

2816;Cyclin, forms a functional kinase complex with Pho85p cyclin-dependent kinase (Cdk), expressed in late M/early G1 phase, activated by Swi5p

2817;Hypothetical protein

2818;Protein of unknown function

2819;Bifunctional enzyme containing both alcohol dehydrogenase and glutathione-dependent formaldehyde dehydrogenase activities, functions in formaldehyde detoxification and formation of long chain and complex alcohols, regulated by Hoglp-Skolp

2820;Hypothetical protein

2821;Protein of the MutS family, forms a dimer with Msh4p that facilitates crossovers between homologs during meiosis; msh5-Y823H mutation confers tolerance to DNA alkylating agents; homologs present in C. elegans and humans

2822;Hypothetical protein

2823;Cardiolipin synthase; produces cardiolipin, which is an important constituent of mitochondrial membranes; required for normal mitochondrial membrane potential and function

2824;Plasma membrane glucose receptor, highly similar to Snf3p; both Rgt2p and Snf3p serve as transmembrane glucose sensors generating an intracellular signal that induces expression of glucose transporter (HXT) genes

2825;Subunit of the nuclear pore complex (NPC), forms a subcomplex with Nup85p, Nup120p, Nup145p-C, Sec13p, and Seh1p that plays a role in nuclear mRNA export and NPC biogenesis

2826;Protein of unknown function, deletion causes hypersensitivity to the K1 killer toxin

2827;Peripherally bound inner membrane protein of the mitochondrial matrix, required for export of C-terminal tail of Cox2p through the inner membrane

2828;Component of U4/U6.U5 snRNP involved in mRNA splicing via spliceosome

2829;Essential protein associated with the U1 snRNP complex; splicing factor involved in recognition of 5' splice site

2830;Pbplp binding protein, interacts strongly with Pab1p-binding protein 1 (Pbp1p) in the yeast two-hybrid system

2831;RNA binding protein required for maturation of tRNA and snRNA precursors; acts as a molecular chaperone for RNAs transcribed by polymerase III; homologous to human La (SS-B) autoantigen

2832;Protein with similarity to Kre9p, which is involved in cell wall beta 1,6-glucan synthesis; overproduction suppresses growth defects of a kre9 null mutant

2833;Subunit of the SF3a splicing factor complex, required for spliceosome assembly 2834;Mitochondrial tRNA pseudouridine synthase involved in pseudouridylation of mitochondrial tRNA at position 32

2835;Ran GTpase binding protein; involved in nuclear protein import and RNA export, ubiquitin-mediated protein degradation during the cell cycle; shuttles between the nucleus and cytoplasm; is essential; homolog of human RanBP1 2836;Phosphoribosylanthranilate isomerase that catalyzes the third step in tryptophan biosynthesis; in 2004, the sequence of TRP1 from strain S228C was updated by changing the previously annotated internal STOP (TAA) to serine (TCA)

2837;Transcriptional regulator involved in activation of the GAL genes in response to galactose; forms a complex with Gal80p and Gal4p to relieve inhibition by Gal80p; binds galactose and ATP but does not have galactokinase activity

2838;Protein of unknown function; mutation confers radiation sensitivity

2839;T subunit of the mitochondrial glycine decarboxylase complex, required for the catabolism of glycine to 5,10-methylene-THF; expression is regulated by levels of levels of 5,10-methylene-THF in the cytoplasm

2840;Lysyl-tRNA synthetase; also identified as a negative regulator of general control of amino acid biosynthesis

2841;Hypothetical protein

2842;Constituent of 66S pre-ribosomal particles, required for large (60S) ribosomal subunit biogenesis; involved in nuclear export of pre-ribosomes; required for maintenance of dsRNA virus; homolog of human CAATT-binding protein

2843;Mitochondrial protein, member of the ATP-binding cassette (ABC) transporter family; transcriptionally activated by Yrmlp along with genes involved in multidrug resistance

2844;Vacuolar membrane protein that is a subunit of the homotypic vacuole fusion and vacuole protein sorting (HOPS) complex; essential for membrane docking and fusion at the Golgi-to-endosome and endosome-to-vacuole stages of protein transport

2845;Telomere end-binding and capping protein, plays a key role with Pol12p in linking telomerase action with completion of lagging strand synthesis, and in a regulatory step required for telomere capping

2846;Non-essential P-type ATPase that is a potential aminophospholipid translocase, localizes to the plasma membrane and late exocytic or early endocytic membranes, likely involved in protein transport; potential Cdc28p substrate

2847;Actin-related protein

2848;Hypothetical protein

2849;Hypothetical protein

2850;Ubiquitin-protein ligase, involved in the proteasome-dependent degradation of aberrant nuclear proteins; san1 mutations suppress sir4, spt16, and cdc68 mutations, suggesting a role in chromatin silencing

2851;Ethanolamine kinase, primarily responsible for phosphatidylethanolamine synthesis via the CDP-ethanolamine pathway; also exhibits choline kinase activity

2852;Nuclear protein, possibly involved in regulation of cation stress responses and/or in the establishment of bipolar budding pattern

2853;RNA-binding protein involved in the cleavage step of mRNA 3'-end formation prior to polyadenylation, and in snoRNA maturation; part of holo-CPF subcomplex APT, which associates with 3'-ends of snoRNA- and mRNA-encoding genes

2854;predicted to catalyze the final step in synthesis of Coenzyme A

2855;Hypothetical protein

2856;Sporulation-specific homolog of the yeast CDC3/10/11/12 family of bud neck microfilament genes; meiotic septin expressed at high levels during meiotic divisions and ascospore formation

2857;U1 snRNP protein involved in splicing, required for U1 snRNP biogenesis; contains multiple tetriatricopeptide repeats

2858;Cytoplasmic serine/threonine protein kinase; identified as a high-copy suppressor of the synthetic lethality of a sis2 sit4 double mutant, suggesting a role in G1/S phase progression; homolog of Sks1p

2859;Beta3 subunit of the heterotrimeric nascent polypeptide-associated complex (alpha, beta1, beta3) which binds ribosomes via its beta-subunits in close proximity to nascent polypeptides

2860;Putative basic leucine zipper (bZIP) transcription factor; overexpression increases sodium and lithium tolerance

2861 hypothetical protein

2862;Essential protein involved in assembly of cytosolic and nuclear iron-sulfur proteins

2863;Protein involved in rRNA processing; component of the exosome 3->5 exonucle ase complex

2864;Protein of unknown function, expression is regulated by phosphate levels

2865;Diacylglycerol pyrophosphate (DGPP) phosphatase, zinc-regulated vacuolar membrane-associated lipid phosphatase, dephosphorylates DGPP to phosphatidate (PA) and Pi, then PA to diacylglycerol; involved in lipid signaling and cell metabolism

2866;Hypothetical protein

2867;Putative DNA helicase

2868;Sphinganine C4-hydroxylase, catalyses the conversion of sphinganine to phytosphingosine in sphingolipid biosyntheis

2869;Gamma-glutamyl kinase, catalyzes the first step in proline biosynthesis

2870;Protein of unknown function involved in initiation of budding and cellular polarization, interacts with Cdc42p via the Cdc42/Rac-interactive binding (CRIB) domain

2871;Transcriptional repressor required for repression of middle sporulation-specific genes during mitosis; regulated by the pachytene checkpoint; a dominant mutation acts as a suppressor of silencing defects of SIR2 mutations

2872;Subunit of TFIIH and nucleotide excision repair factor 3 complexes, required for nucleotide excision repair, target for transcriptional activators

2873;Non-SMC subunit of the condensin complex (Smc2p-Smc4p-Ycs4p-Brn1p-Ycglp); required for establishment and maintenance of chromosome condensation, chromosome segregation and for chromatin binding of the condensin complex

2874;Peroxisomal membrane protein (PMP) required to recruit Pex19p chaperone to peroxisomes; plays selective, essential, direct role in PMP import as a docking factor for Pex19p

2875;UBX (ubiquitin regulatory X) domain-containing protein that interacts with Cdc48p

2876;Hypothetical protein

2877;Hypothetical protein

2878;One of six subunits of RNA polymerase III transcription initiation factor complex (TFIIIC); part of TFIIIC TauB domain that binds BoxB promoter sites of tRNA and other genes; cooperates with Tfc3p in DNA binding; human homolog is TFIIIC-110

2879;Hypothetical protein

2880;Sporulation-specific chitinase

2881;GTPase activating protein (GAP) for Rholp, involved in signaling to the actin cytoskeleton, null mutations suppress tor2 mutations and temperature sensitive mutations in actin; potential Cdc28p substrate

2882;Dimeric hypoxanthine-guanine phosphoribosyltransferase, catalyzes the formation of both inosine monophosphate and guanosine monophosphate; mutations in the human homolog HPRT1 can cause Lesch-Nyhan syndrome and Kelley-Seegmiller syndrome

2883;N-formyltyrosine oxidase, sporulation-specific microsomal enzyme required for spore wall maturation, involved in the production of a soluble LL-dityrosine-containing precursor of the spore wall, homologous to cytochrome P-450s

2884;Farnesyl cysteine-carboxyl methyltransferase, mediates the carboxyl methylation step during C-terminal CAAX motif processing of a-factor and RAS proteins in the endoplasmic reticulum, localizes to the ER membrane

2885;Protein of unknown function, localizes to the ER, contains four transmembrane domains; member of the Derlp-like family

2886;Hypothetical protein

2887;DNA polymerase eta, involved in the predominantly error-free bypass replication of DNA lesions, catalyzes the efficient and accurate synthesis of DNA opposite cyclobutane pyrimidine dimers; homolog of human POLH and bacterial DinB proteins

2888;Transmembrane protein subunit of the glycosylphosphatidylinositol transamidase complex that adds GPIs to newly synthesized proteins; human PIG-Sp homolog

2889;Palmitoyltransferase with autoacylation activity; likely functions in pathway(s) outside Ras; member of a family of putative palmitoyltransferases containing an Asp-His-His-Cys-cysteine rich (DHHC-CRD) domain

2890;Mating pheromone a-factor, made by a cells; interacts with alpha cells to induce cell cycle arrest and other responses leading to mating; biogenesis involves C-terminal modification, N-terminal proteolysis, and export; also encoded by MFA2

2891;Protein involved in negative regulation of expression of spliceosome components PRP4 and PRP3

2892;Identified by gene-trapping, microarray-based expression analysis, and genome-wide homology searching

2893;Arginine methyltransferase; ribosomal protein L12 is a substrate

2894;Subunit of the COMPASS (Set1C) complex, which methylates histone H3 on lysine 4 and is required in transcriptional silencing near telomeres; has similarity to C. elegans Dpy-3 0

2895;One of 10 subunits of the transport protein particle (TRAPP) complex of the cis-Golgi which mediates vesicle docking and fusion; involved in endoplasmic reticulum (ER) to Golgi membrane traffic

2896;Repressible alkaline phosphatase, a glycoprotein localized to the vacuole; regulated by levels of inorganic phosphate and by a system consisting of Pho4p, Pho9p, Pho80p, Pho81p and Pho85p; dephosphorylates phosphotyrosyl peptides

2897;Membrane protein involved in zinc metabolism, member of the four-protein IZH family; transcription is regulated directly by Zap1p, expression induced by zinc deficiency and fatty acids; deletion increases sensitivity to elevated zinc

2898;The authentic, non-tagged protein was localized to the mitochondria

2899;Cytoplasmic protein required for the sorting and processing of soluble vacuolar proteins, acidification of the vacuolar lumen, and assembly of the vacuolar H+-ATPase

2900;Membrane glycoprotein v-SNARE involved in retrograde transport from the Golgi to the ER; required for N- and O-glycosylation in the Golgi but not in the ER; forms a complex with the cytosolic Tip20p

2901;Protein of the mitochondrial intermembrane space, required for acetate utilization and gluconeogenesis; has orthologs in higher eukaryotes

2902;Plasma membrane arginine permease, requires phosphatidyl ethanolamine (PE) for localization, exclusively associated with lipid rafts; mutation confers canavanine resistance

2903;Protein with sequence similarity to the human MybBP1A and weak sequence similar to B-type DNA polymerases, not required for chromosomal DNA replication; required for the synthesis of rRNA

2904;Putative ATPase of the CDC48/PAS1/SEC18 (AAA) family, localized to mitochondria

2905;Transcriptional activator of amino acid biosynthetic genes in response to amino acid starvation; expression is tightly regulated at both the transcriptional and translational levels

2906;Putative mitochondrial NAD+ transporter, member of the mitochondrial carrier subfamily (see also YIA6); has putative human ortholog

2907;Alpha-1,3-mannosyltransferase, integral membrane glycoprotein of the Golgi complex, required for addition of alpha1,3-mannose linkages to N-linked and O-linked oligosaccharides, one of five S. cerevisiae proteins of the MNN1 family

2908;RNA-binding protein, activates mRNA decapping directly by binding to the mRNA substrate and enhancing the activity of the decapping proteins Dcp1p and Dcp2p

2909;Basic leucine zipper (bZIP) transcription factor of the ATF/CREB family, may regulate transcription of genes involved in utilization of non-optimal carbon sources

2910;Aspartate kinase (L-aspartate 4-P-transferase); cytoplasmic enzyme that catalyzes the first step in the common pathway for methionine and threonine biosynthesis; expression regulated by Gcn4p and the general control of amino acid synthesis

2911;Putative regulatory subunit of the protein phosphatase Glc7p, involved in glycogen metabolism; contains a conserved motif (GVNK motif) that is also found in Gac1p, Pig1p, and Pig2p

2912;Protein required for assembly of cytochrome c oxidase

2913;Mitochondrial beta-keto-acyl synthase with possible role in fatty acid synthesis; required for mitochondrial respiration

2914;Anthranilate synthase, catalyzes the initial step of tryptophan biosynthesis, forms multifunctional hetero-oligomeric anthranilate synthase:indole-3-glycerol phosphate synthase enzyme complex with Trp3p

2915;5-phospho-ribosyl-1(alpha)-pyrophosphate synthetase, involved in nucleotide, histidine, and tryptophan biosynthesis; one of a five related enzymes, which are active as heteromultimeric complexes

2916;Monopolin, kinetochore associated protein involved in chromosome attachment to meiotic spindle

2917;Hypothetical protein

2918;Protein that forms a heterodimeric histone-fold NC2 general transcription regulator complex with Ydr1p that binds to TBP and represses RNA pol II transcription during assembly of the preinitiation complex, homologous to human NC2alpha

2919;Non-essential P-type ATPase that is a potential aminophospholipid translocase, localizes to the plasma membrane and late exocytic or early endocytic membranes, likely involved in protein transport

2920;5,10-methenyltetrahydrofolate synthetase, involved in folic acid biosynthesis 2921;Protein of unknown function, nearly identical to Sno2p; expression is induced before the diauxic shift and also in the absence of thiamin

2922;Protein of unknown function, nearly identical to Sno2p; expression is induced before the diauxic shift and also in the absence of thiamin

2923;Putative protein of unknown function; YFL042C is not an essential gene 2924;Glucose-repressible protein kinase involved in signal transduction during cell proliferation in response to nutrients, specifically the establishment of stationary phase; originally identified as a regulator of IME2

2925;F-box protein required for G1/S and G2/M transition, associates with Skp1p and Cdc53p to form a complex, SCFCdc4, which acts as ubiquitin-protein ligase directing ubiquitination of the phosphorylated CDK inhibitor Sic1p

2926;Vacuolar membrane protein involved in vacuolar polyphosphate accumulation; functions as a regulator of vacuolar H+-ATPase activity and vacuolar transporter chaperones; involved in protein localization and non-autophagic vacuolar fusion

2927;Protein involved in meiotic recombination, required for normal levels of crossing over, colocalizes with Zip2p to discrete foci on meiotic chromosomes, has homology to bacterial MutS protein

2928;Nuclear protein involved in asymmetric localization of ASH1 mRNA; binds double-stranded RNA in vitro; constituent of 66S pre-ribosomal particles

2929;Conserved zinc-finger domain protein involved in pre-mRNA splicing, required for assembly of U4 snRNA into the U4/U6 particle

2930;Ubiquitin-specific protease situated in the base subcomplex of the 26S proteasome, releases free ubiquitin from branched polyubiquitin chains; deletion causes hypersensitivity to cycloheximide and other toxic compounds

2931;Calmodulin-dependent protein kinase; may play a role in stress response, many CA++/calmodulan dependent phosphorylation substrates demonstrated in vitro, amino acid sequence similar to Cmk2p and mammalian Cam Kinase II

2932;Hypothetical protein

2933;Putative protein of unknown function; YFR039C is not an essential gene

2934;Cytosolic protein required for sporulation

2935;Mannosyltransferase involved in adding the 4th and 5th mannose residues of O-linked glycans

2936;Transcription factor of the Zn[II]2Cys6 family involved in sterol uptake; involved in induction of hypoxic gene expression

2937;Oxidoreductase, catalyzes NADPH-dependent reduction of the bicyclic diketone bicyclo[2.2.2]octane-2,6-dione (BCO2,6D) to the chiral ketoalcohol (1R,4S,6S)-6-hydroxybicyclo[2.2.2]octane-2-one (BCO2one6ol)

2938;Hypothetical protein

2939;Pho85p cyclin; recruits, activates, and targets Pho85p cyclin-dependent protein kinase to its substrate

2940;Protein required for ubiquinone (coenzyme Q) biosynthesis and for respiratory growth; exhibits genetic interaction with COQ9, suggesting a common function; similar to prokaryotic proteins involved in early steps of ubiquinone biosynthesis

2941;Protein involved in the initiation of DNA replication, required for proper assembly of replication proteins at the origins of replication; interacts with Cdc45p

2942;Constituent of 66S pre-ribosomal particles, involved in 60S ribosomal subunit biogenesis

2943;The authentic, non-tagged protein was localized to the mitochondria

2944;Hypothetical protein

2945;Essential membrane protein localized at the nuclear envelope and spindle pole body (SPB), required for insertion of the newly duplicated SPB into the nuclear envelope; potentially phosphorylated by Cdc28p

2946;Probable 73 kDa subunit of SAGA histone acetyltransferase complex

2947;Mannosyltransferase of the cis-Golgi apparatus, initiates the polymannose outer chain elongation of N-linked oligosaccharides of glycoproteins

2948;Identified by expression profiling and mass spectrometry

2949;Putative cysteine synthase; green fluorescent protein (GFP)-fusion protein localizes to the mitochondrion

2950;Potential regulatory effector of CDC4 function, suppresses a temperature-sensitive allele of CDC4, tripartite protein structure in which a charged region separates two uncharged domains, not essential for mitosis or meiosis

2951;One of 15 subunits of the 'Remodel the Structure of Chromatin' (RSC) complex; required for expression of mid-late sporulation-specific genes; contains two essential bromodomains, a bromo-adjacent homology (BAH) domain, and an AT hook

2952;Protein possibly involved in a post-Golgi secretory pathway; required for the transport of nitrogen-regulated amino acid permease Gaplp from the Golgi to the cell surface

2953;Splicing factor, component of the U4/U6-U5 snRNP complex

2954;Mitochondrial membrane transporter that mediates uptake of the essential cofactor thiamine pyrophosphate (ThPP) into mitochondria; expression appears to be regulated by carbon source; member of the mitochondrial carrier family

2955;Separase with cysteine protease activity (related to caspases) that promotes sister chromatid separation by mediating dissociation of the cohesin Scclp from chromatin; inhibited by Pds1p

2956;Essential DNA-binding protein specific to single-stranded yeast telomeric DNA repeats, required for telomere length regulation and telomere position effect2957;Essential protein that interacts with histones and is involved in nucleosome disassembly and reassembly during transcription elongation

2958;Hypothetical protein

2959;Ammonium permease; belongs to a ubiquitous family of cytoplasmic membrane proteins that transport only ammonium (NH4+); expression is under the nitrogen catabolite repression regulation

2960;Hypothetical protein

2961;Cytoplasmic GTP binding protein, negative regulator of the Ran/Tc4 GTPase cycle downstream of Gtr1p; homolog of human RagC and RagD proteins; component of the EGO complex, which is involved in the regulation of microautophagy

2962;Putative glycosidase of the cell wall, may have a role in cell wall architecture

2963;ATP-binding protein of unknown function; crystal structure resembles that of E.coli pantothenate kinase and other small kinases

2964;Protein involved in 7-aminocholesterol resistance; has seven potential membrane-spanning regions

2965;Protein that functions in a complex with Kel1p to negatively regulate mitotic exit, interacts with Temlp and Ltelp; localizes to regions of polarized growth; potential Cdc28p substrate

2966;Beta subunit of succinyl-CoA ligase, which is a mitochondrial enzyme of the TCA cycle that catalyzes the nucleotide-dependent conversion of succinyl-CoA to succinate

2967;Putative protein of unknown function; deletion mutant has defects in pre-rRNA processing; green fluorescent protein (GFP)-fusion protein localizes to both the nucleus and the nucleolus; YGR251W is an essential gene

2968;Protein of unknown function;predicted to contain a single transmembrane domain; localized to both the mitochondrion and the plasma membrane

2969;Putative RNA helicase related to Ski2p, involved in translation inhibition of non-poly(A) mRNAs; required for repressing propagation of dsRNA viruses

2970;Cytosolic ribosome-associated chaperone, contains a DnaJ domain; together with Ssz1p, acts as a chaperone for nascent polypeptide chains

2971;Meiosis-specific protein that initiates meiotic recombination by catalyzing the formation of double-strand breaks in DNA via a transesterification reaction; required for homologous chromosome pairing and synaptonemal complex formation

2972;Putative protein of unknown function; green fluorescent protein (GFP)-fusion protein localizes to mitochondria

2973;Protein of unknown function; shares weak similarity to E. coli GTP-binding protein gtp1

2974;Mitochondrial ribosomal protein of the small subunit

2975;Member of an oxysterol-binding protein family with seven members in S. cerevisiae; family members have overlapping, redundant functions in sterol metabolism and collectively perform a function essential for viability

2976;Argininosuccinate lyase, catalyzes the final step in the arginine biosynthesis pathway

2977;Subunit c" of the vacuolar ATPase, which functions in acidification of the vacuole; one of three proteolipid subunits of the V0 domain

2978;Essential protein required for the accumulation of box C/D snoRNA

2979;2-deoxyglucose-6-phosphate phosphatase, similar to Dog2p, member of a family of low molecular weight phosphatases; confers 2-deoxyglucose resistance when overexpressed, in vivo substrate has not yet been identified

2980;Metallothionein, binds copper and mediates resistance to high concentrations of copper and cadmium; locus is variably amplified in different strains, with two copies, CUP1-1 and CUP1-2, in the genomic sequence reference strain S288C

2981;Protein of unknown function involved in initiation of budding and cellular polarization, interacts with Cdc42p via the Cdc42/Rac-interactive binding (CRIB) domain

2982;Cyclin, interacts with Pho85p cyclin-dependent kinase (Cdk), induced by Gcn4p at level of transcription, specifically required for Gcn4p degradation, may be sensor of cellular protein biosynthetic capacity

2983;Protein with carboxyl methyl esterase activity that may have a role in demethylation of the phosphoprotein phosphatase catalytic subunit; also identified as a small subunit mitochondrial ribosomal protein

2984;Cytochrome c lysine methyltransferase, trimethylates residue 72 of apo-cytochrome c (Cyc1p) in the cytosol; not required for normal respiratory growth

2985;Protein that binds to cruciform DNA structures

2986;Protein that interacts with Mms22p and is implicated in Mms22-dependent DNA repair during S phase, damage induces phosphorylation by Meclp at one or more SQ/TQ motifs; has four BRCT domains; has a role in regulation of Ty1 transposition

2987;Mitochondrial protein with a potential role in promoting mitochondrial fragmentation during programmed cell death in response to high levels of alpha-factor mating pheromone or the drug amiodarone

2988;Subunit of the THO complex, which functions to connect transcription elongation with mitotic recombination, and of the TREX complex, which is recruited to activated genes and couples transcription to mRNA export

2989;Autophagy-related protein and dual specificity member of the E1 family of ubiquitin-activating enzymes; mediates the conjugation of Atg12p with Atg5p and Atg8p with phosphatidylethanolamine, required steps in autophagosome formation.

2990;Widely conserved NADPH oxidoreductase containing flavin mononucleotide (FMN), homologous to Oye3p with slight differences in ligand binding and catalytic properties; may be involved in sterol metabolismj 2991;Sporulation protein required for prospore membrane formation at selected spindle poles, ensures functionality of all four spindle pole bodies of a cell during meiosis II; not required for meiotic recombination or meiotic chromosome segregation

2992;Essential protein involved in the processing of the ITS2 region of the rRNA locus; required for the maturation and nuclear export of the 60S ribosomal subunit

2993;Exopolyphosphatase, hydrolyzes inorganic polyphosphate (poly P) into Pi residues; located in the cytosol, plasma membrane, and mitochondrial matrix

2994;Invertase, sucrose hydrolyzing enzyme; a secreted, glycosylated form is regulated by glucose repression, and an intracellular, nonglycosylated enzyme is produced constitutively

2995;The authentic, non-tagged protein was localized to the mitochondria

2996;Histidine kinase osmosensor that regulates a MAP kinase cascade; transmembrane protein with an intracellular kinase domain that signals to Ypd1p and Ssk1p, thereby forming a phosphorelay system similar to bacterial two-component regulators

2997;Subunit of DNA polymerase zeta, which is involved in DNA repair; required for mutagenesis induced by DNA damage

2998;Protein required for accurate chromosome segregation during meiosis

2999;Transcription factor of the forkhead family that regulates the cell cycle and pseudohyphal growth; also involved in chromatin silencing at HML and HMR

3000;ATPase component of the ATP-dependent RSC chromatin remodeling complex required for kinetochore function in chromosome segregation; required for expression of early meiotic genes; essential helicase-related protein homologous to Snf2p

3001;Pheromone-regulated protein, predicted to have 1 transmembrane segment; induced during cell integrity signaling

3002;Histidinol-phosphate aminotransferase, catalyzes the seventh step in histidine biosynthesis; responsive to general control of amino acid biosynthesis; mutations cause histidine auxotrophy and sensitivity to Cu, Co, and Ni salts

3003;Subunit of the nuclear pore complex that is found exclusively on the cytoplasmic side, forms a subcomplex with Nup82p and Nsp1p, required for mRNA export

3004;Putative S-adenosylmethionine-dependent methyltransferase of the seven beta-strand family; deletion mutant exhibits a weak vacuolar protein sorting defect and enhanced resistance to the glucan synthase inhibitor caspofungin

3005;Putative metalloprotease

3006;Protein required, along with Dph2p, Kti11p, Jjj3p, and Dph5p, for synthesis of diphthamide, which is a modified histidine residue of translation elongation factor 2 (Eft1p or Eft2p); may act in a complex with Dph2p and Kti11p

3007;Mitochondrial matrix protein, required for assembly or stability at high temperature of the F1 sector of mitochondrial F1F0 ATP synthase

3008;Possible shared subunit of Cop9 signalosome (CSN) and eIF3, binds eIF3b subunit Prt1p, has possible dual functions in transcriptional and translational control, contains a PCI (Proteasome-COP9 signalosome (CSN)-eIF3) domain

3009;Essential 88kDa subunit of the exocyst complex (Sec3p, Sec5p, Sec6p, Sec8p, Sec10p, Sec15p, Exo70p, and Exo84p), which has the essential function of mediating polarized targeting of secretory vesicles to active sites of exocytosis

3010;hypothetical protein

3011;Peptidase that deconjugates Smt3/SUMO-1 peptides from proteins, plays a role in chromosome cohesion at centromeric regions and recovery from checkpoint arrest induced by DNA damage or DNA replication defects; potential Cdc28p substrate

3012;Phosphoribosyl-5-amino-1-phosphoribosyl-4-imidazolecarboxiamide isomerase, catalyzes the fourth step in histidine biosynthesis; mutations cause histidine auxotrophy and sensitivity to Cu, Co, and Ni salts

3013;Cytoplasmic RNA-binding protein, contains an RNA recognition motif (RRM); may have a role in mRNA translation, as suggested by genetic interactions with genes encoding proteins involved in translational initiation

3014;Essential protein involved in a late step of 60S ribosomal subunit assembly or modification; contains multiple WD repeats; interacts with Qsr1p in a two-hybrid assay

3015;Subunit of nuclear RNase P, which cleaves tRNA precursors to generate mature 5' ends; not shared between Rnase MRP and Rnase P, in contrast to all other Rnase P protein subunits

3016;Protein phosphatase involved in vegetative growth at low temperatures, sporulation, and glycogen accumulation; transcription induced by low temperature and nitrogen starvation; member of the dual-specificity family of protein phosphatases

3017;Protein of unknown function, expression is sensitive to nitrogen catabolite repression and regulated by Dal80p; contains transmembrane domain

3018;Putative protein of unknown function; sequence similarity with short-chain dehydrogenase/reductase family members; green fluorescent protein (GFP)-fusion protein localizes to the cytoplasm; YIR036C is a non-essential gene

3019;ER associated glutathione S-transferase capable of homodimerization; expression induced during the diauxic shift and throughout stationary phase; functional overlap with Gtt2p, Grx1p, and Grx2p

3020;Major mitochondrial nuclease, has RNAse and DNA endo- and exonucleolytic activities; has a role in mitochondrial recombination

3021;Low-affinity phosphate transporter; deletion of pho84, pho87, pho89, pho90, and pho91 causes synthetic lethality; transcription independent of Pi and Pho4p activity; overexpression results in vigorous growth

3022;Essential ATP-binding protein required for DNA replication, component of the pre-replicative complex (pre-RC) which requires ORC to associate with chromatin and is in turn required for Mcm2-7p DNA association; homologous to S. pombe Cdc18p

3023;ER-membrane protein; suppressor of pma1-7, deletion of SOP4 slows down the export of wild-type Pmalp and Pma1-7 from the ER

3024;Protein of unknown function, proposed to be involved in the transfer of mannosylphosphate groups onto N-linked oligosaccharides; also proposed to be involved in responding to osmotic stress

3025;Histone methyltransferase with a role in transcriptional elongation, methylates a lysine residue of histone H3; associates with the C-terminal domain of Rpo21p; histone methylation activity is regulated by phosphorylation status of Rpo21p

3026;Integral membrane protein localized to vacuolar intralumenal vesicles, computational analysis of large-scale protein-protein interaction data suggests a possible role in either cell wall synthesis or protein-vacuolar targeting

3027;Putative phosphatidylinositol transfer protein (PITP), exhibits phosphatidylinositol-but not phosphatidylcholine-transfer activity, mainly localized to cytosol and microsomes, similar to Sec14p; may be PITP regulator rather than actual PITP

3028;Self-glucosylating initiator of glycogen synthesis, also glucosylates n-dodecyl-beta-D-maltoside; similar to mammalian glycogenin

3029;Hypothetical protein

3030;Hypothetical protein

3031;Component of the Trk1p-Trk2p potassium transport system; 180 kDa high affinity potassium transporter

3032;Subunit of the cytosolic chaperonin Cct ring complex, related to Tcplp, required for the assembly of actin and tubulins in vivo

3033;Gamma glutamylcysteine synthetase, catalyzes the first step in the gamma-glutamyl cycle for glutathione (GSH) biosynthesis; expression induced by oxidants, cadmium, and mercury

3034;Protein of unknown function; homolog of mammalian PTPLA; involved in sphingolipid biosynthesis; required for cell viability

3035;Cytoplasmic protein of unknown function that interacts with Pcl7p, phosphorylated in vitro; potential Cdc28p substrate

3036;DnaJ-like chaperone required for nuclear membrane fusion during mating, localizes to the ER membrane; exhibits genetic interactions with KAR2

3037;Mitochondrial membrane protein of unknown function, contains no hydrophobic stretches

3038;Hypothetical protein identified by homology. See FEBS Letters [2000] 487:31-36.

3039;Hypothetical protein

3040;Component of the mitochondrial Tim54p-Tim22p complex involved in insertion of polytopic proteins into the inner membrane

3041;Putative protein of unknown function; YJL049W is a non-essential gene

3042;UBX (ubiquitin regulatory X) domain-containing protein that interacts with Cdc48p, transcription is repressed when cells are grown in media containing inositol and choline

3043;GTPase-activating protein (GAP) for the yeast Rab family member, Ypt6p; involved in vesicle mediated protein transport

3044;Protein required for respiratory growth

3045;Subunit of the cytosolic chaperonin Cct ring complex, related to Tcplp, required for the assembly of actin and tubulins in vivo

3046;Beta subunit of C-terminal domain kinase I (CTDK-1), which phosphorylates the C-terminal repeated domain of the RNA polymerase II large subunit (Rpo21p) to affect both transcription and pre-mRNA 3' end processing; has similarity to cyclins

3047;Adenylate cyclase, required for cAMP production and cAMP-dependent protein kinase signaling; involved in cell cycle control and glucose and nitrogen repression of sporulation

3048;Alpha-agglutinin of alpha-cells, binds to Aga1p during agglutination, N-terminal half is homologous to the immunoglobulin superfamily and contains binding site for a-agglutinin, C-terminal half is highly glycosylated and contains GPI anchor

3049;Hypothetical protein

3050;Protein involved in transcription-coupled repair nucleotide excision repair of UV-induced DNA lesions; homolog of human CSB protein

3051;Hypothetical proteinj 3052;Thymidylate and uridylate kinase, functions in de novo biosynthesis of pyrimidine deoxyribonucleotides; converts dTMP to dTDP and dUMP to dUTP; essential for mitotic and meiotic DNA replication; homologous to S. pombe Tmp1p

3053;Hypothetical protein

3054;Essential protein of unknown function

3055;Deoxyhypusine hydroxylase, a HEAT-repeat containing metalloenzyme that catalyses hypusine formation; binds to and is required for the modification of Hyp2p (eIF5A); complements S. pombe mmd1 mutants defective in mitochondrial positioning

3056;Alpha-1,6-mannosyltransferase involved in cell wall mannan biosynthesis; subunit of a Golgi-localized complex that also contains Anplp, Mnn9p, Mnn11p, and Mnn10p; identified as a suppressor of a cell lysis sensitive pkc1-371 allele

3057;Subunit of the Cop9 signalosome, which is required for deneddylation, or removal of the ubiquitin-like protein Rublp from Cdc53p (cullin); involved in adaptation to pheromone signaling

3058;F-box protein component of the SCF ubiquitin-ligase complex, required for Cln1p and Cln2p degradation; involved in carbon catabolite repression, glucose-dependent divalent cation transport, high-affinity glucose transport, and morphogenesis

3059;Component of the ESCRT-II complex, which is involved in ubiquitin-dependent sorting of proteins into the endosome

3060;Non-essential protein of unknown function

3061;Large subunit of carbamoyl phosphate synthetase, which catalyzes a step in the synthesis of citrulline, an arginine precursor

3062;Mitochondrial protein that interacts with Ccr4p in the two-hybrid system; 3'-untranslated region contains a putative mRNA localization element common to genes encoding mitochondrial proteins

3063;Hypothetical protein

3064;Protein involved in mitochondrial genome maintenance; component of the mitochondrial nucleoid, required for the repair of oxidative mtDNA damage

3065;Cell wall mannoprotein with similarity to Tir1p, Tir2p, Tir3p, and Tir4p; expressed under anaerobic conditions, completely repressed during aerobic growth

3066;Lactate transporter, required for uptake of lactate and pyruvate; expression is derepressed by transcriptional activator Cat8p under nonfermentative growth conditions, and repressed in the presence of glucose, fructose, and mannose

3067;ATP-binding cassette (ABC) transporter required for the export of a-factor, catalyzes ATP hydrolysis coupled to a-factor transport, contains 12 transmembrane domains and two ATP binding domains, expressed only in MATa cells

3068;Protein involved in 5' end processing of mitochondrial COB, 15S_rRNA, and RPM1 transcripts; may also have a role in 3' end processing of the COB pre-mRNA; displays genetic interaction with cell cycle-regulated kinase Dbf2p

3069;mRNA transport regulator, essential nuclear protein; Mex67p and Mtr2p form a mRNA export complex which binds to RNA

3070;Protein of unknown function; gene expression increases in cultures shifted to a lower temperature

3071;Receptor for a factor receptor, transcribed in alpha cells and required for mating by alpha cells, couples to MAP kinase cascade to mediate pheromone response; ligand bound receptors are endocytosed and recycled to the plasma membrane; GPCR

3072;Putative protein of unknown function; green fluorescent protein (GFP)-fusion protein localizes to the cytoplasm

3073;Nimlp-related protein kinase that regulates the morphogenesis and septin checkpoints; associates with the assembled septin filament; required along with Hsl7p for bud neck recruitment, phosphorylation, and degradation of Swe1p

3074;GTPase activating factor for Rsr1p/Bud1p required for both axial and bipolar budding patterns; mutants exhibit random budding in all cell types

3075;Putative homolog of Sec14p, which is a phosphatidylinositol/phosphatidylcholine transfer protein involved in lipid metabolism; localizes to the nucleus

3076;Subunit of the nuclear pore complex (NPC) that is localized to both sides of the pore; contains a repetitive GLFG motif that interacts with mRNA export factor Mex67p and with karyopherin Kap95p; homologous to Nup116p

3077;Putative magnesium transporter; has similarity to Alr1p and Alr2p, which mediate influx of Mg2+ and other divalent cations

3078;Mitochondrial 3-oxoacyl-[acyl-carrier-protein] reductase, may comprise a type II mitochondrial fatty acid synthase along with Mct1p

3079;Serine/threonine protein kinase that regulates cellular morphogenesis, septin behavior, and cytokinesis; required for the regulation of other kinases; forms part of the bud neck ring

3080;Putative protein of unknown function; detectable in highly purified mitochondria

3081;Merged open reading frame, does not encode a discreet protein; YKR004C-A was originally annotated as an independent ORF, but was later demonstrated to be an exon of an adjacent ORF, YKR004C

3082;Nonessential mitochondrial protein of unknown function with sequence similarity to Netlp; identified as a topoisomerase I (Toplp) binding protein; displays synthetic genetic interactions with TOP 1 and HPR1

3083;The authentic, non-tagged protein was localized to the mitochondria

3084;Putative protein of unknown function; green fluorescent protein (GFP)-fusion protein localizes to the cytoplasm

3085;Essential protein that forms a dimer with Ntr1p; also forms a trimer, with Ntr2p and the DExD/H-box RNA helicase Prp43p, that is involved in spliceosome disassembly

3086;Dubious open reading frame unlikely to encode a protein, based on available experimental and comparative sequence data; partially overlaps the verified gene DAL80

3087;5-adenosyl-L-methionine uroporphyrinogen III transmethylase, involved in sulfate assimilation, methionine metabolism, and siroheme biosynthesis

3088;LIM domain-containing protein that localizes to sites of polarized growth, required for selection and/or maintenance of polarized growth sites, may modulate signaling by the GTPases Cdc42p and Rholp; has similarity to metazoan paxillin3089;Phosphoenolpyruvate carboxykinase, key enzyme in gluconeogenesis, catalyzes early reaction in carbohydrate biosynthesis, glucose represses transcription and accelerates mRNA degradation, regulated by Mcm1p and Cat8p, located in the cytosol

3090;Protein of unknown function, transcription is activated by paralogous transcription factors Yrmlp and Yrr1p along with genes involved in multidrug resistance

3091;Protein of unknown function, transcription is activated by paralogous transcription factors Yrmlp and Yrr1p along with genes involved in multidrug resistance

3092;Ribonucleoprotein that contains two RNA recognition motifs (RRM)

3093;Splicing factor associated with the spliceosome; contains a U-box, a motif found in a class of ubiquitin ligases

3094;Putative ATPase of the AAA family, required for export of pre-ribosomal large subunits from the nucleus; distributed between the nucleolus, nucleoplasm, and nuclear periphery depending on growth conditions

3095;Identified by gene-trapping, microarray-based expression analysis, and genome-wide homology searching

3096;ABC type transmembrane transporter of MRP/CFTR family, found in vacuolar membrane, involved in the transport of unconjugated bilirubin and in heavy metal detoxification via glutathione conjugates, along with Ycflp

3097;Protein that regulates degradation of specific mRNAs such as COX17, binds almost exclusively to cytoplasmic mRNAs encoding mitochondrial proteins, member of the PUF protein family that contains Pumilio homology domains

3098;Essential subunit of the U3 (box C+D) snRNP complex required for 2' O-methylation of pre-RNA; has similarity to the beta subunit of trimeric G-proteins and the splicing factor Prp4p

3099;Mitochondrial outer membrane protein required for normal mitochondrial morphology and mtDNA stability; involved in tethering mitochondria to the actin cytoskeleton and in anchoring mtDNA nucleoids

3100;Meiosis-specific protein of unknown function, required for spore wall formation during sporulation; dispensable for both nuclear divisions during meiosis

3101;Centrin (Cdc31p)-binding protein required for spindle pole body (SPB) duplication, localizes to the half-bridge of the SPB, required for progression through G(2)-M transition, has similarity to Xenopus laevis XCAP-C

3102;Protein with a role in regulation of Ty1 transposition

3103;Hypothetical protein

3104;Cytoplasmic response regulator, part of a two-component signal transducer that mediates osmosensing via a phosphorelay mechanism; dephosphorylated form is degraded by the ubiquitin-proteasome system; potential Cdc28p substrate

3105;Constituent of the mitochondrial import motor associated with the presequence translocase, along with Ssc1p, Tim44p, Mge1p, and Pam16p; stimulates the ATPase activity of Ssclp to drive mitochondrial import; contains a J domain

3106;Putative NADPH-dependent FMN reductase with strong ferricyanide reductase activity in vitro; gene expression increases in cultures shifted to a lower temperature

3107;Protein containing GATA family zinc finger motifs

3108;Steryl ester hydrolase, catalyzes steryl ester hydrolysis at the plasma membrane; involved in sterol metabolism

3109;Membrane protein involved in zinc metabolism, member of the four-protein IZH family, expression induced by zinc deficiency; deletion reduces sensitivity to elevated zinc and shortens lag phase, overexpression reduces Zap1p activity

3110;Cell wall mannoprotein with similarity to Tir1p, Tir2p, Tir3p, and Tir4p; expressed under anaerobic conditions, completely repressed during aerobic growth

3111;Subunit of the condensin complex, which reorganizes chromosomes during cell division, forms a stable complex with Smc2p that has ATP-hydrolyzing and DNA-binding activity and promotes knotting of circular DNA; potential Cdc28p substrate3112;Putative alanine transaminase (glutamic pyruvic transaminase)

3113;Hypothetical protein

3114;Member of a complex (Iswlb) with Isw1p and Ioc4p that exhibits nucleosome-stimulated ATPase activity and acts within coding regions to coordinate transcription elongation with termination and processing, contains a PHD finger motif

3115;Hypothetical protein

3116;RNA exonuclease; required for maturation of the RNA component of RNase MRP; functions redundantly with Rnh70p and Rex2p in processing of U5 snRNA and RNase P RNA; member of RNase D family of exonucleases

3117;ORF, Uncharacterized

3118;Protein required for cell viability

3119;Subunit of a complex, with Slx1p, that hydrolyzes 5' branches from duplex DNA in response to stalled or converging replication forks; function overlaps with that of Sgslp-Top3p

3120;Proline oxidase, nuclear-encoded mitochondrial protein involved in utilization of proline as sole nitrogen source; PUT1 transcription is induced by Put3p in the presence of proline and the absence of a preferred nitrogen source

3121;Intracellular beta-1,3-endoglucanase, expression is induced during sporulation; may have a role in cortical actin cytoskeleton assembly

3122;Core Sm protein Sm D3; part of heteroheptameric complex (with Smb1p, Smd1p, Smd2p, Sme1p, Smx3p, and Smx2p) that is part of the spliceosomal U1, U2, U4, and U5 snRNPs; homolog of human Sm D3

3123;Pseudouridine synthase, catalyzes only the formation of pseudouridine (Psi)-2819 in mitochondrial 21S rRNA; not essential for viability

3124;Integral peroxisomal membrane receptor for the PTS1 peroxisomal matrix protein signal recognition factor Pex5p, required for the translocation of peroxisomal matrix proteins, also interacts with Pex7p and Pex14p, contains a C-terminal SH3 domain

3125;Protein with a likely role in ribosomal maturation, required for accumulation of wild-type levels of large (60S) ribosomal subunits; binds to the helicase Dbp6p in pre-60S ribosomal particles in the nucleolus

3126;Sterol regulatory element binding protein, regulates transcription of the sterol biosynthetic genes ERG2 and ERG3; member of the fungus-specific Zn[2]-Cys[6] binuclear cluster family of transcription factors; homologous to Upc2p

3127;Lipoyl ligase, involved in the modification of mitochondrial enzymes by the attachment of lipoic acid groups

3128;Cytidine deaminase; catalyzes the modification of cytidine to uridine in vitro but native RNA substrates have not been identified, localizes to both the nucleus and cytoplasm

3129;Subunit of a palmitoyltransferase, composed of Erf2p and Shr5p, that adds a palmitoyl lipid moiety to Ras2p through a thioester linkage; mutants partially mislocalize Ras2p to the vacuole

3130;Hypothetical protein

3131 hypothetical protein

3132;Protein component of the axial elements of the synaptonemal complex, involved in chromosome segregation during the first meiotic division; interacts with Hop1p; required for wild-type levels of Mek1p kinase activity

3133;Protein involved in regulation of nonhomologous end joining; repressed by MAT heterozygosity; associates with Liflp and regulates its cellular distribution3134;Hypothetical protein

3135 ;Hypothetical protein

3136;Beta subunit of the translation initiation factor eIF2B, the guanine-nucleotide exchange factor for eIF2; activity subsequently regulated by phosphorylated eIF2; first identified as a negative regulator of GCN4 expression

3137;Hypothetical protein

3138;Aconitase, required for the tricarboxylic acid (TCA) cycle and also independently required for mitochondrial genome maintenance; component of the mitochondrial nucleoid; mutation leads to glutamate auxotrophy

3139;Chitin deacetylase, together with Cda1p involved in the biosynthesis ascospore wall component, chitosan; required for proper rigidity of the ascospore wa113140;Non-essential kinetochore protein, subunit of the Ctf19 central kinetochore complex (Ctf19p-Mcm21p-Okp1p-Mcm22p-Mcm16p-Ctf3p-Ch14p-Mcm19p-Nkp1p-Nkp2p-Ame1p-Mtw1p)

3141;Actin- and formin-interacting protein, involved in actin cable nucleation and polarized cell growth; isolated as bipolar budding mutant; potential Cdc28p substrate

3142;Protein involved in nucleocytoplasmic transport, binds to either the nucleoplasmic or cytoplasmic faces of the nuclear pore complex depending on Ran-GTP levels; also has a role in chromatin organization

3143;Essential nuclear protein with a possible role in the osmoregulatory glycerol response; interacts with phospholipase C (Plc1p); putative homolog of human NOM1 which is implicated in acute myeloid leukemia

3144;Putative 1,3-beta-glucanosyltransferase, has similarity to Gas1p

3145;Evolutionarily conserved protein with similarity to Orm1p, required for resistance to agents that induce the unfolded protein response; human ortholog is located in the endoplasmic reticulum

3146;Mitochondrial hsp70-type molecular chaperone, required for assembly of iron/sulfur clusters into proteins at a step after cluster synthesis, and for maturation of Yfh1p, which is a homolog of human frataxin implicated in Friedreich's ataxia

3147;Protein of unknown function; deletion results in a mutator phenotype suggesting a role for this protein as a mutational suppressor; deletion increases sensitivity to anticancer drugs oxaliplatin and cisplatin but not mitomycin C

3148:Fructose-1,6-bisphosphatase, key regulatory enzyme in the gluconeogenesis pathway, required for glucose metabolism

3149;Outer kinetochore protein that forms a complex with Mcm16p and Mcm22p; may bind the kinetochore to spindle microtubules

3150;Protein of unknown function, component of the Swr1p complex that incorporates Htz1p into chromatin

3151;Possible U3 snoRNP protein involved in maturation of pre-18S rRNA, based on computational analysis of large-scale protein-protein interaction data

3152;Substituent of the Pafl complex together with RNA polymerase II, Paflp, Hpr1p, Ctr9, Leo1, Rtf1 and Ccr4p, distinct from Srb-containing Pol II complexes; required for the expression of certain genes and modification of some histones

3153;Dihydroorotase, catalyzes the third enzymatic step in the de novo biosynthesis of pyrimidines, converting carbamoyl-L-aspartate into dihydroorotate

3154;Subunit of the 19S regulatory particle of the 26S proteasome lid

3155;Calcineurin A; one isoform (the other is CMP2) of the catalytic subunit of calcineurin, a Ca++/calmodulin-regulated protein phosphatase which regulates Crzlp (a stress-response transcription factor), the other calcineurin subunit is CNB1

3156;Silencing protein that interacts with Sir2p and Sir4p, and histone H3 and H4 tails, to establish a transcriptionally silent chromatin state; required for spreading of silenced chromatin; recruited to chromatin through interaction with Rap1p

3157;Non-essential protein of unknown function

3158;Hypothetical protein

3159;Protein that binds to the Rap1p C-terminus and acts synergistically with Rif1p to help control telomere length and establish telomeric silencing; deletion results in telomere elongation

3160;The authentic, non-tagged protein was localized to the mitochondria

3161;Part of 23-member seripauperin multigene family encoded mainly in subtelomeric regions, active during alcoholic fermentation, regulated by anaerobiosis, negatively regulated by oxygen, repressed by heme

3162;High-affinity inorganic phosphate (Pi) transporter and low-affinity manganese transporter; regulated by Pho4p and Spt7p; mutation confers resistance to arsenate; exit from the ER during maturation requires Pho86p

3163;Putative endonuclease, has a domain similar to a magnesium-dependent endonuclease motif in mRNA deadenylase Ccr4p; similar to Ngl1p and Ngl2p

3164;Component of the mannan polymerase I; forms a complex with Mnn9p, which is involved in in mannan synthesis; mutants are vanadate-resistant

3165;TFIID subunit (65 kDa), involved in RNA polymerase II transcription initiation

3166;defines a new subfamily of the split beta-alpha-beta sandwiches.

3167;Component of the chromatin assembly complex (with Rlf2p and Msilp) that assembles newly synthesized histones onto recently replicated DNA, required for building functional kinetochores, conserved from yeast to humans

3168;TFIID subunit (19 kDa), involved in RNA polymerase II transcription initiation, similar to histone H4 with atypical histone fold motif of Spt3-like transcription factors

3169;Hypothetical protein

3170;Uncharacterized protein with structural resemblance to plant storage and ligand binding proteins (canavalin, glycinin, auxin binding protein), and also to some plant and bacterial enzymes (epimerase, germin)

3171;Homodimeric Zn2Cys6 zinc finger transcription factor; binds to a weak acid response element to induce transcription of PDR12 and FUN34, encoding an acid transporter and a putative ammonia transporter, respectively

3172;Serine esterase that deacylates exogenous lysophospholipids, homolog of human neuropathy target esterase (NTE); mammalian NTE1 deacylates phosphatidylcholine to glycerophosphocholine

3173;Protein involved in the Mec1p-mediated checkpoint pathway that responds to DNA damage or replication arrest, transcription is induced by DNA damage

3174;Subunit of signal peptidase complex (Spclp, Spc2p, Spc3p, Secllp), which catalyzes cleavage of N-terminal signal sequences of proteins targeted to the secretory pathway; homologous to mammalian SPC25

3175,Pheromone-regulated protein, predicted to have 2 transmembrane segments; regulated by Ste12p during mating

3176;Hypothetical protein

3177;AMP deaminase, tetrameric enzyme that catalyzes the deamination of AMP to form IMP and ammonia; may be involved in regulation of intracellular adenine nucleotide pools

3178;Nuclear envelope protein with multiple putative transmembrane domains, required for nuclear pore complex assembly and spindle pole body duplication; required for meiosis II

3179;Protein that interacts in the two-hybrid system with the U1 snRNP-specific protein, Snp1p; may have a role in pre-mRNA splicing

3180;Uracil-DNA glycosylase, required for repair of uracil in DNA formed by spontaneous cytosine deamination, not required for strand-specific mismatch repair, cell-cycle regulated, expressed in late G1, localizes to mitochondria and nucleus

3181;Serine/threonine protein phosphatase Z, isoform of Ppz2p; involved in regulation of potassium transport, which affects osmotic stability, cell cycle progression, and halotolerance

3182;Protein of unknown function; green fluorescent protein (GFP)-fusion protein localizes to the cytoplasm in a punctate pattern

3183;Nuclear protein that plays a regulatory role in the cyclic AMP (cAMP)-dependent protein kinase (PKA) signal transduction pathway; negatively regulates pseudohyphal differentiation; homologous to several transcription factors

3184;Meiosis-specific subunit of the t-SNARE complex, required for prospore membrane formation during sporulation; similar to but not functionally redundant with Sec9p; SNAP-25 homolog

3185;Hypothetical protein

3186;Polyamine oxidase, converts spermine to spermidine, which is required for the essential hypusination modification of translation factor eIF-5A; also involved in pantothenic acid biosynthesis

3187;Subunit of the Cop9 signalosome, which is required for deneddylation, or removal of the ubiquitin-like protein Rublp from Cdc53p (cullin); involved in adaptation to pheromone signaling

3188;Bud neck-localized, SH3 domain-containing protein required for cytokinesis;

regulates actomyosin ring dynamics and septin localization; interacts with the formins, Bni1p and Bnr1p, and with Cyk3p, Vrp1p, and Bni5p

3189;Endoplasmic reticulum transmembrane protein, homolog of human BAP31 protein3190;Subunit of the nuclear pore complex (NPC) that is localized to both sides of the pore; contains a repetitive GLFG motif that interacts with mRNA export factor Mex67p and with karyopherin Kap95p; homologous to Nup100p

3191;Protein required for maturation of the 25S and 5.8S ribosomal RNAs; constituent of 66S pre-ribosomal particles; homologous to mammalian Bop1

3192:Protein required for nuclear membrane fusion during karyogamy, localizes to the membrane with a soluble portion in the endoplasmic reticulum lumen, may form a complex with Jemlp and Kar2p; expression of the gene is regulated by pheromone

3193;Mitochondrial DNA-binding protein involved in mitochondrial DNA replication and recombination, member of HMG1 DNA-binding protein family; activity may be regulated by protein kinase A phosphorylation

3194;Protein required for establishment and maintenance of sister chromatid condensation and cohesion, colocalizes with cohesin on chromosomes in an interdependent manner, may function as a protein-protein interaction scaffold

3195;ATP-dependent RNA helicase of the SFI superfamily, required for nonsense mediated mRNA decay and for efficient translation termination at nonsense codons

3196;Permease of basic amino acids in the vacuolar membrane

3197;Component, with Afg3p, of the mitochondrial inner membrane m-AAA protease that mediates degradation of misfolded or unassembled proteins and is also required for correct assembly of mitochondrial enzyme complexes

3198;Nuclear protein that may have a role in nuclear protein import

3199;Hypothetical protein

3200;Protein of unknown function, transcription is activated by paralogous transcription factors Yrm1p and Yrr1p along with genes involved in multidrug resistance

3201;Protein kinase with similarity to serine/threonine protein kinase Ypklp; functionally redundant with YPK1 at the genetic level; participates in a signaling pathway required for optimal cell wall integrity; homolog of mammalian kinase SGK

3202;Putative fatty aldehyde dehydrogenase, located in the mitochondrial outer membrane and also in lipid particles; has similarity to human fatty aldehyde dehydrogenase (FALDH) which is implicated in Sjogren-Larsson syndrome

3203;Hypothetical protein

3204;Dihydrofolate synthetase, involved in folic acid biosynthesis; catalyzes the conversion of dihydropteroate to dihydrofolate in folate coenzyme biosynthesis3205;The authentic, non-tagged protein was localized to the mitochondria

3206;Hypothetical protein

3207;DNA 3'-phosphatase that functions in repair of endogenous damage of double-stranded DNA, activity is specific for removal of 3' phosphates at strand breaks; has similarity to the 1-2-haloacid dehalogenase superfamily

3208;Co-chaperone for Hsp40p, anchored in the ER membrane; with its homolog Hdj1p promotes ER-associated protein degradation (ERAD) of integral membrane substrates; similar to E. coli DnaJ

3209;Putative metal transporter involved in mitochondrial iron accumulation; closely related to Mmt2p

3210;Hypothetical protein

3211;Putative transcriptional repressor with proline-rich zinc fingers; overproduction impairs cell growth

3212;Mitochondrial ribosomal protein of the small subunit

3213;Essential splicing factor; associated with Prp19p and the spliceosome, contains an N-terminal c-Myb DNA binding motif necessary for cell viability but not for Prp19p association, evolutionarily conserved and homologous to S. pombe Cdc5p

3214;Serine hydrolase that localizes to both the nucleus and cytoplasm; sequence is similar to Fsh1p and Fsh3p

3215;Peripheral vacuolar membrane protein required for protein trafficking and vacuole biogenesis; forms complex with Pep3p that promotes vesicular docking/fusion reactions in conjunction with SNARE proteins, also interacts with Pep7p

3216;Subunit (17 kDa) of TFIID and SAGA complexes, involved in RNA polymerase II transcription initiation and in chromatin modification, similar to histone H3

3217;Hypothetical protein

3218;Omega class glutathione transferase

3219;Endoplasmic reticulum membrane protein that recruits the ubiquitin-conjugating enzyme Ubc7p to the ER where it functions in protein degradation; contains a CUE domain that binds ubiquitin to facilitate intramolecular monoubiquitination3220;Protein involved in promoting high level transcription of rDNA, subunit of UAF (upstream activation factor) for RNA polymerase I

3221;Ubiquitin-binding component of the Rsp5p E3-ubiquitin ligase complex, functional homolog of Bu12p, disruption causes temperature-sensitive growth, overexpression causes missorting of amino acid permeases

3222;Carboxy-terminal domain (CTD) phosphatase, essential for dephosphorylation of the repeated C-terminal domain of the RNApolymerase II large subunit (Rpo21p)

3223;Zinc cluster transcriptional activator necessary for derepression of a variety of genes under non-fermentative growth conditions, active after diauxic shift, binds carbon source responsive elements

3224;ER membrane protein involved in the second step of glycosylphosphatidylinositol (GPI) anchor assembly, the de-N-acetylation of the N-acetylglucosaminylphosphatidylinositol intermediate; functional homolog of human PIG-Lp

3225;Mitochondrial ribosomal protein of the large subunit

>

3226;Hypothetical protein 3227;Membrane protein of the plasma membrane and ER, involved in translocation of phospholipids and alkylphosphocholine drugs across the plasma membrane

3228;Essential nucleolar protein required for 40S ribosome biogenesis; physically and functionally interacts with Krr1p

3229;Gamma subunit of coatomer, a heptameric protein complex that together with Arf1p forms the COPI coat; involved in ER to Golgi transport of selective cargo

3230;Part of the evolutionarily-conserved CCR4-NOT transcriptional regulatory complex involved in controlling mRNA initiation, elongation, and degradation

3231;Plasma membrane protein that binds HCO3-, I-, Br-, N03- and Cl- ; putative boron efflux transporter with similarity to A. thaliana BOR1 which is a known boron efflux transporter

3232;Subunit of a replication-pausing checkpoint complex (Tof1p-Mrc1p-Csm3p) that acts at the stalled replication fork to promote sister chromatid cohesion after DNA damage, facilitating gap repair of damaged DNA; interacts with the MCM helicase

3233;Phosphatidylinositol transfer protein (PITP), downregulates Plblp-mediated turnover of phosphatidylcholine, found in the cytosol and microsomes, homologous to Pdr16p, deletion affects phospholipid composition

3234;Catalytic subunit of DNA polymerase epsilon, one of the major chromosomal DNA replication polymerases characterized by processivity and proofreading exonuclease activity; also involved in DNA synthesis during DNA repair

3235;Protein required for cell viability

3236;Peripheral membrane protein required for the formation of cytosolic sequestering vesicles involved in vacuolar import through both the Cvt pathway and autophagy; interacts with Atg9p and is necessary for its trafficking

3237;Subunit of the Mediator complex; interacts with the RNA polymerase II holoenzyme to postively or negatively regulate transcription; dispensible for basal transcription

3238;Nitrogen catabolite repression regulator that acts by inhibition of GLN3 transcription in good nitrogen source; altered form of Ure2p creates [URE3] prion 3239;Protein that contains a 70 amino acid J-domain, may function as a co-chaperone to recruit Hsp70-like activity to specific sites; mutation causes defects in fluid-phase endocytosis

3240;Protein with DNA-dependent ATPase and ssDNA annealing activities involved in maintenance of genome; interacts functionally with DNA polymerase delta; homolog of human Werner helicase interacting protein (WHIP)

3241;Peroxisomal membrane protein component of the peroxisomal translocation machinery, required for peroxisome biogenesis, binds Pex14p

3242;Hypothetical protein

3243;Peroxisomal 2,4-dienoyl-CoA reductase, auxiliary enzyme of fatty acid beta-oxidation; homodimeric enzyme required for growth and sporulation on petroselineate medium; expression induced during late sporulation and in the presence of oleate

3244;Hypothetical protein

3245;Protein required for cell viability; computational analysis of large-scale protein-protein interaction data suggests a possible role in assembly of the ribosomal large subunit

3246;Largest subunit of the Anaphase-Promoting Complex/Cyclosome (APC/C), which is a ubiquitin-protein ligase required for degradation of anaphase inhibitors, including mitotic cyclins, during the metaphase/anaphase transition

3247;Essential protein required for maturation of Gaslp and Pho8p; green fluorescent protein (GFP)-fusion protein localizes to the nuclear periphery; has synthetic genetic interactions with secretory pathway genes

3248;Protein required for cell viability

3249;ORF, Uncharacterized

3250;Protein of unknown function, required for survival upon exposure to K1 killer toxin; proposed to regulate double-strand break repair via non-homologous end-joining

3251;Putative S-adenosylmethionine-dependent methyltransferase of the seven beta-strand family

3252;Hypothetical protein

3253;Serine-rich protein of unknown function; overproduction suppresses the growth inhibition caused by exposure to the immunosuppressant leflunomide

3254;Protein of unknown function, localizes to the vacuole; potential Cdc28p substrate

3255;Alpha-1,2-mannosyltransferase, catalyzes addition of the terminal alpha 1,2-Man to the Man5GlcNAc2-PP-dolichol intermediate during asparagine-linked glycosylation in the ER

3256;Essential subunit of RNA polymerase III transcription factor (TFIIIB), which is involved in transcription of genes encoding tRNAs, 5S rRNA, U6 snRNA, and other small RNAs

3257;Hypothetical protein

3258;Non-essential protein with similarity to S. pombe hypothetical protein E349594

3259;Poly(A)+ RNA-binding protein, abundant mRNP-component protein hypothesized to bind a pool of non-translatable mRNAs; not reported to associate with polyribosomes

3260;Translational elongation factor EF-3; paralog of YEF3 and member of the ABC superfamily; stimulates EF-1 alpha-dependent binding of aminoacyl-tRNA by the ribosome; normally expressed in zinc deficient cells

3261;S-adenosylmethionine transporter of the mitochondrial inner membrane, member of the mitochondrial carrier family; required for biotin biosynthesis and respiratory growth

3262;Probable RNA-binding protein, functions in protein translation to promote G1 progression and differentiation, required for meiotic cell division

3263;RNA-dependent ATPase in the DEAH-box family, required for activation of the spliceosome before the first transesterification step in RNA splicing

3264;Hypothetical protein

3265;Guanine nucleotide exchange factor (GEF); glycosylated integral membrane protein of the endoplasmic reticulum, important for the initiation of transport vesicle budding from the endoplasmic reticulum through activation of the GTPase Sar1p

3266;Hypothetical protein

3267;Hypothetical protein

3268;Anchorage subunit of a-agglutinin of a-cells, highly O-glycosylated protein with N-terminal secretion signal and C-terminal signal for addition of GPI anchor to cell wall, linked to adhesion subunit Aga2p via two disulfide bonds

3269;Probable component of the secretory vesicle docking complex, acts at a late step in secretion; shows genetic and physical interactions with Sec1p and is enriched in microsomal membrane fractions; required for sporulation

3270;Dethiobiotin synthetase, catalyzes the third step in the biotin biosynthesis pathway; BIO4 is in a cluster of 3 genes (BIO3, BIO4, and BIO5) that mediate biotin synthesis; expression appears to be repressed at low iron levels

3271;Ferric reductase, reduces a specific subset of siderophore-bound iron prior to uptake by transporters; expression induced by low iron levels

3272;Epoxide hydrolase, member of the alpha/beta hydrolase fold family; may have a role in detoxification of epoxides

3273;Hypothetical proteinj 3274;Part of 23-member seripauperin multigene family encoded mainly in subtelomeric regions, active during alcoholic fermentation, regulated by anaerobiosis, negatively regulated by oxygen, repressed by heme

3275:Hypothetical protein

3276;Nucleolar protein required for 60S ribosomal subunit biogenesis

3277;Subunit of the RNA polymerase II mediator complex; associates with core polymerase subunits to form the RNA polymerase II holoenzyme; essential for transcriptional regulation

3278;Putative 1,3-beta-glucanosyltransferase, has similarity to Gaslp; localizes to the cell wall

3279;Subunit of a palmitoyltransferase, composed of Shr5p and Erf2p, that adds a palmitoyl lipid moiety to Ras2p through a thioester linkage; palmitoylation is required for Ras2p localization to the plasma membrane

3280;Myo-inositol transporter with strong similarity to the major myo-inositol transporter Itr1p, member of the sugar transporter superfamily; expressed constitutively

3281;tRNA 2'-phosphotransferase, catalyzes the final step in yeast tRNA splicing: the transfer of the 2'-PO(4) from the splice junction to NAD(+) to form ADP-ribose 1"-2"cyclic phosphate and nicotinamide

3282;Cytoplasmic tryptophanyl-tRNA synthetase, aminoacylates tryptophanyl-tRNA

3283;O-methyltransferase, catalyzes two different O-methylation steps in ubiquinone (Coenzyme Q) biosynthesis; component of a mitochondrial ubiquinone-synthesizing complex

3284;Subunit of heteropentameric Replication factor C (RF-C), which is a DNA binding protein and ATPase that acts as a clamp loader of the proliferating cell nuclear antigen (PCNA) processivity factor for DNA polymerases delta and epsilon

3285;GTPase-activating protein that negatively regulates RAS by converting it from the GTP- to the GDP-bound inactive form, required for reducing cAMP levels under nutrient limiting conditions, has similarity to Iralp and human neurofibromin

3286;Nuclear pore-associated protein, forms a complex with Sac3p that is involved in transcription and in mRNA export from the nucleus; contains a PAM domain implicated in protein-protein binding

3287;Non-essential protein of unknown function required for transcriptional induction of the early meiotic-specific transcription factor IME1, also required for sporulation

3288;Protein required for normal hydroxyurea resistance; one of consitutive RNR transcription (CRT) regulators

3289;Protein required for normal hydroxyurea resistance; one of consitutive RNR transcription (CRT) regulators

3290;5-phospho-ribosyl-l(alpha)-pyrophosphate synthetase, involved in nucleotide, histidine, and tryptophan biosynthesis; one of a five related enzymes, which are active as heteromultimeric complexes

3291;Homolog of Gpm1p phosphoglycerate mutase which converts 3-phosphoglycerate to 2-phosphoglycerate in glycolysis; may be non-functional derivative of a gene duplication event

3292;Structural maintenance of chromosomes (SMC) protein, interacts with Smc6p and Nse1p to form a large complex; S. pombe homolog forms a heterodimer with S. pombe Rad18p that is involved in DNA repair

3293;Hypothetical protein

3294;Protein involved in telomeric and mating-type locus silencing, interacts with Sir2p and also interacts with the Gal11p, which is a component of the RNA pol II mediator complex

3295;Calmodulin-dependent protein kinase; may play a role in stress response, many CA++/calmodulan dependent phosphorylation substrates demonstrated in vitro, amino acid sequence similar to Cmk1p and mammalian Cam Kinase II

3296;Mitochondrial outer membrane protein, required for transmission of mitochondria to daughter cells; exists in a complex with Mmm1p and Mdm10p

3297;Coenzyme Q binding protein, required for function of coenzyme Q in respiration by functioning in the delivery of Q 6 to its proper location for electron transport, START domain protein with homologs in bacteria and eukaryotes

3298;RNA polymerase II subunit B12.5; part of central core; similar to Rpc19p and bacterial alpha subunit

3299;DNA binding subunit of Sin3p-Rpd3p histone deacetylase complex, involved in transcriptional repression of meiosis-specific genes during vegetative growth and silencing

3300;DNA ligase required for nonhomologous end-joining (NHEJ), forms stable heterodimer with required cofactor Liflp, catalyzes DNA ligation as part of a complex with Liflp and Nej1p; involved in meiosis, not essential for vegetative growth3301;Hypothetical protein

3302;Copper-binding metallothionein, required for wild-type copper resistance

3303;Protein containing a CUE domain that binds ubiquitin, which may facilitate intramolecular monoubiquitination; green fluorescent protein (GFP)-fusion protein localizes to the cytoplasm in a punctate pattern

3304;Nuclear protein that inhibits replication of Brome mosaic virus in S. cerevisiae, which is a model system for studying replication of positive-strand RNA viruses in their natural hosts

3305;t-SNARE required for ER membrane fusion and vesicular traffic, integral membrane protein that constitutes with Sec20p and Uselp the trimeric acceptor for R/v-SNAREs on Golgi-derived vesicles at the ER; part of Dsl1p complex

3306;Contains three calcium and lipid binding domains; green fluorescent protein (GFP)-fusion protein localizes to the cell periphery; C-terminal portion of Tcb1p, Tcb2p and Tcb3p interact

3307;Alpha-isopropylmalate synthase II (2-isopropylmalate synthase), catalyzes the first step in the leucine biosynthesis pathway; the minor isozyme, responsible for the residual alpha-IPMS activity detected in a leu4 null mutant

3308;GTPase-activating protein for the polarity-establishment protein Cdc42p; implicated in control of septin organization, pheromone response, and haploid invasive growth

3309;Hypothetical protein

3310;Protein of unassigned function involved in activation of the Pmalp plasma membrane H+-ATPase by glucose

3311;Protein involved in regulation of Rsp5p, which is an essential HECT ubiquitin ligase; required for binding of Rsp5p to Ubp2p; contains an UBA domain

3312;Essential protein that promotes the first step of splicing and is required for the final stages of spliceosome maturation; interacts with Prp2p, which may release Spp2p from the spliceosome following the first cleavage reaction

3313;Alpha 1,2-mannosyltransferase involved in glycosyl phosphatidyl inositol (GPI) biosynthesis; required for addition of the fourth, side branching mannose to the GPI core structure

3314;Mitochondrial ribosomal protein of the large subunit

3315;Mitochondrial ribosomal protein of the small subunit; PET123 exhibits genetic interactions with PET122, which encodes a COX3 mRNA-specific translational activator

3316;Zn2-Cys6 zinc-finger transcription factor that activates genes involved in multidrug resistance; paralog of Yrr1p, acting on an overlapping set of target genes

3317;Sporulation-specific exo-1,3-beta-glucanase; contributes to ascospore thermoresistance

3318;Peripheral peroxisomal membrane protein involved in controlling peroxisome size and number, interacts with homologous protein Pex25p

3319;Protein involved in biosynthesis of the coenzyme lipoic acid, has similarity to E. coli lipoic acid synthase

3320;Nicotinate phosphoribosyltransferase, acts in the salvage pathway of NAD+ biosynthesis; required for silencing at rDNA and telomeres and has a role in silencing at mating-type loci; localized to the nucleus

3321;Hypothetical protein

3322;Hypothetical protein

3323;Hypothetical protein

3324;Mannoprotein that exhibits a tight association with the cell wall, required for cell wall stability in the absence of GPI-anchored mannoproteins; has a high serine-threonine content; expression is induced in cell wall mutants

3325;Protein of unknown function required for the construction of the outer spore wall layers

3326;Daughter cell-specific protein, may help establish daughter fate

3327;Subunit of vacuolar-ATPase V0 domain, one of two isoforms (Vphlp and Stvlp); Vphlp is located in V-ATPase complexes of the vacuole while Stvlp is located in V-ATPase complexes of the Golgi and endosomes

3328;Phosphoprotein of the mRNA cap-binding complex involved in translational control, repressor of cap-dependent translation initiation, competes with eIF4G for binding to eIF4E

3329;Uroporphyrinogen III synthase, catalyzes the conversion of hydroxymethylbilane to uroporphyrinogen III, the fourth step in the heme biosynthetic pathway

3330;Member of the imitation-switch (ISWI) class of ATP-dependent chromatin remodeling complexes; ATPase component that, with Itc1p, forms a complex required for repression of a-specific genes, INO1, and early meiotic genes during mitotic growth

3331;Vacuolar transporter that mediates zinc transport into the vacuole; overexpression confers resistance to cobalt and rhodium

3332;Tail-anchored endoplasmic reticulum membrane protein that is a substrate of the phosphatase calcineurin, interacts with homolog Frt2p, promotes cell growth in conditions of high Na+, alkaline pH, and cell wall stress

3333;Hypothetical protein

3334;Ubiquitin-conjugating enzyme most similar in sequence to Xenopus ubiquitin-conjugating enzyme E2-C, but not a true functional homolog of this E2; unlike E2-C, not required for the degradation of mitotic cyclin Clb2

3335;Mitochondrial protein similar to Lucilia illustris mitochondrial cytochrome oxidase

3336;Hypothetical protein

3337;Protein of unknown function, green fluorescent protein (GFP)-fusion protein localizes to mitochondria; msc6 mutants are defective in directing meiotic recombination events to homologous chromatids

3338;Sorting nexin required to maintain late-Golgi resident enzymes in their proper location by recycling molecules from the prevacuolar compartment; contains a PX domain and sequence similarity to human Snx3p

3339;Hypothetical protein; open reading frame overlaps 5' end of essential SUI3 gene encoding a translation initiation factor subunit

3340;The authentic, non-tagged protein was localized to the mitochondria.

3341;Bifunctional enzyme with thiamine-phosphate pyrophosphorylase and 4-methyl-5-beta-hydroxyethylthiazole kinase activities, required for thiamine biosynthesis; GFP-fusion protein localizes to the cytoplasm in a punctate pattern

3342;tRNA:pseudouridine synthase, introduces pseudouridines at positions 26-28, 34-36, 65, and 67 of tRNA; nuclear protein that appears to be involved in tRNA export; also acts on U2 snRNA

3343;Aurora kinase involved in regulating kinetochore-microtubule attachments, associates with Sli5p, which stimulates Ipllp kinase activity and promotes its association with the mitotic spindle, potential Cdc28p substrate

3344;Iron-regulated transcriptional activator, required for iron homeostasis and resistance to oxidative stress; similar to Aft1p

3345;Hypothetical protein

3346;Protein that relieves transcriptional repression by binding to the Cyc8p-Tuplp corepressor and recruiting the SAGA complex to the repressed promoter; contains a PHD finger domain

3347;Large subunit of the dynactin complex, which is involved in partitioning the mitotic spindle between mother and daughter cells; putative ortholog of mammalian p150(glued)

3348;Heme A:farnesyltransferase, catalyzes the first step in the conversion of protoheme to the heme A prosthetic group required for cytochrome c oxidase activity; human ortholog is associated with mitochondrial disorders

3349;Protein of unknown function; green fluorescent protein (GFP)-fusion protein localizes to the cytoplasm in a punctate pattern

3350;Kinesin-related motor protein involved in mitotic spindle positioning, stabilizes microtubules by targeting Biklp to the plus end; Kip2p levels are controlled during the cell cycle

3351;Mitogen-activated kinase kinase involved in protein kinase C signaling pathway that controls cell integrity; upon activation by Bcklp phosphorylates downstream target, S1t2p; functionally redundant with Mkk1p

3352;Glc7-interacting protein whose overexpression relocalizes Glc7p from the nucleus

3353;Mitochondrial inner membrane protein required for delivery of copper to the Cox1p subunit of cytochrome c oxidase; association with mitochondrial ribosomes suggests that copper delivery may occur during translation of Cox1p

3354;Karyopherin with a role in the assembly or export of 60S ribosomal subunits

3355;Meiosis specific protein involved in DMC1-dependent meiotic recombination, forms heterodimer with Sae3p; proposed to be an assembly factor for Dmc1p

3356;Phosphatidylinositol 3,5-bisphosphate-binding protein required for maturation of pro-aminopeptidase I, predicted to fold as a seven-bladed beta-propeller; displays punctate cytoplasmic localizationj 3357;Mitochondrial tyrosyl-tRNA synthetase

3358;Plasma membrane sulfite pump involved in sulfite metabolism and required for efficient sulfite efflux; major facilitator superfamily protein

3359;Putative aryl alcohol dehydrogenase; transcription is activated by paralogous transcription factors Yrm1p and Yrr1p along with genes involved in multidrug resistance

3360;Protein involved in the synthesis of N-acetylglucosaminyl phosphatidylinositol (GlcNAc-PI), the first intermediate in the synthesis of glycosylphosphatidylinositol (GPI) anchors; homologous to the human PIG-C protein

3361;Hypothetical protein

3362;Plasma membrane weak-acid-inducible ATP-binding cassette (ABC) transporter, required for weak organic acid resistance, strongly induced by sorbate and benzoate, regulated by War1p, mutants exhibit sorbate hypersensitivity

3363;Probable mannosylphosphate transferase involved in the synthesis of core oligosaccharides in protein glycosylation pathway; member of the KRE2/MNT1 mannosyltransferase family

3364;Conserved nuclear protein required to establish sister-chromatid pairing during S-phase, probable DNA helicase with similarity to human BACH1, which associates with tumor suppressor BRCA1; associates with acetyltransferase Ctf7p

3365;Mitochondrial protein that participates in respiration, induced by diauxic shift; homologous to E. coli PrpD, may take part in the conversion of 2-methylcitrate to 2-methylisocitrate

3366;Electron transfer flavoprotein complex subunit ETF-alpha; contains a FAD binding domain; interacts with YFH1, the yeast frataxin homolog

3367;Cytoplasmic protein involved in halotolerance; decreases intracellular Na+ (via Enalp) and increases intracellular K+ by decreasing efflux; expression repressed by Ssn6p-Tup1p and Skolp and induced by NaCl, KCl, and sorbitol through Gcn4p

3368;Mitochondrial transporter, acts both as a glutamate uniporter and as an aspartate-glutamate exchanger; involved in nitrogen metabolism, ornithine synthesis, and the malate-aspartate NADH shuttle

3369;Cyclin associated with protein kinase Kin28p, which is the TFIIH-associated carboxy-terminal domain (CTD) kinase involved in transcription initiation at RNA polymerase II promoters

3370;Acid trehalase required for utilization of extracellular trehalose

3371;Hypothetical protein

3372;Component of the histone acetyltransferase complex

3373;Translation initiation factor eIF-5; N-terminal domain functions as a GTPase-activating protein to mediate hydrolysis of ribosome-bound GTP; C-terminal domain is the core of ribosomal preinitiation complex formation

3374;Catalytic subunit of N-terminal acetyltransferase of the NatC type; required for replication of dsRNA virus

3375;snRNP protein component of spliceosomal snRNPs, required for pre-mRNA splicing and snRNP biogenesis; in null mutant newly-synthesized snRNAs are destabilized and 3'-end processing is slowed

3376;Hypothetical protein

3377;Protein with sequence similarity to Grs1p, which is a glycyl-tRNA synthetase; cannot substitute for Grs1p; possible pseudogene that is expressed at very low levels

3378;Hypothetical protein

3379;Hypothetical protein

3380;Methylthioribose-1-phosphate isomerase, catalyzes the isomerization of 5-methylthioribose-1-phosphate to 5-methylthioribulose-1-phosphate in the methionine salvage pathway

3381;B-type cyclin involved in DNA replication during S phase; activates Cdc28p to promote initiation of DNA synthesis; functions in formation of mitotic spindles along with Clb3p and Clb4p; most abundant during late G1 phase

3382;Nuclear encoded protein needed for efficient splicing of mitochondrial COX1 aI5beta intron; mss18 mutations block cleavage of 5' exon - intron junction; phenotype of intronless strain suggests additional functions

3383;Chromatin-associated protein, required for sister chromatid cohesion; interacts with DNA polymerase alpha (Po11p) and may link DNA synthesis to sister chromatid cohesion

3384;Minus-end-directed microtubule motor that functions in mitosis and meiosis, localizes to the spindle pole body and localization is dependent on functional Cik1p, required for nuclear fusion during mating; potential Cdc28p substrate

3385;Protein of unknown function; green fluorescent protein (GFP)-fusion protein localizes to the cytoplasm in a punctate pattern

3386;Mitochondrial protein required for degradation of unstable forms of cytochrome c

3387;Second largest subunit of DNA polymerase II (DNA polymerase epsilon), required for normal yeast chromosomal replication; expression peaks at the G1/S phase boundary; potential Cdc28p substrate

3388;Phosphorylated protein that interacts with Vac8p, required for the cytoplasm-to-vacuole targeting (Cvt) pathway and autophagy

3389;Hypothetical protein

3390;Questionable ORF from MIPS

3391;Protein of unknown function, localized in the nucleoplasm and the nucleolus, genetically interacts with MTR2 in 60S ribosomal protein subunit export

3392;Proposed beta subunit of the heterotrimeric G protein that interacts with the receptor Grp1p, has signaling role in response to nutrients; involved in regulation of pseudohyphal growth through cAMP levels; homolog of Gpb1p

3393;Oleate-activated transcription factor, acts alone and as a heterodimer with Pip2p; activates genes involved in beta-oxidation of fatty acids and peroxisome organization and biogenesis

3394;Oleate-activated transcription factor, acts alone and as a heterodimer with Pip2p; activates genes involved in beta-oxidation of fatty acids and peroxisome organization and biogenesis 3395;Component of the cytoplasmic Tub4p (gamma-tubulin) complex, binds spindle pole bodies and links them to microtubules; has roles in astral microtubule formation and stabilization

3396;Hypothetical protein

3397;Similar to pombe uvi31, putative DNA repair protein

3398;H subunit of the mitochondrial glycine decarboxylase complex, required for the catabolism of glycine to 5,10-methylene-THF; expression is regulated by levels of levels of 5,10-methylene-THF in the cytoplasm

3399;Subunit of holo-CPF, a multiprotein complex and functional homolog of mammalian CPSF, required for the cleavage and polyadenylation of mRNA and snoRNA, 3' ends; involved in pre-tRNA processing; binds to the phosphorylated CTD of RNAPII

3400;Hypothetical protein

3401;Hypothetical protein

3402;Identified by gene-trapping, microarray-based expression analysis, and genome-wide homology searching

3403;Non-essential protein of unknown function

3404;Questionable ORF from MIPS

3405;Vesicle membrane receptor protein (v-SNARE) involved in the fusion between Golgi-derived secretory vesicles with the plasma membrane; proposed to be involved in endocytosis; member of the synaptobrevin/VAMP family of R-type v-SNARE proteins

3406;Vesicle membrane receptor protein (v-SNARE) involved in the fusion between Golgi-derived secretory vesicles with the plasma membrane; proposed to be involved in endocytosis; member of the synaptobrevin/VAMP family of R-type v-SNARE proteins

3407;Nucleoside transporter with broad nucleoside selectivity; localized to intracellular membranes

3408;Transcriptional modulator involved in the regulation of structural genes involved in phospholipid biosynthesis, also participates in regulation of metabolically unrelated genes as well as maintenance of mating efficiency and sporulation

3409;Protein of unknown; function, component of the Swr1p complex that incorporates Htz1p into chromatin; required for formation of nuclear-associated array of smooth endoplasmic reticulum known as karmellae

3410;Hypothetical protein

3411;Hypothetical protein

3412;Translation elongation factor 1 beta; stimulates nucleotide exchange to regenerate EF-1 alpha-GTP for the next elongation cycle; part of the EF-1 complex, which facilitates binding of aminoacyl-tRNA to the ribosomal A site

3413;Translation elongation factor 1 beta; stimulates nucleotide exchange to regenerate EF-1 alpha-GTP for the next elongation cycle; part of the EF-1 complex, which facilitates binding of aminoacyl-tRNA to the ribosomal A site

3414;Membrane-associated hydrophilic protein that interacts with the small GTPase, Vps21p, to facilitate soluble vacuolar protein localization; required for localization and trafficking of the CPY sorting receptor; contains a RING finger motif

3415;Largest of six subunits of the RNA polymerase III transcription initiation factor complex (TFIIIC); part of the TauB domain of TFIIIC that binds DNA at the BoxB promoter sites of tRNA and similar genes; cooperates with Tfc6p in DNA binding

3416;Subunit of the tRNA splicing endonuclease, which is composed of Sen2p, Sen15p, Sen34p, and Sen54p; Sen34p contains the active site for tRNA 3' splice site cleavage and has similarity to Sen2p and to Archaeal tRNA splicing endonuclease

3417;Protein involved in bud-site selection, Bud14p-Glc7p complex functions as a cortical regulator of dynein; diploid mutants display a random budding pattern instead of the wild-type bipolar pattern

3418;Putative integral membrane protein of unknown function; interacts with Ulplp at the nuclear periphery; member of DUP240 gene family

3419;Putative integral membrane protein of unknown function; interacts with Ulp1p at the nuclear periphery; member of DUP240 gene family

3420;Putative integral membrane protein of unknown function; interacts with Ulp1p at the nuclear periphery; member of DUP240 gene family

3421;Outer mitochondrial carnitine acetyltransferase, minor ethanol-inducible enzyme involved in transport of activated acyl groups from the cytoplasm into the mitochondrial matrix

3422;Outer mitochondrial carnitine acetyltransferase, minor ethanol-inducible enzyme involved in transport of activated acyl groups from the cytoplasm into the mitochondrial matrix

3423;Merged open reading frame, does not encode a discrete protein; YAR044W was originally annotated as an independent ORF, but as a result of a sequence change, it was merged with an adjacent ORF into a single reading frame, designated YAR042W

3424;Lectin-like protein involved in flocculation, cell wall protein that binds to mannose chains on the surface of other cells, confers floc-forming ability that is chymotrypsin sensitive and heat resistant; similar to Flo5p

3425;Lectin-like protein involved in flocculation, cell wall protein that binds to mannose chains on the surface of other cells, confers floc-forming ability that is chymotrypsin sensitive and heat resistant; similar to Flo5p

3426;Hypothetical protein

3427;Hypothetical protein

3428;Dubious open reading frame

3429;Kynurenine 3-mono oxygenase, required for biosynthesis of nicotinic acid from tryptophan via kynurenine pathway

3430;Essential protein required for chromosome condensation, likely to function as an intrinsic component of the condensation machinery, may influence multiple aspects of chromosome transmission and dynamics

3431;Hypothetical protein

3432;Hypothetical protein

3433;Protein component of the large (60S) ribosomal subunit, has similarity to rat L32 ribosomal protein; overexpression disrupts telomeric silencing

3434;Protein involved in regulation of phospholipid metabolism; homolog of Scs2p2; similar to D. melanogaster inturned protein

3435;Putative transporter, member of a family of seven S. cerevisiae genes (AVT1-7) related to vesicular GABA-glycine transporters

3436;Protein component of the large (60S) ribosomal subunit, identical to Rp123Bp and has similarity to E. coli L14 and rat L23 ribosomal proteins

3437;Protein of unknown function; green fluorescent protein (GFP)-fusion protein localizes to the cell periphery

3438;Dolichol-P-Man dependent alpha(1-3) mannosyltransferase, involved in the synthesis of dolichol-linked oligosaccharide donor for N-linked glycosylation of proteins

3439;Hypothetical protein

3440;Hypothetical protein

3441;Abundant subunit of the nuclear pore complex (NPC), required for proper localization of specific nucleoporins within the NPC, involved in nuclear envelope permeability and in chromosome segregation, has similarity to Nup157p

3442;Abundant subunit of the nuclear pore complex (NPC), required for proper localization of specific nucleoporins within the NPC, involved in nuclear envelope permeability and in chromosome segregation, has similarity to Nup157p

3443;Hypothetical protein; open reading frame overlaps 3' end of essential ILS1 gene encoding cytoplasmic isoleucine-tRNA synthetase

3444;ATPase involved in protein folding and the response to stress; plays a role in SRP-dependent cotranslational protein-membrane targeting and translocation; member of the heat shock protein 70 (HSP70) family; localized to the cytoplasm

3445;ATPase involved in protein folding and the response to stress; plays a role in SRP-dependent cotranslational protein-membrane targeting and translocation; member of the heat shock protein 70 (HSP70) family; localized to the cytoplasm

3446;Component of the U5 snRNP, required for splicing of U3 precursors; originally described as a splicing factor specifically required for splicing pre-mRNA of the MATa1 cistron

3447;Component of the U5 snRNP, required for splicing of U3 precursors; originally described as a splicing factor specifically required for splicing pre-mRNA of the MATa1 cistron

3448;Hypothetical protein

3449;5-phospho-ribosyl-1(alpha)-pyrophosphate synthetase, involved in nucleotide, histidine, and tryptophan biosynthesis; one of a five related enzymes, which are active as heteromultimeric complexes

3450;Putative ubiquitin-specific protease

3451;Putative transcription factor, has homolog in Kluyveromyces lactis

3452;Hypothetical protein

3453;Activator of Chs3p (chitin synthase III), recruits Chs3p to the bud neck via interaction with Bni4p; has similarity to Shc1p, which activates Chs3p during sporulation

3454;Activator of Chs3p (chitin synthase III), recruits Chs3p to the bud neck via interaction with Bni4p; has similarity to Shc1p, which activates Chs3p during sporulation

3455;Hypothetical protein

3456;Hypothetical protein

3457;One of two (see also PTH1) mitochondrially-localized peptidyl-tRNA hydrolases; dispensable for cell growth

3458;Hypothetical protein

3459;Peripheral membrane protein required for vesicular transport between ER and Golgi and for the 'priming' step in homotypic vacuole fusion, part of the cis-SNARE complex; has similarity to alpha-SNAP

3460;Key endocytic protein involved in a network of interactions with other endocytic proteins, binds membranes in a ubiquitin-dependent manner, may also bind ubiquitinated membrane-associated proteins

3461;Key endocytic protein involved in a network of interactions with other endocytic proteins, binds membranes in a ubiquitin-dependent manner, may also bind ubiquitinated membrane-associated proteins

3462;Integral membrane protein that binds to the HDEL motif in proteins destined for retention in the endoplasmic reticulum; has a role in maintenance of normal levels of ER-resident proteins

3463;Component of the mitotic spindle that binds to interpolar microtubules via its association with beta-tubulin (Tub2p); required for interpolar microtubules to provide an outward force on the spindle poles

3464;Component of the mitotic spindle that binds to interpolar microtubules via its association with beta-tubulin (Tub2p); required for interpolar microtubules to provide an outward force on the spindle poles

3465;Mitochondrial ATP-dependent protease involved in intramitochondrial proteolysis; involved in degradation of misfolded proteins in mitochondria; required for bigenesis and maintenance of mitochondria

3466;Mitochondrial ATP-dependent protease involved in intramitochondrial proteolysis; involved in degradation of misfolded proteins in mitochondria; required for bigenesis and maintenance of mitochondria

3467;Subunit of both RNase MRP, which cleaves pre-rRNA, and nuclear RNase P, which cleaves tRNA precursors to generate mature 5' ends

3468;Mitogen-activated protein kinase involved in mating pheromone response; activated by phoshporylation by Ste7p; provides specificity during the mating vs. filamentous growth response by phosphorylating transcriptional and cytoplasmic targets

3469;Acetyl-coA hydrolase, primarily localized to mitochondria; required for acetate utilization and for diploid pseudohyphal growth

3470;Protein involved in the transcription of 35S rRNA genes by RNA polymerase I; component of the core factor (CF) complex also composed of Rrn11p, Rrn7p and TATA-binding protein

3471;Methionyl-tRNA formyltransferase, catalyzes the formylation of initiator Met-tRNA in mitochondria; potential Cdc28p substrate

3472;Protein of unknown function; null mutant shows a reduced affinity for the alcian blue dye suggesting a decreased net negative charge of the cell surface

3473;Galactokinase, phosphorylates alpha-D-galactose to alpha-D-galactose-1-phosphate in the first step of galactose catabolism; expression regulated by Gal4p

3474;Galactokinase, phosphorylates alpha-D-galactose to alpha-D-galactose-1-phosphate in the first step of galactose catabolism; expression regulated by Gal4p

3475;N-terminally acetylated protein component of the large (60S) ribosomal subunit, nearly identical to Rp14Bp and has similarity to E. coli L4 and rat L4 ribosomal proteins

3476;Non-essential protein of unknown function

3477;Chitin synthase II, requires activation from zymogenic form in order to catalyze the transfer of N-acetylglucosamine (GlcNAc) to chitin; required for the synthesis of chitin in the primary septum during cytokinesis

3478;Chitin synthase II, requires activation from zymogenic form in order to catalyze the transfer of N-acetylglucosamine (GlcNAc) to chitin; required for the synthesis of chitin in the primary septum during cytokinesis

3479;Fatty acid transporter and very long-chain fatty acyl-CoA synthetase, may form a complex with Faa1p or Faa4p that imports and activates exogenous fatty acids

3480;Hypothetical protein

348 hypothetical protein

3482;Multidrug transporter required for resistance to quinidine, barban, cisplatin, and bleomycin; member of the major facilitator superfamily of transporters conferring multiple drug resistance (MFS-MDR)

3483;Multidrug transporter required for resistance to quinidine, barban, cisplatin, and bleomycin; member of the major facilitator superfamily of transporters conferring multiple drug resistance (MFS-MDR)

3484;Protein involved in the assembly of the mitochondrial succinate dehydrogenase complex; putative chaperone

3485;Meiosis-specific regulatory subunit of the Glc7p protein phosphatase, regulates spore wall formation and septin organization, required for expression of some late meiotic genes and for normal localization of Glc7p

3486;Protein component of the small (40S) ribosomal subunit; identical to Rps11Ap and has similarity to E. coli S 17 and rat S 11 ribosomal proteins

3487;Splicing factor, component of the U4/U6-U5 snRNP complex 3488;Ubiquitin-specific protease that specifically disassembles unanchored ubiquitin chains; involved in fructose-1,6-bisphosphatase (Fbp1p) degradation; similar to human isopeptidase T

3489;Ser-Thr protein kinase, member (with Arklp and Prk1p) of the Ark kinase family; involved in endocytosis and actin cytoskeleton organization

3490;Ser-Thr protein kinase, member (with Arklp and Prklp) of the Ark kinase family; involved in endocytosis and actin cytoskeleton organization

3491;Component of UDP-GlcNAc transferase required for the second step of dolichyl-linked oligosaccharide synthesis; anchors the catalytic subunit Alg13p to the ER membrane; similar to bacterial and human glycosyltransferases

3492;DNA-dependent ATPase, stimulates strand exchange by modifying the topology of double-stranded DNA; involved in the recombinational repair of double-strand breaks in DNA during mitosis and meiosis; proposed to be involved in crossover interference

3493;Merged open reading frame, does not encode a discrete protein; YBR075W was originally annotated as an independent ORF, but as a result of a sequence change, it was merged with an adjacent ORF into a single reading frame, designated YBR074W

3494;Non-essential protein of unknown function

3495;Component of the EGO complex, which is involved in the regulation of microautophagy; gene exhibits synthetic genetic interaction with MSS4 encoding phosphatidylinositol 4-phosphate kinase

3496;Component of the EGO complex, which is involved in the regulation of microautophagy; gene exhibits synthetic genetic interaction with MSS4 encoding phosphatidylinositol 4-phosphate kinase

3497;GPI-anchored protein of unknown function, has a possible role in apical bud growth; GPI-anchoring on the plasma membrane crucial to function; similar to Sps2p and Pst1p

3498;Ubiquitin-conjugating enzyme that mediates degradation of short-lived and abnormal proteins; interacts with E3-CaM in ubiquitinating calmodulin; interacts with many SCF ubiquitin protein ligases; component of the cellular stress response

3499;Hypothetical protein

3500;Hypothetical protein

3501;Hypothetical protein

3502;Protein possibly involved in cell fusion and invasive growth

3503;Myristoylated serine/threonine protein kinase involved in vacuolar protein sorting; functions as a membrane-associated complex with Vps34p; active form recruits Vps34p to the Golgi membrane; also detected in mitochondria

3504;Myristoylated serine/threonine protein kinase involved in vacuolar protein sorting; functions as a membrane-associated complex with Vps34p; active form recruits Vps34p to the Golgi membrane; also detected in mitochondria

3505;Hypothetical protein

3506;WD40 repeat-containing subunit of the Set3C histone deacetylase complex, which represses early/middle sporulation genes; antagonizes telomeric silencing; binds specifically to the Sir4p N-terminus

3507;WD40 repeat-containing subunit of the Set3C histone deacetylase complex, which represses early/middle sporulation genes; antagonizes telomeric silencing; binds specifically to the Sir4p N-terminus

3508;Hypothetical protein identified by homology. See FEBS Letters [2000] 487:31-36.

3509;Protein interacting with Rsv167p

3510;Calmodulin; Ca++ binding protein that regulates Ca++ independent processes (mitosis, bud growth, actin organization, endocytosis, etc.) and Ca++ dependent processes (stress-activated pathways), targets include Nuf1p, Myo2p and calcineurin

3511;Nudix hydrolase family member with ADP-ribose pyrophosphatase activity

3512;Protein that recognizes and binds damaged DNA in an ATP-dependent manner (with Rad7p) during nucleotide excision repair; subunit of Nucleotide Excision Repair Factor 4 (NEF4); member of the SWI/SNF family

3513;Protein that recognizes and binds damaged DNA in an ATP-dependent manner (with Rad7p) during nucleotide excision repair; subunit of Nucleotide Excision Repair Factor 4 (NEF4); member of the SWI/SNF family

3514;Translational elongation factor EF-1 alpha; also encoded by TEF1; functions in the binding reaction of aminoacyl-tRNA (AA-tRNA) to ribosomes

3515;U1 snRNP A protein, homolog of human U1-A; involved in nuclear mRNA splicing

3516;Mitochondrial ribosomal protein of the large subunit; overproduction suppresses mutations in the COX2 leader peptide-encoding region

3517;One of six subunits of the RNA polymerase III transcription initiation factor complex (TFIIIC); part of the TauA globular domain of TFIIIC that binds DNA at the BoxA promoter sites of tRNA and similar genes; human homolog is TFITIC-63

3518;One of six subunits of the RNA polymerase III transcription initiation factor complex (TFIIIC); part of the TauA globular domain of TFIIIC that binds DNA at the BoxA promoter sites of tRNA and similar genes; human homolog is TFIIIC-63

3519;Cytoplasmic type 2C protein phosphatase; identified as a high-copy number suppressor of the synthetic lethality of a cnb1 mpk1 double deletion; overexpression decreases high-osmolarity induced Hoglp phosphorylation and kinase activity

3520;Protein of unknown function, overproduction blocks cell cycle arrest in the presence of mating pheromone

3521;Genome integrity checkpoint protein and PI kinase superfamily member; signal transducer required for cell cycle arrest and transcriptional responses prompted by damaged or unreplicated DNA; monitors and participates in meiotic recombination

3522;Genome integrity checkpoint protein and PI kinase superfamily member; signal transducer required for cell cycle arrest and transcriptional responses prompted by damaged or unreplicated DNA; monitors and participates in meiotic recombination

3523;Putative serine type Carboxypeptidase; green fluorescent protein (GFP)-fusion protein localizes to the vacuole; YBR139W is not an essential gene

3524;Probable Zn-finger protein

3525;Probable Zn-finger protein

3526;Protein of unknown function; null mutation does not confer any obvious defects in growth, spore germination, viability, or carbohydrate utilization

3527;Protein required for daughter cell separation, multiple mitotic checkpoints, and chromosome stability; contains 12 degenerate leucine-rich repeat motifs; expression is induced by the Mitotic Exit Network (MEN)

3528;Protein required for daughter cell separation, multiple mitotic checkpoints, and chromosome stability; contains 12 degenerate leucine-rich repeat motifs; expression is induced by the Mitotic Exit Network (MEN)

3529;Protein of unknown function that interacts with Myo2p; has similarity to S. pombe Mpd2

3530;Dubious open reading frame unlikely to encode a protein, based on available experimental and comparative sequence data; partially overlaps the verified ORF YBR175W; null mutant is viable and sporulation defective

3531;Possible serine hydrolase, may be involved in lipid metabolism, null mutant slightly temperature sensitive at 37C

3532;Mitochondrial integral membrane protein involved in mitochondrial fusion and maintenance of the mitochondrial genome; contains N-terminal GTPase domain

3533;Protein component of the small (40S) ribosomal subunit; identical to Rps6Ap and has similarity to rat S6 ribosomal protein

3534;Protein involved in assembly of mitochondrial respiratory complexes; may act as a receptor for proteins destined for export from the mitochondrial matrix to the inner membrane tive proteasomal subunits in other species; null mutant is temperature sensitive and exhibits cell cycle and proteasome assembly defects

5798;Non-ATPase regulatory subunit of the 26S proteasome, has similarity to putative proteasomal subunits in other species; null mutant is temperature sensitive and exhibits cell cycle and proteasome assembly defects

5799;RNA-binding protein that carries poly(A)+ mRNA from the nucleus into the cytoplasm; phosphorylation by Sky1p in the cytoplasm may promote release of mRNAs

5800;Apparent pseudogene, not transcribed or translated under normal conditions; encodes a protein with similarity to adenine phosphoribosyltransferase, but artificially expressed protein exhibits no enzymatic activity

5801;Nucleolar protein, component of the small subunit (SSU) processome containing the U3 snoRNA that is involved in processing of pre-185 rRNA

5802;Stress inducible cytoplasmic thioredoxin peroxidase; cooperates with Tsalp in the removal of reactive oxygen, nitrogen and sulfur species using thioredoxin as hydrogen donor; deletion enhances the mutator phenotype of tsa1 mutants

5803;Transcription factor, activated by proteolytic processing in response to signals from the SPS sensor system for external amino acids; activates transcription of amino acid permease genes and may have a role in tRNA processing

5804:Subunit of RNase MRP, which cleaves pre-rRNA; not shared between Rnase MRP and nuclear Rnase P, in contrast to all other Rnase MRP protein subunits; binds to the NME1 RNA subunit of Rnase MRP

5805;Component of a complex containing Ceflp, putatively involved in pre-mRNA splicing; may bind RNA; has similarity to S. pombe Cwf21p

5806;Hypothetical protein

5807;Hypothetical protein

5808;Protein involved in regulation of cell wall composition and integrity and response to osmotic stress; overproduction suppresses a lysis sensitive PKC mutation; similar to Lre1p, which functions antagonistically to protein kinase A

5809;Fumurate ReDuctase Soluble

5810;Identified by fungal homology and RT-PCR

5811;Hypothetical protein

5812;Microtubule effector required for tubulin heterodimer formation, binds alpha-tubulin, required for normal microtubule function, null mutant exhibits cold-sensitive microtubules and sensitivity to benomyl

5813;Probable transcriptional repressor involved in response to toxic agents such as hydroxyurea that inhibit ribonucleotide reductase; phosphorylation by Snf1p or the Mec1p pathway inactivates Mig3p, allowing induction of damage response genes

5814;Purine-cytosine permease, mediates purine (adenine, guanine, and hypoxanthine) and cytosine accumulation

5815;Questionable ORF from MIPS

5816;Phosphotyrosine-specific protein phosphatase involved in the inactivation of mitogen-activated protein kinase (MAPK) during osmolarity sensing; dephosporylates Hoglp MAPK and regulates its localization; localized to the cytoplasm

5817;Hypothetical protein

5818;3-phosphoglycerate dehydrogenase, catalyzes the first step in serine and glycine biosynthesis; isozyme of Ser33p

5819;Questionable ORF from MIPS

5820;Questionable ORF from MIPS

5821;Hypothetical protein

5822;Transmembrane osmosensor, participates in activation of both the Cdc42p- and MAP kinase-dependent filamentous growth pathway and the high-osmolarity glycerol response pathway

5823;Questionable ORF from MIPS

5824;Questionable ORF from MIPS

5825;Subunit of cohesin loading factor (Scc2p-Scc4p), a complex required for the loading of cohesin complexes onto chromosomes; involved in establishing sister chromatid cohesion during double-strand break repair via phosphorylated histone H2AX

5826;Hypothetical protein

5827;Questionable ORF from MIPS

5828;Questionable ORF from MIPS

5829;Questionable ORF from MIPS

5830;The authentic, non-tagged protein was localized to the mitochondria

5831;Probable Mg(2+) transporter; overexpression confers increased tolerance to Al(3+) and Ga(3+) ions

5832;Putative protein of unknown function; green fluorescent protein (GFP)-fusion protein localizes to the nucleus; YFL049W is not an essential gene; abundantly expressed in many growth conditions

5833;Protein involved in bud-site selection, nutrient signaling, and gene expression controlled by the TOR kinase; diploid mutants display a random budding pattern instead of the wild-type bipolar pattern

5834;Questionable ORF from MIPS

5835;Protein required for the negative regulation by ammonia of Gaplp, which is a general amino acid permease 5836;Hypothetical protein

5837;Dubious open reading frame unlikely to encode a protein based on available experimental and comparative sequence data; partially overlaps the uncharacterized gene YFR055W

5838;Putative cystathionine beta-lyase; involved in copper ion homeostasis and sulfur metabolism; YFR055W is not an essential gene

5839;Protein with sequence similarity to iron/copper reductases (FRE1-8), possibly involved in iron homeostasis

5840;Putative protein of unknown function; deletion mutant has no detectable phenotype

5841;Dubious open reading frame unlikely to encode a protein, based on available experimental and comparative sequence data; partially overlaps the verified ORF INO80/YGL150C.

5842;Protein component of the large (60S) ribosomal subunit, nearly identical to Rp19Bp and has similarity to E. coli L6 and rat L9 ribosomal proteins

5843;Protein with similarity to hect domain E3 ubiquitin-protein ligases, not essential for viability

5844;Protein with similarity to hect domain E3 ubiquitin-protein ligases, not essential for viability

5845;hypothetical protein

5846;Hypothetical protein

5847;Mitochondrial 21S rRNA methyltransferase, required for methylation of U(279 1) in 21S rRNA; MRM2 deletion confers thermosensitive respiration and loss of mitochondrial DNA; has similarity to Spblp and Trm7p, and to E. coli FtsJ/RrmJ

5848;Component of the ATP-dependent Isw2p-Itc1p chromatin remodeling complex, required for repression of a-specific genes, repression of early meiotic genes during mitotic growth, and repression of INO1

5849;Component of the ATP-dependent Isw2p-Itc1p chromatin remodeling complex, required for repression of a-specific genes, repression of early meiotic genes during mitotic growth, and repression of INO1

5850;Protein required for inositol prototrophy, appears to be involved in the synthesis of inositol phospholipids from inositol but not in the control of inositol synthesis

5851;Nuclear polyadenylated RNA-binding protein; autoregulates mRNA levels; related to human hnRNPs; has nuclear localization signal sequence that binds to Kap104p; required for poly(A) tail length control and nuclear RNA export

5852;Mitochondrial protein required for sporulation

5853;Hypothetical protein; open reading frame overlaps 3' end of essential RPL28 gene encoding a large subunit ribosomal protein

5854;Essential nucleoporin, catalyzes its own cleavage in vivo to generate a C-terminal fragment that assembles into the Nup84p subcomplex of the nuclear pore complex, and an N-terminal fragment of unknown function that is homologous to Nup100p

5855;Essential nucleoporin, catalyzes its own cleavage in vivo to generate a C-terminal fragment that assembles into the Nup84p subcomplex of the nuclear pore complex, and an N-terminal fragment of unknown function that is homologous to Nup100p

5856;Protein involved in DNA double-strand break repair; physically interacts with DNA ligase 4 (Lig4p); homologous to mammalian XRCC4 protein

5857;Protein involved in DNA double-strand break repair; physically interacts with DNA ligase 4 (Lig4p); homologous to mammalian XRCC4 protein

5858;Hypothetical protein; open reading frame overlaps 5' end of essential HSF1 gene encoding heat shock transcription factor

5859;Identified by gene-trapping, microarray-based expression analysis, and genome-wide homology searching

5860;Catalytic component of UDP-GlcNAc transferase, required for the second step of dolichyl-linked oligosaccharide synthesis; anchored to the ER membrane via interaction with Alg14p; similar to bacterial and human glycosyltransferases

5861;Catalytic component of UDP-GlcNAc transferase, required for the second step of dolichyl-linked oligosaccharide synthesis; anchored to the ER membrane via interaction with Alg14p; similar to bacterial and human glycosyltransferases

5862;Hypothetical protein

5863;Hypothetical protein

5864;Subunit of the Mediator global transcriptional cofactor complex, which is part of the RNA polymerase II holoenzyme and plays an essential role in basal and activated transcription; direct target of the Cyc8p-Tup1p transcriptional corepressor

5865;Member of the PUF protein family, which is defined by the presence of Pumilio homology domains that confer RNA binding activity; preferentially binds mRNAs encoding nucleolar ribosomal RNA-processing factors 5866;Member of the PUF protein family, which is defined by the presence of Pumilio homology domains that confer RNA binding activity; preferentially binds mRNAs encoding nucleolar ribosomal RNA-processing factors

5867;Zinc cluster protein that is a master regulator involved in recruiting other zinc cluster proteins to pleiotropic drug response elements (PDREs) to fine tune the regulation of multidrug resistance genes

5868;Protein with similarity to methyltransferase family members; required for replication of Brome mosaic virus in S. cerevisiae, which is a model system for studying replication of positive-strand RNA viruses in their natural hosts

5869;Protein with similarity to methyltransferase family members; required for replication of Brome mosaic virus in S. cerevisiae, which is a model system for studying replication of positive-strand RNA viruses in their natural hosts

5870;Hypothetical protein

5871;Protein with both structural and functional similarity to Mid2p, which is a plasma membrane sensor required for cell integrity signaling during pheromone-induced morphogenesis; suppresses rgd1 null mutations

5872;tRNAHis guanylyltransferase, adds a guanosine residue to the 5' end of tRNAHis after transcription and RNase P cleavage; couples nuclear division and migration to cell budding and cytokinesis; essential enzyme conserved among eukaryotes

5873;Putative protein of unknown function; green fluorescent protein (GFP)-fusion protein localizes to the mitochondrion

5874;Dubious open reading frame unlikely to encode a protein, based on available experimental and comparative sequence data; completely overlaps the uncharacterized ORF YGR031 W

5875;Catalytic subunit of 1,3-beta-glucan synthase, has similarity to an alternate catalytic subunit, Fkslp (Gsclp); Rholp encodes the regulatory subunit; involved in cell wall synthesis and maintenance

5876;Catalytic subunit of 1,3-beta-glucan synthase, has similarity to an alternate catalytic subunit, Fkslp (Gsclp); Rholp encodes the regulatory subunit; involved in cell wall synthesis and maintenance

5877;Zinc finger protein involved in control of meiosis; prevents meiosis by repressing IME1 expression and promotes mitosis by activating CLN2 expression; directly repressed by a1-a2 regulator; mediates cell type control of sporulation

5878;Zinc finger protein involved in control of meiosis; prevents meiosis by repressing IME1 expression and promotes mitosis by activating CLN2 expression; directly repressed by a1-a2 regulator; mediates cell type control of sporulation

5879;Protein that forms a complex with Spt5p and mediates both activation and inhibition of transcription elongation, and plays a role in pre-mRNA processing; in addition, Spt4p is involved in kinetochore function and gene silencing

5880;Hypothetical protein; has similarity to Adr1p DNA-binding domain

5881;Putative protein of unknown function; deletion mutant has increased glycogen accumulation; green fluorescent protein (GFP)-fusion protein localizes to the nucleus

5882;Putative protein of unknown function; deletion mutant has increased glycogen accumulation; green fluorescent protein (GFP)-fusion protein localizes to the nucleus

5883;Hypothetical protein; open reading frame extensively overlaps essential SMD1 gene encoding a U6 snRNP protein

5884;Part of the heteromeric co-chaperone GimC/prefoldin complex, which promotes efficient protein folding

5885;Hypothetical protein

5886;Protein of unknown function; deletion mutant has synthetic fitness defect with an sgs1 deletion mutant

5887;Possible U3 snoRNP protein involved in maturation of pre-18S rRNA, based on computational analysis of large-scale protein-protein interaction data

5888;Possible U3 snoRNP protein involved in maturation of pre-18S rRNA, based on computational analysis of large-scale protein-protein interaction data

5889;Protein serine/threonine phosphatase with similarity to human phosphatase PP5; present in both the nucleus and cytoplasm; expressed during logarithmic growth

5890;Vacuolar protein sorting (VPS) protein required for cytoplasm to vacuole targeting of proteins

5891;Identified by gene-trapping, microarray-based expression analysis, and genome-wide homology searching

5892;Hypothetical protein

5893;3'5' exoribonuclease, exosome subunit; nucleolar protein involved in export of mRNA and ribosomal subunits; homologous to the E. coli exonuclease RNase PH

5894;Hypothetical protein

5895;Integral membrane protein required for the biogenesis of ER-derived COPII transport vesicles; interacts with Yiflp and Yos1p; localizes to the Golgi, the ER, and COPII vesicles

5896;Hypothetical protein; open reading frame overlaps 5' end of essential HIP1 gene encoding histidine permease

5897;Protein involved in nitrosoguanidine (MNNG) resistance; expression is regulated by transcription factors involved in multidrug resistance

5898;Putative protein of unknown function; green fluorescent protein (GFP)-fusion protein localizes to the cytoplasm in a punctate pattern; YGR198W is an essential gene

5899;Putative protein of unknown function; deletion mutant is sensitive to rapamycin and nystatin; green fluorescent protein (GFP)-fusion protein localizes to the endosome

5900;Putative protein of unknown function; green fluorescent protein (GFP)-fusion protein localizes to the cytoplasm

5901;Protein component of the small (40S) ribosomal subunit, nearly identical to Rps0Bp; required for maturation of 18S rRNA along with Rps0Bp; deletion of either RPS0 gene reduces growth rate, deletion of both genes is lethal

5902;Protein required for splicing of the COX1 intron AI5 beta; also specifically required, together with Pet122p and Pet494p, for translation of the COX3 mRNA; located in the mitochondrial inner membrane

5903;Phosphatidylinositol 3,5-bisphosphate-binding protein, predicted to fold as a seven-bladed beta-propeller; displays punctate cytoplasmic localization

5904;Activator of meiotic anaphase promoting complex (APC/C); Cdc20p family member; required for initiation of spore wall assembly; required for Clb1p degradation during meiosis

5905;Dubious open reading frame unlikely to encode a protein, based on available experimental and comparative sequence data; partially overlaps the verified ORF SMI1/YGR229C

5906;Nitric oxide oxidoreductase, flavohemoglobin involved in nitric oxide detoxification; plays a role in the oxidative and nitrosative stress responses

5907;Protein involved in clathrin cage assembly; binds Panlp and clathrin; homologous to Yap1801p, member of the AP180 protein family

5908;Dubious open reading frame unlikely to encode a protein, based on available experimental and comparative sequence data; partially overlaps the verified ORF YAP1802/YGR241C

5909;Highly conserved nuclear protein required for actin cytoskeleton organization and passage through Start, plays a critical role in G1 events, binds Naplp, also involved in 60S ribosome biogenesis

5910;6-phosphogluconate dehydrogenase (decarboxylating), catalyzes an NADPH regenerating reaction in the pentose phosphate pathway; required for growth on D-glucono-delta-lactone

5911;Single-stranded DNA endonuclease, cleaves single-stranded DNA during nucleotide excision repair to excise damaged DNA; subunit of Nucleotide Excision Repair Factor 3 (NEF3); homolog of human XPG protein

5912;Transporter, member of the ARN family of transporters that specifically recognize siderophore-iron chelates; responsible for uptake of iron bound to ferrirubin, ferrirhodin, and related siderophores

5913;Questionable ORF from MIPS

5914;Questionable ORF from MIPS

5915;ER membrane protein involved in the translocation of soluble secretory proteins and insertion of membrane proteins into the ER membrane; may also have a role in the stress response but has only partial functional overlap with WSC 1-3

5916;Putative protein of unknown function, does not appear to be involved in monocarboxylic acid transport; green fluorescent protein (GFP)-fusion protein localizes to the vacuole

5917;Questionable ORF from MIPS

5918;Essential protein that interacts with proteasome components and has a potential role in proteasome substrate specificity; also copurifies with 66S pre-ribosomal particles 5919;Questionable ORF from MIPS

5920;Hypothetical protein

5921;Hypothetical protein

5922;Hypothetical protein

5923;Hypothetical protein

5924;Hypothetical protein

5925;Meiosis specific protein involved in DMC1-dependent meiotic recombination, forms heterodimer with Mei5p; proposed to be an assembly factor for Dmc1p

5926;Substrate-specific nuclear cofactor for exosome activity in the processing of stable RNAs; homolog of mammalian nuclear matrix protein CID, which is involved in regulation of DNA repair and recombination

5927;Transcription factor that is activated by a MAP kinase signaling cascade, activates genes involved in mating or pseudohyphal/invasive growth pathways; cooperates with Tec1p transcription factor to regulate genes specific for invasive growth

5928;Transcription factor that is activated by a MAP kinase signaling cascade, activates genes involved in mating or pseudohyphal/invasive growth pathways; cooperates with Tec1p transcription factor to regulate genes specific for invasive growth

5929;Hexose transporter with moderate affinity for glucose, may function in accumulation of reserve carbohydrates during stress, expression induced by a decrease in growth rate, contains an extended N-terminal domain relative to other HXTs

5930;Endosomal protein of unknown function that contains a phox (PX) homology domain and binds to both phosphatidylinositol-3-phosphate (PtdIns(3)P) and proteins involved in ER-Golgi or vesicular transport

5931;Hypothetical protein

5932;Putative metalloprotease with similarity to the zinc carboxypeptidase family, required for normal cell wall assembly

5933;Aromatic aminotransferase, catalyzes the first step of tryptophan, phenylalanine, and tyrosine catabolism

5934;Identified by gene-trapping, microarray-based expression analysis, and genome-wide homology searching

5935;Homologous to PBI2

5936;Daughter cell-specific secreted protein with similarity to glucanases, degrades cell wall from the daughter side causing daughter to separate from mother; expression is repressed by cAMP

5937;Protein of unknown function, required for spore formation

5938;Protein required for proper cell fusion and cell morphology; functions in a complex with Kel2p to negatively regulate mitotic exit, interacts with Temlp and Ltelp; localizes to regions of polarized growth; potential Cdc28p substrate

5939;Protein required for proper cell fusion and cell morphology; functions in a complex with Kel2p to negatively regulate mitotic exit, interacts with Temlp and Ltelp; localizes to regions of polarized growth; potential Cdc28p substrate

5940;Questionable ORF from MIPS

5941;Enolase II, a phosphopyruvate hydratase that catalyzes the conversion of 2-phosphoglycerate to phosphoenolpyruvate during glycolysis and the reverse reaction during gluconeogenesis; expression is induced in response to glucose

5942;ORF, Uncharacterized

5943;ORF, Uncharacterized

5944;Hypothetical protein

5945;Alpha subunit of the heteromeric nascent polypeptide-associated complex (NAC) involved in protein sorting and translocation, associated with cytoplasmic ribosomes

5946;Putative protein of unknown function; green fluorescent protein (GFP)-fusion protein localizes to the cytoplasm; mRNA is enriched in Scp160p-associated mRNPs; YIL161 W is a non-essential gene

5947;Myosin-like protein associated with the nuclear envelope, connects the nuclear pore complex with the nuclear interior; involved in the Tel1p pathway that controls telomere length

5948;Subunit Vb of cytochrome c oxidase, which is the terminal member of the mitochondrial inner membrane electron transport chain; predominantly expressed during anaerobic growth while its isoform Va (Cox5Ap) is expressed during aerobic growth

5949;Protein of unknown function, required for survival upon exposure to K1 killer toxin; involved in proteasome-dependent catabolite inactivation of fructose-1,6-bisphosphatase; contains CTLH domain

>

5950;Questionable ORF from MIPS 5951;Questionable ORF from MIPS

5952;Ubiquitin-protein ligase of the ER/nuclear envelope, required for degradation of Alpha2p and other proteins containing a Deg1 degradation signal; ssm4 mutation suppresses mRNA instability caused by an rna14 mutation

5953;Questionable ORF from MIPS

5954;Questionable ORF from MIPS

5955;Questionable ORF from MIPS

5956;GPI-anchored cell surface glycoprotein required for diploid pseudohyphal formation and haploid invasive growth, transcriptionally regulated by the MAPK pathway (via Ste12p and Teclp) and the cAMP pathway (via Flo8p)

5957;Questionable ORF from MIPS

5958;Putative protein of unknown function; GFP-fusion protein localizes to mitochondria and native protein copurifies with mitochondria; interacts with Arhlp, a mitochondrial oxidoreductase; deletion mutant has a respiratory growth defect

5959;Hypothetical protein

5960;Hypothetical protein

5961;Hypothetical protein

5962;Hypothetical protein

5963;Hypothetical protein

5964;Hypothetical protein

5965;Mitochondrial elongation factor involved in translational elongation

5966;Mitochondrial elongation factor involved in translational elongation

5967;Putative K+/H+ antiporter

5968;Putative K+/H+ antiporter

5969;Protein of unknown function, green fluorescent protein (GFP)-fusion protein localizes to the cytoplasm and nucleus

5970;Hypothetical protein

5971; hypothetical protein

5972;Putative protein of unknown function; expression induced in respiratory-deficient petite cells, in carbon-limited chemostat cultures, during meiosis, sporulation and after alpha factor treatment; similar to adjacent ORF, YJL038C

5973;Hypothetical protein

5974;Subunit of the cytosolic chaperonin Cct ring complex, related to Tcplp, required for the assembly of actin and tubulins in vivo

5975;Component of the SSU processome, which is required for pre-18S rRNA processing, interacts with and controls the stability of Imp3p and Imp4p, essential for viability; similar to human Mpp10p

5976;ATP sulfurylase, catalyzes the primary step of intracellular sulfate activation, essential for assimilatory reduction of sulfate to sulfide, involved in methionine metabolism

5977;Hypothetical protein; open reading frame overlaps promoter and 5' end of essential GPI14 gene encoding glycosylphosphatidylinositol-alpha 1,4 mannosyltransferase I involved in GPI anchor biosynthesis

5978;Hypothetical protein

5979;Hypothetical protein

5980;Hypothetical protein

5981;Hypothetical protein; open reading frame overlaps essential LSM8 gene encoding an snRNP protein required for RNA processing and splicing

5982;Helix-loop-helix protein that binds the motif CACRTG, which is present at several sites including MET gene promoters and centromere DNA element I (CDEI); required for nucleosome positioning at this motif; targets Isw1p to DNA 5983;Protein involved in bud-site selection and required for axial budding pattern; localizes with septins to bud neck in mitosis and may constitute an axial landmark for next round of budding; potential Cdc28p substrate

5984;Protein of unknown function, required for normal microtubule organization

5985;Protein of unknown function; may be involved in survival during stationary phase; substrate for the N-myristoyltransferase Nmt1p

5986;Protein of unknown function; may be involved in survival during stationary phase; substrate for the N-myristoyltransferase Nmt1p

5987;Protein of unknown function; may be involved in mitochondrial DNA maintenance; required for slowed DNA synthesis-induced filamentous growth

5988;Hypothetical protein

5989;Putative protein of unknown function; green fluorescent protein (GFP)-fusion protein localizes to the cytoplasm

5990;Putative protein of unknown function; green fluorescent protein (GFP)-fusion protein localizes to the cytoplasm

5991;Putative aryl-alcohol dehydrogenase with similarity to P. chrysosporium aryl-alcohol dehydrogenase; mutational analysis has not yet revealed a physiological role

5992;Protein of unknown function with similarity to hexose transporter family members, expression is repressed by high levels of glucose

5993;Putative protein of unknown function; green fluorescent protein (GFP)-fusion protein localizes to the cytoplasm

5994;Putative positive regulator of mannosylphosphate transferase (Mnn6p), involved in mannosylphosphorylation of N-linked oligosaccharides; expression increases in late-logarithmic and stationary growth phases

5995;Essential protein required for the maturation of 25S rRNA and 60S ribosomal subunit assembly, localizes to the nucleolus; constituent of 66S pre-ribosomal particles

5996;Essential protein required for the maturation of 25S rRNA and 60S ribosomal subunit assembly, localizes to the nucleolus; constituent of 66S pre-ribosomal particles

5997;Essential protein required for the maturation of 25S rRNA and 60S ribosomal subunit assembly, localizes to the nucleolus; constituent of 66S pre-ribosomal particles

5998;RNA lariat debranching enzyme, involved in intron turnover; required for efficient Ty1 transposition

5999;Dubious open reading frame, unlikely to encode a protein; not conserved in closely related Saccharomyces species; partially overlaps the verified gene AVT3

6000;One of six ATPases of the 19S regulatory particle of the 26S proteasome involved in the degradation of ubiquitinated substrates; required for optimal CDC20 transcription; interacts with Rpn12p and the E3 ubiquitin-protein ligase Ubr1p

6001;Protein required for growth at low temperature

6002;Protein required for growth at low temperature

6003;Hypothetical protein

6004;Putative protein of unknown function

6005;Putative protein of unknown function

6006;Putative protein of unknown function; identified by homology to uncharacterized proteins in other fungi

6007;Predicted phosphopantothenoylcysteine decarboxylase, may be involved in coenzyme A biosynthesis; interacts with Sis2p and Vhs3p

6008;Predicted phosphopantothenoylcysteine decarboxylase, may be involved in coenzyme A biosynthesis; interacts with Sis2p and Vhs3p

6009;Essential protein, constituent of 66S pre-ribosomal particles; interacts with proteins involved in ribosomal biogenesis and cell polarity; member of the SURF-6 family

6010;Subunit C of the eight-subunit V1 peripheral membrane domain of vacuolar H+-ATPase (V-ATPase), an electrogenic proton pump found throughout the endomembrane system; required for the V1 domain to assemble onto the vacuolar membrane

6011;Protein involved in early pre-mRNA splicing; component of the pre-mRNA-U1 snRNP complex, the commitment complex; interacts with Ms15p/BBP splicing factor and Sub2p; similar to metazoan splicing factor U2AF65

6012;Protein involved in early pre-mRNA splicing; component of the pre-mRNA-U1 snRNP complex, the commitment complex; interacts with Ms15p/BBP splicing factor and Sub2p; similar to metazoan splicing factor U2AF65

6013;RNAPII degradation factor, forms a complex with Rad26p in chromatin, enables ubiquitination and proteolysis of RNAPII present in an elongation complex

6014;Centromere protein that resembles histones, required for proper kinetochore function; homolog of human CENP-A

6015;Subunit of DNA primase, which is required for DNA synthesis and double-strand break repair

6016;Dubious open reading frame, unlikely to encode a protein; not conserved in closely related Saccharomyces species; partially overlaps the essential gene UGP1 gene and uncharacterized ORF YKL037W

6017;GTPase, similar to Ypt51p and Ypt53p and to mammalian rab5; required for vacuolar protein sorting and endocytosis

6018;Putative protein of unknown function

6019;Protein that forms a complex with the Sit4p protein phosphatase and is required for its function; member of a family of similar proteins including Sap4p, Sap155p, and Sap185p

6020;Phospholipase D, catalyzes the hydrolysis of phosphatidylcholine, producing choline and phosphatidic acid; involved in Sec14p-independent secretion; required for meiosis and spore formation; differently regulated in secretion and meiosis

6021;Negative regulator of genes in multiple nitrogen degradation pathways; expression is regulated by nitrogen levels and by Gln3p; member of the GATA-binding family, forms homodimers and heterodimers with Deh1p

6022;ORF, Dubious

6023;ORF, Dubious

6024;Component of Dam1p complex, important for spindle and kinetochore integrity; localized to nuclear side of spindle pole body and along mitotic spindle

6025;Hypothetical protein

6026;Myosin-like protein associated with the nuclear envelope, connects the nuclear pore complex with the nuclear interior; involved with Tel1p in telomere length control; involved with Pml1p and Pml39p in nuclear retention of unspliced mRNAs

6027;Lectin-like protein with similarity to Flo1p, thought to be involved in flocculation

6028;Lectin-like protein with similarity to Flo1p, thought to be involved in flocculation

6029;Hypothetical protein

6030;E2-like conjugating enzyme that mediates formation of the Atg12p-Atg5p conjugate, which is a critical step in autophagy

6031;Protein required for cytochrome c oxidase assembly, located in the cytosol and mitochondrial intermembrane space; putative copper metallochaperone that delivers copper to cytochrome c oxidase

6032;Essential subunit of the U3 (box C+D) snRNP complex required for 2' O-methylation of pre-RNA; has similarity to the beta subunit of trimeric G-proteins and the splicing factor Prp4p

6033;Essential subunit of the U3 (box C+D) snRNP complex required for 2' O-methylation of pre-RNA; has similarity to the beta subunit of trimeric G-proteins and the splicing factor Prp4p

6034;Subunit of the COMPASS (Set1C) complex, which methylates histone H3 on lysine 4 and is required in transcriptional silencing near telomeres; has similarity to the trithorax-group protein ASH2L

6035;Cytoplasmic ubiquitin-protein ligase (E3)

6036;Cytoplasmic ubiquitin-protein ligase (E3)

6037;Hypothetical protein

6038;Protein required for fermentation at low temperature

6039;Subunit of the GPI (glycosylphosphatidylinositol):protein transamidase complex, removes the GPI-anchoring signal and attaches GPI to proteins in the ER

6040;Cell wall protein, mutants are defective in mating and agglutination, expression is downregulated by alpha-factor

6041;Mitogen-activated protein kinase involved in osmoregulation via three independent osmosensors; mediates the recruitment and activation of RNA Pol II at Hotlp-dependent promoters; localization regulated by Ptp2p and Ptp3p

6042;Mutation is syntheticly lethal with apl2 vps1 double mutant

6043;Essential splicesome assembly factor; contains multiple tetratricopeptide repeat (TPR) protein-binding motifs and interacts specifically with many spliceosome components, may serve as a scaffold during splicesome assembly

6044;Essential splicesome assembly factor; contains multiple tetratricopeptide repeat (TPR) protein-binding motifs and interacts specifically with many spliceosome components, may serve as a scaffold during splicesome assembly

6045;Essential splicesome assembly factor; contains multiple tetratricopeptide repeat (TPR) protein-binding motifs and interacts specifically with many spliceosome components, may serve as a scaffold during splicesome assembly

6046;YLR164Wp is homologous to TIM18p

6047;Cytosolic NADP-specific isocitrate dehydrogenase, catalyzes oxidation of isocitrate to alpha-ketoglutarate; levels are elevated during growth on non-fermentable carbon sources and reduced during growth on glucose

6048;Multivesicular body (MVB) protein involved in endosomal protein sorting; binds to Vps20p and Vps4p; may regulate Vps4p function; bindsVps60p and may act at a late step in MVB formation; mutants show class E vacuolar-protein sorting defects

6049;Half-type ATP-binding cassette (ABC) transporter of the inner mitochondrial membrane, mediates export of peptides generated upon proteolysis of mitochondrial proteins, plays a role in the regulation of cellular resistance to oxidative stress

6050;Hypothetical protein

6051;Mitochondrial protein of unknown function

6052;Mitochondrial protein of unknown function

6053;Putative glycoside hydrolase of the spore wall envelope; required for normal spore wall assembly, possibly for cross-linking between the glucan and chitosan layers; expressed during sporulation

6054;Structural component of the meiotic outer plaque, which is a membrane-organizing center that assembles on the cytoplasmic face of the spindle pole body during meiosis II and triggers the formation of the prospore membrane

6055;Hypothetical protein

6056;Hypothetical protein

6057;Protein of unknown function, overproduction suppresses a pam1 slv3 double null mutation

6058;Hypothetical protein

6059;Protein of unknown function, localizes to the nucleus; potential Cdc28p substrate

6060;Hypothetical protein

6061;Hypothetical protein

6062;Hypothetical protein

6063;Hypothetical protein

6064;Protein component of the large (60S) ribosomal subunit, has similarity to rat L38 ribosomal protein

6065;Hypothetical protein

6066;Protein required for synaptonemal complex formation, may have a role in meiotic recombination; localizes to synapsis initiation sites on meiotic chromosomes; potential Cdc28p substrate

6067;Transcription factor that controls expression of many ribosome biogenesis genes in response to nutrients and stress, regulates G2/M transitions during mitotic cell cycle and DNA-damage response, involved in cell size modulation

6068;Transcription factor that controls expression of many ribosome biogenesis genes in response to nutrients and stress, regulates G2/M transitions during mitotic cell cycle and DNA-damage response, involved in cell size modulation

6069;Identified by fungal homology and RT-PCR

6070;Protein of unknown function; green fluorescent protein (GFP)-fusion protein localizes to the cell periphery

6071;Peripheral membrane protein, required for autophagy and for the cytoplasm-to-vacuole targeting (Cvt) pathway

6072;Identified by gene-trapping, microarray-based expression analysis, and genome-wide homology searching

6073;L-ornithine transaminase (OTAse), catalyzes the second step of arginine degradation, expression is dually-regulated by allophanate induction and a specific arginine induction process; not nitrogen catabolite repression sensitive

6074;Hypothetical protein

6075;One of two orotate phosphoribosyltransferase isozymes (see also URA10) that catalyze the fifth enzymatic step in de novo biosynthesis of pyrimidines, converting orotate into orotidine-5'-phosphate

6076;Hypothetical protein

6077;Protein component of mitochondrial RNase P, along with the mitochondrially-encoded RNA subunit RPM1; Rnase P removes 5' extensions from tRNA precursors; Rpm2p is also involved in maturation of RPM1 and in translation of mitochondrial mRNAs

6078;Protein component of mitochondrial RNase P, along with the mitochondrially-encoded RNA subunit RPM1; Rnase P removes 5' extensions from tRNA precursors; Rpm2p is also involved in maturation of RPM1 and in translation of mitochondrial mRNAs

6079;F-box receptor protein, subunit of the Skp1-Cdc53-F-box receptor (SCF) E3 ubiquitin ligase complex; binds to phosphorylated Ho endonuclease, allowing its ubiquitylation by SCF and subsequent degradation

6080;One of two isozymes of HMG-CoA reductase that catalyzes the conversion of HMG-CoA to mevalonate, which is a rate-limiting step in sterol biosynthesis; localizes to the nuclear envelope; overproduction induces the formation of karmellae

6081;Dihydroxyacetone kinase, required for detoxification of dihydroxyacetone (DHA); involved in stress adaptation

6082;Hypothetical protein

6083;Questionable ORF from MIPS

6084;tRNA methyltransferase, catalyzes the esterification of modified uridine nucleotides in tRNAs, creating 5-methylcarbonylmethyluridine in tRNA(Arg3) and 5-methylcarbonylmethyl-2-thiouridine in tRNA(Glu); may have a role in stress response

6085;Hypothetical protein

6086;Phospholipase B (lysophospholipase) involved in phospholipid metabolism; displays transacylase activity in vitro; overproduction confers resistance to lysophosphatidylcholine

6087;Hypothetical protein

6088;Hypothetical protein

6089;Protein required for expression of the mitochondrial OLI1 gene encoding subunit 9 of F1-FO ATP synthase

6090;Component of a complex containing the Tor2p kinase and other proteins, which may have a role in regulation of cell growth

6091;Serine-rich, hydrophilic protein with similarity to Mbrlp; overexpression suppresses growth defects of hap2, hap3, and hap4 mutants; expression is under glucose control; cotranscribed with NAM7 in a cyp 1 mutant

6092;Protein of unknown function; green fluorescent protein (GFP)-fusion protein localizes to the cell periphery

6093;Hypothetical protein

6094;Protein component of the large (60S) ribosomal subunit, nearly identical to Rpl15Ap and has similarity to rat L15 ribosomal protein; binds to 5.8 S rRNA

6095;Hypothetical protein

6096;Hypothetical protein

6097;Hypothetical protein

6098;Protein required for normal transcription at several loci including HTA2-HTB2 and HHF2-HHT2, but not required at the other histone loci; functionally related to Spt10p

6099;RNA 5'-triphosphatase, localizes to both the nucleus and cytoplasm

6100;Nucleolar DNA helicase of the RecQ family involved in maintenance of genome integrity, regulates chromosome synapsis and meiotic crossing over; has similarity to human BLM and WRN helicases implicated in Bloom and Werner syndromes

6101;Phosphomevalonate kinase), an essential cytosolic enzyme that acts in the biosynthesis of isoprenoids and sterols, including ergosterol, from mevalonate

6102;Hypothetical protein

6103;Coiled-coiled protein of unknown function, identified as a high-copy suppressor of a dbp5 mutation

6104;Hypothetical protein

6105;Identified by gene-trapping, microarray-based expression analysis, and genome-wide homology searching

6106;Protein that interacts with silencing proteins at the telomere, involved in transcriptional silencing; has a role in localization of Bcy1p, a regulatory subunit of protein kinase A; implicated in mRNA nuclear export

6107;2'-O-ribosyl phosphate transferase, modifies the initiator methionine tRNA at position 64 to distinguish it from elongator methionine tRNA

6108;Protein involved in 5.8S rRNA processing; Ccr4p-like RNase required for correct 3'-end formation of 5.8S rRNA at site E; similar to Ngl1p and Ngl3p

6109;Component of the yeast dynactin complex, consisting of Nip100p, Jnm1p, and Arp1p; required for proper nuclear migration and spindle partitioning during mitotic anaphase B

6110;Putative aryl-alcohol dehydrogenase with similarity to P. chrysosporium aryl-alcohol dehydrogenase; mutational analysis has not yet revealed a physiological role

6111;Glycosylphosphatidylinositol (GPI)-anchored cell wall endoglucanase required for proper cell separation after cytokinesis, expression is activated by Swi5p and tightly regulated in a cell cycle-dependent manner

6112;Hypothetical protein

6113;Hypothetical protein

6114;GTPase-activating protein for Sec4p and several other Rab GTPases, regulates exocytosis via its action on Sec4p, also required for proper actin organization; similar to Msb4p; both Msb3p and Msb4p localize to sites of polarized growth

6115;Subunit of heteropentameric Replication factor C (RF-C), which is a DNA binding protein and ATPase that acts as a clamp loader of the proliferating cell nuclear antigen (PCNA) processivity factor for DNA polymerases delta and epsilon

6116;Subunit of both RNase MRP, which cleaves pre-rRNA, and nuclear RNase P, which cleaves tRNA precursors to generate mature 5' ends

6117;Hypothetical protein

6118;Subtilisin-like protease (proprotein convertase), a calcium-dependent serine protease involved in the activation of proproteins of the secretory pathway

6119;Hypothetical protein

6120;Phosphatidylinositol transfer protein (PITP) controlled by the multiple drug resistance regulator Pdr1p, localizes to lipid particles and microsomes, controls levels of various lipids, may regulate lipid synthesis, homologous to Pdr17p

6121;Nuclear protein of unknown function; deletion results in sensitivity to anticancer drugs oxaliplatin and cisplatin, but not mitomycin C; deletion is synthetically lethal with a chitin synthase (CHS1) null mutant

6122;Hypothetical protein

6123;Chitin synthase I, requires activation from zymogenic form in order to catalyze the transfer of N-acetylglucosamine (GlcNAc) to chitin; required for repairing the chitin septum during cytokinesis; transcription activated by mating factor

6124;Karyopherin alpha homolog, forms a dimer with karyopherin beta Kap95p to mediate import of nuclear proteins, binds the nuclear localization signal of the substrate during import; may also play a role in regulation of protein degradation

6125;Essential protein involved in karyogamy during mating and in spindle pole body duplication during mitosis, localizes to the half-bridge of the spindle pole body, interacts with Spc72p during karyogamy, also interacts with Cdc31p

6126;Protein kinase that stabilizes several plasma membrane amino acid transporters by antagonizing their ubiquitin-mediated degradation

6127;Hypothetical protein

6128;Hypothetical protein

6129;Hypothetical protein; open reading frame extensively overlaps essential RPC19 gene encoding an RNA polymerase subunit

>

6130;Hypothetical protein 6131;Hypothetical protein

6132;Protein localized in the bud neck at G2/M phase; physically interacts with septins; possibly involved in a mitotic signaling network

6133;Putative chaperone of the HSP40 (DNAJ) family; overexpression interferes with propagation of the [Psi+] prion

6134;Cell wall protein related to glucanases, possibly involved in cell wall septation; member of the SUN family

6135;S-adenosylmethionine-dependent Methyltransferase; methylates translational release factor Mrf1p; similar to E.coli PrmC; is not an essential gene

6136;Subunit of tRNA (1-methyladenosine) methyltransferase with Gcd14p, required for the modification of the adenine at position 58 in tRNAs, especially tRNAi-Met; first identified as a negative regulator of GCN4 expression

6137;Probable RNA m(5)C methyltransferase, essential for processing and maturation of 27S pre-rRNA and large ribosomal subunit biogenesis; localized to the nucleolus; constituent of 66S pre-ribosomal particles

>

613 hypothetical protein 6139;Hypothetical protein

6140;Hypothetical protein

6141 hypothetical protein

6142;Hypothetical protein

6143;MAP kinase kinase kinase of the HOG1 mitogen-activated signaling pathway; interacts with Ssk1p, leading to autophosphorylation and activation of Ssk2p which phosphorylates Pbs2p; also mediates actin cytoskeleton recovery from osmotic stress

6144;Hypothetical proteinj 6145;Component of the Paflp complex, which is a large complex that binds to and modulates the activity of RNA polymerase II and is required for expression of a subset of genes, including cyclin genes; contains TPR repeats

6146;Hypothetical protein; open reading frame extensively overlaps essential HRT1 gene encoding a subunit of Skip1-Cullin-F-box ubiquitin protein ligases

6147;Cytoplasmic malate dehydrogenase, one of the three isozymes that catalyze interconversion of malate and oxaloacetate; involved in gluconeogenesis during growth on ethanol or acetate as carbon source; interacts with Pck1p and Fbp1p

6148;Cytoplasmic malate dehydrogenase, one of the three isozymes that catalyze interconversion of malate and oxaloacetate; involved in gluconeogenesis during growth on ethanol or acetate as carbon source; interacts with Pck1p and Fbp1p

6149;Membrane protein involved in zinc metabolism, member of the four-protein IZH family, expression induced by fatty acids and altered zinc levels; deletion reduces sensitivity to excess zinc; possible role in sterol metabolism

6150;Hypothetical protein identified by homology. See FEBS Letters [2000] 487:31-36.

6151 hypothetical protein

6152;Bisphosphate-3'-nucleotidase, involved in salt tolerance and methionine biogenesis; dephosphorylates 3'-phosphoadenosine-5'-phosphate and 3'-phosphoadenosine-5'-phosphosulfate, intermediates of the sulfate assimilation pathway

6153;Protein required for normal mitochondrial morphology, has similarity to hemolysins

6154;NAD-dependent glycerol 3-phosphate dehydrogenase, homolog of Gpd1p, expression is controlled by an oxygen-independent signaling pathway required to regulate metabolism under anoxic conditions; located in cytosol and mitochondria

6155;S-adenosylmethionine decarboxylase, required for the biosynthesis of spermidine and spermine; cells lacking Spe2p require spermine or spermidine for growth in the presence of oxygen but not when grown anaerobically

6156;One of two (see also PSK1) PAS domain containing S/T protein kinases; regulates sugar flux and translation in response to an unknown metabolite by phosphorylating Ugp1p and Gsy2p (sugar flux) and Caf20p, Tif11p and Sro9p (translation)

6157;Hypothetical protein

6158;Cell wall mannoprotein of the Srplp/Tiplp family of serine-alanine-rich proteins; expressed under anaerobic conditions and required for anaerobic growth; transcription is also induced by cold shock

6159;Hypothetical protein

6160;5'-3' exonuclease and flap-endonuclease involved in recombination, double-strand break repair and DNA mismatch repair; member of the Rad2p nuclease family, with conserved N and I nuclease domains

6161;5'-3' exonuclease and flap-endonuclease involved in recombination, double-strand break repair and DNA mismatch repair; member of the Rad2p nuclease family, with conserved N and I nuclease domains

6162;Ankyrin repeat-containing protein similar to Akr1p; member of a family of putative palmitoyltransferases containing an Asp-His-His-Cys-cysteine rich (DHHC-CRD) domain; possibly involved in constitutive endocytosis of Ste3p

6163;Mitochondrial protein required for normal abundance of mitochondrial cytochrome c (Cyc1p) and for mitochondrial osmotic stability; may be involved in regulating the activity of cytochrome c heme lyase (Cyc3p); potential Cdc28p substrate

6164;Glucosyl transferase, involved in N-linked glycosylation; adds glucose to the dolichol-linked oligosaccharide precursor prior to transfer to protein during lipid-linked oligosaccharide biosynthesis; similar to Alg6p

6165;Nexin-1 homolog required for localizing membrane proteins from a prevacuolar/late endosomal compartment back to the late Golgi apparatus; structural component of the retromer membrane coat complex; forms a retromer subcomplex with Vps17p

6166;Cis-golgi GTPase-activating protein (GAP) for the Rab family members Ypt1p (in vivo) and for Ypt1p, Sec4p, Ypt7p, and Ypt51p (in vitro); involved in vesicle docking and fusion

6167;Component of small ribosomal subunit (SSU) processosome that contains U3 snoRNA; originally isolated as bud-site selection mutant that displays a random budding pattern

6168;Component of small ribosomal subunit (SSU) processosome that contains U3 snoRNA; originally isolated as bud-site selection mutant that displays a random budding pattern

6169;Protein that regulates the critical cell size required for passage through Start and commitment to cell division; may act upstream of SCB binding factor (SBF) and MCB binding factor (MBF); periodically expressed in G1

6170;Syntaxin-related protein required for vacuolar assembly; functions with Vam7p in vacuolar protein trafficking; member of the syntaxin family of proteins

6171;Elongation factor 2 (EF-2), also encoded by EFT2; catalyzes ribosomal translocation during protein synthesis; contains diphthamide, the unique posttranslationally modified histidine residue specifically ADP-ribosylated by diphtheria toxin

6172;Rho GTPase activating protein (RhoGAP), stimulates the intrinsic GTPase activity of Rholp, which plays a role in actin cytoskeleton organization and control of cell wall synthesis; structurally and functionally related to Sac7p

6173;Hypothetical protein

6174;Hypothetical protein

6175;Transcription repressor involved in regulation of flocculation-related genes, inhibits transcription by recruiting general corepressor Cyc8p-Tup1p to different promoters; negatively regulated by cAMP-dependent protein kinase A subunit Tpk2p

6176;Component of the meiotic outer plaque, a membrane-organizing center which is assembled on the cytoplasmic face of the spindle pole body during meiosis II and triggers the formation of the prospore membrane; potential Cdc28p substrate

6177;Regulatory subunit for Glc7p type-1 protein phosphatase (PP1), tethers Glc7p to Gsy2p glycogen synthase, binds Hsflp heat shock transcription factor, required for induction of some HSF-regulated genes under heat shock

6178;Kinetochore-associated protein required for normal segregation of chromosomes in meiosis and mitosis; component of the FEAR regulatory network, which promotes Cdc14p release from the nucleolus during anaphase; potential Cdc28p substrate

6179;Subunit of the SAS complex (Sas2p, Sas4p, Sas5p), which acetylates free histones and nucleosomes and regulates transcriptional silencing; stimulates Sas2p HAT activity

6180;Subunit of heteropentameric Replication factor C (RF-C), which is a DNA binding protein and ATPase that acts as a clamp loader of the proliferating cell nuclear antigen (PCNA) processivity factor for DNA polymerases delta and epsilon

6181;Hypothetical protein; open reading frame overlaps 3' end of essential RFC1 gene encoding a replication factor complex subunit

6182;Protein involved in nuclear migration, part of the dynein/dynactin pathway; targets dynein to microtubule tips, which is necessary for sliding of microtubules along bud cortex; synthetic lethal with bni1; homolog of human LIS 1

6183;Hypothetical protein; open reading frame overlaps 5' end of essential PLP2 gene

6184;Component of the U4/U6.U5 snRNP complex involved in pre-mRNA splicing via spliceosome; has homology to human SART-1 and to an S. pombe protein; snu66 null mutation confers cold-sensitivity but is not lethal at normal growth temperatures

6183;Component of the U4/U6.U5 snRNP complex involved in pre-mRNA splicing via spliceosome; has homology to human SART-1 and to an S. pombe protein; snu66 null mutation confers cold-sensitivity but is not lethal at normal growth temperatures

6186;Endoplasmic reticulum (ER)-resident membrane protein, overproduction induces enlargement of ER-like membrane structures and suppresses a temperature-sensitive sly1 mutation

6187;Hypothetical protein

6188;Hypothetical protein

6189;Hypothetical protein

6190;Hypothetical protein

6191;Putative metal transporter involved in mitochondrial iron accumulation; closely related to Mmt1p

6192;Hydrophilin of unknown function; stress induced (osmotic, ionic, oxidative, heat shock and heavy metals); regulated by the HOG pathway

6193;N-terminally acetylated protein component of the large (60S) ribosomal subunit, nearly identical to Rpl1Bp and has similarity to E. coli L1 and rat L10a ribosomal proteins; rpl1a rpl1b double null mutation is lethal

6194;GTPase, GTP-binding protein of the ARF family, component of COPII coat of vesicles; required for transport vesicle formation during ER to Golgi protein transport

6195;Hypothetical protein

6196;Hypothetical protein

6197;Hypothetical protein

6198;Hypothetical protein

6199;Hypothetical proteinj 6200;Delta adaptin-like subunit of the clathrin associated protein complex (AP-3); functions in transport of alkaline phosphatase to the vacuole via the alternate pathway, suppressor of loss of casein kinase 1 function

6201;Widely conserved NADPH oxidoreductase containing flavin mononucleotide (FMN), homologous to Oye2p with slight differences in ligand binding and catalytic properties; may be involved in sterol metabolism

6202;Widely conserved NADPH oxidoreductase containing flavin mononucleotide (FMN), homologous to Oye2p with slight differences in ligand binding and catalytic properties; may be involved in sterol metabolism

6203;Protein involved in DNA mismatch repair; forms a complex with Mlh1p to promote meiotic crossing-over; mammalian homolog is implicated mammalian microsatellite instability

6204;Hypothetical protein

6205;Hypothetical protein

6206;Mitochondrial inner membrane transporter, exports 2-oxoadipate and 2-oxoglutarate from the mitochondrial matrix to the cytosol for use in lysine and glutamate biosynthesis and in lysine catabolism

6207;RNAse; member of the T(2) family of endoribonucleases

6208;Conserved peptide N-glycanase required for deglycosylation of misfolded glycoproteins during proteasome-dependent degradation, localizes to the cytoplasm and nucleus, interacts with the DNA repair protein Rad23p

6209;Conserved peptide N-glycanase required for deglycosylation of misfolded glycoproteins during proteasome-dependent degradation, localizes to the cytoplasm and nucleus, interacts with the DNA repair protein Rad23p

6210;Protein component of the small (40S) ribosomal subunit; identical to Rps6Bp and has similarity to rat S6 ribosomal protein

6211;COPII vesicle coat protein required for ER transport vesicle budding and autophagosome formation; Sec16p is bound to the periphery of ER membranes and may act to stabilize initial COPII complexes; interacts with Sec23p, Sec24p and See31p

6212;essential abundant protein involved in regulation of transcription, removes Spt15p (TBP) from DNA via its C-terminal ATPase activity, forms a complex with TBP that binds TATA DNA with high affinity but with altered specificity

6213;Hypothetical protein

6214;Hypothetical protein

6215;Regulatory protein of unknown function, constitutively-expressed, involved in the regulation of mating-specific genes and the invasive growth pathway, required for MAP-kinase imposed repression, inhibits pheromone-responsive transcription

6216;Regulatory protein of unknown function, constitutively-expressed, involved in the regulation of mating-specific genes and the invasive growth pathway, required for MAP-kinase imposed repression, inhibits pheromone-responsive transcription

6217;Hypothetical protein

6218;Plasma membrane H+-ATPase, isoform of Pmalp, involved in pumping protons out of the cell; regulator of cytoplasmic pH and plasma membrane potential

6219;Isozyme of methylenetetrahydrofolate reductase, catalyzes the reduction of 5,10-methylenetetrahydrofolate to 5-methyltetrahydrofolate in the methionine biosynthesis pathway

6220;Hypothetical protein identified by homology. See FEBS Letters [2000] 487:31-36.

6221;Largest subunit (p90) of the Chromatin Assembly Complex (CAF-I) with Cac2p and Msilp that assembles newly synthesized histones onto recently replicated DNA; involved in the maintenance of transcriptionally silent chromatin

6222;Hypothetical protein

6223;Hypothetical protein

6224;Hypothetical protein

6225;Hypothetical protein identified by homology. See FEBS Letters [2000] 487:31-36.

6226;Protein of unknown function, overproduction blocks cell cycle arrest in the presence of mating pheromone

6227;Translational elongation factor EF-1 alpha; also encoded by TEF2; functions in the binding reaction of aminoacyl-tRNA (AA-tRNA) to ribosomes

6228;Zinc cluster protein involved in pre-mRNA splicing and cycloheximide resistance

6229;component of NineTeen complex (NTC) containing Prp19p involved in mRNA splicing, interacts physically and genetically with Prp19p

6230;Essential conserved protein that associates with 35S precursor rRNA and is required for its initial processing at the A(0)-A(2) cleavage sites, shows partial nucleolar localization, contains five consensus RNA-binding domains

623 hypothetical protein

6232;Cytoplasmic protein of unknown function involved in vacuolar protein sorting.

6233;Hypothetical protein; open reading frame overlaps 5' end of essential RRP15 gene required for ribosomal RNA processing

6234;Identified by fungal homology and RT-PCR

6235;Identified by expression profiling and mass spectrometry

6236;Subunit of a possibly tetrameric trichostatin A-sensitive class II histone deacetylase complex that contains an Hda1p homodimer and an Hda2p-Hda3p heterodimer; required for the activity of the complex; has similarity to Hda2p

### (SEQ ID NOs.: 166154-166181)

166154;Subunit I of cytochrome c oxidase, which is the terminal member of the mitochondrial inner membrane electron transport chain; one of three mitochondrially-encoded subunits

166155;Reverse transcriptase required for splicing of the COX1 pre-mRNA, encoded by a mobile group II intron within the mitochondrial COX1 gene

166156;Cytochrome b, mitochondrially encoded subunit of the ubiquinol-cytochrome c reductase complex which includes Cobp, Riplp, Cytlp, Corlp, Qcr2p, Qcr6p, Qcr7p, Qcr8p, Qcr9p, and Qcr10p

166157;Subunit 8 of the F0 sector of mitochondrial inner membrane F1-F0 ATP synthase, encoded on the mitochondrial genome

166158;Mitochondrially encoded subunit 6 of the F0 sector of mitochondrial F1F0 ATP synthase, which is a large, evolutionarily conserved enzyme complex required for ATP synthesis

166159;F0-ATP synthase subunit 9 (ATPase-associated proteolipid), encoded on the mitochondrial genome; mutation confers oligomycin resistance; expression is specifically dependent on the nuclear genes AEP1 and AEP2

166160;Mitochondrial ribosomal protein of the small subunit, mitochondrially-encoded; polymorphic in different strains due to variation in number of AAT (asparagine) codons; translated near the mitochondrial inner membrane

166161;Subunit III of cytochrome c oxidase, which is the terminal member of the mitochondrial inner membrane electron transport chain; one of three mitochondrially-encoded subunits

166162;Intron within the mitochondrial COX1 gene

166163;Intron within the mitochondrial COX1 gene

166164;Intron within the mitochondrial COX1 gene

166165;Intron within the mitochondrial COB1 gene

166166;Hypothetical protein

166167;Identified by MPSS

166168;Identified by MPSS

166169;Identified by MPSS

166170;Identified by MPSS

166171;Identified by MPSS

166172;Identified by MPSS

166173;Identified by MPSS

166174;Identified by MPSS

166175;Identified by MPSS

166176;Identified by MPSS

166177;Identified by MPSS

166178;Identified by MPSS

166179;Identified by MPSS

166180;Identified by MPSS

166181;Identified by MPSS

### Industrial Applicability

Since the method of the present invention allows efficient identification of genes associated with desired brewing characteristics and proteins encoded by the genes, the present method is useful for comprehensive function analysis of translation products of the genes contained in the genome of the bottom fermenting yeast.

The present invention is also useful for the production of alcohol or alcoholic beverages having improved productivity and quality, since the present invention allows control of brewing characteristics of the yeast by genetically disrupting the gene and/or by making the gene to be expressed in a high level.

## Claims

1. A method for identifying a target protein of yeast or a gene encoding the target protein comprising the steps of:
(A) a step of determining the amino acid sequence of the target protein selected from the following steps (A1) to (A4):
Step (A1)
(1) cultivating yeast under a predetermined cultivation condition;
(2) extracting a protein sample from the cultivation product of the yeast;
(3) separating the protein sample by a protein separation means, selecting a target peak or spot, and recovering the target protein or a fragment thereof contained in the peak or spot; and
(4) determining the amino acid sequence of the target protein;
Step (A2)
(1) cultivating yeast under a predetermined cultivation condition;
(2) extracting a protein sample from cultivated products of the yeast; and
(3) determining the amino acid sequence of the one or more proteins contained in the protein sample;
Step (A3)
(1) cultivating the same strain of yeast under different cultivation conditions;
(2) extracting a protein sample from each cultivation product obtained in step (1);
(3) analyzing the protein sample and identifying a highly expressed or low expressed protein under each cultivation condition; and
(4) determining the amino acid sequence of the protein identified in step (3);
Step (A4)
(1) cultivating different strains of yeast under the same cultivation condition;
(2) extracting a protein sample from each cultivation product obtained in step (1);
(3) analyzing the protein sample and identifying a protein whose expression level is different in each cultivation product; and
(4) determining the amino acid sequence of the protein identified in step (3);
(B) comparing the amino acid sequence determined in step (A) with the amino acid sequence determined in advance based on all or a part of genome sequence information of bottom fermenting yeast;
(C) identifying the gene encoding the target protein based on the results of comparison; and
(D) analyzing functions of the identified gene to identify characters given to the yeast by the gene;
wherein all or a part of the genome sequence information of the bottom fermenting yeast comprises two or more nucleotide sequences of:
SEQ ID Nos.: 33 to 6236,
SEQ ID Nos.: 75337 to 82784,
SEQ ID Nos.: 166154 to 166181,
SEQ ID Nos.: 166490 to 167042; and
SEQ ID Nos.: 173125 to 174603.

2. A method for identifying a target protein of yeast or a gene encoding the target protein comprising the steps of:
(1) referring to a database comprising all or a part of genome sequence information of bottom fermenting yeast based on the amino acid sequence of the target protein of the yeast;
(2) identifying a gene encoding the target protein based on the result of reference; and
(3) analyzing functions of the identified gene to identify characters given to the yeast by the gene;
wherein all or a part of the genome sequence information of the bottom fermenting yeast comprises two or more nucleotide sequences of:
SEQ ID Nos.: 33 to 6236,
SEQ ID Nos.: 75337 to 82784,
SEQ ID Nos.: 166154 to 166181,
SEQ ID Nos.: 166490 to 167042; and
SEQ ID Nos.: 173125 to 174603.

3. The method according to claim 2, wherein the amino acid sequence of the target protein is obtained by separating one or more proteins from a protein extract derived from the yeast and determining the amino acid sequences of the one or more proteins.

4. The method according to any one of Claims 1 to 3, wherein all or a part of the genome sequence information of the bottom fermenting yeast comprises 40 or more nucleotide sequences selected from:
SEQ ID Nos.: 33 to 6236,
SEQ ID Nos.: 75337 to 82784,
SEQ ID Nos.: 166154 to 166181,
SEQ ID Nos.: 166490 to 167042; and
SEQ ID Nos.: 173125 to 174603.

5. The method according to any one of Claims 1 to 3, wherein all or a part of the genome sequence information of the bottom fermenting yeast comprises 50 or more nucleotide sequences selected from:
SEQ ID Nos.: 33 to 6236,
SEQ ID Nos.: 75337 to 82784,
SEQ ID Nos.: 166154 to 166181,
SEQ ID Nos.: 166490 to 167042; and
SEQ ID Nos.: 173125 to 174603.

6. The method according to any one of Claims 1 to 3, wherein all or a part of the genome sequence information of the bottom fermenting yeast comprises the nucleotide sequences selected from:
SEQ ID Nos.: 33 to 6236,
SEQ ID Nos.: 75337 to 82784,
SEQ ID Nos.: 166154 to 166181,
SEQ ID Nos.: 166490 to 167042; and
SEQ ID Nos.: 173125 to 174603.

7. The method according to any one of Claims 1 to 3, wherein all or a part of the genome sequence information of the bottom fermenting yeast comprises the nucleotide sequences of SEQ ID Nos.: 33 to 6236.

8. The method according to any one of Claims 1 to 3, wherein all or a part of genome sequence information of the bottom fermenting yeast comprises two or more nucleotide sequences selected from SEQ ID Nos.: 33 to 6236.

9. The method according to any one of Claims 1 to 3, wherein all or a part of genome sequence information of the bottom fermenting yeast comprises nucleotide sequences of SEQ ID Nos.: 166154 to 166181.

10. The method according to any one of Claims 1 to 3, wherein all or a part of genome sequence information of the bottom fermenting yeast comprises two or more nucleotide sequences selected from SEQ ID Nos.: 166154 to 166181.

## Patentansprüche

1. Verfahren zum Identifizieren eines Zielproteins von Hefe oder eines Gens, das das Zielprotein codiert, umfassend die Schritte:
(A) einen Schritt des Bestimmens der Aminosäuresequenz des Zielproteins ausgewählt aus den folgenden Schritten (A1) bis (A4):
Schritt (A1):
(1) Züchten von Hefe unter einer zuvor definierten Kulturbedingung;
(2) Extrahieren einer Proteinprobe aus dem Produkt der Hefekultur;
(3) Trennen der Proteinprobe mithilfe eines Mittels zur Proteintrennung, Selektieren eines Zielpeaks oder eines Zielspots und Gewinnen des in dem Zielpeak oder in dem Zielspot enthalten Zielproteins oder eines Fragments davon; und
(4) Bestimmen der Aminosäuresequenz des Zielproteins;
Schritt (A2):
(1) Züchten der Hefe unter einer zuvor definierten Kulturbedingung;
(2) Extrahieren einer Proteinprobe aus den Produkten der Hefekultur; und
(3) Bestimmen der Aminosäuresequenz des einen oder der mehreren Proteine, die in der Proteinprobe enthalten sind;
Schritt (A3):
(1) Züchten des gleichen Hefestamms unter anderen Kulturbedingungen;
(2) Extrahieren einer Proteinprobe aus jedem in Schritt (1) erhaltenen Produkt der Kultur;
(3) Analysieren der Proteinprobe und Identifizieren eines hoch oder gering exprimierten Proteins unter der jeweiligen Kulturbedingung; und
(4) Bestimmen der Aminosäuresequenz des in Schritt (3) identifizierten Proteins;
Schritt (A4):
(1) Züchten verschiedener Hefestämme unter der gleichen Kulturbedingung;
(2) Extrahieren einer Proteinprobe aus jedem in Schritt (1) erhaltenen Produkt der Kultur;
(3) Analysieren der Proteinprobe und Identifizieren eines Proteins, dessen Expressionsspiegel in jedem Produkt der Kultur anders ist; und
(4) Bestimmen der Aminosäuresequenz des in Schritt (3) identifizierten Proteins;
(B) Vergleichen der in Schritt (A) bestimmten Aminosäuresequenz mit der zuvor bestimmten Aminosäuresequenz auf der Grundlage der gesamten oder eines Teils der Genomsequenzinformation von untergäriger Hefe;
(C) Identifizieren des Gens, das das Zielprotein codiert, auf der Grundlage der Vergleichsergebnisse; und
(D) Analysieren der Funktionen des identifizierten Gens, um die Eigenschaften zu bestimmen, die der Hefe durch das Gen verliehen werden;
wobei die gesamte oder ein Teil der Genomsequenzinformation der untergärigen Hefe zwei oder mehr Nucleotidsequenzen aus:
SEQ ID NOs:33 bis 6236,
SEQ ID NOs:75337 bis 82784,
SEQ ID NOs:166154 bis 166181,
SEQ ID NOs:166490 bis 167042; und
SEQ ID NOs:173125 bis 174603
umfasst.

2. Verfahren zum Identifizieren eines Zielproteins von Hefe oder eines Gens, das das Zielprotein codiert, umfassend die Schritte:
(1) Abgleich mit einer Datenbank, die die gesamte oder einen Teil der Genomsequenzinformation untergäriger Hefe umfasst, auf der Grundlage der Aminosäuresequenz des Zielproteins der Hefe;
(2) Identifizieren eines Gens, das das Zielprotein codiert, auf der Grundlage des Abgleichsergebnisses; und
(3) Analysieren der Funktionen des identifizierten Gens, um die Eigenschaften zu identifizieren, die der Hefe durch das Gen verliehen werden;
wobei die gesamte oder ein Teil der Genomsequenzinformation der untergärigen Hefe zwei oder mehr Nucleotidsequenzen aus:
SEQ ID NOs:33 bis 6236,
SEQ ID NOs:75337 bis 82784,
SEQ ID NOs:166154 bis 166181,
SEQ ID NOs:166490 bis 167042; und
SEQ ID NOs:173125 bis 174603
umfasst.

3. Verfahren nach Anspruch 2, wobei die Aminosäuresequenz des Zielproteins erhalten wird durch Abtrennen eines oder mehrerer Proteine aus einem Proteinextrakt, der von der Hefe stammt, und durch Bestimmen der Aminosäuresequenzen des einen oder der mehreren Proteine.

4. Verfahren nach einem der Ansprüche 1 bis 3, wobei die gesamte oder ein Teil der Genomsequenzinformation der untergärigen Hefe 40 oder mehr Nucleotidsequenzen ausgewählt aus:
SEQ ID NOs:33 bis 6236,
SEQ ID NOs:75337 bis 82784,
SEQ ID NOs:166154 bis 166181,
SEQ ID NOs:166490 bis 167042; und
SEQ ID NOs:173125 bis 174603
umfasst.

5. Verfahren nach einem der Ansprüche 1 bis 3, wobei die gesamte oder ein Teil der Genomsequenzinformation der untergärigen Hefe 50 oder mehr Nucleotidsequenzen ausgewählt aus:
SEQ ID NOs:33 bis 6236,
SEQ ID NOs:75337 bis 82784,
SEQ ID NOs:166154 bis 166181,
SEQ ID NOs:166490 bis 167042; und
SEQ ID NOs:173125 bis 174603
umfasst.

6. Verfahren nach einem der Ansprüche 1 bis 3, wobei die gesamte oder ein Teil der Genomsequenzinformation der untergärigen Hefe die Nucleotidsequenzen ausgewählt aus:
SEQ ID NOs:33 bis 6236,
SEQ ID NOs:75337 bis 82784,
SEQ ID NOs:166154 bis 166181,
SEQ ID NOs:166490 bis 167042; und
SEQ ID NOs:173125 bis 174603
umfasst.

7. Verfahren nach einem der Ansprüche 1 bis 3, wobei die gesamte oder ein Teil der Genomsequenzinformation der untergärigen Hefe die Nucleotidsequenzen der SEQ ID NOs: 33 bis 6236 umfasst.

8. Verfahren nach einem der Ansprüche 1 bis 3, wobei die gesamte oder ein Teil der Genomsequenzinformation der untergärigen Hefe zwei oder mehr Nucleotidsequenzen ausgewählt aus den SEQ ID NOs:33 bis 6236 umfasst.

9. Verfahren nach einem der Ansprüche 1 bis 3, wobei die gesamte oder ein Teil der Genomsequenzinformation der untergärigen Hefe Nucleotidsequenzen der SEQ ID NOs:166154 bis 166181 umfasst.

10. Verfahren nach einem der Ansprüche 1 bis 3, wobei die gesamte oder ein Teil der Genomsequenzinformation der untergärigen Hefe zwei oder mehr Nucleotidsequenzen ausgewählt aus den SEQ ID NOs:166154 bis 166181 umfasst.

## Revendications

1. Procédé destiné à identifier une protéine cible de levure ou un gène codant pour la protéine cible comprenant les étapes de :
(A) une étape de détermination de la séquence d'acides aminés de la protéine cible choisie parmi les étapes (A1) à (A4) suivantes :
Etape (A1)
(1) culture de levure dans des conditions de culture prédéterminées ;
(2) extraction d'un échantillon de protéine à partir du produit de culture de la levure ;
(3) séparation de l'échantillon de protéine par des moyens de séparation de protéines, sélection d'un pic ou point cible, et récupération de la protéine cible ou d'un fragment de celle-ci contenu dans le pic ou point ; et
(4) détermination de la séquence d'acides aminés de la protéine cible ;
Etape (A2)
(1) culture de levure dans des conditions de culture prédéterminées ;
(2) extraction d'un échantillon de protéine à partir de produits cultivés de la levure ; et
(3) détermination de la séquence d'acides aminés des une ou plusieurs protéines contenues dans l'échantillon de protéine ;
Etape (A3)
(1) culture de la même souche de levure dans différentes conditions de culture ;
(2) extraction d'un échantillon de protéine à partir de chaque produit de culture obtenu dans l'étape (1) ;
(3) analyse de l'échantillon de protéine et identification d'une protéine fortement exprimée ou faiblement exprimée dans chaque condition de culture ; et
(4) détermination de la séquence d'acides aminés de la protéine identifiée dans l'étape (3) ;
Etape (A4)
(1) culture de différentes souches de levure dans la même condition de culture ;
(2) extraction d'un échantillon de protéine à partir de chaque produit de culture obtenu dans l'étape (1) ;
(3) analyse de l'échantillon de protéine et identification d'une protéine dont le niveau d'expression est différent dans chaque produit de culture ; et
(4) détermination de la séquence d'acides aminés de la protéine identifiée dans l'étape (3) ;
(B) comparaison de la séquence d'acides aminés déterminée dans l'étape (A) avec la séquence d'acides aminés déterminée à l'avance sur la base de tout ou une partie des informations de séquence génomique de levure de fermentation basse ;
(C) identification du gène codant pour la protéine cible sur la base des résultats de comparaison ; et
(D) analyse de fonctions du gène identifié pour identifier des caractères conférés à la levure par le gène ;
dans lequel tout ou une partie des informations de séquence génomique de la levure de fermentation basse comprend deux séquences nucléotidiques ou plus de :
SEQ ID Nos.: 33 à 6236,
SEQ ID Nos.: 75337 à 82784,
SEQ ID Nos.: 166154 à 166181,
SEQ ID Nos.: 166490 à 167042 ; et
SEQ ID Nos.: 173125 à 174603.

2. Procédé destiné à identifier une protéine cible de levure ou un gène codant pour la protéine cible comprenant les étapes de :
(1) référence à une base de données comprenant tout ou une partie des informations de séquence génomique de levure de fermentation basse sur la base de la séquence d'acides aminés de la protéine cible de la levure ;
(2) identification d'un gène codant pour la protéine cible sur la base du résultat de référence ; et
(3) analyse de fonctions du gène identifié pour identifier des caractères conférés à la levure par le gène ;
dans lequel tout ou une partie des informations de séquence génomique de la levure de fermentation basse comprend deux séquences nucléotidiques ou plus de :
SEQ ID Nos.: 33 à 6236,
SEQ ID Nos.: 75337 à 82784.
SEQ ID Nos.: 166154 à 166181,
SEQ ID Nos.: 166490 à 167042 ; et
SEQ ID Nos.: 173125 à 174603.

3. Procédé selon la revendication 2, dans lequel la séquence d'acides aminés de la protéine cible est obtenue par séparation d'une ou plusieurs protéines à partir d'un extrait de protéine dérivé de la levure et détermination des séquences d'acides aminés des une ou plusieurs protéines.

4. Procédé selon l'une quelconque des revendications 1 à 3, dans lequel tout ou une partie des informations de séquence génomique de la levure de fermentation basse comprend 40 séquences nucléotidiques ou plus choisies parmi :
SEQ ID Nos: 33 à 6236,
SEQ ID Nos: 75337 à 82784,
SEQ ID Nos: 166154 à 166181,
SEQ ID Nos: 166490 à 167042 ; et
SEQ ID Nos: 173125 à 174603.

5. Procédé selon l'une quelconque des revendications 1 à 3, dans lequel tout ou une partie des informations de séquence génomique de la levure de fermentation basse comprend 50 séquences nucléotidiques ou plus choisies parmi :
SEQ ID Nos.: 33 à 6236,
SEQ ID Nos.: 75337 à 82784,
SEQ ID Nos.: 166154 à 166181,
SEQ ID Nos.: 166490 à 167042 ; et
SEQ ID Nos.: 173125 à 174603.

6. Procédé selon l'une quelconque des revendications 1 à 3, dans lequel tout ou une partie des informations de séquence génomique de la levure de fermentation basse comprend les séquences nucléotidiques choisies parmi :
SEQ ID Nos.: 33 à 6236,
SEQ ID Nos.: 75337 à 82784.
SEQ ID Nos.: 166154 à 166181,
SEQ ID Nos.: 166490 à 167042 ; et
SEQ ID Nos.: 173125 à 174603.

7. Procédé selon l'une quelconque des revendications 1 à 3, dans lequel tout ou une partie des informations de séquence génomique de la levure de fermentation basse comprend les séquences nucléotidiques de SEQ ID Nos.: 33 à 6236.

8. Procédé selon l'une quelconque des revendications 1 à 3, dans lequel tout ou une partie des informations de séquence génomique de la levure de fermentation basse comprend deux séquences nucléotidiques ou plus choisies parmi SEQ ID Nos.: 33 à 6236.

9. Procédé selon l'une quelconque des revendications 1 à 3, dans lequel tout ou une partie des informations de séquence génomique de la levure de fermentation basse comprend les séquences nucléotidiques de SEQ ID Nos.: 166154 à 166181.

10. Procédé selon l'une quelconque des revendications 1 à 3, dans lequel tout ou une partie des informations de séquence génomique de la levure de fermentation basse comprend deux séquences nucléotidiques ou plus choisies parmi SEQ ID Nos.: 166154 à 166181.
